# EUROPEAN PATENT SPECIFICATION

(11) **EP 4 301 748 B1**
(45) Date of publication and mention of the grant of the patent: **07.05.2025**
(21) Application number: 22708569.3
(22) Date of filing: 02.03.2022
(51) Int. Cl.: C07D 405/14, A61P 1/00, A61P 9/00, A61P 13/12, A61P 27/02, A61P 29/00, A61P 35/00, A61P 37/00, A61K 31/4192

(54) **TRIAZOLYL-METHYL SUBSTITUTED ALPHA-D-GALACTOPYRANOSIDE DERIVATIVES**
TRIAZOLYLMETHYL-SUBSTITUIERTE ALPHA-D-GALACTOPYRANOSIDDERIVATE
DÉRIVÉS D'ALPHA-D-GALACTOPYRANOSIDE SUBSTITUÉS PAR TRIAZOLYL-MÉTHYLE

(30) Priority: 03.03.2021 WO PCT/EP2021/055348
(43) Date of publication of application: 10.01.2024
(73) Proprietor: Idorsia Pharmaceuticals Ltd, 4123 Allschwil (CH)
(72) Inventor: BOLLI, Martin, 4123 Allschwil (CH); GATFIELD, John, 4123 Allschwil (CH); GRISOSTOMI, Corinna, 4123 Allschwil (CH); REMEN, Lubos, 4123 Allschwil (CH); SAGER, Christoph, 4123 Allschwil (CH); ZUMBRUNN, Cornelia, 4123 Allschwil (CH)
(74) Representative: Meyer Mojzes, Melinda
(86) International application number: PCT/EP2022/055224
(87) International publication number: WO 2022/184755

(56) References cited:
- WO-A1-2018/209255
- WO-A1-2019/067702
- WO-A1-2020/210308
- WO-A1-2021/038068

## Description

The present invention relates to compounds of Formula (I) which are galectin-3 inhibitors and their use in the treatment of diseases and disorders that are related to galectin-3 binding to natural ligands. The invention also concerns related aspects including processes for the preparation of the compounds, pharmaceutical compositions containing one or more compounds of Formula (I), and their medical use as Galectin-3 inhibitors. The compounds of Formula (I)) may especially be used as single agents or in combination with one or more therapeutic agents.

Galectins are defined as a protein family based on conserved β-galactoside-binding sites found within their characteristic ~130 amino acid (aa) carbohydrate recognition domains (CRDs) (Barondes SH et al., Cell 1994; 76, 597-598). Human, mouse and rat genome sequences reveal the existence of at least 16 conserved galectins and galectin-like proteins in one mammalian genome (Leffler H. et al., Glycoconj. J. 2002, 19, 433-440). So far, three galectin subclasses were identified, the prototypical galectins containing one carbohydrate-recognition domain (CRD); the chimaera galectin consisting of unusual tandem repeats of proline- and glycine-rich short stretches fused onto the CRD; and the tandem -repeat-type galectins, containing two distinct CRDs in tandem connected by a linker (Zhong X., Clin Exp Pharmacol Physiol. 2019; 46:197-203). As galectins can bind either bivalently or multivalently, they can e.g. cross-link cell surface glycoconjugates to trigger cellular signaling events. Through this mechanism, galectins modulate a wide variety of biological processes (Sundblad V. et al., Histol Histopathol 2011; 26: 247-265).

Galectin-3 (Gal-3), the only chimaera type in the galectin family, has a molecular weight of 32-35 kDa and consists of 250 amino acid residues in humans, a highly conserved CRD and an atypical N- terminal domain (ND). Galectin-3 is monomeric up to high concentrations (100 µM), but can aggregate with ligands at much lower concentrations, which is promoted by its N-terminal non-CRD region via an oligomerisation mechanism that is not yet completely understood (Johannes, L. et al., Journal of Cell Science 2018; 131, jcs208884).

Gal-3 is widely distributed in the body, but the expression level varies among different organs. Depending on its extracellular or intracellular localization, it can display a broad diversity of biological functions, including immunomodulation, host-pathogen interactions, angiogenesis, cell migration, wound healing and apoptosis (Sundblad V. et al., Histol Histopathol 2011; 26: 247-265). Gal-3 is highly expressed in many human tumours and cell types, such as myeloid cells, inflammatory cells (macrophages, mast cells, neutrophils, T cells, eosinophils, etc.), fibroblasts and cardiomyocytes (Zhong X. et al., Clin Exp Pharmacol Physiol. 2019; 46:197-203), indicating that Gal-3 is involved in the regulation of inflammatory and fibrotic processes (Henderson NC. Et al., Immunological Reviews 2009; 230: 160-171; Sano H. et al., J Immunol. 2000; 165(4):2156-64). Furthermore, Gal-3 protein expression levels are up-regulated under certain pathological conditions, such as neoplasms and inflammation (Chiariotti L. et al., Glycoconjugate Journal 2004 19, 441-449; Farhad M. et al., Oncolmmunology 2018, 7:6, e1434467).

There are multiple lines of evidence supporting functional involvement of Gal-3 in the development of inflammatory / autoimmune diseases, such as asthma, rheumatoid arthritis, multiple sclerosis and diabetes (Liu FT et al., Ann N Y Acad Sci. 2012; 1253:80-91; Henderson NC, et al., Immunol Rev. 2009;230(1):160-71; Li P et al., Cell 2016; 167:973-984), cardiovascular diseases, such as atherosclerosis, heart failure and thrombosis (Nachtigal M. et al., Am J Pathol. 1998; 152(5):1199-208; Gehlken C. et al., Heart Fail Clin. 2018,14(1):75-92; DeRoo EP. Et al., Blood. 2015, 125(11):1813-21), organ fibrosis, such as lung fibrosis, liver fibrosis, kidney fibrosis, eye fibrosis and skin fibrosis (Mackinnon AC et al., Am. J. Respir. Crit. Care Med 2012; 185: 537-546; Henderson NC et al., PNAS 2006; 103: 5060-5065; Henderson NC et al., Am. J. Pathol. 2008; 172:288-298; Chen WS. Et al., Investigative Ophthalmology & Visual Science 2017, Vol.58, 9-20; Taniguchi T. et al., The Journal of Rheumatology 2012, jrheum.110755; Arciniegas E. et al., The American Journal of dermatopathology 2019; 41(3):193-204) and cancer (Farhad M. et al., Oncoimmunology. 2018; 7(6): e1434467; Vuong L. et al., Cancer Res 2019 (79) (7) 1480-1492).

Recently, Gal-3 inhibitors have shown to have positive effects when used in combination immunotherapy (Galectin Therapeutics. Press Release, February 7, 2017) and idiopathic pulmonary fibrosis (Galectin Therapeutics. Press Release, March 10, 2017). WO20180209276, WO2018209255 and WO2019089080 disclose compounds having binding affinity with galectin proteins for the treatment of systemic insulin resistance disorders. Thus, Gal-3 inhibitors, alone or in combination with other therapies, may be useful for the prevention or treatment of diseases or disorders such as acute or chronic heart failure, cancer, chronic and acute kidney disease, idiopathic pulmonary fibrosis, type 2 diabetes, rheumatoid arthritis, psoriasis, scarring, systemic sclerosis, systemic lupus erythematosus and dry eye disease.

Several publications and patent applications describe synthetic inhibitors of Gal-3 that are being explored as antifibrotic agents (see for example WO2005/113568, WO2005/113569, WO2014/067986, WO2016/120403, US2014/0099319, WO2019/067702, WO2019/075045 and WO2014/078655). WO2002/057284, WO2005/113569, WO2014/078655, WO2021/028336, WO2021/028323, and WO2021/028570 disclose beta-configured galectin inhibitors. WO2016120403, WO2020104335, WO2021001528, WO2021038068 and WO2021004940 disclose a broad generic scope of alpha-D-galactoside inhibitors of galectins.

The present invention provides novel compounds of Formula (I) which are alpha-configured galectin-3 inhibitors. The present compounds may, thus, be useful for the prevention / prophylaxis or treatment of diseases and disorders where modulation of Gal-3 binding to its natural carbohydrate ligands is indicated.
1) In a first embodiment, the invention relates to compounds of the Formula (I), 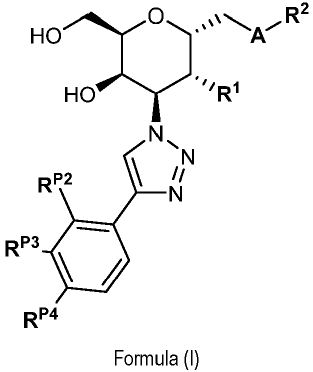 wherein
   **R^{P2}** represents halogen (especially fluoro);
   **R^{P3}** represents halogen (especially fluoro);
   **R^{P4}** represents halogen (especially fluoro, chloro, bromo), methyl or cyano;
   **R¹** represents
      - hydroxy;
      - C₁₋₄-alkoxy (especially methoxy);
      - -O-CO-C₁₋₃-alkyl;
      - O-CO-NH-**R^{N11}** wherein **R^{N11}** represents hydrogen or C₁₋₃-alkyl;
      - -O-CH₂-C₁-fluoroalkyl;
      - -O-CH₂-**HET¹** wherein **HET¹** represents a 5-membered heteroaryl (especially oxazolyl, thiazolyl, or imidazolyl) wherein said 5-membered heteroaryl independently is unsubstituted or mono-substituted with methyl; or
      - -O-CH₂-CO-**R^{1X}** wherein **R^{1X}** represents
         ➢ -hydroxy;
         ➢ C₁₋₃-alkoxy (especially methoxy);
         ➢ morpholin-4-yl; or

         ❖ -N**R^{N21}R^{N22}** wherein **R^{N21}** and **R^{N22}** both independently represent hydrogen or methyl; or **R^{N21}** and **R^{N22}** together with the nitrogen atom to which they are attached form a 4- to 6-membered mono-cyclic heterocycloalkyl selected from azetidine-1-yl, pyrrolidine-1-yl, and piperidine-1-yl, wherein said 4- to 6-membered heterocycloalkyl is mono-substituted with hydroxy;
   [wherein, in a sub-embodiment, **R¹** especially represents methoxy];
   A represents [1,2,3]triazol-1,4-diyl (wherein it is understood that **R²** may be attached to position 1 or to position 4 of said [1,2,3]triazol-1,4-diyl); and
   - **R²** represents branched C₃₋₆-alkyl (especially isopropyl or tert-butyl) wherein said branched C₃₋₆-alkyl is
      ➢ mono-substituted with hydroxy [especially such group is 2-hydroxy-1,1-dimethyl-ethyl or 1-hydroxy-1-methyl-ethyl],
      ➢ mono-substituted with -CO-O-C₁₋₄-alkyl [especially such group is 1-methoxy-2-methyl-1-oxopropan-2-yl], or
      ➢ mono-substituted with C₁-fluoroalkyl [especially such group is 2,2-difluoro-1,1-dimethyl-ethyl];
   - or **R²** represents a saturated 3- to 8-membered mono- or bicyclic group, wherein said mono- or bicyclic group is
      ❖ mono-cyclic C₃₋₆-cycloalkyl (especially cyclopropyl, cyclobutyl or cyclopentyl),
      ❖ mono-cyclic 4- to 6-membered heterocycloalkyl containing one ring oxygen atom (especially oxetan-3-yl, tetrahydro-2H-pyran-3-yl),
      ❖ bridged bicyclic C₅₋₈-cycloalkyl (especially bicyclo[1.1.1]pentan-1-yl, bicyclo[2.2.2]octan-1-yl),
      ❖ fused bicyclic C₆₋₈-cycloalkyl (especially bicyclo[3.1.0]hexan-6-yl),
      ❖ spiro-bicyclic C₆₋₈-cycloalkyl (especially spiro[2.3]hexan-5-yl), or
      ❖ spiro-bicyclic 7- or 8-membered heterocycloalkyl containing one ring oxygen atom (especially oxaspiro[3.3]heptan-6-yl);

      wherein said mono-cyclic or bicyclic group independently is unsubstituted, or mono-, di-, or tri-substituted wherein the substituents independently are selected from hydroxy; C₁₋₃-alkyl (especially methyl; or ethyl or isopropyl); C₁₋₃-alkoxy (especially methoxy); -C₁₋₃-alkylene-OH (especially hydroxymethyl, or 2-hydroxyethyl); C₁-fluoroalkyl (especially trifluoromethyl); -NR^{N1}R^{N2}, wherein R^{N1} represents hydrogen, and R^{N2} represents hydrogen or -CO-O-C₁₋₄-alkyl (especially -CO-O-tert.-butyl); and fluoro;
      wherein in a sub-embodiment, especially said mono-cyclic or bicyclic group independently is
         ➢ unsubstituted;
         ➢ mono-substituted with hydroxy;
         ➢ mono-substituted with C₁₋₃-alkyl (especially methyl; or ethyl or isopropyl);
         ➢ mono-substituted with C₁₋₃-alkoxy (especially methoxy);
         ➢ mono-substituted with -C₁₋₃-alkylene-OH (especially hydroxymethyl, 2-hydroxyethyl);
         ➢ mono-substituted with -C₁₋₃-alkylene-O-C₁₋₃-alkyl (especially methoxymethyl);
         ➢ mono-substituted with C₁-fluoroalkyl (especially trifluoromethyl);
         ➢ mono-substituted with -NR^{N1}R^{N2}, wherein R^{N1} represents hydrogen, and R^{N2} represents hydrogen or -CO-O-C₁₋₄-alkyl (especially -CO-O-tert.-butyl);
         ➢ mono- or di-substituted with fluoro;
         ➢ di-substituted wherein one substituent is hydroxy and the other is C₁₋₃-alkyl (especially methyl); or
         ➢ tri-substituted wherein two of said substituents are fluoro (which are notably attached to the same ring carbon atom); and the remaining substituent is C₁₋₃-alkyl (especially methyl) or -C₁₋₃-alkylene-OH (especially hydroxymethyl).
2) In a second embodiment, the invention relates to compounds of the Formula (I) according to embodiment 1), wherein
   **R^{P2}** represents halogen (especially fluoro);
   **R^{P3}** represents halogen (especially fluoro);
   **R^{P4}** represents halogen (especially fluoro, chloro, bromo), methyl or cyano;
   **R¹** represents
      - hydroxy;
      - C₁₋₄-alkoxy (especially methoxy);
      - -O-CO-C₁₋₃-alkyl;
      - O-CO-NH-**R^{N11}** wherein **R^{N11}** represents hydrogen or C₁₋₃-alkyl;
      - -O-CH₂-C₁-fluoroalkyl;
      - -O-CH₂-**HET¹** wherein **HET¹** represents a 5-membered heteroaryl (especially oxazolyl, thiazolyl, or imidazolyl) wherein said 5-membered heteroaryl independently is unsubstituted or mono-substituted with methyl; or
      - -O-CH₂-CO-**R^{1X}** wherein **R^{1X}** represents
         ➢ -hydroxy;
         ➢ C₁₋₃-alkoxy (especially methoxy);
         ➢ morpholin-4-yl; or

         ❖ -N**R^{N21}R^{N22}** wherein **R^{N21}** and **R^{N22}** both independently represent hydrogen or methyl; or **R^{N21}** and **R^{N22}** together with the nitrogen atom to which they are attached form a 4- to 6-membered mono-cyclic heterocycloalkyl selected from azetidine-1-yl, pyrrolidine-1-yl, and piperidine-1-yl, wherein said 4- to 6-membered heterocycloalkyl is mono-substituted with hydroxy;
   [wherein, in a sub-embodiment, **R¹** especially represents methoxy];

A represents [1,2,3]triazol-1,4-diyl (wherein it is understood that **R²** may be attached to position 1 or to position 4 of said [1,2,3]triazol-1,4-diyl); and
- **R²** represents branched C₃₋₆-alkyl (especially isopropyl or tert-butyl) wherein said branched C₃₋₆-alkyl is
   ➢ mono-substituted with hydroxy [especially such group is 2-hydroxy-1,1-dimethyl-ethyl or 1-hydroxy-1-methyl-ethyl],
   ➢ mono-substituted with -CO-O-C₁₋₄-alkyl [especially such group is 1-methoxy-2-methyl-1-oxopropan-2-yl], or
   ➢ mono-substituted with C₁-fluoroalkyl [especially such group is 2,2-difluoro-1,1-dimethyl-ethyl];
- or **R²** represents a saturated 3- to 8-membered mono- or bicyclic group, wherein said mono- or bicyclic group is
   ❖ mono-cyclic C₃₋₆-cycloalkyl (especially cyclopropyl, cyclobutyl or cyclopentyl),
   ❖ mono-cyclic 4- to 6-membered heterocycloalkyl containing one ring oxygen atom (especially oxetan-3-yl, tetrahydro-2H-pyran-3-yl),
   ❖ bridged bicyclic C₅₋₈-cycloalkyl (especially bicyclo[1.1.1]pentan-1-yl, bicyclo[2.2.2]octan-1-yl),
   ❖ fused bicyclic C₆₋₈-cycloalkyl (especially bicyclo[3.1.0]hexan-6-yl),
   ❖ spiro-bicyclic C₆₋₈-cycloalkyl (especially spiro[2.3]hexan-5-yl), or
   ❖ spiro-bicyclic 7- or 8-membered heterocycloalkyl containing one ring oxygen atom (especially oxaspiro[3.3]heptan-6-yl);

   wherein said mono-cyclic or bicyclic group independently is
      ➢ unsubstituted;
      ➢ mono-substituted with hydroxy;
      ➢ mono-substituted with C₁₋₃-alkyl (especially methyl; or ethyl or isopropyl);
      ➢ mono-substituted with C₁₋₃-alkoxy (especially methoxy);
      ➢ mono-substituted with -C₁₋₃-alkylene-OH (especially hydroxymethyl, 2-hydroxyethyl);
      ➢ mono-substituted with -C₁₋₃-alkylene-O-C₁₋₃-alkyl (especially methoxymethyl);
      ➢ mono-substituted with C₁-fluoroalkyl (especially trifluoromethyl);
      ➢ mono-substituted with -NR^{N1}R^{N2}, wherein R^{N1} represents hydrogen, and R^{N2} represents hydrogen or -CO-O-C₁₋₄-alkyl (especially -CO-O-tert.-butyl);
      ➢ mono- or di-substituted with fluoro;
      ➢ di-substituted wherein one substituent is hydroxy and the other is C₁₋₃-alkyl (especially methyl); or
      ➢ tri-substituted wherein two of said substituents are fluoro (which are notably attached to the same ring carbon atom); and the remaining substituent is C₁₋₃-alkyl (especially methyl) or -C₁₋₃-alkylene-OH (especially hydroxymethyl).

It is understood that in the compounds of Formula (I) any non-aromatic oxygen or nitrogen atom will preferably be distanced from another oxygen or nitrogen atom by at least two carbon atoms. In particular, an oxygen atom or a nitrogen atom which is part of a group **R²** (whether part of said saturated 3- to 8-membered mono- or bicyclic group, or substituent or part of a substituent of said saturated 3- to 8-membered mono- or bicyclic group) is preferably distanced by at least two carbon atoms from another oxygen atom or nitrogen atom which is part of a group **R²** (wherein it is understood that said other oxygen atom or nitrogen atom may be part of said saturated 3- to 8-membered mono- or bicyclic group, or part of a substituent of said saturated 3- to 8-membered mono- or bicyclic group), as well as distanced by at least two carbon atoms from an aromatic nitrogen atom which is part of the ring **A** (e.g. in case such ring A is [1,2,3]triazol-1,4-diyl wherein **R²** is attached to position 1). In particular, in a group **R²** representing a branched C₃₋₆-alkyl which is mono-substituted with hydroxy such hydroxy substituent will be distanced by at least two carbon atoms from a ring nitrogen part of ring A; in a group **R²** representing any saturated mono- or bicyclic carbocyclic group which is mono-substituted with hydroxy such hydroxy substituent will be distanced by at least two carbon atoms from a ring nitrogen part of ring A; in a group **R²** representing any saturated mono- or bicyclic carbocyclic group which is mono-substituted with C₁₋₃-alkoxy the oxygen atom of such C₁₋₃-alkoxy substituent will be distanced by at least two carbon atoms from a ring nitrogen part of ring A; in a group **R²** representing any saturated mono- or bicyclic heterocycloalkyl group which is mono-substituted with hydroxy such hydroxy substituent will be distanced by at least two carbon atoms from an aromatic ring nitrogen part of ring A and from the ring oxygen atom part of such saturated mono- or bicyclic heterocycloalkyl group; and in a group **R²** representing any saturated mono- or bicyclic heterocycloalkyl group which is mono-substituted with C₁₋₃-alkoxy the oxygen atom of such C₁₋₃-alkoxy substituent will be distanced by at least two carbon atoms from an aromatic ring nitrogen part of ring A and from the ring oxygen atom part of such saturated mono- or bicyclic heterocycloalkyl group.

The compounds of Formula (I) contain five stereogenic or asymmetric centers, which are situated on the tetrahydropyran moiety and which are in the absolute configuration as drawn for Formula (I). In addition, the compounds of Formula (I) may contain contain one, and possibly more, further stereogenic or asymmetric centers, such as one or more additional asymmetric carbon atoms. The compounds of Formula (I) may thus be present as mixtures of stereoisomers or preferably as pure stereoisomers. Mixtures of stereoisomers may be separated in a manner known to a person skilled in the art.

In case a particular compound (or generic structure) is designated as being in a certain absolute configuration, e.g. as (R)- or (S)-enantiomer, such designation is to be understood as referring to the respective compound (or generic structure) in enriched, especially essentially pure, enantiomeric form. Likewise, in case a specific asymmetric center in a compound is designated as being in (R)- or (S)-configuration or as being in a certain relative configuration, such designation is to be understood as referring to the compound that is in enriched, especially essentially pure, form with regard to the respective configuration of said asymmetric center. Likewise, in case such stereogenic or asymmetric center is designated as being in (RS)-configuration, this means that such stereogenic or asymmetric center in such compound may be present in (R)-configuration, in (S)-configuration, or in any mixture of epimers with regard to such center. In case two or more such stereogenic or asymmetric centers (in undesignated or designated (RS)-configuration) are present in one molecule, it is understood that, if not explicitly defined otherwise, the order of absolute configuration does not indicate any defined relative configuration with regard to the two or more centers. It is understood that explicitly designated (R)- or (S)-configuration(s) and undesignated or designated (RS)-configuration(s), can co-exist in one and the same molecule and are to be interpreted accordingly. In analogy, cis- or trans-designations (or (R*,R*) / (R*,S*) designations) are to be understood as referring to the respective stereoisomer of the respective relative configuration in enriched form, especially in essentially pure form. The relative configuration of stereoisomers is denoted as follows: for example, the compound 2,3-difluoro-4-(1-((2R,3R,4S,5R,6R)-2-((1-((3R*,4S*)-3-fluorotetrahydro-2H-pyran-4-yl)-1H-1,2,3-triazol-4-yl)methyl)-5-hydroxy-6-(hydroxymethyl)-3-methoxytetrahydro-2H-pyran-4-yl)-1H-1,2,3-triazol-4-yl)benzonitrile; denominates 2,3-difluoro-4-(1-((2R,3R,4S,5R,6R)-2-((1-((3R,4S)-3-fluorotetrahydro-2H-pyran-4-yl)-1H-1,2,3-triazol-4-yl)methyl)-5-hydroxy-6-(hydroxymethyl)-3-methoxytetrahydro-2H-pyran-4-yl)-1H-1,2,3-triazol-4-yl)benzonitrile, 2,3-difluoro-4-(1-((2R,3R,4S,5R,6R)-2-((1-((3S,4R)-3-fluorotetrahydro-2H-pyran-4-yl)-1H-1,2,3-triazol-4-yl)methyl)-5-hydroxy-6-(hydroxymethyl)-3-methoxytetrahydro-2H-pyran-4-yl)-1H-1,2,3-triazol-4-yl)benzonitrile, or any mixtures thereof. Likewise, the compound (2R,3R,4S,5R,6R)-4-(4-(4-chloro-2,3-difluorophenyl)-1H-1,2,3-triazol-1-yl)-6-((1-((3R*,4R*)-4-fluorotetrahydrofuran-3-yl)-1H-1,2,3-triazol-4-yl)methyl)-2-(hydroxymethyl)-5-methoxytetrahydro-2H-pyran-3-ol denominates (2R,3R,4S,5R,6R)-4-(4-(4-chloro-2,3-difluorophenyl)-1H-1,2,3-triazol-1-yl)-6-((1-((3R,4R)-4-fluorotetrahydrofuran-3-yl)-1H-1,2,3-triazol-4-yl)methyl)-2-(hydroxymethyl)-5-methoxytetrahydro-2H-pyran-3-ol I, (2R,3R,4S,5R,6R)-4-(4-(4-chloro-2,3-difluorophenyl)-1H-1,2,3-triazol-1-yl)-6-((1-((3S,4S)-4-fluorotetrahydrofuran-3-yl)-1H-1,2,3-triazol-4-yl)methyl)-2-(hydroxymethyl)-5-methoxytetrahydro-2H-pyran-3-ol , or any mixtures thereof.

In this patent application, a bond drawn as a dotted line, or interrupted by a wavy line, shows the point of attachment of the radical drawn. For example, the radicals drawn below 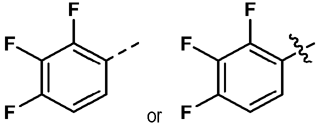 describe a 2,3,4-trifluorophenyl group.

The term "enriched", when used in the context of stereoisomers, is to be understood in the context of the present invention to mean that the respective stereoisomer is present in a ratio of at least 70:30, especially of at least 90:10 (i.e., in a purity of at least 70% by weight, especially of at least 90% by weight), with regard to the respective other stereoisomer / the entirety of the respective other stereoisomers.

The term "essentially pure", when used in the context of stereoisomers, is to be understood in the context of the present invention to mean that the respective stereoisomer is present in a purity of at least 95% by weight, especially of at least 99% by weight, with regard to the respective other stereoisomer / the entirety of the respective other stereoisomers.

The present invention also includes isotopically labelled, especially ²H (deuterium) labelled compounds of Formula (I) according to embodiments 1) to 25), which compounds are identical to the compounds of Formula (I) except that one or more atoms have each been replaced by an atom having the same atomic number but an atomic mass different from the atomic mass usually found in nature. Isotopically labelled, especially ²H (deuterium) labelled compounds of formulae (I), (II) and (III) and salts thereof are within the scope of the present invention. Substitution of hydrogen with the heavier isotope ²H (deuterium) may lead to greater metabolic stability, resulting e.g. in increased *in-vivo* half-life or reduced dosage requirements, or may lead to reduced inhibition of cytochrome P450 enzymes, resulting e.g. in an improved safety profile. In one embodiment of the invention, the compounds of Formula (I) are not isotopically labelled, or they are labelled only with one or more deuterium atoms. In a sub-embodiment, the compounds of Formula (I) are not isotopically labelled at all. Isotopically labelled compounds of Formula (I) may be prepared in analogy to the methods described hereinafter, but using the appropriate isotopic variation of suitable reagents or starting materials.

Where the plural form is used for compounds, salts, pharmaceutical compositions, diseases and the like, this is intended to mean also a single compound, salt, or the like.

Any reference to compounds of Formula (I) according to embodiments 1) to 25) is to be understood as referring also to the salts (and especially the pharmaceutically acceptable salts) of such compounds, as appropriate and expedient.

The term "pharmaceutically acceptable salts" refers to salts that retain the desired biological activity of the subject compound and exhibit minimal undesired toxicological effects. Such salts include inorganic or organic acid and/or base addition salts depending on the presence of basic and/or acidic groups in the subject compound. For reference see for example "Handbook of Pharmaceutical Salts. Properties, Selection and Use.", P. Heinrich Stahl, Camille G. Wermuth (Eds.), Wiley-VCH, 2008; and "Pharmaceutical Salts and Co-crystals", Johan Wouters and Luc Quéré (Eds.), RSC Publishing, 2012.

Definitions provided herein are intended to apply uniformly to the compounds of Formula (I), as defined in any one of embodiments 1) to 20), and, *mutatis mutandis,* throughout the description and the claims unless an otherwise expressly set out definition provides a broader or narrower definition. It is well understood that a definition or preferred definition of a term defines and may replace the respective term independently of (and **in** combination with) any definition or preferred definition of any or all other terms as defined herein.

In this patent application, the compounds are named using IUPAC nomenclature, but can also be named using carbohydrate nomenclature. Thus, the moiety: 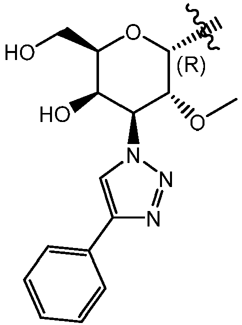 can be named (2R,3R4R5R,6R)-5-hydroxy-6-(hydroxymethyl)-3-methoxy-4-(4-phenyl-1H-1,2,3-triazol-1-yl)tetrahydro-2H-pyran-2-yl or, alternatively, 1,2,3-tri-deoxy-2-methoxy-3-[4-phenyl-1H-1,2,3-triazol-1-yl]-α-D-galactopyranoside-1-yl, wherein the absolute configuration of carbon atom carrying the point of attachment to the rest of the molecule is (2R)- , respectively, alpha. For example, the compound (2R,3R,4S,5R,6R)-6-((4-cyclopentyl-1H-1,2,3-triazol-1-yl)methyl)-2-(hydroxymethyl)-5-methoxy-4-(4-(2,3,4-trifluorophenyl)-1H-1,2,3-triazol-1-yl)tetrahydro-2H-pyran-3-ol is to be understood as also referring to: 1-(1,2,3-tri-deoxy-2-methoxy-3-[4-(2,3,4-trifluorophenyl)-1H-1,2,3-triazol-1-yl]-α-D-galacto-pyranose)-1-(4-cyclopentyl-1H-triazol-1-yl)-methane.

Whenever a substituent is denoted as optional, it is understood that such substituent may be absent (i.e. the respective residue is unsubstituted with regard to such optional substituent), in which case all positions having a free valency (to which such optional substituent could have been attached to; such as for example in an aromatic ring the ring carbon atoms and / or the ring nitrogen atoms having a free valency) are substituted with hydrogen where appropriate. Likewise, in case the term "optionally" is used in the context of (ring) heteroatom(s), the term means that either the respective optional heteroatom(s), or the like, are absent (i.e. a certain moiety does not contain heteroatom(s) / is a carbocycle / or the like), or the respective optional heteroatom(s), or the like, are present as explicitly defined. If not explicitly defined otherwise in the respective embodiment or claim, groups defined herein are unsubstituted.

The term "halogen" means fluorine/fluoro, chlorine/chloro, bromine/bromo or iodine/iodo; especially fluoro, chloro, or bromo; in particular fluoro. For the substituent **R^{P4}** the term especially means fluoro, chloro, or bromo.

The term "alkyl", used alone or in combination, refers to a saturated straight or branched chain hydrocarbon group containing one to six carbon atoms. The term "C_{x-y}-alkyl" (x and y each being an integer), refers to an alkyl group as defined before, containing x to y carbon atoms. For example, a C₁₋₆-alkyl group contains from one to six carbon atoms.

Examples of alkyl groups are methyl, ethyl, propyl, isopropyl, butyl, isobutyl, tert.-butyl, pentyl, 3-methyl-butyl, 2,2-dimethyl-propyl and 3,3-dimethyl-butyl. For avoidance of any doubt, in case a group is referred to as e.g. propyl or butyl, it is generally referring to n-propyl, respectively, n-butyl. Examples of branched C₃₋₆-alkyl as used for the group R² are the above-listed branched alkyl groups, especially isopropyl and tert-butyl.

The term "-C_{x-y}-alkylene-", used alone or in combination, refers to bivalently bound alkyl group as defined before containing x to y carbon atoms. The term "-C_{0-y}-alkylene-" refers to a direct bond, or to a -(C_{1-y})alkylene- as defined before. Preferably, the points of attachment of a -C_{1-y}-alkylene group are in 1,1-diyl, or in 1,2-diyl, or in 1,3-diyl arrangement. In case a C_{0 y}-alkylene group is used in combination with another substituent, the term means that either said substituent is linked through a C_{1 y}-alkylene group to the rest of the molecule, or it is directly attached to the rest of the molecule (i.e. a C₀-alkylene group represents a direct bond linking said substituent to the rest of the molecule). The alkylene group -C₂H₄- refers to -CH₂-CH₂- if not explicitly indicated otherwise. Examples of -C₁₋₃-alkylene as used for example in -C₁₋₃-alkylene-OH or -C₁₋₃-alkylene-O-C₁₋₃-alkyl are especially methylene, and ethylene (-CH₂-CH₂-).

The term "fluoroalkyl", used alone or in combination, refers to an alkyl group as defined before containing one to three carbon atoms in which one or more (and possibly all) hydrogen atoms have been replaced with fluorine. The term "C_{x-y}-fluoroalkyl" (x and y each being an integer) refers to a fluoroalkyl group as defined before containing x to y carbon atoms. For example, a C₁₋₃-fluoroalkyl group contains from one to three carbon atoms in which one to seven hydrogen atoms have been replaced with fluorine. C₁-fluoroalkyl especially refers to trifluoromethyl or difluoromethyl.

The term "cycloalkyl", used alone or in combination, refers to a saturated mono- or bicyclic (e.g. bridged bicyclic, fused bicyclic, or spiro-bicyclic) hydrocarbon ring containing three to eight carbon atoms. The term "C_{x-y}-cycloalkyl" (x and y each being an integer), refers to a cycloalkyl group as defined before containing x to y carbon atoms. For example, a C₃₋₆-cycloalkyl group contains from three to six carbon atoms. Examples of cycloalkyl groups are mono-cyclic C₃₋₆-cycloalkyl groups such as cyclopropyl, cyclobutyl, cyclopentyl, and cyclohexyl (especially cyclopropyl, cyclobutyl, and cyclopentyl); bridged bicyclic C₅₋₈-cycloalkyl groups such as bicyclo[1.1.1]pentan-1-yl or bicyclo[2.2.2]octan-1-yl; spiro-bicyclic C₆₋₈-cycloalkyl groups such as spiro[2.3]hexan-5-yl; and fused bicyclic C₆₋₈-cycloalkyl groups such as bicyclo[3.1.0]hexan-6-yl (especially (1*R*,5*S*)-bicyclo[3.1.0]hexan-6-yl).

The term "heterocycloalkyl", used alone or in combination, and if not explicitly defined in a more narrow way, refers to a saturated cycloalkyl as defined before, wherein said cycloalkyl contains one or two ring heteroatoms independently selected from nitrogen, sulfur, and oxygen (especially one oxygen atom; or one sulfur atom, one nitrogen atom, two nitrogen atoms, two oxygen atoms, or one nitrogen atom and one oxygen atom). The term "x- to y-membered heterocycloalkyl" refers to such a heterocycloalkyl containing a total of x to y ring atoms. Heterocycloalkyl groups are unsubstituted or substituted as explicitly defined. Examples of heterocycloalkyl groups are mono-cyclic 4- to 6-membered heterocycloalkyl containing one ring oxygen atom such as oxetan-3-yl and tetrahydro-2H-pyran-4-yl; and spiro-bicyclic 7- or 8-membered heterocycloalkyl containing one ring oxygen atom such as 2-oxaspiro[3.3]heptan-6-yl.

The term "alkoxy", used alone or in combination, refers to an alkyl-O- group wherein the alkyl group is as defined before. The term "C_{x-y}-alkoxy" (x and y each being an integer) refers to an alkoxy group as defined before containing x to y carbon atoms. Preferred are ethoxy and especially methoxy.

The term "heteroaryl", used alone or in combination, and if not explicitly defined in a broader or more narrow way, means a 5- to 10-membered monocyclic or bicyclic aromatic ring containing one to a maximum of four heteroatoms, each independently selected from oxygen, nitrogen and sulfur. Examples of such heteroaryl groups are furanyl, oxazolyl, isoxazolyl, thiophenyl, thiazolyl, pyrrolyl, imidazolyl, pyrazolyl, triazolyl, tetrazolyl, pyridinyl, pyrimidinyl, pyridazinyl, pyrazinyl, indazolyl, benzo[d]imidazolyl, benzo[d]oxazolyl and indolyl. The above-mentioned heteroaryl groups are unsubstituted or substituted as explicitly defined. For the substituent **HET¹** examples of 5-membered heteroaryl groups are especially oxazolyl, thiazolyl, and imidazolyl.

The term "cyano" refers to a group -CN.

The term "oxo" refers to a group =O which is preferably attached to a chain or ring carbon (or sulfur) atom as for example in a carbonyl group -(CO)- (or a sulfonyl group -(SO₂)-).

Whenever the word "between" is used to describe a numerical range, it is to be understood that the end points of the indicated range are explicitly included in the range. For example: if a temperature range is described to be between 40 °C and 80 °C, this means that the end points 40 °C and 80 °C are included in the range; or if a variable is defined as being an integer between 1 and 4, this means that the variable is the integer 1, 2, 3, or 4.

Unless used regarding temperatures, the term "about" placed before a numerical value "X" refers in the current application to an interval extending from X minus 10% of X to X plus 10% of X, and preferably to an interval extending from X minus 5% of X to X plus 5% of X. In the particular case of temperatures, the term "about" placed before a temperature "Y" refers in the current application to an interval extending from the temperature Y minus 10°C to Y plus 10°C, and preferably to an interval extending from Y minus 5°C to Y plus 5°C. Besides, the term "room temperature" as used herein refers to a temperature of about 25°C.

Further embodiments of the invention are presented hereinafter:
3) Another embodiment relates to compounds according to embodiments 1) or 2), wherein
   ➢ **R^{P2}** represents fluoro or chloro (especially fluoro);
   ➢ **R^{P3}** represents fluoro or chloro (especially fluoro); and
   ➢ **R^{P4}** represents halogen (especially fluoro, chloro, bromo), methyl or cyano.
4) Another embodiment relates to compounds according to embodiments 1) or 2), wherein
   ➢ **R^{P2}** represents fluoro;
   ➢ **R^{P3}** represents fluoro; and
   ➢ **R^{P4}** represents fluoro, chloro, bromo, methyl or cyano.
5) Another embodiment relates to compounds according to embodiments 1) or 2), wherein
   ➢ **R^{P2}** represents fluoro or chloro (especially fluoro);
   ➢ **R^{P3}** represents fluoro or chloro (especially fluoro); and
   ➢ **R^{P4}** represents halogen (especially fluoro, chloro, bromo), or methyl.
6) Another embodiment relates to compounds according to embodiments 1) or 2), wherein
   ➢ **R^{P2}** represents fluoro;
   ➢ **R^{P3}** represents fluoro; and
   ➢ **R^{P4}** represents fluoro, chloro, bromo, or methyl.
7) Another embodiment relates to compounds according to any one of embodiments 1) to 6), wherein **R¹** represents hydroxy; or C₁₋₄-alkoxy (especially methoxy).
8) Another embodiment relates to compounds according to any one of embodiments 1) to 6), wherein **R¹** represents methoxy.
9) Another embodiment relates to compounds according to any one of embodiments 1) to 8), wherein
   - **R²** represents branched C₃₋₆-alkyl wherein said branched C₃₋₆-alkyl is
      ➢ mono-substituted with hydroxy [especially such group is 2-hydroxy-1,1-dimethyl-ethyl or 1-hydroxy-1-methyl-ethyl],
      ➢ mono-substituted with -CO-O-C₁₋₄-alkyl [especially such group is 1-methoxy-2-methyl-1-oxopropan-2-yl], or
      ➢ mono-substituted with C₁-fluoroalkyl [especially such group is 2,2-difluoro-1,1-dimethyl-ethyl];
   - or **R²** represents a saturated 3- to 8-membered mono- or bicyclic group, wherein said mono- or bicyclic group is
      ❖ mono-cyclic C₃₋₆-cycloalkyl (especially cyclopropyl, cyclobutyl or cyclopentyl), wherein said mono-cyclic C₃₋₆-cycloalkyl is
         ➢ unsubstituted;
         ➢ mono-substituted with hydroxy;
         ➢ mono-substituted with C₁₋₃-alkyl (especially methyl; or isopropyl);
         ➢ mono-substituted with -C₁₋₃-alkylene-OH (especially hydroxymethyl);
         ➢ mono-substituted with -C₁₋₃-alkylene-O-C₁₋₃-alkyl (especially methoxymethyl);
         ➢ di-substituted wherein one substituent is hydroxy and the other is C₁₋₃-alkyl (especially methyl); or
         ➢ tri-substituted wherein two of said substituents are fluoro, wherein said fluoro substituents both are attached to the same ring carbon atom; and the remaining substituent is C₁₋₃-alkyl (especially methyl) or -C₁₋₃-alkylene-OH (especially hydroxymethyl);
      ❖ mono-cyclic 4- to 6-membered heterocycloalkyl containing one ring oxygen atom (especially oxetan-3-yl, tetrahydro-2H-pyran-3-yl), wherein said mono-cyclic 4- to 6-membered heterocycloalkyl group is
         ➢ mono-substituted with hydroxy;
         ➢ mono-substituted with C₁₋₃-alkyl (especially methyl; or ethyl);
         ➢ mono-substituted with -C₁₋₃-alkylene-OH (especially hydroxymethyl, 2-hydroxyethyl); or
         ➢ mono-substituted with -C₁₋₃-alkylene-O-C₁₋₃-alkyl (especially methoxymethyl);
      ❖ bridged bicyclic C₅₋₈-cycloalkyl (especially bicyclo[1.1.1]pentan-1-yl, bicyclo[2.2.2]octan-1-yl), wherein said bridged bicyclic C₅₋₈-cycloalkyl group is
         ➢ unsubstituted;
         ➢ mono-substituted with hydroxy;
         ➢ mono-substituted with C₁₋₃-alkyl (especially methyl; or ethyl or isopropyl);
         ➢ mono-substituted with C₁₋₃-alkoxy (especially methoxy);
         ➢ mono-substituted with C₁-fluoroalkyl (especially trifluoromethyl);
         ➢ mono-substituted with -NR^{N1}R^{N2}, wherein R^{N1} represents hydrogen, and R^{N2} represents hydrogen or -CO-O-C₁₋₄-alkyl (especially -CO-O-tert.-butyl); or
         ➢ mono--substituted with fluoro;
      ❖ unsubstituted fused bicyclic C₆₋₈-cycloalkyl (especially bicyclo[3.1.0]hexan-6-yl);
      ❖ unsubstituted spiro-bicyclic C₆₋₈-cycloalkyl (especially spiro[2.3]hexan-5-yl); or
      ❖ unsubstituted spiro-bicyclic 7- or 8-membered heterocycloalkyl containing one ring oxygen atom (especially oxaspiro[3.3]heptan-6-yl).
10) Another embodiment relates to compounds according to any one of embodiments 1) to 9), wherein A represents [1,2,3]triazol-1,4-diyl wherein **R²** is attached to position 1 of said [1,2,3]triazol-1,4-diyl.
11) Another embodiment relates to compounds according to any one of embodiments 1) to 9), wherein A represents [1,2,3]triazol-1,4-diyl wherein **R²** is attached to position 4 of said [1,2,3]triazol-1,4-diyl.
12) Another embodiment relates to compounds according to any one of embodiments 1) to 8), wherein
   a) the group **A-R²** represents a group 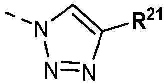 selected from:
      (i) 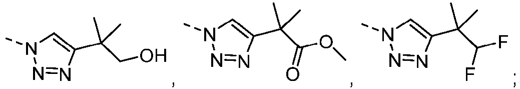
      (ii) 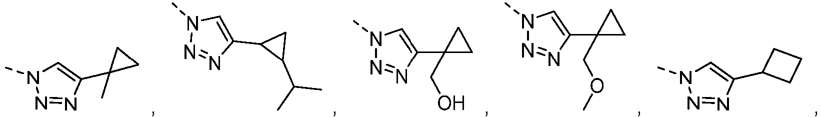 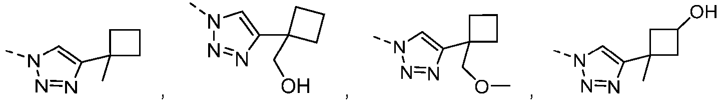 (especially 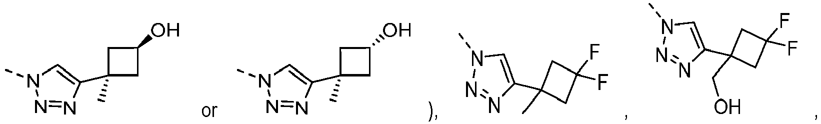 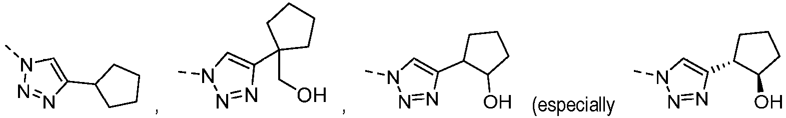 or 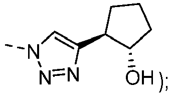
      (iii) 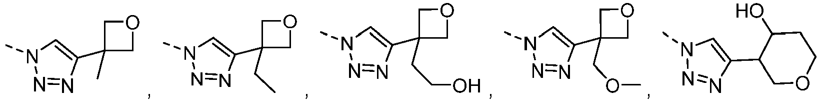 (especially 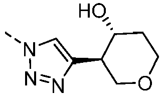 );
      (iv) 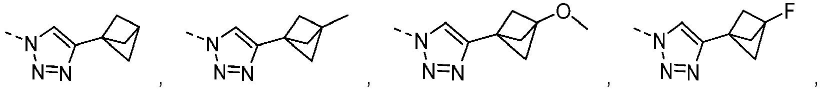 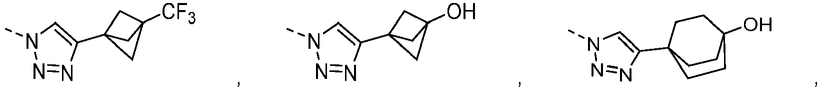 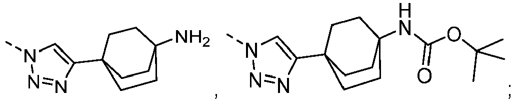 or
      (v) 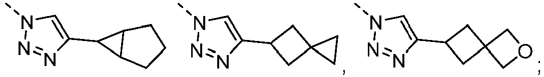 or
   b) the group **A-R²** represents a group 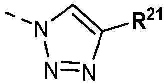 selected from:
      (i) 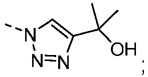
      (ii) 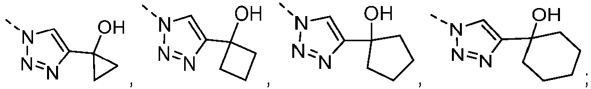 or
      (iii) 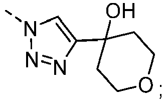 or
   c) the group **A-R²** represents a group 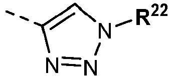 selected from:
      (i) 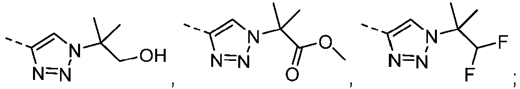
      (ii) 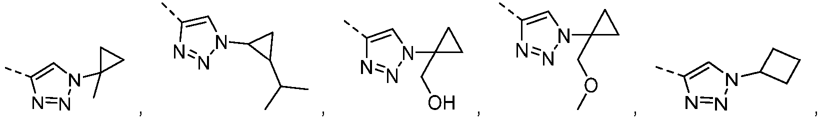 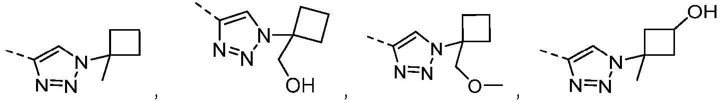 (especially 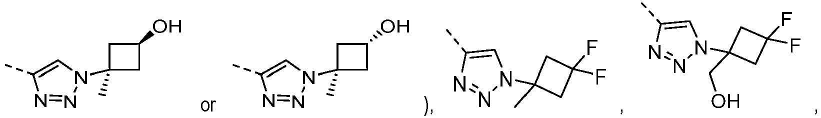 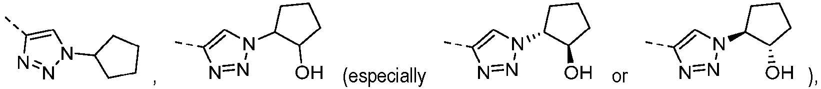 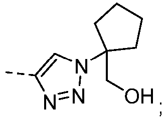
      (iii) 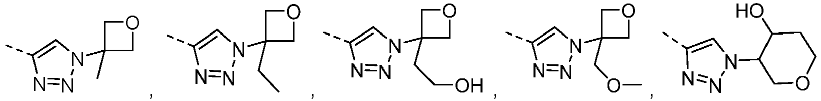 (especially 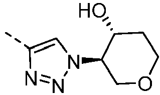 );
      (iv) 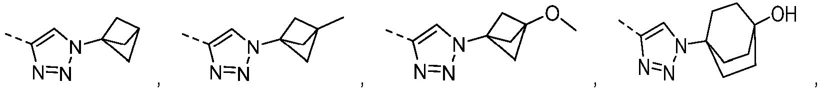 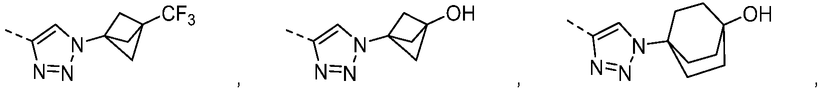 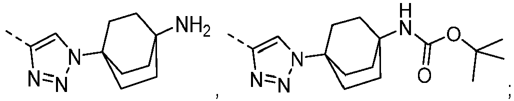 or
      (v) 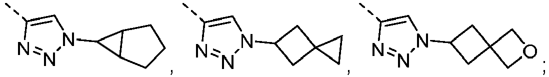
   wherein each of the groups a), b) and c) forms a separate sub-embodiment.
13) Another embodiment relates to compounds according to any one of embodiments 1) to 8), wherein
   a) the group **A-R²** represents a group 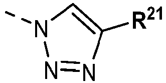 selected from:
      (i) 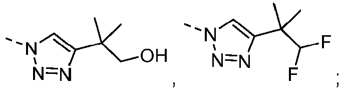
      (ii) 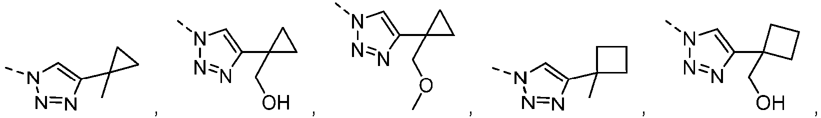 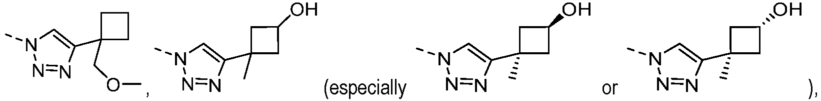 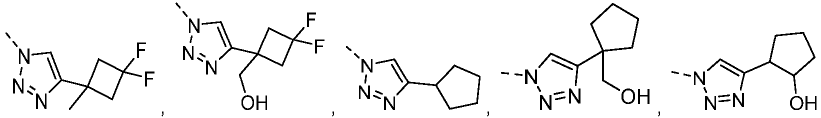 (especially 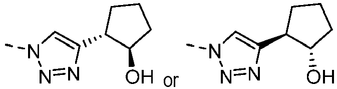 );
      (iii) 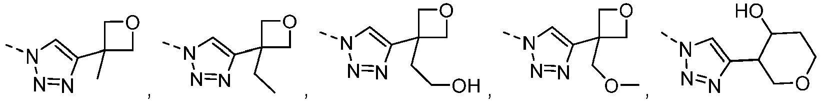 (especially 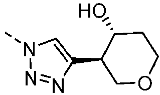 ); or
      (iv) 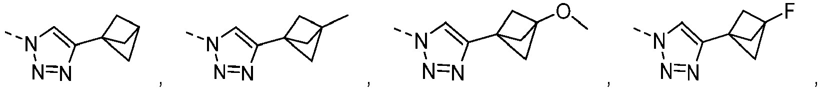 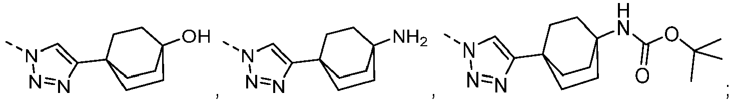 or
   b) the group **A-R²** represents a group 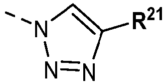 selected from:
      (i) 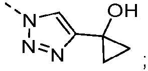 or
      (ii) 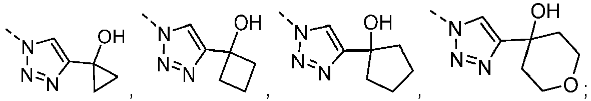 or
   c) the group **A-R²** represents a group 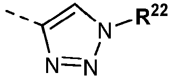 selected from:
      (i) 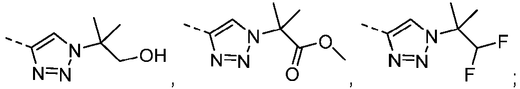
      (ii) 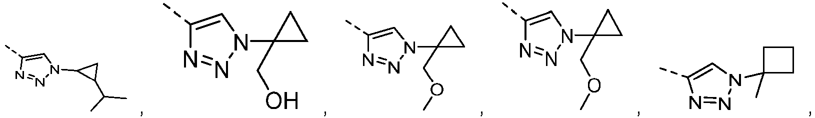 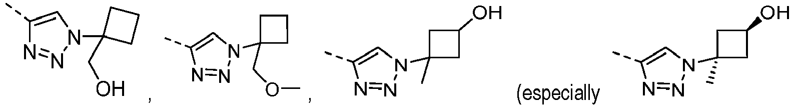 or 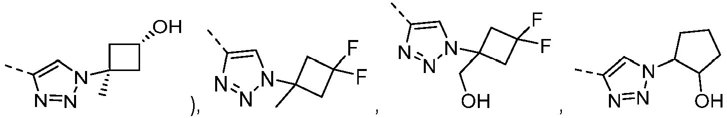 (especially 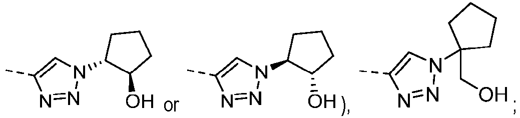
      (iii) 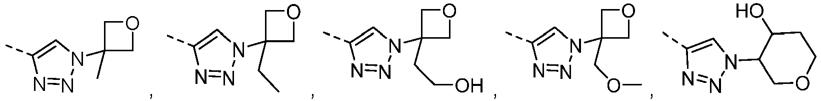 (especially 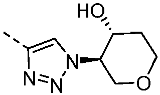 );
      (iv) 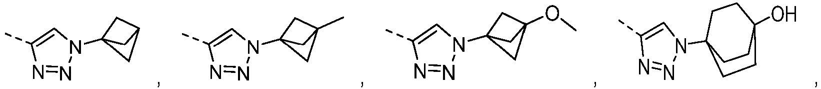 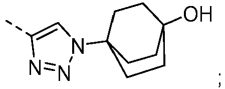 or
      (vi) 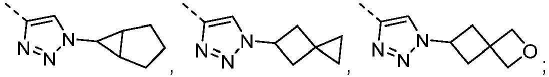
   wherein each of the groups a), b) and c) forms a separate sub-embodiment.
14) Another embodiment relates to compounds according to any one of embodiments 1) to 8), wherein
   a) the group **A-R²** represents a group 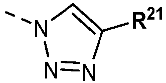 selected from:
      (i) 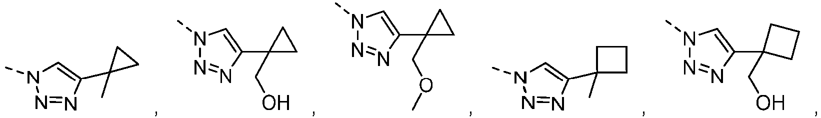 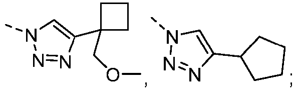
      (ii) 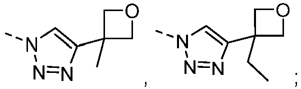 or
      (iii) 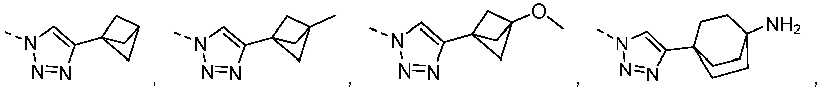 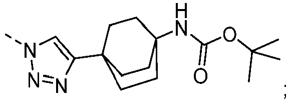 or
   b) the group **A-R²** represents a group 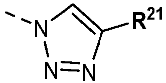 selected from:
      (i) 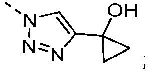 or
      (ii) 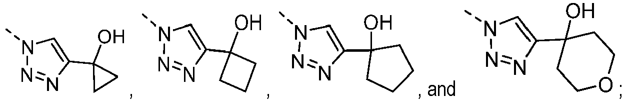 or
   c) the group **A-R²** represents a group 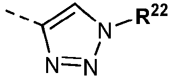 selected from:
      (i) 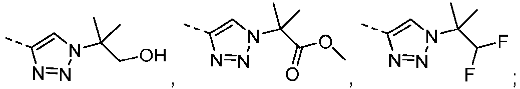
      (ii) 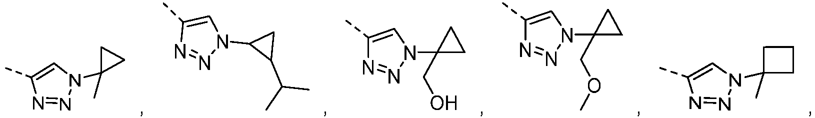 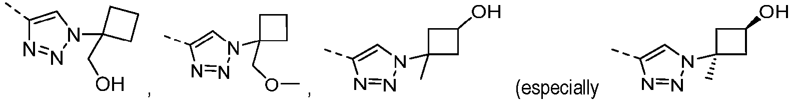 or 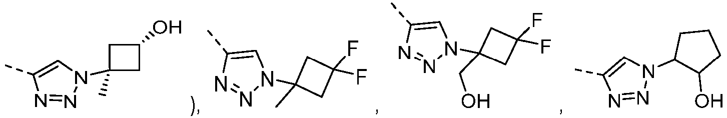 (especially 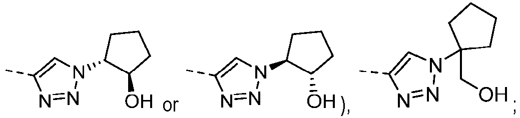 (iii) 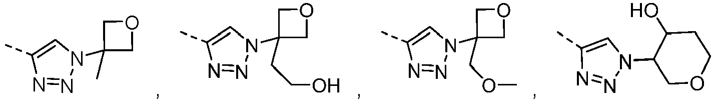 (especially 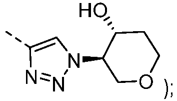
      (iv) 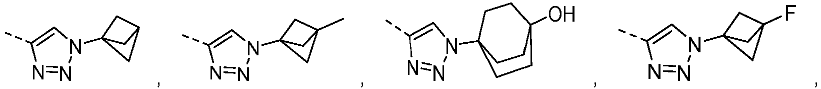 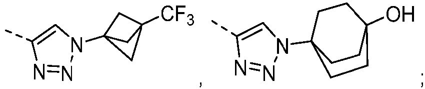 or
      (vii) 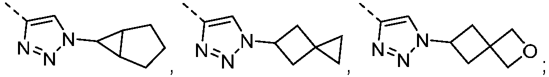
   wherein each of the groups a), b) and c) forms a separate sub-embodiment.
15) Another embodiment relates to compounds according to any one of embodiments 1) to 8), wherein
   a) the group **A-R²** represents a group 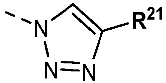 selected from:
      (i) 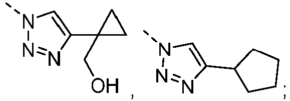
      (ii) 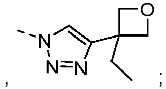 or
      (iii) 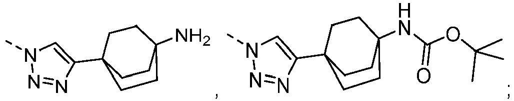 or
   b) the group **A-R²** represents a group 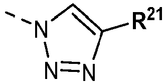 selected from:
      (i) 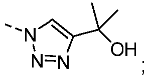 or
      (ii) 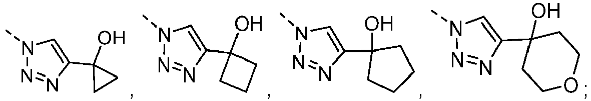 or
   c) the group **A-R²** represents a group 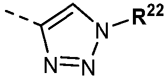 selected from:
      (i) 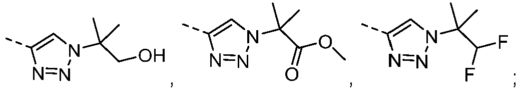
      (ii) 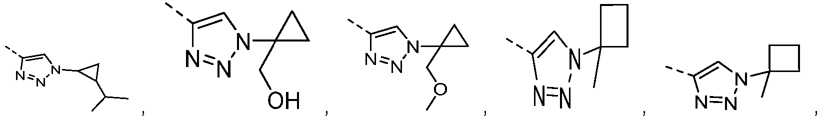 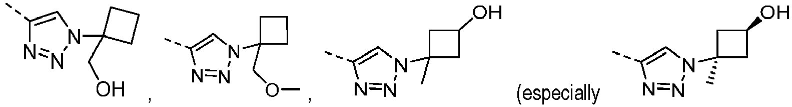 or 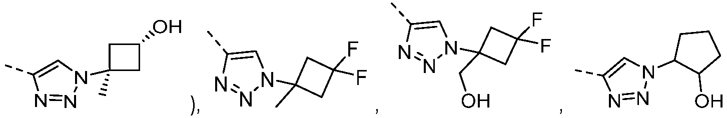 (especially 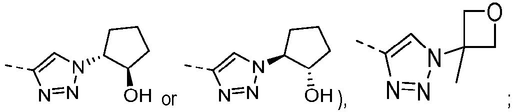
      (iii) 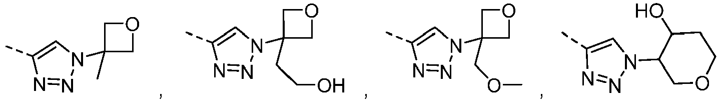 (especially 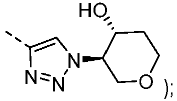
      (iv) 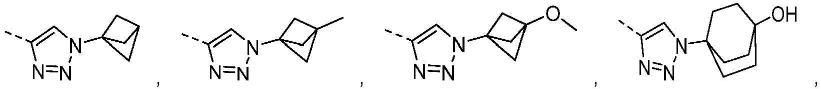 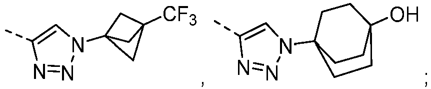 or
      (v) 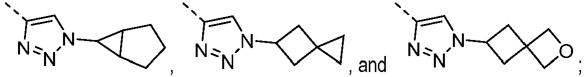
   wherein each of the groups a), b) and c) forms a separate sub-embodiment.
16) Another embodiment relates to compounds according to any one of embodiments 1) to 8), wherein
   a) the group **A-R²** represents a group 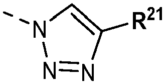 selected from:
      (i) 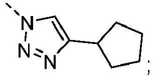
      (ii) 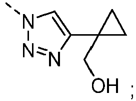 or
      (iii) 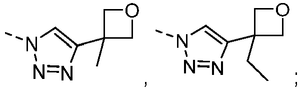 or
   b) the group **A-R²** represents a group 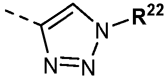 selected from:
      (i) 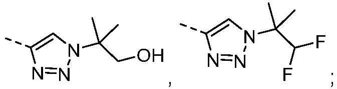
      (ii) 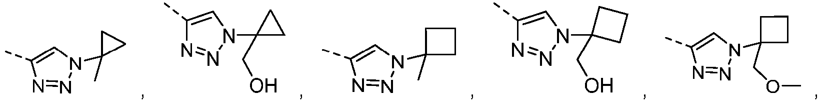 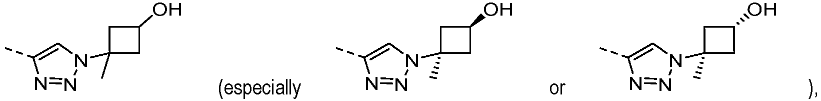 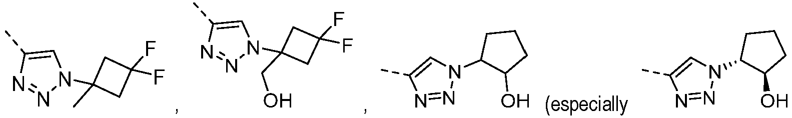 or 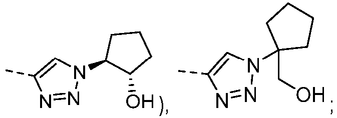
      (iii) 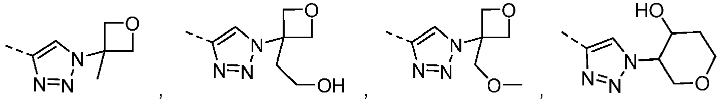 (especially 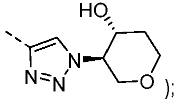 or
      (iv) 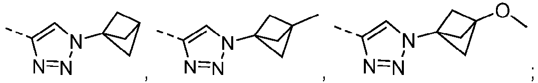
   wherein each of the groups a) and b) forms a separate sub-embodiment.
17) Another embodiment relates to compounds according to any one of embodiments 1) to 8), wherein
   a) the group **A-R²** represents a group 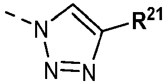 selected from:
      (i) 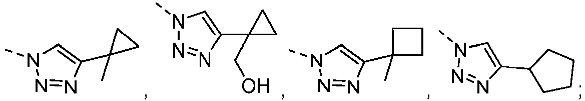
      (ii) 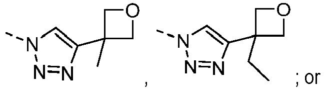
      (iii) 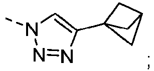 or
   b) the group **A-R²** represents a group 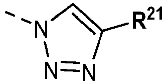 selected from:
      (i) 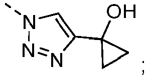 or
      (ii) 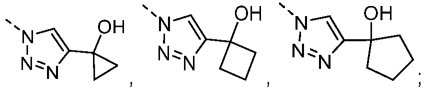 or
   c) the group **A-R²** represents a group 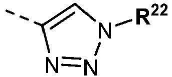 selected from:
      (i) 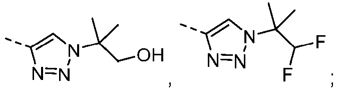
      (ii) 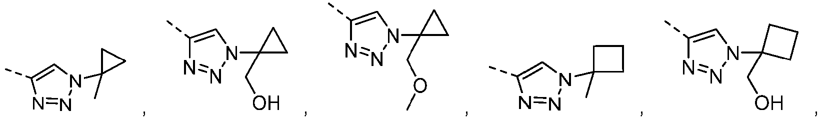 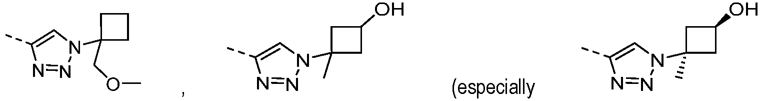 or 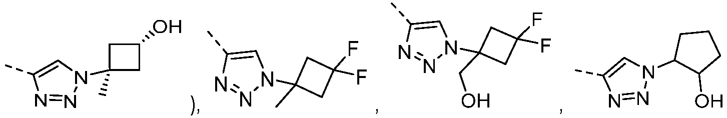 (especially 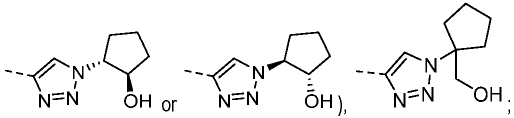
      (iii) 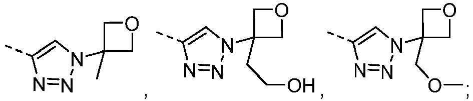
      (iv) 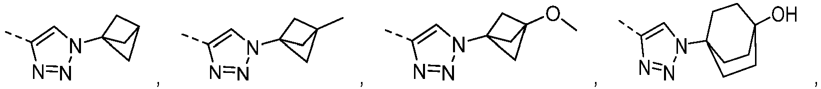 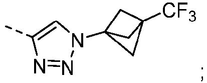
   wherein each of the groups a), b) and c) forms a separate sub-embodiment.
18) In a further embodiment, the invention relates to compounds of the Formula (I) which are also compounds of the Formula (II), 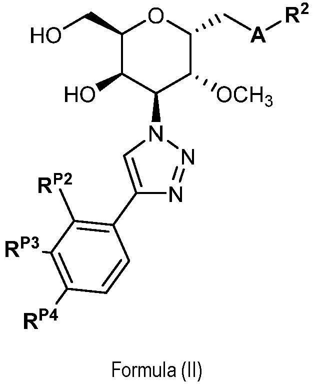 wherein
   **R^{P2}** represents halogen (especially fluoro);
   **R^{P3}** represents halogen (especially fluoro);
   **R^{P4}** represents halogen (especially fluoro, chloro, bromo), methyl or cyano; and
   the group **-A-R²** represents a group 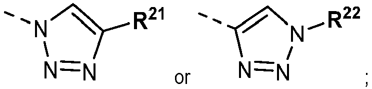 wherein
      - **R²¹** and **R²¹** independently represent branched C₃₋₆-alkyl (especially isopropyl or tert-butyl) wherein said branched C₃₋₆-alkyl is
         ➢ mono-substituted with hydroxy [especially such group is 2-hydroxy-1,1-dimethyl-ethyl or 1-hydroxy-1-methyl-ethyl],
         ➢ mono-substituted with -CO-O-C₁₋₄-alkyl [especially such group is 1-methoxy-2-methyl-1-oxopropan-2-yl], or
         ➢ mono-substituted with C₁-fluoroalkyl [especially such group is 2,2-difluoro-1,1-dimethyl-ethyl];
      - or **R²¹** and **R²¹** independently represent a saturated 3- to 8-membered mono- or bicyclic group, wherein said mono- or bicyclic group is
         ➢ mono-cyclic C₃₋₆-cycloalkyl (especially cyclopropyl, cyclobutyl or cyclopentyl),
         ➢ mono-cyclic 4- to 6-membered heterocycloalkyl containing one ring oxygen atom (especially oxetan-3-yl, tetrahydro-2H-pyran-3-yl),
         ➢ bridged bicyclic C₅₋₈-cycloalkyl (especially bicyclo[1.1.1]pentan-1-yl, bicyclo[2.2.2]octan-1-yl),
         ➢ fused bicyclic C₆₋₈-cycloalkyl (especially bicyclo[3.1.0]hexan-6-yl),
         ➢ spiro-bicyclic C₆₋₈-cycloalkyl (especially spiro[2.3]hexan-5-yl), or
         ➢ spiro-bicyclic 7- or 8-membered heterocycloalkyl containing one ring oxygen atom (especially oxaspiro[3.3]heptan-6-yl);

         wherein said mono-cyclic or bicyclic group independently is unsubstituted, or mono-, di-, or tri-substituted wherein the substituents independently are selected from hydroxy; C₁₋₃-alkyl (especially methyl; or ethyl or isopropyl); C₁₋₃-alkoxy (especially methoxy); -C₁₋₃-alkylene-OH (especially hydroxymethyl, or 2-hydroxyethyl); C₁-fluoroalkyl (especially trifluoromethyl); -NR^{N1}R^{N2}, wherein R^{N1} represents hydrogen, and R^{N2} represents hydrogen or -CO-O-C₁₋₄-alkyl (especially -CO-O-tert.-butyl); and fluoro;
         In a sub-embodiment, said mono-cyclic or bicyclic group independently is
            ➢ unsubstituted;
            ➢ mono-substituted with hydroxy;
            ➢ mono-substituted with C₁₋₃-alkyl (especially methyl; or ethyl or isopropyl);
            ➢ mono-substituted with C₁₋₃-alkoxy (especially methoxy);
            ➢ mono-substituted with -C₁₋₃-alkylene-OH (especially hydroxymethyl, 2-hydroxyethyl);
            ➢ mono-substituted with -C₁₋₃-alkylene-O-C₁₋₃-alkyl (especially methoxymethyl);
            ➢ mono-substituted with C₁-fluoroalkyl (especially trifluoromethyl);
            ➢ mono-substituted with -NR^{N1}R^{N2}, wherein R^{N1} represents hydrogen, and R^{N2} represents hydrogen or -CO-O-C₁₋₄-alkyl (especially -CO-O-tert.-butyl);
            ➢ mono- or di-substituted with fluoro;
            ➢ di-substituted wherein one substituent is hydroxy and the other is C₁₋₃-alkyl (especially methyl); or
            ➢ tri-substituted wherein two of said substituents are fluoro (which are notably attached to the same ring carbon atom); and the remaining substituent is C₁₋₃-alkyl (especially methyl) or -C₁₋₃-alkylene-OH (especially hydroxymethyl).
   wherein the characteristics disclosed in embodiments 3) to 6) and 9) to 17) are intended to apply *mutatis mutandis* also to the compounds formula (II) according to embodiment 18).
19) A further embodiment relates to the compounds of Formula (II) according to embodiment 18), wherein the group 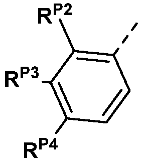 is as defined in embodiment 3); wherein especially such group is: 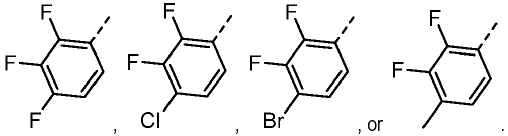
20) A further embodiment relates to the compounds of Formula (II) according to embodiment 18) or 19), wherein the group **-A-R²** is as defined in embodiment 12), 13), 14), 15), 16), or 17).
21) Another embodiment relates to compounds of Formula (I) according to embodiment 1), which are selected from the following compounds:
   (2R,3R,4S,5R,6R)-4-(4-(2,3-difluoro-4-methylphenyl)-1H-1,2,3-triazol-1-yl)-2-(hydroxymethyl)-6-((1-((3R,4R)-4-hydroxytetrahydro-2H-pyran-3-yl)-1H-1,2,3-triazol-4-yl)methyl)-5-methoxytetrahydro-2H-pyran-3-ol;
   (2R,3R,4S,5R,6R)-4-(4-(2,3-difluoro-4-methylphenyl)-1H-1,2,3-triazol-1-yl)-6-((1-((1R,2R)-2-hydroxycyclopentyl)-1H-1,2,3-triazol-4-yl)methyl)-2-(hydroxymethyl)-5-methoxytetrahydro-2H-pyran-3-ol;
   (2R,3R,4S,5R,6R)-4-(4-(2,3-difluoro-4-methylphenyl)-1H-1,2,3-triazol-1-yl)-6-((1-((1S,2S)-2-hydroxycyclopentyl)-1H-1,2,3-triazol-4-yl)methyl)-2-(hydroxymethyl)-5-methoxytetrahydro-2H-pyran-3-ol;
   (2R,3R,4S,5R,6R)-4-(4-(2,3-difluoro-4-methylphenyl)-1H-1,2,3-triazol-1-yl)-2-(hydroxymethyl)-6-((1-(1-(hydroxymethyl)cyclopropyl)-1H-1,2,3-triazol-4-yl)methyl)-5-methoxytetrahydro-2H-pyran-3-ol;
   (2R,3R,4S,5R,6R)-4-(4-(2,3-difluoro-4-methylphenyl)-1H-1,2,3-triazol-1-yl)-2-(hydroxymethyl)-6-((1-(1-(hydroxymethyl)cyclopentyl)-1H-1,2,3-triazol-4-yl)methyl)-5-methoxytetrahydro-2H-pyran-3-ol;
   (2R,3R,4S,5R,6R)-4-(4-(2,3-difluoro-4-methylphenyl)-1H-1,2,3-triazol-1-yl)-2-(hydroxymethyl)-5-methoxy-6-((1-(3-methyloxetan-3-yl)-1H-1,2,3-triazol-4-yl)methyl)tetrahydro-2H-pyran-3-ol;
   (2R,3R,4S,5R,6R)-4-(4-(2,3-difluoro-4-methylphenyl)-1H-1,2,3-triazol-1-yl)-2-(hydroxymethyl)-5-methoxy-6-((1-(1-(methoxymethyl)cyclopropyl)-1H-1,2,3-triazol-4-yl)methyl)tetrahydro-2H-pyran-3-ol;
   (2R,3R,4S,5R,6R)-4-(4-(2,3-difluoro-4-methylphenyl)-1H-1,2,3-triazol-1-yl)-6-((1-(4-hydroxybicyclo[2.2.2]octan-1-yl)-1H-1,2,3-triazol-4-yl)methyl)-2-(hydroxymethyl)-5-methoxytetrahydro-2H-pyran-3-ol;
   (2R,3R,4S,5R,6R)-4-(4-(2,3-difluoro-4-methylphenyl)-1H-1,2,3-triazol-1-yl)-6-((1-(1-hydroxy-2-methylpropan-2-yl)-1H-1,2,3-triazol-4-yl)methyl)-2-(hydroxymethyl)-5-methoxytetrahydro-2H-pyran-3-ol;
   methyl 2-(4-(((2R,3R,4S,5R,6R)-4-(4-(2,3-difluoro-4-methylphenyl)-1H-1,2,3-triazol-1-yl)-5-hydroxy-6-(hydroxymethyl)-3-methoxytetrahydro-2H-pyran-2-yl)methyl)-1H-1,2,3-triazol-1-yl)-2-methylpropanoate;
   (2R,3R,4S,5R,6R)-4-(4-(2,3-difluoro-4-methylphenyl)-1H-1,2,3-triazol-1-yl)-2-(hydroxymethyl)-6-((1-(1-(hydroxymethyl)cyclobutyl)-1H-1,2,3-triazol-4-yl)methyl)-5-methoxytetrahydro-2H-pyran-3-ol;
   (2R,3R,4S,5R,6R)-6-((1-(bicyclo[1.1.1]pentan-1-yl)-1H-1,2,3-triazol-4-yl)methyl)-4-(4-(2,3-difluoro-4-methylphenyl)-1H-1,2,3-triazol-1-yl)-2-(hydroxymethyl)-5-methoxytetrahydro-2H-pyran-3-ol;
   (2R,3R,4S,5R,6R)-4-(4-(2,3-difluoro-4-methylphenyl)-1H-1,2,3-triazol-1-yl)-2-(hydroxymethyl)-5-methoxy-6-((1-(1-methylcyclobutyl)-1H-1,2,3-triazol-4-yl)methyl)tetrahydro-2H-pyran-3-ol;
   (2R,3R,4S,5R,6R)-4-(4-(2,3-difluoro-4-methylphenyl)-1H-1,2,3-triazol-1-yl)-2-(hydroxymethyl)-5-methoxy-6-((1-(3-methylbicyclo[1.1.1]pentan-1-yl)-1H-1,2,3-triazol-4-yl)methyl)tetrahydro-2H-pyran-3-ol;
   (2R,3R,4S,5R,6R)-6-((1-(3,3-difluoro-1-methylcyclobutyl)-1H-1,2,3-triazol-4-yl)methyl)-4-(4-(2,3-difluoro-4-methylphenyl)-1H-1,2,3-triazol-1-yl)-2-(hydroxymethyl)-5-methoxytetrahydro-2H-pyran-3-ol;
   (2R,3R,4S,5R,6R)-6-((1-(2-oxaspiro[3.3]heptan-6-yl)-1H-1,2,3-triazol-4-yl)methyl)-4-(4-(2,3-difluoro-4-methylphenyl)-1H-1,2,3-triazol-1-yl)-2-(hydroxymethyl)-5-methoxytetrahydro-2H-pyran-3-ol;
   (2R,3R,4S,5R,6R)-4-(4-(2,3-difluoro-4-methylphenyl)-1H-1,2,3-triazol-1-yl)-2-(hydroxymethyl)-6-((1-((1RS,2SR)-2-isopropylcyclopropyl)-1H-1,2,3-triazol-4-yl)methyl)-5-methoxytetrahydro-2H-pyran-3-ol;
   (2R,3R,4S,5R,6R)-6-((1-(3,3-difluoro-1-(hydroxymethyl)cyclobutyl)-1H-1,2,3-triazol-4-yl)methyl)-4-(4-(2,3-difluoro-4-methylphenyl)-1H-1,2,3-triazol-1-yl)-2-(hydroxymethyl)-5-methoxytetrahydro-2H-pyran-3-ol;
   (2R,3R,4S,5R,6R)-4-(4-(2,3-difluoro-4-methylphenyl)-1H-1,2,3-triazol-1-yl)-6-((1-((1S,3S)-3-hydroxy-1-methylcyclobutyl)-1H-1,2,3-triazol-4-yl)methyl)-2-(hydroxymethyl)-5-methoxytetrahydro-2H-pyran-3-ol;
   (2R,3R,4S,5R,6R)-6-((1-((1R,5S)-bicyclo[3.1.0]hexan-6-yl)-1H-1,2,3-triazol-4-yl)methyl)-4-(4-(2,3-difluoro-4-methylphenyl)-1H-1,2,3-triazol-1-yl)-2-(hydroxymethyl)-5-methoxytetrahydro-2H-pyran-3-ol;
   (2R,3R,4S,5R,6R)-4-(4-(2,3-difluoro-4-methylphenyl)-1H-1,2,3-triazol-1-yl)-2-(hydroxymethyl)-5-methoxy-6-((1-(3-(methoxymethyl)oxetan-3-yl)-1H-1,2,3-triazol-4-yl)methyl)tetrahydro-2H-pyran-3-ol;
   (2R,3R,4S,5R,6R)-4-(4-(2,3-difluoro-4-methylphenyl)-1H-1,2,3-triazol-1-yl)-6-((1-(3-(2-hydroxyethyl)oxetan-3-yl)-1H-1,2,3-triazol-4-yl)methyl)-2-(hydroxymethyl)-5-methoxytetrahydro-2H-pyran-3-ol;
   (2R,3R,4S,5R,6R)-4-(4-(2,3-difluoro-4-methylphenyl)-1H-1,2,3-triazol-1-yl)-2-(hydroxymethyl)-5-methoxy-6-((1-(1-methylcyclopropyl)-1H-1,2,3-triazol-4-yl)methyl)tetrahydro-2H-pyran-3-ol;
   (2R,3R,4S,5R,6R)-4-(4-(2,3-difluoro-4-methylphenyl)-1H-1,2,3-triazol-1-yl)-2-(hydroxymethyl)-5-methoxy-6-((1-(3-(trifluoromethyl)bicyclo[1.1.1]pentan-1-yl)-1H-1,2,3-triazol-4-yl)methyl)tetrahydro-2H-pyran-3-ol;
   (2R,3R,4S,5R,6R)-4-(4-(2,3-difluoro-4-methylphenyl)-1H-1,2,3-triazol-1-yl)-6-((1-(3-fluorobicyclo[1.1.1]pentan-1-yl)-1H-1,2,3-triazol-4-yl)methyl)-2-(hydroxymethyl)-5-methoxytetrahydro-2H-pyran-3-ol;
   (2R,3R,4S,5R,6R)-4-(4-(2,3-difluoro-4-methylphenyl)-1H-1,2,3-triazol-1-yl)-2-(hydroxymethyl)-5-methoxy-6-((1-(spiro[2.3]hexan-5-yl)-1H-1,2,3-triazol-4-yl)methyl)tetrahydro-2H-pyran-3-ol;
   (2R,3R,4S,5R,6R)-4-(4-(2,3-difluoro-4-methylphenyl)-1H-1,2,3-triazol-1-yl)-6-((1-((1r,3R)-3-hydroxy-1-methylcyclobutyl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-2-(hydroxymethyl)-5-methoxy-6-((1-(1-(methoxymethyl)cyclopropyl)-1H-1,2,3-triazol-4-yl)methyl)tetrahydro-2H-pyran-3-ol;
   (2R,3R,4S,5R,6R)-4-(4-(4-chloro-2,3-difluorophenyl)-1H-1,2,3-triazol-1-yl)-6-((1-(1,1-difluoro-2-methylpropan-2-yl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-2-(hydroxymethyl)-6-((1-(1-(hydroxymethyl)cyclopropyl)-1H-1,2,3-triazol-4-yl)methyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-2-(hydroxymethyl)-6-((1-(1-(hydroxymethyl)cyclopentyl)-1H-1,2,3-triazol-4-yl)methyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-2-(hydroxymethyl)-6-((1-(1-(hydroxymethyl)cyclobutyl)-1H-1,2,3-triazol-4-yl)methyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-6-((1-(4-hydroxybicyclo[2.2.2]octan-1-yl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-6-((1-(1-hydroxy-2-methylpropan-2-yl)-1H-1,2,3-triazol-4-yl)methyl)-2-(hydroxymethyl)-5-methoxytetrahydro-2H-pyran-3-ol;
   methyl 2-(4-(((2R,3R,4S,5R,6R)-4-(4-(4-chloro-2,3-difluorophenyl)-1H-1,2,3-triazol-1-yl)-5-hydroxy-6-(hydroxymethyl)-3-methoxytetrahydro-2H-pyran-2-yl)methyl)-1H-1,2,3-triazol-1-yl)-2-methylpropanoate;
   (2R,3R,4S,5R,6R)-6-((1-(bicyclo[1.1.1]pentan-1-yl)-1H-1,2,3-triazol-4-yl)methyl)-4-(4-(4-chloro-2,3-difluorophenyl)-1H-1,2,3-triazol-1-yl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-2-(hydroxymethyl)-5-methoxy-6-((1-(1-methylcyclobutyl)-1H-1,2,3-triazol-4-yl)methyl)tetrahydro-2H-pyran-3-ol;
   (2R,3R,4S,5R,6R)-4-(4-(4-chloro-2,3-difluorophenyl)-1H-1,2,3-triazol-1-yl)-2-(hydroxymethyl)-5-methoxy-6-((1-(3-methyloxetan-3-yl)-1H-1,2,3-triazol-4-yl)methyl)tetrahydro-2H-pyran-3-ol;
   (2R,3R,4S,5R,6R)-4-(4-(4-chloro-2,3-difluorophenyl)-1H-1,2,3-triazol-1-yl)-2-(hydroxymethyl)-5-methoxy-6-((1-(3-methylbicyclo[1.1.1]pentan-1-yl)-1H-1,2,3-triazol-4-yl)methyl)tetrahydro-2H-pyran-3-ol;
   (2R,3R,4S,5R,6R)-4-(4-(4-chloro-2,3-difluorophenyl)-1H-1,2,3-triazol-1-yl)-2-(hydroxymethyl)-5-methoxy-6-((1-(3-methoxybicyclo[1.1.1]pentan-1-yl)-1H-1,2,3-triazol-4-yl)methyl)tetrahydro-2H-pyran-3-ol;
   (2R,3R,4S,5R,6R)-4-(4-(4-chloro-2,3-difluorophenyl)-1H-1,2,3-triazol-1-yl)-6-((1-(3,3-difluoro-1-methylcyclobutyl)-1H-1,2,3-triazol-4-yl)methyl)-2-(hydroxymethyl)-5-methoxytetrahydro-2H-pyran-3-ol;
   (2R,3R,4S,5R,6R)-6-((1-(2-oxaspiro[3.3]heptan-6-yl)-1H-1,2,3-triazol-4-yl)methyl)-4-(4-(4-chloro-2,3-difluorophenyl)-1H-1,2,3-triazol-1-yl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-2-(hydroxymethyl)-6-((1-(2-isopropylcyclopropyl)-1H-1,2,3-triazol-4-yl)methyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-6-((1-(3,3-difluoro-1-(hydroxymethyl)cyclobutyl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-6-((1-((1S,3S)-3-hydroxy-1-methylcyclobutyl)-1H-1,2,3-triazol-4-yl)methyl)-2-(hydroxymethyl)-5-methoxytetrahydro-2H-pyran-3-ol;
   (2R,3R,4S,5R,6R)-6-((1-((1R,5S)-bicyclo[3.1.0]hexan-6-yl)-1H-1 ,2,3-triazol-4-yl)methyl)-4-(4-(4-chloro-2,3-difluorophenyl)-1H-1,2,3-triazol-1-yl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-2-(hydroxymethyl)-5-methoxy-6-((1-(3-(methoxymethyl)oxetan-3-yl)-1H-1,2,3-triazol-4-yl)methyl)tetrahydro-2H-pyran-3-ol;
   (2R,3R,4S,5R,6R)-4-(4-(4-chloro-2,3-difluorophenyl)-1H-1,2,3-triazol-1-yl)-6-((1-(3-(2-hydroxyethyl)oxetan-3-yl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-2-(hydroxymethyl)-5-methoxy-6-((1-(1-methylcyclopropyl)-1H-1,2,3-triazol-4-yl)methyl)tetrahydro-2H-pyran-3-ol;
   (2R,3R,4S,5R,6R)-4-(4-(4-chloro-2,3-difluorophenyl)-1H-1,2,3-triazol-1-yl)-2-(hydroxymethyl)-5-methoxy-6-((1-(3-(trifluoromethyl)bicyclo[1.1.1]pentan-1-yl)-1H-1,2,3-triazol-4-yl)methyl)tetrahydro-2H-pyran-3-ol;
   (2R,3R,4S,5R,6R)-4-(4-(4-chloro-2,3-difluorophenyl)-1H-1,2,3-triazol-1-yl)-6-((1-(3-fluorobicyclo[1.1.1]pentan-1-yl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-2-(hydroxymethyl)-5-methoxy-6-((1-(spiro[2.3]hexan-5-yl)-1H-1,2,3-triazol-4-yl)methyl)tetrahydro-2H-pyran-3-ol;
   (2R,3R,4S,5R,6R)-4-(4-(4-chloro-2,3-difluorophenyl)-1H-1,2,3-triazol-1-yl)-6-((1-((1R,3R)-3-hydroxy-1-methylcyclobutyl)-1H-1,2,3-triazol-4-yl)methyl)-2-(hydroxymethyl)-5-methoxytetrahydro-2H-pyran-3-ol;
   (2R,3R,4S,5R,6R)-2-(hydroxymethyl)-6-((1-(1-(hydroxymethyl)cyclopentyl)-1H-1,2,3-triazol-4-yl)methyl)-5-methoxy-4-(4-(2,3,4-trifluorophenyl)-1H-1,2,3-triazol-1-yl)tetrahydro-2H-pyran-3-ol;
   (2R,3R,4S,5R,6R)-2-(hydroxymethyl)-6-((1-(1-(hydroxymethyl)cyclopropyl)-1H-1,2,3-triazol-4-yl)methyl)-5-methoxy-4-(4-(2,3,4-trifluorophenyl)-1H-1,2,3-triazol-1-yl)tetrahydro-2H-pyran-3-ol;
   (2R,3R,4S,5R,6R)-2-(hydroxymethyl)-5-methoxy-6-((1-(1-(methoxymethyl)cyclopropyl)-1 H-1,2,3-triazol-4-yl)methyl)-4-(4-(2,3,4-trifluorophenyl)-1H-1,2,3-triazol-1-yl)tetrahydro-2H-pyran-3-ol;
   (2R,3R,4S,5R,6R)-6-((1-(bicyclo[1.1.1]pentan-1-yl)-1H-1,2,3-triazol-4-yl)methyl)-2-(hydroxymethyl)-5-methoxy-4-(4-(2,3,4-trifluorophenyl)-1H-1,2,3-triazol-1-yl)tetrahydro-2H-pyran-3-ol;
   (2R,3R,4S,5R,6R)-2-(hydroxymethyl)-6-((1-(1-(hydroxymethyl)cyclobutyl)-1H-1,2,3-triazol-4-yl)methyl)-5-methoxy-4-(4-(2,3,4-trifluorophenyl)-1H-1,2,3-triazol-1-yl)tetrahydro-2H-pyran-3-ol;
   (2R,3R,4S,5R,6R)-6-((1-(3-fluorobicyclo[1.1.1]pentan-1-yl)-1H-1,2,3-triazol-4-yl)methyl)-2-(hydroxymethyl)-5-methoxy-4-(4-(2,3,4-trifluorophenyl)-1H-1,2,3-triazol-1-yl)tetrahydro-2H-pyran-3-ol;
   (2R,3R,4S,5R,6R)-2-(hydroxymethyl)-5-methoxy-6-((1-(3-(trifluoromethyl)bicyclo[1.1.1]pentan-1-yl)-1H-1,2,3-triazol-4-yl)methyl)-4-(4-(2,3,4-trifluorophenyl)-1H-1,2,3-triazol-1-yl)tetrahydro-2H-pyran-3-ol;
   (2R,3R,4S,5R,6R)-6-((1-(3,3-difluoro-1-methylcyclobutyl)-1H-1,2,3-triazol-4-yl)methyl)-2-(hydroxymethyl)-5-methoxy-4-(4-(2,3,4-trifluorophenyl)-1H-1,2,3-triazol-1-yl)tetrahydro-2H-pyran-3-ol;
   (2R,3R,4S,5R,6R)-6-((1-(3,3-difluoro-1-(hydroxymethyl)cyclobutyl)-1H-1,2,3-triazol-4-yl)methyl)-2-(hydroxymethyl)-5-methoxy-4-(4-(2,3,4-trifluorophenyl)-1H-1,2,3-triazol-1-yl)tetrahydro-2H-pyran-3-ol;
   (2R,3R,4S,5R,6R)-6-((1-(2-oxaspiro[3.3]heptan-6-yl)-1H-1,2,3-triazol-4-yl)methyl)-2-(hydroxymethyl)-5-methoxy-4-(4-(2,3,4-trifluorophenyl)-1H-1,2,3-triazol-1-yl)tetrahydro-2H-pyran-3-ol;
   (2R,3R,4S,5R,6R)-6-((1-((1R,3R)-3-hydroxy-1-methylcyclobutyl)-1H-1,2,3-triazol-4-yl)methyl)-2-(hydroxymethyl)-5-methoxy-4-(4-(2,3,4-trifluorophenyl)-1H-1,2,3-triazol-1-yl)tetrahydro-2H-pyran-3-ol;
   (2R,3R,4S,5R,6R)-6-((1-((1S,3S)-3-hydroxy-1-methylcyclobutyl)-1H-1,2,3-triazol-4-yl)methyl)-2-(hydroxymethyl)-5-methoxy-4-(4-(2,3,4-trifluorophenyl)-1H-1,2,3-triazol-1-yl)tetrahydro-2H-pyran-3-ol;
   (2R,3R,4S,5R,6R)-2-(hydroxymethyl)-5-methoxy-6-((1-(3-(methoxymethyl)oxetan-3-yl)-1H-1,2,3-triazol-4-yl)methyl)-4-(4-(2,3,4-trifluorophenyl)-1H-1,2,3-triazol-1-yl)tetrahydro-2H-pyran-3-ol;
   (2R,3R,4S,5R,6R)-6-((1-(3-(2-hydroxyethyl)oxetan-3-yl)-1H-1,2, 3-triazol-4-yl)methyl)-2-(hydroxymethyl)-5-methoxy-4-(4-(2,3,4-trifluorophenyl)-1H-1,2,3-triazol-1-yl)tetrahydro-2H-pyran-3-ol;
   (2R,3R,4S,5R,6R)-2-(hydroxymethyl)-5-methoxy-6-((1-(1-methylcyclopropyl)-1H-1,2,3-triazol-4-yl)methyl)-4-(4-(2,3,4-trifluorophenyl)-1H-1,2,3-triazol-1-yl)tetrahydro-2H-pyran-3-ol;
   (2R,3R,4S,5R,6R)-4-(4-(4-bromo-2,3-difluorophenyl)-1H-1,2,3-triazol-1-yl)-2-(hydroxymethyl)-5-methoxy-6-((1-(3-methyloxetan-3-yl)-1H-1,2,3-triazol-4-yl)methyl)tetrahydro-2H-pyran-3-ol;
   (2R,3R,4S,5R,6R)-4-(4-(4-bromo-2,3-difluorophenyl)-1H-1,2,3-triazol-1-yl)-2-(hydroxymethyl)-5-methoxy-6-((1-(1-methylcyclobutyl)-1H-1,2,3-triazol-4-yl)methyl)tetrahydro-2H-pyran-3-ol;
   (2R,3R,4S,5R,6R)-6-((1-(bicyclo[1.1.1]pentan-1-yl)-1H-1,2,3-triazol-4-yl)methyl)-4-(4-(4-bromo-2,3-difluorophenyl)-1H-1,2,3-triazol-1-yl)-2-(hydroxymethyl)-5-methoxytetrahydro-2H-pyran-3-ol;
   (2R,3R,4S,5R,6R)-4-(4-(4-bromo-2,3-difluorophenyl)-1H-1,2,3-triazol-1-yl)-2-(hydroxymethyl)-5-methoxy-6-((1-(1-(methoxymethyl)cyclobutyl)-1H-1,2,3-triazol-4-yl)methyl)tetrahydro-2H-pyran-3-ol;
   (2R,3R,4S,5R,6R)-4-(4-(4-bromo-2,3-difluorophenyl)-1H-1,2,3-triazol-1-yl)-2-(hydroxymethyl)-6-((1-(1-(hydroxymethyl)cyclopropyl)-1H-1,2,3-triazol-4-yl)methyl)-5-methoxytetrahydro-2H-pyran-3-ol;
   (2R,3R,4S,5R,6R)-4-(4-(4-bromo-2,3-difluorophenyl)-1H-1,2,3-triazol-1-yl)-6-((1-((1S,2S)-2-hydroxycyclopentyl)-1H-1,2,3-triazol-4-yl)methyl)-2-(hydroxymethyl)-5-methoxytetrahydro-2H-pyran-3-ol;
   (2R,3R,4S,5R,6R)-4-(4-(2,3-difluoro-4-methylphenyl)-1H-1,2,3-triazol-1-yl)-6-((4-(1-hydroxycyclobutyl)-1H-1,2,3-triazol-1-yl)methyl)-2-(hydroxymethyl)-5-methoxytetrahydro-2H-pyran-3-ol;
   (2R,3R,4S,5R,6R)-6-((4-cyclopentyl-1H-1,2,3-triazol-1-yl)methyl)-4-(4-(2,3-difluoro-4-methylphenyl)-1H-1,2,3-triazol-1-yl)-2-(hydroxymethyl)-5-methoxytetrahydro-2H-pyran-3-ol;
   (2R,3R,4S,5R,6R)-4-(4-(2,3-difluoro-4-methylphenyl)-1H-1,2,3-triazol-1-yl)-6-((4-(1-hydroxycyclopentyl)-1H-1,2,3-triazol-1-yl)methyl)-2-(hydroxymethyl)-5-methoxytetrahydro-2H-pyran-3-ol;
   (2R,3R,4S,5R,6R)-4-(4-(2,3-difluoro-4-methylphenyl)-1H-1,2,3-triazol-1-yl)-2-(hydroxymethyl)-6-((4-(2-hydroxypropan-2-yl)-1H-1,2,3-triazol-1-yl)methyl)-5-methoxytetrahydro-2H-pyran-3-ol;
   (2R,3R,4S,5R,6R)-4-(4-(2,3-difluoro-4-methylphenyl)-1H-1,2,3-triazol-1-yl)-2-(hydroxymethyl)-5-methoxy-6-((4-(3-methyloxetan-3-yl)-1H-1,2,3-triazol-1-yl)methyl)tetrahydro-2H-pyran-3-ol;
   (2R,3R,4S,5R,6R)-4-(4-(2,3-difluoro-4-methylphenyl)-1H-1,2,3-triazol-1-yl)-2-(hydroxymethyl)-6-((4-(4-hydroxytetrahydro-2H-pyran-4-yl)-1H-1,2,3-triazol-1-yl)methyl)-5-methoxytetrahydro-2H-pyran-3-ol;
   (2R,3R,4S,5R,6R)-4-(4-(2,3-difluoro-4-methylphenyl)-1H-1,2,3-triazol-1-yl)-6-((4-(1-hydroxycyclopropyl)-1H-1,2,3-triazol-1-yl)methyl)-2-(hydroxymethyl)-5-methoxytetrahydro-2H-pyran-3-ol;
   (2R,3R,4S,5R,6R)-4-(4-(2,3-difluoro-4-methylphenyl)-1H-1,2,3-triazol-1-yl)-2-(hydroxymethyl)-6-((4-(1-(hydroxymethyl)cyclopropyl)-1H-1,2,3-triazol-1-yl)methyl)-5-methoxytetrahydro-2H-pyran-3-ol;
   tert-butyl (4-(1-(((2R,3R,4S,5R,6R)-4-(4-(2,3-difluoro-4-methylphenyl)-1H-1,2,3-triazol-1-yl)-5-hydroxy-6-(hydroxymethyl)-3-methoxytetrahydro-2H-pyran-2-yl)methyl)-1H-1,2,3-triazol-4-yl)bicyclo[2.2.2]octan-1-yl)carbamate;
   (2R,3R,4S,5R,6R)-4-(4-(2,3-difluoro-4-methylphenyl)-1H-1,2,3-triazol-1-yl)-6-((4-(3-ethyloxetan-3-yl)-1H-1,2,3-triazol-1-yl)methyl)-2-(hydroxymethyl)-5-methoxytetrahydro-2H-pyran-3-ol;
   (2R,3R,4S,5R,6R)-6-((1-(4-aminobicyclo[2.2.2]octan-1-yl)-1H-1,2,3-triazol-4-yl)methyl)-4-(4-(2,3-difluoro-4-methylphenyl)-1H-1,2,3-triazol-1-yl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-6-((4-(1-hydroxycyclobutyl)-1H-1,2,3-triazol-1-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-6-((4-cyclopentyl-1H-1,2,3-triazol-1-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-6-((4-(1-hydroxycyclopentyl)-1H-1,2,3-triazol-1-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-2-(hydroxymethyl)-6-((4-(2-hydroxypropan-2-yl)-1H-1,2,3-triazol-1-yl)methyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-2-(hydroxymethyl)-5-methoxy-6-((4-(3-methyloxetan-3-yl)-1H-1,2,3-triazol-1-yl)methyl)tetrahydro-2H-pyran-3-ol;
   (2R,3R,4S,5R,6R)-4-(4-(4-chloro-2,3-difluorophenyl)-1H-1,2,3-triazol-1-yl)-2-(hydroxymethyl)-6-((4-(4-hydroxytetrahydro-2H-pyran-4-yl)-1H-1,2,3-triazol-1-yl)methyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-6-((4-(1-hydroxycyclopropyl)-1H-1,2,3-triazol-1-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-2-(hydroxymethyl)-6-((4-(1-(hydroxymethyl)cyclopropyl)-1H-1,2,3-triazol-1-yl)methyl)-5-methoxytetrahydro-2H-pyran-3-ol;
   tert-butyl (4-(1-(((2R,3R,4S,5R,6R)-4-(4-(4-chloro-2,3-difluorophenyl)-1H-1,2,3-triazol-1-yl)-5-hydroxy-6-(hydroxymethyl)-3-methoxytetrahydro-2H-pyran-2-yl)methyl)-1H-1,2,3-triazol-4-yl)bicyclo[2.2.2]octan-1-yl)carbamate;
   (2R,3R,4S,5R,6R)-4-(4-(4-chloro-2,3-difluorophenyl)-1H-1,2,3-triazol-1-yl)-6-((4-(3-ethyloxetan-3-yl)-1H-1,2,3-triazol-1-yl)methyl)-2-(hydroxymethyl)-5-methoxytetrahydro-2H-pyran-3-ol; and
   (2R,3R,4S,5R,6R)-6-((1-(4-aminobicyclo[2.2.2]octan-1-yl)-1H-1,2,3-triazol-4-yl)methyl)-4-(4-(4-chloro-2,3-difluorophenyl)-1H-1,2,3-triazol-1-yl)-2-(hydroxymethyl)-5-methoxytetrahydro-2H-pyran-3-ol.
22) In addition to the compounds listed in embodiment 21), further compounds of Formula (I) according to embodiment 1) are selected from:
   (2R,3R,4S,5R,6R)-6-((4-(1-hydroxycyclobutyl)-1H-1,2,3-triazol-1-yl)methyl)-2-(hydroxymethyl)-5-methoxy-4-(4-(2,3,4-trifluorophenyl)-1H-1,2,3-triazol-1-yl)tetrahydro-2H-pyran-3-ol;
   (2R,3R,4S,5R,6R)-6-((4-cyclopentyl-1H-1,2,3-triazol-1-yl)methyl)-2-(hydroxymethyl)-5-methoxy-4-(4-(2,3,4-trifluorophenyl)-1H-1,2,3-triazol-1-yl)tetrahydro-2H-pyran-3-ol;
   (2R,3R,4S,5R,6R)-6-((4-(1-hydroxycyclopentyl)-1H-1,2,3-triazol-1-yl)methyl)-2-(hydroxymethyl)-5-methoxy-4-(4-(2,3,4-trifluorophenyl)-1H-1,2,3-triazol-1-yl)tetrahydro-2H-pyran-3-ol;
   (2R,3R,4S,5R,6R)-2-(hydroxymethyl)-6-((4-(2-hydroxypropan-2-yl)-1H-1,2,3-triazol-1-yl)methyl)-5-methoxy-4-(4-(2,3,4-trifluorophenyl)-1H-1,2,3-triazol-1-yl)tetrahydro-2H-pyran-3-ol;
   (2R,3R,4S,5R,6R)-2-(hydroxymethyl)-5-methoxy-6-((4-(3-methyloxetan-3-yl)-1H-1,2,3-triazol-1-yl)methyl)-4-(4-(2,3,4-trifluorophenyl)-1H-1,2,3-triazol-1-yl)tetrahydro-2H-pyran-3-ol;
   (2R,3R,4S,5R,6R)-2-(hydroxymethyl)-6-((4-(4-hydroxytetrahydro-2H-pyran-4-yl)-1H-1,2,3-triazol-1-yl)methyl)-5-methoxy-4-(4-(2,3,4-trifluorophenyl)-1H-1,2,3-triazol-1-yl)tetrahydro-2H-pyran-3-ol;
   (2R,3R,4S,5R,6R)-6-((4-(1-hydroxycyclopropyl)-1H-1,2,3-triazol-1-yl)methyl)-2-(hydroxymethyl)-5-methoxy-4-(4-(2,3,4-trifluorophenyl)-1H-1,2,3-triazol-1-yl)tetrahydro-2H-pyran-3-ol;
   (2R,3R,4S,5R,6R)-2-(hydroxymethyl)-6-((4-(1-(hydroxymethyl)cyclopropyl)-1H-1,2,3-triazol-1-yl)methyl)-5-methoxy-4-(4-(2,3,4-trifluorophenyl)-1H-1,2,3-triazol-1-yl)tetrahydro-2H-pyran-3-ol;
   (2R,3R,4S,5R,6R)-6-((4-(3-ethyloxetan-3-yl)-1H-1,2,3-triazol-1-yl)methyl)-2-(hydroxymethyl)-5-methoxy-4-(4-(2,3,4-trifluorophenyl)-1H-1,2,3-triazol-1-yl)tetrahydro-2H-pyran-3-ol;
   tert-butyl (4-(1-(((2R,3R,4S,5R,6R)-5-hydroxy-6-(hydroxymethyl)-3-methoxy-4-(4-(2,3,4-trifluorophenyl)-1H-1,2,3-triazol-1-yl)tetrahydro-2H-pyran-2-yl)methyl)-1H-1,2,3-triazol-4-yl)bicyclo[2.2.2]octan-1-yl)carbamate; and
   (2R,3R,4S,5R,6R)-6-((1-(4-aminobicyclo[2.2.2]octan-1-yl)-1H-1,2,3-triazol-4-yl)methyl)-2-(hydroxymethyl)-5-methoxy-4-(4-(2,3,4-trifluorophenyl)-1H-1,2,3-triazol-1-yl)tetrahydro-2H-pyran-3-ol; as well as
   (2R,3R,4S,5R,6R)-6-((4-(bicyclo[1.1.1]pentan-1-yl)-1H-1,2,3-triazol-1-yl)methyl)-4-(4-(4-chloro-2,3-difluorophenyl)-1H-1,2,3-triazol-1-yl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-2-(hydroxymethyl)-5-methoxy-6-((4-(1-methylcyclopropyl)-1H-1,2,3-triazol-1-yl)methyl)tetrahydro-2H-pyran-3-ol; and
   (2R,3R,4S,5R,6R)-4-(4-(4-chloro-2,3-difluorophenyl)-1H-1,2,3-triazol-1-yl)-2-(hydroxymethyl)-5-methoxy-6-((4-(1-methylcyclobutyl)-1H-1,2,3-triazol-1-yl)methyl)tetrahydro-2H-pyran-3-ol.
23) In addition to the compounds listed in embodiments 21) and 22), further compounds of Formula (I) according to embodiment 1) are selected from:
   (2R,3R,4S,5R,6R)-4-(4-(4-chloro-2,3-difluorophenyl)-1H-1,2,3-triazol-1-yl)-6-((1-(1-(fluoromethyl)cyclobutyl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-2-(hydroxymethyl)-5-methoxy-6-((1-(3-(trifluoromethyl)cyclobutyl)-1H-1,2,3-triazol-4-yl)methyl)tetrahydro-2H-pyran-3-ol;
   (2R,3R,4S,5R,6R)-4-(4-(4-chloro-2,3-difluorophenyl)-1H-1,2,3-triazol-1-yl)-2-(hydroxymethyl)-5-methoxy-6-((1-(3-methyltetrahydrofuran-3-yl)-1H-1,2,3-triazol-4-yl)methyl)tetrahydro-2H-pyran-3-ol;
   (2R,3R,4S,5R,6R)-4-(4-(4-chloro-2,3-difluorophenyl)-1H-1,2,3-triazol-1-yl)-6-((1-(1-(difluoromethyl)cyclobutyl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-6-((1-(1-(fluoromethyl)cyclopropyl)-1H-1,2,3-triazol-4-yl)methyl)-2-(hydroxymethyl)-5-methoxytetrahydro-2H-pyran-3-ol;
   (2R,3R,4S,5R,6R)-2-(hydroxymethyl)-5-methoxy-6-((1-(spiro[2.3]hexan-5-yl)-1H-1,2,3-triazol-4-yl)methyl)-4-(4-(2,3,4-trifluorophenyl)-1H-1,2,3-triazol-1-yl)tetrahydro-2H-pyran-3-ol;
   (2R,3R,4S,5R,6R)-2-(hydroxymethyl)-5-methoxy-6-((1-(3-methylbicyclo[1.1.1]pentan-1-yl)-1H-1,2,3-triazol-4-yl)methyl)-4-(4-(2,3,4-trifluorophenyl)-1H-1,2,3-triazol-1-yl)tetrahydro-2H-pyran-3-ol;
   (2R,3R,4S,5R,6R)-2-(hydroxymethyl)-5-methoxy-6-((1-(1-methylcyclobutyl)-1H-1,2,3-triazol-4-yl)methyl)-4-(4-(2,3,4-trifluorophenyl)-1H-1,2,3-triazol-1-yl)tetrahydro-2H-pyran-3-ol;
   (2R,3R,4S,5R,6R)-2-(hydroxymethyl)-5-methoxy-6-((1-(spiro[3.3]heptan-1-yl)-1H-1,2,3-triazol-4-yl)methyl)-4-(4-(2,3,4-trifluorophenyl)-1H-1,2,3-triazol-1-yl)tetrahydro-2H-pyran-3-ol;
   (2R,3R,4S,5R,6R)-6-((1-(1-(difluoromethyl)cyclopropyl)-1H-1,2,3-triazol-4-yl)methyl)-2-(hydroxymethyl)-5-methoxy-4-(4-(2,3,4-trifluorophenyl)-1H-1,2,3-triazol-1-yl)tetrahydro-2H-pyran-3-ol;
   (2R,3R,4S,5R,6R)-4-(4-(4-bromo-2,3-difluorophenyl)-1H-1,2,3-triazol-1-yl)-6-((1-(3,3-difluoro-1-methylcyclobutyl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-bromo-2,3-difluorophenyl)-1H-1,2,3-triazol-1-yl)-6-((1-((1S,3S)-3-hydroxy-1-methylcyclobutyl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-bromo-2,3-difluorophenyl)-1H-1,2,3-triazol-1-yl)-6-((1-(3-(2-hydroxyethyl)oxetan-3-yl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-bromo-2,3-difluorophenyl)-1H-1,2,3-triazol-1-yl)-2-(hydroxymethyl)-5-methoxy-6-((1-(1-methylcyclopropyl)-1H-1,2,3-triazol-4-yl)methyl)tetrahydro-2H-pyran-3-ol;
   (2R,3R,4S,5R,6R)-4-(4-(4-bromo-2,3-difluorophenyl)-1H-1,2,3-triazol-1-yl)-2-(hydroxymethyl)-5-methoxy-6-((1-(3-(trifluoromethyl)bicyclo[1.1.1]pentan-1-yl)-1H-1,2,3-triazol-4-yl)methyl)tetrahydro-2H-pyran-3-ol;
   (2R,3R,4S,5R,6R)-4-(4-(4-bromo-2,3-difluorophenyl)-1H-1,2,3-triazol-1-yl)-2-(hydroxymethyl)-5-methoxy-6-((1-(3-methoxybicyclo[1.1.1]pentan-1-yl)-1H-1,2,3-triazol-4-yl)methyl)tetrahydro-2H-pyran-3-ol;
   (2R,3R,4S,5R,6R)-4-(4-(4-chloro-2,3-difluorophenyl)-1H-1,2,3-triazol-1-yl)-6-((1-((3R,4R)-4-fluorotetrahydro-2H-pyran-3-yl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-6-((1-(3-(fluoromethyl)oxetan-3-yl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-6-((1-(3-(difluoromethyl)oxetan-3-yl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-2-(hydroxymethyl)-5-methoxy-6-((1-(3-(trifluoromethyl)oxetan-3-yl)-1H-1,2,3-triazol-4-yl)methyl)tetrahydro-2H-pyran-3-ol;
   (2R,3R,4S,5R,6R)-4-(4-(4-chloro-2,3-difluorophenyl)-1H-1,2,3-triazol-1-yl)-6-((1-((R)-2,2-difluorocyclobutyl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-6-((1-((S)-2,2-difluorocyclobutyl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-6-((1-((R)-2,2-difluorocyclopentyl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-6-((1-((S)-2,2-difluorocyclopentyl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-6-((1-((3S,4R)-3-fluorotetrahydro-2H-pyran-4-yl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-6-((1-((3R,4S)-3-fluorotetrahydro-2H-pyran-4-yl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-2-(hydroxymethyl)-5-methoxy-6-((1-(4-(trifluoromethyl)tetrahydro-2H-pyran-4-yl)-1H-1,2,3-triazol-4-yl)methyl)tetrahydro-2H-pyran-3-ol;
   (2R,3R,4S,5R,6R)-4-(4-(4-chloro-2,3-difluorophenyl)-1H-1,2,3-triazol-1-yl)-6-((1-((3R,4R)-4-fluorotetrahydrofuran-3-yl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-6-((1-((3S,4S)-4-fluorotetrahydrofuran-3-yl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-6-((1-((3R,4S)-4-fluorotetrahydrofuran-3-yl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-6-((1-((3S,4R)-4-fluorotetrahydrofuran-3-yl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-6-((1-(4,4-difluorospiro[2.2]pentan-1-yl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-2-(hydroxymethyl)-5-methoxy-6-((1-((R)-3-(trifluoromethyl)tetrahydrofuran-3-yl)-1H-1,2,3-triazol-4-yl)methyl)tetrahydro-2H-pyran-3-ol;
   (2R,3R,4S,5R,6R)-4-(4-(4-chloro-2,3-difluorophenyl)-1H-1,2,3-triazol-1-yl)-2-(hydroxymethyl)-5-methoxy-6-((1-((S)-3-(trifluoromethyl)tetrahydrofuran-3-yl)-1H-1,2,3-triazol-4-yl)methyl)tetrahydro-2H-pyran-3-ol;
   (2R,3R,4S,5R,6R)-4-(4-(4-chloro-2,3-difluorophenyl)-1H-1,2,3-triazol-1-yl)-6-((1-((R)-3-(difluoromethyl)tetrahydrofuran-3-yl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-6-((1-((S)-3-(difluoromethyl)tetrahydrofuran-3-yl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-6-((1-((1R,2S)-2-fluorocyclopentyl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-6-((1-((1S,2R)-2-fluorocyclopentyl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-6-((1-((1R,2R)-2-fluorocyclopentyl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-6-((1-((1S,2S)-2-fluorocyclopentyl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-6-((1-((R)-2,2-difluorocyclohexyl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-6-((1-((S)-2,2-difluorocyclohexyl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-6-((1-((1S,2R)-2-fluorocyclohexyl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-6-((1-((1R,2R)-2-fluorocyclohexyl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-6-((1-((1S,2S)-2-fluorocyclohexyl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-6-((1-((1R,2S)-2-fluorocyclobutyl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-6-((1-((1S,2R)-2-fluorocyclobutyl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-6-((1-((1R,2R)-2-fluorocyclobutyl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-6-((1-((1S,2S)-2-fluorocyclobutyl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-6-((1-((R)-3,3-difluorotetrahydro-2H-pyran-4-yl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-6-((1-((S)-3,3-difluorotetrahydro-2H-pyran-4-yl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-6-((1-((R)-4,4-difluorotetrahydrofuran-3-yl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-6-((1-((S)-4,4-difluorotetrahydrofuran-3-yl)-1H-1,2,3-triazol-4-yl)methyl)-2-(hydroxymethyl)-5-methoxytetrahydro-2H-pyran-3-ol;
   (2R,3R,4S,5R,6R)-2-(hydroxymethyl)-5-methoxy-6-((1-(3-methyloxetan-3-yl)-1H-1,2,3-triazol-4-yl)methyl)-4-(4-(2,3,4-trifluorophenyl)-1H-1,2,3-triazol-1-yl)tetrahydro-2H-pyran-3-ol;
   (2R,3R,4S,5R,6R)-6-((1-(1-(fluoromethyl)cyclopropyl)-1H-1,2,3-triazol-4-yl)methyl)-2-(hydroxymethyl)-5-methoxy-4-(4-(2,3,4-trifluorophenyl)-1H-1,2,3-triazol-1-yl)tetrahydro-2H-pyran-3-ol;
   (2R,3R,4S,5R,6R)-6-((1-(1-(difluoromethyl)cyclobutyl)-1H-1,2,3-triazol-4-yl)methyl)-2-(hydroxymethyl)-5-methoxy-4-(4-(2,3,4-trifluorophenyl)-1H-1,2,3-triazol-1-yl)tetrahydro-2H-pyran-3-ol;
   (2R,3R,4S,5R,6R)-6-((1-(1,1-difluoro-2-methylpropan-2-yl)-1H-1,2,3-triazol-4-yl)methyl)-2-(hydroxymethyl)-5-methoxy-4-(4-(2,3,4-trifluorophenyl)-1H-1,2,3-triazol-1-yl)tetrahydro-2H-pyran-3-ol;
   (2R,3R,4S,5R,6R)-6-((1-(1-(fluoromethyl)cyclobutyl)-1H-1,2,3-triazol-4-yl)methyl)-2-(hydroxymethyl)-5-methoxy-4-(4-(2,3,4-trifluorophenyl)-1H-1,2,3-triazol-1-yl)tetrahydro-2H-pyran-3-ol;
   (2R,3R,4S,5R,6R)-2-(hydroxymethyl)-5-methoxy-6-((1-(1-(trifluoromethyl)cyclobutyl)-1H-1,2,3-triazol-4-yl)methyl)-4-(4-(2,3,4-trifluorophenyl)-1H-1,2,3-triazol-1-yl)tetrahydro-2H-pyran-3-ol;
   (2R,3R,4S,5R,6R)-2-(hydroxymethyl)-5-methoxy-6-((1-(3-(trifluoromethyl)oxetan-3-yl)-1H-1,2,3-triazol-4-yl)methyl)-4-(4-(2,3,4-trifluorophenyl)-1H-1,2,3-triazol-1-yl)tetrahydro-2H-pyran-3-ol;
   (2R,3R,4S,5R,6R)-6-((1-((3S,4R)-3-fluorotetrahydro-2H-pyran-4-yl)-1H-1,2,3-triazol-4-yl)methyl)-2-(hydroxymethyl)-5-methoxy-4-(4-(2,3,4-trifluorophenyl)-1H-1,2,3-triazol-1-yl)tetrahydro-2H-pyran-3-ol;
   (2R,3R,4S,5R,6R)-6-((1-((3R,4S)-3-fluorotetrahydro-2H-pyran-4-yl)-1H-1,2,3-triazol-4-yl)methyl)-2-(hydroxymethyl)-5-methoxy-4-(4-(2,3,4-trifluorophenyl)-1H-1,2,3-triazol-1-yl)tetrahydro-2H-pyran-3-ol;
   (2R,3R,4S,5R,6R)-4-(4-(4-bromo-2,3-difluorophenyl)-1H-1,2,3-triazol-1-yl)-6-((1-(1-(fluoromethyl)cyclopropyl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-bromo-2,3-difluorophenyl)-1H-1,2,3-triazol-1-yl)-6-((1-(3-(fluoromethyl)oxetan-3-yl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-bromo-2,3-difluorophenyl)-1H-1,2,3-triazol-1-yl)-6-((1-(3-fluorobicyclo[1.1.1]pentan-1-yl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-bromo-2,3-difluorophenyl)-1H-1,2,3-triazol-1-yl)-2-(hydroxymethyl)-5-methoxy-6-((1-(1-(trifluoromethyl)cyclobutyl)-1H-1,2,3-triazol-4-yl)methyl)tetrahydro-2H-pyran-3-ol;
   (2R,3R,4S,5R,6R)-4-(4-(4-bromo-2,3-difluorophenyl)-1H-1,2,3-triazol-1-yl)-6-((1-(1-(fluoromethyl)cyclobutyl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-bromo-2,3-difluorophenyl)-1H-1,2,3-triazol-1-yl)-6-((1-((R)-2,2-difluorocyclopentyl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-bromo-2,3-difluorophenyl)-1H-1,2,3-triazol-1-yl)-6-((1-((S)-2,2-difluorocyclopentyl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-bromo-2,3-difluorophenyl)-1H-1,2,3-triazol-1-yl)-2-(hydroxymethyl)-5-methoxy-6-((1-(3-methylbicyclo[1.1.1]pentan-1-yl)-1H-1,2,3-triazol-4-yl)methyl)tetrahydro-2H-pyran-3-ol;
   (2R,3R,4S,5R,6R)-4-(4-(4-bromo-2,3-difluorophenyl)-1H-1,2,3-triazol-1-yl)-6-((1-((1S,2S)-2-fluorocyclopentyl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-bromo-2,3-difluorophenyl)-1H-1,2,3-triazol-1-yl)-6-((1-((1R,2R)-2-fluorocyclopentyl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-bromo-2,3-difluorophenyl)-1H-1,2,3-triazol-1-yl)-6-((1-(2,2-difluorocyclobutyl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-bromo-2,3-difluorophenyl)-1H-1,2,3-triazol-1-yl)-6-((1-((1R,2S)-2-fluorocyclobutyl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-bromo-2,3-difluorophenyl)-1H-1,2,3-triazol-1-yl)-6-((1-((1S,2R)-2-fluorocyclobutyl)-1H-1,2,3-triazol-4-yl)methyl)-2-(hydroxymethyl)-5-methoxytetrahydro-2H-pyran-3-ol;
   (2R,3R,4S,5R,6R)-4-(4-(2,3-difluoro-4-methylphenyl)-1H-1,2,3-triazol-1-yl)-6-((1-(3-(difluoromethyl)oxetan-3-yl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-6-((1-(1-fluoro-2-methylpropan-2-yl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-2-(hydroxymethyl)-5-methoxy-6-((1-(1-(trifluoromethyl)cyclopropyl)-1H-1,2,3-triazol-4-yl)methyl)tetrahydro-2H-pyran-3-ol;
   (2R,3R,4S,5R,6R)-4-(4-(4-chloro-2,3-difluorophenyl)-1H-1,2,3-triazol-1-yl)-2-(hydroxymethyl)-5-methoxy-6-((1-(1-(trifluoromethyl)cyclobutyl)-1H-1,2,3-triazol-4-yl)methyl)tetrahydro-2H-pyran-3-ol;
   (2R,3R,4S,5R,6R)-4-(4-(4-chloro-2,3-difluorophenyl)-1H-1,2,3-triazol-1-yl)-2-(hydroxymethyl)-5-methoxy-6-((1-(1,1,1-trifluoro-2-methylpropan-2-yl)-1H-1,2,3-triazol-4-yl)methyl)tetrahydro-2H-pyran-3-ol;
   (2R,3R,4S,5R,6R)-4-(4-(4-chloro-2,3-difluorophenyl)-1H-1,2,3-triazol-1-yl)-2-(hydroxymethyl)-5-methoxy-6-((1-(1-(trifluoromethyl)cyclopentyl)-1H-1,2,3-triazol-4-yl)methyl)tetrahydro-2H-pyran-3-ol;
   (2R,3R,4S,5R,6R)-4-(4-(4-chloro-2,3-difluorophenyl)-1H-1,2,3-triazol-1-yl)-2-(hydroxymethyl)-5-methoxy-6-((1-((1R,3R,5S)-3-(trifluoromethyl)bicyclo[3.1.0]hexan-3-yl)-1H-1,2,3-triazol-4-yl)methyl)tetrahydro-2H-pyran-3-ol;
   (2R,3R,4S,5R,6R)-4-(4-(4-chloro-2,3-difluorophenyl)-1H-1,2,3-triazol-1-yl)-2-(hydroxymethyl)-5-methoxy-6-((1-(2-(trifluoromethyl)bicyclo[2.2.1]heptan-2-yl)-1H-1,2,3-triazol-4-yl)methyl)tetrahydro-2H-pyran-3-ol;
   (2R,3R,4S,5R,6R)-4-(4-(4-chloro-2,3-difluorophenyl)-1H-1,2,3-triazol-1-yl)-6-((1-((3R,4R)-3-fluorotetrahydro-2H-pyran-4-yl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-6-((1-((3S,4S)-3-fluorotetrahydro-2H-pyran-4-yl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-6-((1-((R)-4,4-difluorotetrahydro-2H-pyran-3-yl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-6-((1-((S)-4,4-difluorotetrahydro-2H-pyran-3-yl)-1H-1,2,3-triazol-4-yl)methyl)-2-(hydroxymethyl)-5-methoxytetrahydro-2H-pyran-3-ol;
   (2R,3R,4S,5R,6R)-2-(hydroxymethyl)-5-methoxy-6-((1-(3-methoxybicyclo[1.1.1]pentan-1-yl)-1H-1,2,3-triazol-4-yl)methyl)-4-(4-(2,3,4-trifluorophenyl)-1H-1,2,3-triazol-1-yl)tetrahydro-2H-pyran-3-ol;
   (2R,3R,4S,5R,6R)-6-((1-(3-(fluoromethyl)oxetan-3-yl)-1H-1,2,3-triazol-4-yl)methyl)-2-(hydroxymethyl)-5-methoxy-4-(4-(2,3,4-trifluorophenyl)-1H-1,2,3-triazol-1-yl)tetrahydro-2H-pyran-3-ol;
   (2R,3R,4S,5R,6R)-6-((1-(3-(difluoromethyl)oxetan-3-yl)-1H-1,2,3-triazol-4-yl)methyl)-2-(hydroxymethyl)-5-methoxy-4-(4-(2,3,4-trifluorophenyl)-1H-1,2,3-triazol-1-yl)tetrahydro-2H-pyran-3-ol;
   (2R,3R,4S,5R,6R)-4-(4-(4-bromo-2,3-difluorophenyl)-1H-1,2,3-triazol-1-yl)-6-((1-(1,1-difluoro-2-methylpropan-2-yl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-bromo-2,3-difluorophenyl)-1H-1,2,3-triazol-1-yl)-6-((1-(1-(difluoromethyl)cyclobutyl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-bromo-2,3-difluorophenyl)-1H-1,2,3-triazol-1-yl)-6-((1-(3-(difluoromethyl)oxetan-3-yl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-bromo-2,3-difluorophenyl)-1H-1,2,3-triazol-1-yl)-2-(hydroxymethyl)-5-methoxy-6-((1-(3-(trifluoromethyl)oxetan-3-yl)-1H-1,2,3-triazol-4-yl)methyl)tetrahydro-2H-pyran-3-ol;
   (2R,3R,4S,5R,6R)-4-(4-(2,3-difluoro-4-methylphenyl)-1H-1,2,3-triazol-1-yl)-2-(hydroxymethyl)-5-methoxy-6-((1-(3-(trifluoromethyl)oxetan-3-yl)-1H-1,2,3-triazol-4-yl)methyl)tetrahydro-2H-pyran-3-ol;
   (2R,3R,4S,5R,6R)-4-(4-(4-chloro-2,3-difluorophenyl)-1H-1,2,3-triazol-1-yl)-6-((1-(1-(difluoromethyl)cyclopropyl)-1H-1,2,3-triazol-4-yl)methyl)-2-(hydroxymethyl)-5-methoxytetrahydro-2H-pyran-3-ol;
   (2R,3R,4S,5R,6R)-2-(hydroxymethyl)-5-methoxy-6-((1-(1-(trifluoromethyl)cyclopropyl)-1H-1,2,3-triazol-4-yl)methyl)-4-(4-(2,3,4-trifluorophenyl)-1H-1,2,3-triazol-1-yl)tetrahydro-2H-pyran-3-ol;
   (2R,3R,4S,5R,6R)-4-(4-(4-bromo-2,3-difluorophenyl)-1H-1,2,3-triazol-1-yl)-6-((1-(1-(difluoromethyl)cyclopropyl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-bromo-2,3-difluorophenyl)-1H-1,2,3-triazol-1-yl)-2-(hydroxymethyl)-5-methoxy-6-((1-(1-(trifluoromethyl)cyclopropyl)-1H-1,2,3-triazol-4-yl)methyl)tetrahydro-2H-pyran-3-ol;
   (2R,3R,4S,5R,6R)-4-(4-(4-chloro-2,3-difluorophenyl)-1H-1,2,3-triazol-1-yl)-2-(hydroxymethyl)-5-methoxy-6-((1-(tert-pentyl)-1H-1,2,3-triazol-4-yl)methyl)tetrahydro-2H-pyran-3-ol;
   (2R,3R,4S,5R,6R)-4-(4-(4-chloro-2,3-difluorophenyl)-1H-1,2,3-triazol-1-yl)-2-(hydroxymethyl)-5-methoxy-6-((1-(3-methylpentan-3-yl)-1H-1,2,3-triazol-4-yl)methyl)tetrahydro-2H-pyran-3-ol;
   4-(1-((2R,3R,4S,5R,6R)-2-((1-(3-(difluoromethyl)oxetan-3-yl)-1H-1,2,3-triazol-4-yl)methyl)-5-hydroxy-6-(hydroxymethyl)-3-methoxytetrahydro-2H-pyran-4-yl)-1H-1,2,3-triazol-4-yl)-2,3-difluorobenzonitrile;
   2,3-difluoro-4-(1-((2R,3R,4S,5R,6R)-3-hydroxy-2-(hydroxymethyl)-5-methoxy-6-((1-(3-(trifluoromethyl)oxetan-3-yl)-1H-1,2,3-triazol-4-yl)methyl)tetrahydro-2H-pyran-4-yl)-1H-1,2,3-triazol-4-yl)benzonitrile;
   2,3-difluoro-4-(1-((2R,3R,4S,5R,6R)-3-hydroxy-2-(hydroxymethyl)-5-methoxy-6-((1-(3-methyloxetan-3-yl)-1H-1,2,3-triazol-4-yl)methyl)tetrahydro-2H-pyran-4-yl)-1H-1,2,3-triazol-4-yl)benzonitrile;
   4-(1-((2R,3R,4S,5R,6R)-2-((1-(1-(difluoromethyl)cyclobutyl)-1H-1,2,3-triazol-4-yl)methyl)-5-hydroxy-6-(hydroxymethyl)-3-methoxytetrahydro-2H-pyran-4-yl)-1H-1,2,3-triazol-4-yl)-2,3-difluorobenzonitrile;
   2,3-difluoro-4-(1-((2R,3R,4S,5R,6R)-2-((1-((3R,4S)-3-fluorotetrahydro-2H-pyran-4-yl)-1H-1,2,3-triazol-4-yl)methyl)-5-hydroxy-6-(hydroxymethyl)-3-methoxytetrahydro-2H-pyran-4-yl)-1H-1,2,3-triazol-4-yl)benzonitrile;
   2,3-difluoro-4-(1-((2R,3R,4S,5R,6R)-2-((1-((3S,4R)-3-fluorotetrahydro-2H-pyran-4-yl)-1H-1,2,3-triazol-4-yl)methyl)-5-hydroxy-6-(hydroxymethyl)-3-methoxytetrahydro-2H-pyran-4-yl)-1H-1,2,3-triazol-4-yl)benzonitrile;
   (2R,3R,4S,5R,6R)-4-(4-(4-chloro-2,3-difluorophenyl)-1H-1,2,3-triazol-1-yl)-6-((4-(3,3-difluorocyclobutyl)-1H-1,2,3-triazol-1-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-6-((4-(6,6-difluorospiro[3.3]heptan-2-yl)-1H-1,2,3-triazol-1-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-6-((4-(1-(difluoromethyl)cyclopropyl)-1H-1,2,3-triazol-1-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-6-((4-(1-(fluoromethyl)cyclopropyl)-1H-1,2,3-triazol-1-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-2-(hydroxymethyl)-5-methoxy-6-((4-(1-(trifluoromethyl)cyclopropyl)-1H-1,2,3-triazol-1-yl)methyl)tetrahydro-2H-pyran-3-ol;
   tert-butyl ((1S,3S)-3-(1-(((2R,3R,4S,5R,6R)-4-(4-(4-chloro-2,3-difluorophenyl)-1H-1,2,3-triazol-1-yl)-5-hydroxy-6-(hydroxymethyl)-3-methoxytetrahydro-2H-pyran-2-yl)methyl)-1H-1,2,3-triazol-4-yl)cyclobutyl)carbamate;
   (2R,3R,4S,5R,6R)-6-((4-((1S,3S)-3-aminocyclobutyl)-1H-1,2,3-triazol-1-yl)methyl)-4-(4-(4-chloro-2,3-difluorophenyl)-1H-1,2,3-triazol-1-yl)-2-(hydroxymethyl)-5-methoxytetrahydro-2H-pyran-3-ol;
   (2R,3R,4S,5R,6R)-2-(hydroxymethyl)-5-methoxy-6-((4-(1-methylcyclopropyl)-1H-1,2,3-triazol-1-yl)methyl)-4-(4-(2,3,4-trifluorophenyl)-1H-1,2,3-triazol-1-yl)tetrahydro-2H-pyran-3-ol;
   (2R,3R,4S,5R,6R)-2-(hydroxymethyl)-5-methoxy-6-((4-(1-methylcyclobutyl)-1H-1,2,3-triazol-1-yl)methyl)-4-(4-(2,3,4-trifluorophenyl)-1H-1,2,3-triazol-1-yl)tetrahydro-2H-pyran-3-ol;
   (2R,3R,4S,5R,6R)-6-((4-(bicyclo[1.1.1]pentan-1-yl)-1H-1,2,3-triazol-1-yl)methyl)-2-(hydroxymethyl)-5-methoxy-4-(4-(2,3,4-trifluorophenyl)-1H-1,2,3-triazol-1-yl)tetrahydro-2H-pyran-3-ol;
   (2R,3R,4S,5R,6R)-4-(4-(4-bromo-2,3-difluorophenyl)-1H-1,2,3-triazol-1-yl)-6-((4-(1-hydroxycyclobutyl)-1H-1,2,3-triazol-1-yl)methyl)-2-(hydroxymethyl)-5-methoxytetrahydro-2H-pyran-3-ol;
   (2R,3R,4S,5R,6R)-4-(4-(4-bromo-2,3-difluorophenyl)-1H-1,2,3-triazol-1-yl)-6-((4-cyclopentyl-1H-1,2,3-triazol-1-yl)methyl)-2-(hydroxymethyl)-5-methoxytetrahydro-2H-pyran-3-ol;
   (2R,3R,4S,5R,6R)-4-(4-(4-bromo-2,3-difluorophenyl)-1H-1,2,3-triazol-1-yl)-6-((4-(1-hydroxycyclopentyl)-1H-1,2,3-triazol-1-yl)methyl)-2-(hydroxymethyl)-5-methoxytetrahydro-2H-pyran-3-ol;
   (2R,3R,4S,5R,6R)-4-(4-(4-bromo-2,3-difluorophenyl)-1H-1,2,3-triazol-1-yl)-2-(hydroxymethyl)-6-((4-(2-hydroxypropan-2-yl)-1H-1,2,3-triazol-1-yl)methyl)-5-methoxytetrahydro-2H-pyran-3-ol;
   (2R,3R,4S,5R,6R)-4-(4-(4-bromo-2,3-difluorophenyl)-1H-1,2,3-triazol-1-yl)-2-(hydroxymethyl)-5-methoxy-6-((4-(3-methyloxetan-3-yl)-1H-1,2,3-triazol-1-yl)methyl)tetrahydro-2H-pyran-3-ol;
   (2R,3R,4S,5R,6R)-4-(4-(4-bromo-2,3-difluorophenyl)-1H-1,2,3-triazol-1-yl)-2-(hydroxymethyl)-6-((4-(4-hydroxytetrahydro-2H-pyran-4-yl)-1H-1,2,3-triazol-1-yl)methyl)-5-methoxytetrahydro-2H-pyran-3-ol;
   (2R,3R,4S,5R,6R)-4-(4-(4-bromo-2,3-difluorophenyl)-1H-1,2,3-triazol-1-yl)-6-((4-(1-hydroxycyclopropyl)-1H-1,2,3-triazol-1-yl)methyl)-2-(hydroxymethyl)-5-methoxytetrahydro-2H-pyran-3-ol;
   (2R,3R,4S,5R,6R)-4-(4-(4-bromo-2,3-difluorophenyl)-1H-1,2,3-triazol-1-yl)-2-(hydroxymethyl)-6-((4-(1-(hydroxymethyl)cyclopropyl)-1H-1,2,3-triazol-1-yl)methyl)-5-methoxytetrahydro-2H-pyran-3-ol;
   (2R,3R,4S,5R,6R)-4-(4-(4-bromo-2,3-difluorophenyl)-1H-1,2,3-triazol-1-yl)-6-((4-(3-ethyloxetan-3-yl)-1H-1,2,3-triazol-1-yl)methyl)-2-(hydroxymethyl)-5-methoxytetrahydro-2H-pyran-3-ol;
   tert-butyl (4-(1-(((2R,3R,4S,5R,6R)-4-(4-(4-bromo-2,3-difluorophenyl)-1H-1,2,3-triazol-1-yl)-5-hydroxy-6-(hydroxymethyl)-3-methoxytetrahydro-2H-pyran-2-yl)methyl)-1H-1,2,3-triazol-4-yl)bicyclo[2.2.2]octan-1-yl)carbamate; (2R,3R,4S,5R,6R)-6-((4-(4-aminobicyclo[2.2.2]octan-1-yl)-1H-1,2,3-triazol-1-yl)methyl)-4-(4-(4-bromo-2,3-difluorophenyl)-1H-1,2,3-triazol-1-yl)-2-(hydroxymethyl)-5-methoxytetrahydro-2H-pyran-3-ol;
   (2R,3R,4S,5R,6R)-4-(4-(4-bromo-2,3-difluorophenyl)-1H-1,2,3-triazol-1-yl)-2-(hydroxymethyl)-5-methoxy-6-((4-(1-methylcyclopropyl)-1H-1,2,3-triazol-1-yl)methyl)tetrahydro-2H-pyran-3-ol;
   (2R,3R,4S,5R,6R)-4-(4-(4-bromo-2,3-difluorophenyl)-1H-1,2,3-triazol-1-yl)-2-(hydroxymethyl)-5-methoxy-6-((4-(1-methylcyclobutyl)-1H-1,2,3-triazol-1-yl)methyl)tetrahydro-2H-pyran-3-ol; and
   (2R,3R,4S,5R,6R)-6-((4-(bicyclo[1.1.1]pentan-1-yl)-1H-1,2,3-triazol-1-yl)methyl)-4-(4-(4-bromo-2,3-difluorophenyl)-1H-1,2,3-triazol-1-yl)-2-(hydroxymethyl)-5-methoxytetrahydro-2H-pyran-3-ol.
24) In addition to the compounds listed in embodiments 21), 22), and 23), further compounds of Formula (I) according to embodiment 1) are selected from:
   (2R,3R,4S,5R,6R)-4-(4-(4-chloro-2,3-difluorophenyl)-1H-1,2,3-triazol-1-yl)-6-((1-((2R,3R)-2,3-dimethyltetrahydrofuran-3-yl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-6-((1-((2S,3R)-2,3-dimethyltetrahydrofuran-3-yl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-6-((1-(4,4-difluoro-1-(trifluoromethyl)cyclohexyl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-6-((1-(2,2-difluoro-1-(fluoromethyl)cyclopropyl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-6-((1-((1S,3S)-3-fluoro-1-methylcyclobutyl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-6-((1-((1R,3R)-3-fluoro-1-methylcyclobutyl)-1H-1,2,3-triazol-4-yl)methyl)-2-(hydroxymethyl)-5-methoxytetrahydro-2H-pyran-3-ol;
   (2R,3R,4S,5R,6R)-6-((1-(3,3-bis(trifluoromethyl)cyclobutyl)-1H-1,2,3-triazol-4-yl)methyl)-4-(4-(4-chloro-2,3-difluorophenyl)-1H-1,2,3-triazol-1-yl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-6-((1-((1R,3R)-3-fluorocyclobutyl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-6-((1-((1S,3S)-3-fluorocyclobutyl)-1H-1,2,3-triazol-4-yl)methyl)-2-(hydroxymethyl)-5-methoxytetrahydro-2H-pyran-3-ol; and
   (2R,3R,4S,5R,6R)-4-(4-(4-chloro-2,3-difluorophenyl)-1H-1,2,3-triazol-1-yl)-6-((4-(3,3-difluoro-1-hydroxycyclobutyl)-1H-1,2,3-triazol-1-yl)methyl)-2-(hydroxymethyl)-5-methoxytetrahydro-2H-pyran-3-ol.
25) In addition to the compounds listed in embodiments 21), 22), 23), and 24), further compounds of Formula (I) according to embodiment 1) are selected from:
   (2R,3R,4S,5R,6R)-4-(4-(4-chloro-2,3-difluorophenyl)-1H-1,2,3-triazol-1-yl)-6-((1-(3-(fluoromethyl)oxetan-3-yl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-6-((1-(3-(difluoromethyl)oxetan-3-yl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-2-(hydroxymethyl)-5-methoxy-6-((1-(3-(trifluoromethyl)oxetan-3-yl)-1H-1,2,3-triazol-4-yl)methyl)tetrahydro-2H-pyran-3-ol;
   (2R,3R,4S,5R,6R)-4-(4-(4-chloro-2,3-difluorophenyl)-1H-1,2,3-triazol-1-yl)-6-((1-((3R,4S)-3-fluorotetrahydro-2H-pyran-4-yl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-6-((1-((3R,4R)-4-fluorotetrahydrofuran-3-yl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-6-((1-((3S,4S)-4-fluorotetrahydrofuran-3-yl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-6-((1-((R)-3,3-difluorotetrahydro-2H-pyran-4-yl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-6-((1-((S)-3,3-difluorotetrahydro-2H-pyran-4-yl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-6-((1-((R)-4,4-difluorotetrahydrofuran-3-yl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-6-((1-((S)-4,4-difluorotetrahydrofuran-3-yl)-1H-1,2,3-triazol-4-yl)methyl)-2-(hydroxymethyl)-5-methoxytetrahydro-2H-pyran-3-ol;
   (2R,3R,4S,5R,6R)-6-((1-((3S,4R)-3-fluorotetrahydro-2H-pyran-4-yl)-1H-1,2,3-triazol-4-yl)methyl)-2-(hydroxymethyl)-5-methoxy-4-(4-(2,3,4-trifluorophenyl)-1H-1,2,3-triazol-1-yl)tetrahydro-2H-pyran-3-ol;
   (2R,3R,4S,5R,6R)-6-((1-((3R,4S)-3-fluorotetrahydro-2H-pyran-4-yl)-1H-1,2,3-triazol-4-yl)methyl)-2-(hydroxymethyl)-5-methoxy-4-(4-(2,3,4-trifluorophenyl)-1H-1,2,3-triazol-1-yl)tetrahydro-2H-pyran-3-ol;
   (2R,3R,4S,5R,6R)-4-(4-(4-chloro-2,3-difluorophenyl)-1H-1,2,3-triazol-1-yl)-6-((1-((3R,4R)-3-fluorotetrahydro-2H-pyran-4-yl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-chloro-2,3-difluorophenyl)-1H-1,2,3-triazol-1-yl)-6-((1-((3S,4S)-3-fluorotetrahydro-2H-pyran-4-yl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-bromo-2,3-difluorophenyl)-1H-1,2,3-triazol-1-yl)-6-((1-(3-(difluoromethyl)oxetan-3-yl)-1H-1,2,3-triazol-4-yl)methyl)-2-(hydroxymethyl)-5-methoxytetrahydro-2H-pyran-3-ol;
   (2R,3R,4S,5R,6R)-4-(4-(4-bromo-2,3-difluorophenyl)-1H-1,2,3-triazol-1-yl)-2-(hydroxymethyl)-5-methoxy-6-((1-(3-(trifluoromethyl)oxetan-3-yl)-1H-1,2,3-triazol-4-yl)methyl)tetrahydro-2H-pyran-3-ol;
   2,3-difluoro-4-(1-((2R,3R,4S,5R,6R)-2-((1-((3R,4S)-3-fluorotetrahydro-2H-pyran-4-yl)-1H-1,2,3-triazol-4-yl)methyl)-5-hydroxy-6-(hydroxymethyl)-3-methoxytetrahydro-2H-pyran-4-yl)-1H-1,2,3-triazol-4-yl)benzonitrile; and
   2,3-difluoro-4-(1-((2R,3R,4S,5R,6R)-2-((1-((3S,4R)-3-fluorotetrahydro-2H-pyran-4-yl)-1H-1,2,3-triazol-4-yl)methyl)-5-hydroxy-6-(hydroxymethyl)-3-methoxytetrahydro-2H-pyran-4-yl)-1H-1,2,3-triazol-4-yl)benzonitrile.
      I) Further disclosed are compounds of Formula (III) 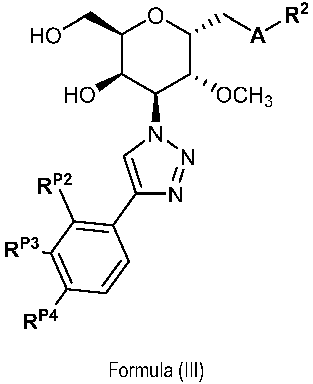 wherein
         **R^{P2}** represents halogen (especially fluoro);
         **R^{P3}** represents halogen (especially fluoro);
         **R^{P4}** represents halogen (especially fluoro, chloro, bromo), methyl or cyano; and
         A represents [1,2,3]triazol-1,4-diyl (wherein it is understood that **R²** may be attached to position 1 or to position 4 of said [1,2,3]triazol-1,4-diyl); and

         - **R²** represents
            ❖ bridged bicyclic 6- or 7-membered heterocycloalkyl containing one ring oxygen atom (especially 2-oxabicyclo[2.1.1]hexan-4-yl, or 2-oxabicyclo[3.1.1]heptan-5-yl);
            wherein said bridged bicyclic 6- or 7-membered heterocycloalkyl containing one ring oxygen atom independently is unsubstituted, or mono-, di-, or tri-substituted wherein the substituents independently are selected from hydroxy; C₁₋₃-alkyl (especially methyl; or ethyl or isopropyl); C₁₋₃-alkoxy (especially methoxy); - C₁₋₃-alkylene-OH (especially hydroxymethyl, or 2-hydroxyethyl); C₁-fluoroalkyl (especially trifluoromethyl); - NR^{N1}R^{N2}, wherein R^{N1} represents hydrogen, and R^{N2} represents hydrogen or -CO-O-C₁₋₄-alkyl (especially -COO-tert.-butyl); and fluoro.
      II) Further disclosure relates to compounds of Formula (III) according to disclosure I), wherein **A** represents [1,2,3]triazol-1,4-diyl wherein **R²** is attached to position 4 of said [1,2,3]triazol-1,4-diyl.
      III) Further disclosure relates to compounds of Formula (III) according to disclosure I) or II), wherein **R²** represents
         ❖ bridged bicyclic 6- or 7-membered heterocycloalkyl containing one ring oxygen atom (especially 2-oxabicyclo[2.1.1]hexan-4-yl, or 2-oxabicyclo[3.1.1]heptan-5-yl), wherein said bridged bicyclic 6- or 7-membered heterocycloalkyl containing one ring oxygen atom is unsubstituted or mono-substituted with C₁₋₃-alkyl (especially methyl).
      IV) Further disclosed are compounds of Formula (III) according to disclosure I), which are selected from the following compounds:
         (2R,3R,4S,5R,6R)-4-(4-(4-chloro-2,3-difluorophenyl)-1H-1,2,3-triazol-1-yl)-2-(hydroxymethyl)-5-methoxy-6-((1-(1-methyl-2-oxabicyclo[3.1.1]heptan-5-yl)-1H-1,2,3-triazol-4-yl)methyl)tetrahydro-2H-pyran-3-ol;
         (2R,3R,4S,5R,6R)-4-(4-(4-chloro-2,3-difluorophenyl)-1H-1,2,3-triazol-1-yl)-2-(hydroxymethyl)-5-methoxy-6-((1-(1-methyl-2-oxabicyclo[2.1.1]hexan-4-yl)-1H-1,2,3-triazol-4-yl)methyl)tetrahydro-2H-pyran-3-ol;
         (2R,3R,4S,5R,6R)-6-((1-(2-oxabicyclo[2.1.1]hexan-4-yl)-1H-1,2,3-triazol-4-yl)methyl)-2-(hydroxymethyl)-5-methoxy-4-(4-(2,3,4-trifluorophenyl)-1H-1,2,3-triazol-1-yl)tetrahydro-2H-pyran-3-ol;
         (2R,3R,4S,5R,6R)-4-(4-(4-bromo-2,3-difluorophenyl)-1H-1,2,3-triazol-1-yl)-2-(hydroxymethyl)-5-methoxy-6-((1-(1-methyl-2-oxabicyclo[2.1.1]hexan-4-yl)-1H-1,2,3-triazol-4-yl)methyl)tetrahydro-2H-pyran-3-ol;
         (2R,3R,4S,5R,6R)-6-((1-(2-oxabicyclo[2.1.1]hexan-4-yl)-1H-1,2,3-triazol-4-yl)methyl)-4-(4-(4-bromo-2,3-difluorophenyl)-1H-1,2,3-triazol-1-yl)-2-(hydroxymethyl)-5-methoxytetrahydro-2H-pyran-3-ol;
         (2R,3R,4S,5R,6R)-2-(hydroxymethyl)-5-methoxy-6-((1-(1-methyl-2-oxabicyclo[2.1.1]hexan-4-yl)-1H-1,2,3-triazol-4-yl)methyl)-4-(4-(2,3,4-trifluorophenyl)-1H-1,2,3-triazol-1-yl)tetrahydro-2H-pyran-3-ol;
         (2R,3R,4S,5R,6R)-4-(4-(4-bromo-2,3-difluorophenyl)-1H-1,2,3-triazol-1-yl)-2-(hydroxymethyl)-5-methoxy-6-((1-(1-methyl-2-oxabicyclo[3.1.1]heptan-5-yl)-1H-1,2,3-triazol-4-yl)methyl)tetrahydro-2H-pyran-3-ol;
         (2R,3R,4S,5R,6R)-2-(hydroxymethyl)-5-methoxy-6-((1-(1-methyl-2-oxabicyclo[3.1.1]heptan-5-yl)-1H-1,2,3-triazol-4-yl)methyl)-4-(4-(2,3,4-trifluorophenyl)-1H-1,2,3-triazol-1-yl)tetrahydro-2H-pyran-3-ol; and
         (2R,3R,4S,5R,6R)-6-((1-(2-oxabicyclo[2.1.1]hexan-4-yl)-1H-1,2,3-triazol-4-yl)methyl)-4-(4-(4-chloro-2,3-difluorophenyl)-1H-1,2,3-triazol-1-yl)-2-(hydroxymethyl)-5-methoxytetrahydro-2H-pyran-3-ol.

The production of the pharmaceutical compositions can be effected in a manner which will be familiar to any person skilled in the art (see for example Remington, The Science and Practice of Pharmacy, 21st Edition (2005), Part 5, "Pharmaceutical Manufacturing" [published by Lippincott Williams & Wilkins]) by bringing the described compounds of Formula (I) or their pharmaceutically acceptable salts, optionally in combination with other therapeutically valuable substances, into a galenical administration form together with suitable, non-toxic, inert, therapeutically compatible solid or liquid carrier materials and, if desired, usual pharmaceutical adjuvants.

For avoidance of any doubt, if compounds are described as useful for the prevention / prophylaxis or treatment of certain diseases, such compounds are likewise suitable for use in the preparation of a medicament for the prevention / prophylaxis or treatment of said diseases. Likewise, such compounds are also suitable in a method for the prevention / prophylaxis or treatment of such diseases, comprising administering to a subject (mammal, especially human) in need thereof, an effective amount of such compound.

26) Another embodiment relates to the compounds of formula (I) as defined in any one of embodiments 1) to 25) which are useful for the prevention / prophylaxis or treatment of diseases and disorders that are related to galectin-3 binding to natural ligands.

Such diseases and disorders that are related to galectin-3 binding to natural ligands are especially diseases and disorders in which inhibition of the physiological activity of Gal-3 is useful, such as diseases in which a Gal-3 receptor participates, is involved in the etiology or pathology of the disease or is otherwise associated with at least one symptom of the disease.

Diseases or disorders that are related to galectin-3 binding to natural ligands may in particular be defined as including:
- fibrosis of organs comprising:
   ➢ all forms of lung / pulmonary fibrosis including all forms of fibrosing interstitial lung diseases, especially idiopathic pulmonary fibrosis (alternatively named cryptogenic fibrosing alveolitis); pulmonary fibrosis secondary to systemic inflammatory disease such as rheumatoid arthritis, scleroderma (systemic sclerosis, SSc), lupus (systemic lupus erythematosus, SLE), polymyositis, or mixed connective tissue disease (MCTD); pulmonary fibrosis secondary to sarcoidosis; iatrogenic pulmonary fibrosis including radiation-induced fibrosis; silicosis-induced pulmonary fibrosis; asbestos-induced pulmonary fibrosis; and pleural fibrosis;
   ➢ renal / kidney fibrosis, including renal fibrosis caused by / associated with chronic kidney disease (CKD), (acute or chronic) renal failure, tubulointerstitial nephritis, and/or chronic nephropathies such as (primary) glomerulonephritis and glomerulonephritis secondary to systemic inflammatory diseases such as SLE or SSc, diabetes, focal segmental glomerular sclerosis, IgA nephropathy, hypertension, renal allograft, and Alport syndrome;
   ➢ all forms of liver / hepatic fibrosis (associated or not with portal hypertension) including cirrhosis, alcoholinduced liver fibrosis, nonalcoholic steatohepatitis, biliary duct injury, primary biliary cirrhosis (also known as primary biliary cholangitis), infection- or viral-induced liver fibrosis (e.g. chronic HCV infection), and autoimmune hepatitis;
   ➢ all forms of heart / cardiac fibrosis, including heart / cardiac fibrosis associated with cardiovascular diseases, heart failure, Fabry disease, CKD; diabetes, hypertension, or hypercholesterolemia;
   ➢ gut fibrosis, including gut fibrosis secondary to SSc, and radiation-induced gut fibrosis;
   ➢ skin fibrosis, including SSc and skin scarring;
   ➢ head and neck fibrosis, including radiation-induced head and neck fibrosis;
   ➢ eye / corneal fibrosis, including scarring (e.g. sequelae of laser-assisted in situ keratomileusis, or trabeculectomy);
   ➢ hypertrophic scarring and keloids, including burn-induced or surgical hypertrophic scarring and keloids;
   ➢ fibrosis sequelae of organ transplant (including corneal transplant);
   ➢ and other fibrotic diseases including endometriosis, spinal cord fibrosis, myelofibrosis, perivascular and aterial fibrosis; as well as formation of scar tissue, Peyronie's disease, abdominal or bowel adhesions, bladder fibrosis, fibrosis of the nasal passages, and fibrosis mediated by fibroblasts;
- (acute or chronic) liver diseases and disorders including acute and chronic viral hepatitis; cirrhosis caused by / associated with arthritis and vasculitis; metabolic liver diseases caused by / associated with arthritis, myocarditis, diabetes, or neurologic symptoms; cholestatic diseases caused by / associated with hyperlipidaemia, inflammatory bowel disease (IBD), or ulcerative colitis; liver tumors; autoimmune hepatitis and cirrhosis caused by / associated with celiac disease, autoimmune haemolytic anaemia, IBD, autoimmune thyroiditis, ulcerative colitis, diabetes, glomerulonephritis, pericarditis, autoimmune thyroiditis, hyperthyroidism, polymyositis, Sjörgen syndrome, panniculitis, alveolitis or alcoholic steatosis; cirrhosis associated with dementia; cirrhosis associated with peripheral neuropathy; cirrhosis caused by / associated with oral or oesophageal cancer; non-alcoholic fatty liver disease (especially non-alcoholic steatohepatitis) caused by / associated with obesity, metabolic syndrome or type 2 diabetes; hepatic blood vessel disorders (including Budd-Chiari syndrome, portal vein thrombosis, sinusoidal obstruction syndrome); acute and chronic liver failure (associated or not with portal hypertension); liver hypofunction;
- acute kidney injury and chronic kidney disease (CKD) [especially CKD of stages 1 to 5 as defined by the Kidney Disease Improving Global Outcomes (KDIGO) Guidelines], in particular CKD (notably of these stages) caused by / associated with cardiac diseases (also referred to as cardio-renal syndrome type 1 and type 2), or caused by / associated with hypertension, or caused by / associated with diabetes (also referred to as diabetic kidney disease (DKD), including DKD associated with hypertension), wherein such diabetes especially is type 1 or type 2 diabetes), or caused by / associated with inflammatory diseases and disorders (such as glomerulonephritis and glomerulonephritis secondary to systemic inflammatory diseases such as SLE or SSc, tubulo-interstitial nephritis, vasculitis, sepsis, urinary tract infection), or caused by / associated with polycystic kidney disease, or caused by / associated with obstructive nephropathy (including calculi, benign prostatic hyperplasia, prostate cancer, retroperitoneal pelvic tumor), or caused by / associated with symptoms associated with neuropathic bladder disease); as well as acute and chronic renal failure;
- cardiovascular diseases and disorders (including atherosclerosis caused by / associated with hypertension, hypercholesterolemia, diabetes, inflammation, obesity, elderly/age; peripheral arterial disease caused by / associated with hypertension, hypercholesterolemia, diabetes, elderly/age; deep venous thrombosis; pulmonary embolism caused by / associated with obesity or cancer; aortic aneurysm and dissection caused by / associated with elderly/age, hypertension, Marfan syndrome, congenital heart disorders, inflammatory or infectious disorders; cerebrovascular disease caused by / associated with hypertension, atrial fibrillation, hypercholesterolemia, diabetes, elderly/age; coronary heart disease caused by / associated with hypertension, hypercholesterolemia, diabetes, elderly/age, or CKD (especially CKD of stages 1 to 5 as defined by the Kidney Disease Improving Global Outcomes (KDIGO) Guidelines); rheumatic heart disease caused by / associated with bacterial infection; heart and vascular tumors; cardiomyopathy and arrythmias; valvular heart disease (including valvular calcification and degenerative aortic stenosis); inflammatory heart disease caused by / associated with infection, carditis, glomerulonephitis, cancer; heart failure (HF) defined as including especially congestive HF, including in particular systolic HF / HF with reduced ejection fraction (HFrEF), and diastolic HF / HF with preserved ejection fraction (HFpEF);
- interstitial lung diseases and disorders (including smoking-related interstitial lung disease; interstitial lung disease associated with / caused by chronic obstructive pulmonary disease; interstitial pneumonia associated with collagen vascular disease (including usual interstitial pneumonia), or pneumonia);
- cell proliferative diseases and cancers (including solid tumors, solid tumor metastasis, carcinoma, sarcoma, myeloma (and multiple myeloma), leukemia, lymphoma, mixed types of cancers, vascular fibroma, Kaposi's sarcoma, chronic lymphocytic leukemia (CLL), spinal cord tumors and invasive metastasis of cancer cells; in particular said cell proliferative diseases and cancers are cancers of the thyroid gland, the central nervous system, the tongue, the breast, the gastric system, the head and neck squamous cell, the pancreas, the bladder, the kidney, the liver, the parathyroid, or the salivary glands; or lymphoma; carcinoma, non-small cell lung cancer, melanoma or neuroblastoma);
- inflammatory and autoimmune diseases and disorders including chronic and acute inflammatory and autoimmune diseases and disorders (in particular including sepsis, Q-fever, asthma, rheumatoid arthritis, multiple sclerosis (MS), systemic lupus erythematosus (SLE), systemic sclerosis (SSc), polymyositis, plaque psoriasis (including psoriasis caused by / associated with NASH), atopic dermatitis, inflammatory renal / kidney diseases such as nephropathy (including diabetic nephropathy, glomerulonephritis, tubulointerstitial nephritis), inflammatory cardiac / heart diseases, inflammatory lung / lung related diseases; inflammatory liver / liver related diseases; diabetes (type 1 or type 2) and diabetes related diseases such as diabetic vasculopathy, diabetic nephropathy, diabetic retinopathy, diabetic peripheral neuropathy or skin related condition; viral encephalitis; and COVID-19 and sequelae thereof);
- gastrointestinal tract diseases and disorders (including irritable bowel syndrome (IBS), inflammatory bowel disease (IBD), gastritis, and abnormal pancreatic secretion);
- pancreatic diseases and disorders (including pancreatitis, e.g. associated with cystic fibrosis);
- abnormal angiogenesis-associated diseases and disorders (including arterial obstruction);
- brain-associated diseases and disorders (including stroke and cerebral haemorrhage);
- neuropathic pain and peripheral neuropathy;
- ocular diseases and disorders (including dry eye disease (dry eye syndrome), macular degeneration (AMD) associated with age, diabetes related disease (diabetic retinopathy), proliferative vitreoretinopathy (PVR), cicatricial pemphigoid, and glaucoma (including glaucoma associated with elevated intraocular pressure, and ocular scarring after glaucoma filtration surgery), and corneal angiogenesis/neovascularization); and
- transplant rejection comprising rejection of transplanted organs such as kidney, liver, heart, lung, pancreas, cornea, and skin; graft-versus-host diseases brought about by hematopoietic stem cell transplantation; chronic allograft rejection and chronic allograft vasculopathy; and sequelae of such transplant rejection.

27) A further embodiment relates to the compounds of formula (I) for use according to embodiment 26) wherein said compounds are for use in the prevention / prophylaxis or treatment of fibrosis of organs including liver / hepatic fibrosis, renal / kidney fibrosis, lung / pulmonary fibrosis ,heart / cardiac fibrosis, eye / corneal fibrosis, and skin fibrosis; as well as gut fibrosis, head and neck fibrosis, hypertrophic scarring and keloids; and fibrosis sequelae of organ transplant.

28) A further embodiment relates to the compounds of formula (I) for use according to embodiment 26) wherein said compounds are for use in the prevention / prophylaxis or treatment of cardiovascular diseases and disorders.

29) A further embodiment relates to the compounds of formula (I) for use according to embodiment 26) wherein said compounds are for use in the prevention / prophylaxis or treatment of acute kidney injury and chronic kidney disease (CKD).

30) A further embodiment relates to the compounds of formula (I) for use according to embodiment 26) wherein said compounds are for use in the prevention / prophylaxis or treatment of (acute or chronic) liver diseases and disorders.

31) A further embodiment relates to the compounds of formula (I) for use according to embodiment 26) wherein said compounds are for use in the prevention / prophylaxis or treatment of interstitial lung diseases and disorders.

32) A further embodiment relates to the compounds of formula (I) for use according to embodiment 26) wherein said compounds are for use in the prevention / prophylaxis or treatment of ocular diseases and disorders.

33) A further embodiment relates to the compounds of formula (I) for use according to embodiment 26) wherein said compounds are for use in the prevention / prophylaxis or treatment of cell proliferative diseases and cancers.

Especially such cell proliferative diseases and cancers are cancers of the thyroid gland, the central nervous system, the tongue, the breast, the gastric system, the head and neck squamous cell, the pancreas, the bladder, the kidney, the liver, the parathyroid, or the salivary glands; or lymphoma; carcinoma, non-small cell lung cancer, melanoma or neuroblastoma.

34) A further embodiment relates to the compounds of formula (I) for use according to embodiment 26) wherein said compounds are for use in the prevention / prophylaxis or treatment of chronic or acute inflammatory and autoimmune diseases and disorders.

35) A further embodiment relates to the compounds of formula (I) for use according to embodiment 26) wherein said compounds are for use in the prevention / prophylaxis or treatment of gastrointestinal tract diseases and disorders.

36) A further embodiment relates to the compounds of formula (I) for use according to embodiment 26) wherein said compounds are for use in the prevention / prophylaxis or treatment of pancreatic diseases and disorders.

37) A further embodiment relates to the compounds of formula (I) for use according to embodiment 26) wherein said compounds are for use in the prevention / prophylaxis or treatment of abnormal angiogenesis-associated diseases and disorders.

38) A further embodiment relates to the compounds of formula (I) for use according to embodiment 26) wherein said compounds are for use in the prevention / prophylaxis or treatment of brain-associated diseases and disorders.

39) A further embodiment relates to the compounds of formula (I) for use according to embodiment 26) wherein said compounds are for use in the prevention / prophylaxis or treatment of neuropathic pain and peripheral neuropathy.

40) A further embodiment relates to the compounds of formula (I) for use according to embodiment 26) wherein said compounds are for use in the treatment of transplant rejection.

For avoidance of any doubt, if compounds are described as useful for the prevention / prophylaxis or treatment of certain diseases, such compounds are likewise suitable for use in the preparation of a medicament for the prevention / prophylaxis or treatment of said diseases. Likewise, such compounds are also suitable in a method for the prevention / prophylaxis or treatment of such diseases, comprising administering to a subject (mammal, especially human) in need thereof, an effective amount of such compound.

Besides, any preferences and (sub-)embodiments indicated for the compounds of Formula (I) (whether for the compounds themselves, salt thereof, compositions containing the compounds or salts thereof, or uses of the compounds or salts thereof, etc.) apply *mutatis mutandis* to compounds of Formula (II).

### Preparation of compounds of Formula (I):

The compounds of Formula (I) can be prepared by well-known literature methods, by the methods given below, by the methods given in the experimental part below or by analogous methods. Optimum reaction conditions may vary with the particular reactants or solvents used, but such conditions can be determined by a person skilled in the art by routine optimisation procedures. In some cases, the order of carrying out the following reaction schemes, and/or reaction steps, may be varied to facilitate the reaction or to avoid unwanted reaction products. In the general sequence of reactions outlined below, the generic groups **R¹**, **R²**, **A**, and **Ar¹** are as defined for Formula (I). Other abbreviations used herein are explicitly defined or are as defined in the experimental section. In some instances, the generic groups **R¹**, **R²**, **A**, and **Ar¹** might be incompatible with the assembly illustrated in the schemes below and so will require the use of protecting groups (Pg). The use of protecting groups is well known in the art (see for example "Protective Groups in Organic Synthesis", T.W. Greene, P.G.M. Wuts, Wiley-Interscience, 1999). For the purposes of this discussion, it will be assumed that such protecting groups as necessary are in place. In some cases, the final product may be further modified, for example, by manipulation of substituents to give a new final product. These manipulations may include, but are not limited to, reduction, oxidation, alkylation, acylation, hydrolysis and transition-metal catalysed cross-coupling reactions which are commonly known to those skilled in the art. The compounds obtained may also be converted into salts, especially pharmaceutically acceptable salts, in a manner known *per* se.

Compounds of the Formula (I) of the present invention can be prepared according to the general sequence of reactions outlined below. Only a few of the synthetic possibilities leading to compounds of Formula (I) are described.

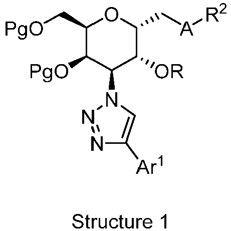

Compounds of Formula (I) are prepared by deprotecting a compound of Structure 1 in which R represents, hydrogen, a suitable protective group such as acetyl, trimethylsilyl, TBDMS or R¹, as defined in Formula (I).

Compounds of Structure 1 in which A represents a 1,4-disubstituted 1,2,3-triazole (Structure 2a or Structure 2b) can be prepared by copper-catalysed 1,3-dipolar cycloadditions of alkynes of structures 3 or 6 with azides of structures 4 or 5 (Click Chemistry in Glycoscience: New Development and Strategies, 1st Edition, 2013, John Wiley& Sons, WO 2017/007689 A1) following a batch procedure, alternatively the reaction can be run on a commercial continuous-flow reactor (Vapourtec) using a copper coil in a solvent such as THF and as shown below.

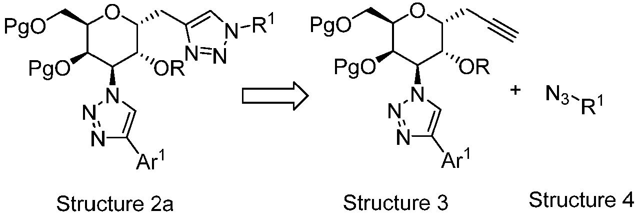

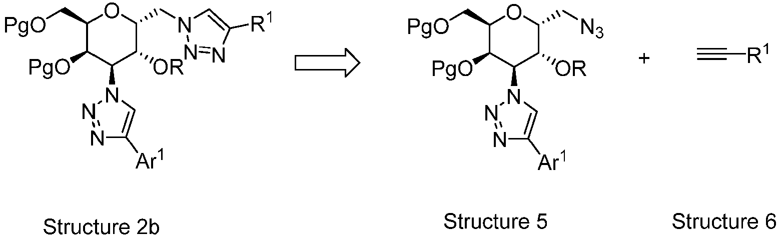

Azides of Structure 4 are either commercially available or can be prepared according to procedures known to a person skilled in the art *(*Org. Biomol. Chem. 2014, 12, 4397-4406, Nature 2019, 574, 86-89, Org. Lett., 2007, 9, 3797-3800).

### Experimental Part

The following examples illustrate the invention.

All temperatures are stated in °C. Commercially available starting materials are used as received without further purification. Unless otherwise specified, all reactions are carried out under an atmosphere of nitrogen or argon. Compounds are purified by flash chromatography on silica gel (Combiflash, ISCO), by prep TLC (TLC-plates from Merck, Silica gel 60 F₂₅₄) or by preparative HPLC. Compounds described in the invention are characterized by ¹H-NMR spectra, which are recorded on a Bruker Avance II, 400 MHz Ultra ShieldTM or Brooker Avance III HD, Descend 500 MHz ; chemical shifts are given in ppm relative to the solvent used; multiplicities: s = singlet, d = doublet, t = triplet, q = quadruplet, quint = quintuplet, hex = hexet, hept = heptet, m = multiplet, br = broad, coupling constants are given in Hz) and/or by LCMS (retention time t_{R} is given in min; molecular weight obtained for the mass spectrum is given in g/mol) using the conditions listed below and / or by chiral analytical HPLC (retention time t_{R} is given in min).

Characterization methods used:
The LC-MS retention times are obtained using the following elution conditions:

### A) LC-MS (A):

Zorbax RRHD SB-Aq, 1.8µm, 2.1x50 mm column thermostated at 40°C. The two elution solvents are as follows: solvent A= water + 0.04% TFA; solvent B = acetonitrile. The eluent flow rate is 0.8 mL/min and the characteristics of the eluting mixture proportion in function of the time t from start of the elution are summarized in the table below (a linear gradient being used between two consecutive time points):

| t (min) | 0 | 0.01 | 1.20 | 1.90 | 2.10 |
|---|---|---|---|---|---|
| Solvent A (%) | 95 | 95 | 5 | 5 | 95 |
| Solvent B (%) | 5 | 5 | 95 | 95 | 5 |

| | | | | | |
|---|---|---|---|---|---|
| Detection: UV at 210 nm. | | | | | |

### B) LC-MS (B):

Waters BEH C18, 1.8µm, 1.2*50 mm column thermostated at 40°C. The two elution solvents are as follows: solvent A= water + 13mM NH₄OH; solvent B = acetonitrile. The eluent flow rate is 0.8 mL/min and the characteristics of the eluting mixture proportion in function of the time t from start of the elution are summarized in the table below (a linear gradient being used between two consecutive time points):

| t (min) | 0 | 0.01 | 1.20 | 1.90 | 2.00 |
|---|---|---|---|---|---|
| Solvent A (%) | 95 | 95 | 5 | 5 | 95 |
| Solvent B (%) | 5 | 5 | 95 | 95 | 5 |

| | | | | | |
|---|---|---|---|---|---|
| Detection: UV at 210 nm. | | | | | |

The purifications by preparative LC-MS are performed using the conditions described hereafter.

### C) Preparative LC-MS (I):

A Waters column (Waters XBridge C18, 10 µm OBD, 30x75 mm) is used. The two elution solvents are as follows: solvent A = water + 0.5% of a solution of 25% NH₄OH in water; solvent B = acetonitrile. The eluent flow rate is 75 mL/min and the characteristics of the eluting mixture proportion in function of the time t from start of the elution are summarized in the tables below (a linear gradient being used between two consecutive time points):

| t (min) | 0 | 0.01 | 4.0 | 6.0 | 6.2 | 6.6 |
|---|---|---|---|---|---|---|
| Solvent A (%) | 90 | 90 | 5 | 5 | 90 | 90 |
| Solvent B (%) | 10 | 10 | 95 | 95 | 10 | 10 |

| | | | | | | |
|---|---|---|---|---|---|---|
| Detection 210 nm. | | | | | | |

### D) Preparative LC-MS (II):

A Waters column (Zorbax SB-AQ 30x75 mm 5 µm) is used. The two elution solvents are as follows: solvent A = water + HCOOH 0.5%; solvent B = acetonitrile. The eluent flow rate is 75 mL/min and the characteristics of the eluting mixture proportion in function of the time t from start of the elution are summarized in the tables below (a linear gradient being used between two consecutive time points):

| t (min) | 0 | 0.01 | 4.0 | 6.0 | 6.2 | 6.6 |
|---|---|---|---|---|---|---|
| Solvent A (%) | 90 | 90 | 5 | 5 | 90 | 90 |
| Solvent B (%) | 10 | 10 | 95 | 95 | 10 | 10 |

| | | | | | | |
|---|---|---|---|---|---|---|
| Detection 210 nm. | | | | | | |

### Chiral preparative HPLC methods used:

### E) Chiral preparative HPLC (I):

ChiralPack IC, 5µm, 30 x 250mm is used, column thermostated at 40°C. The two elution solvents are as follows: solvent A = CO₂; solvent B = (MeCN/EtOH) = (1/1). The eluent flow rate is 160 mL/min. The elution is done isocratic using 60% of the solvent A and 40% of the solvent B. The injection V= 1.0 mL, 10 mg/mL EtOH.

### F) Chiral preparative HPLC (II):

Chiralcel OJ-H, 5µm, 30 x 250mm is used, column thermostated at 40°C. The two elution solvents are as follows: solvent A = CO₂; solvent B = (MeCN/EtOH) = (1/1). The eluent flow rate is 160 mL/min. The elution is done isocratic using 80% of the solvent A and 20% of the solvent B. The injection V= 1.0 mL, 10 mg/mL EtOH.

### G) Chiral preparative HPLC (III):

ChiralPack IB, 5µm, 30 x 250mm is used, column thermostated at 40°C. The two elution solvents are as follows: solvent A = CO₂; solvent B = EtOH. The eluent flow rate is 160 mL/min. The elution is done isocratic using 75% of the solvent A and 25% of the solvent B. The injection V= 2.0 mL, 10 mg/mL EtOH.

### H) Chiral preparative HPLC (IV):

Chiralcel OJ-H, 5µm, 30 x 250mm is used, column thermostated at 40°C. The two elution solvents are as follows: solvent A = CO₂; solvent B = EtOH. The eluent flow rate is 160 mL/min. The elution is done isocratic using 80% of the solvent A and 20% of the solvent B. The injection V= 1.5 mL, 10 mg/mL EtOH.

### I) Chiral analytical HPLC (I):

Diastereomers of diastereomer mixtures are characterized by chiral analytical HPLC. Conditions vary for each diastereomer mixture. Several columns have been used, all have the same size: 4.6 x 250 mm, 5 um. Elution is done, if not specified otherwise, at isocratic conditions: Eluent A is usually CO₂, if not otherwise specified, eluent B is either an organic solvent or a mixture thereof. Runs last from 2.5 to 5 min.

Column type, B solvent, wherever needed also Solvent A and the length of the elution are mentioned for each diastereomer in the corresponding Table shown herewith.

### Abbreviations (as used herein):

- Ac₂O: acetic anhydride
- AcOH: acetic acid
- ADMP: 2-azido-1,3-dimethylimidazolinium hexafluorophosphate
- aq.: aqueous
- BB: building block
- BF₃OEt₂: boron trifluoride diethyletherate
- BOC: tert-butoxycarbonyl
- Bu: butyl (such as in nBuLi = n-butyl lithium)
- CC: column chromatography on silica
- conc.: concentrated
- DCM: dichloromethane
- dil.: dilute
- DIPEA: N-ethyl diisopropyl amine
- DMAP: 4-dimethylamino pyridine
- DMF: dimethylformamide
- DMSO: dimethylsulfoxide
- EA: ethyl acetate
- eq: (molar) equivalent(s)
- Et: ethyl
- EtOH: ethanol
- Et₂O: diethyl ether
- FC: flash chromatography
- h: hour(s)
- Hept: heptane
- HPLC: high performance liquid chromatography
- M: molarity [mol L⁻¹]
- Me: methyl
- MeCN: acetonitrile
- MeOH: methanol
- MS: mass spectroscopy
- min.: minute(s)
- N: normality
- K₂[OsO₂(OH)₄]: potassium osmate (VI) dihydrate
- NaIO₄: sodium(meta)periodate
- NaOAc: sodium acetate
- NaOMe: sodium methoxide
- o/n: over night
- org.: organic
- Pg: protective group
- Ph: phenyl
- PTSA: *p*-Toluenesulfonic acid
- rt: room temperature
- sat.: saturated
- TBME: tert-butylmethylether
- tBu: tert-butyl = tertiary butyl
- Tf: trifluoromethanesulfonyl
- Tf₂O: trifluoromethanesulfonic anhydride
- TFA: trifluoroacetic acid
- THF: tetrahydrofuran
- TMEDA: tetramethylethylenediamine
- TMSCI: trimethylsilyl chloride
- TMSOTf: trimethylsilyl trifluoromethanesulfonate
- T3P: propylphosphonic anhydride
- t_{R}: retention time

### A- Preparation of precursors and intermediates

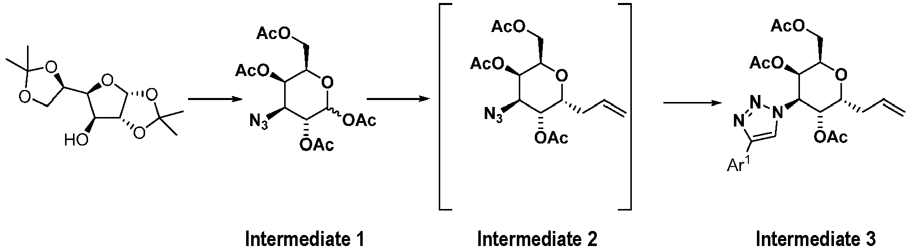

### Intermediate 1: (3R,4S,5R,6R)-6-(Acetoxymethyl)-4-azidotetrahydro-2H-pyran-2,3,5-triyl triacetate

(3R,4S,5R,6R)-6-(acetoxymethyl)-4-azidotetrahydro-2H-pyran-2,3,5-triyl triacetate is synthesized from (3aR,5S,6S,6aR)-5-((R)-2,2-dimethyl-1,3-dioxolan-4-yl)-2,2-dimethyltetrahydrofuro[2,3-d][1,3]dioxol-6-ol following the literature procedures from Ref: Carbohydrate Research 1994, 251, 33-67 and references cited therein.

### Intermediate 2: (2R,3R,4R,5R,6R)-2-(acetoxymethyl)-6-allyl-4-azidotetrahydro-2H-pyran-3,5-diyl diacetate

To a cooled (0°C) solution of Intermediate 1 (10 g, 26.8 mmol) in MeCN (100 mL) are added allyltrimethylsilane 98% (13 mL, 80.4 mmol, 3 eq) and dropwise TMSOTf (99%, 2.45 mL, 13.4 mmol, 0.5 eq). The ice bath is removed after the addition is finished and the mixture is stirred at rt for 72 h. The mixture is then poured on aq. sat. NaHCO₃ solution and extracted with TBME. The phases are separated and the combined organic phase is washed with brine, dried over MgSO₄ and concentrated *in vacuo.* The crude is purified by filtration over SiO₂ (10% TBME in DCM) to give the title intermediate (as a 9:1 mixture of alpha/beta isomers) as a colorless oil which is used in the next step without further purification.
major isomer: 1H NMR (500 MHz, DMSO-d6) δ: 5.70-5.78 (m, 1 H), 5.31 (dd, J1 = 1.6 Hz, J2 = 3.4 Hz, 1 H), 5.06-5.14 (m, 2 H), 5.00 (dd, J¹ = 5.6 Hz, J² = 10.5 H, 1 H), 4.39 (dd, J¹ = 3.4 Hz, J² = 10.6 Hz, 1 H), 4.15 (quint, J = 4.7 Hz,1 H), 3.91-4.09 (m, 3 H), 2.56-2.65 (m, 1 H), 2.22-2.28 (m, 1 H), 2.11 (s, 3 H), 2.09 (s, 3 H), 1.99 (s, 3 H)

### Intermediate 3: (2R,3R,4R,5R,6R)-2-(acetoxymethyl)-6-allyl-4-(4-(4-chloro-2,3-difluorophenyl)-1H-1,2,3-triazol-1-yl)tetrahydro-2H-pyran-3,5-diyl diacetate

To a warmed (60°C) solution of Intermediate 2 (18.75 g, 52.8 mmol) in DMF (120 mL) are added copper(I) iodide (0.5 g, 2.64 mmol, 0.05 eq), DIPEA (27.1 mL, 158 mmol, 3.0 eq) and 1-chloro-4-ethynyl-2,3-difluorobenzene (9.6 g, 55.4 mmol, 1.05 eq). Stirring is continued at 60°C for 2 h, then at rt over 15 h. The mixture is diluted with EA, the phases are separated and the org. phase is washed with aq. sat. NH₄Cl, water and aq. sat. NaCl, dried over MgSO₄ and concentrated *in vacuo.* Purification by Combiflash (330 g cartridge, elution with 0 -> 50% EA in Hept) yields the desired product as a beige solid (26.1 g, 94%, 12% β-anomer). LCMS (A): t_{R} = 1.04 min; [M+H]⁺ = 528.08.

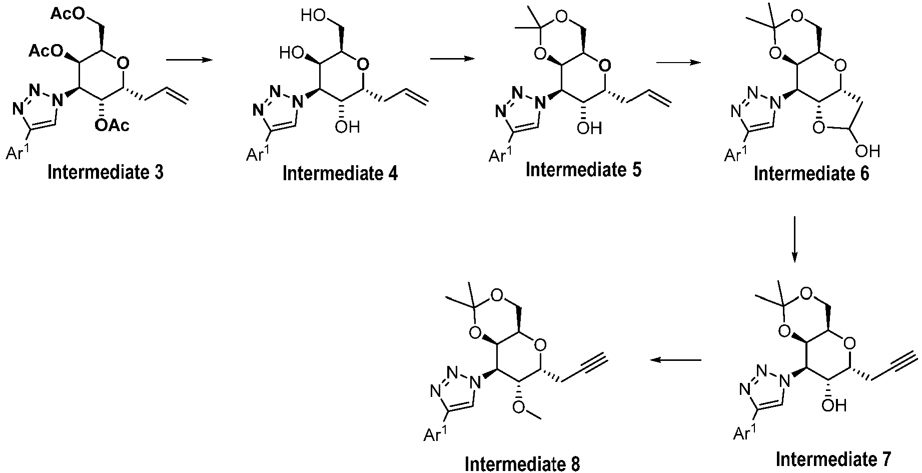

### Intermediate 4: (2R,3R,4R,5R,6R)-2-allyl-4-(4-(4-chloro-2,3-difluorophenyl)-1H-1,2,3-triazol-1-yl)-6-(hydroxymethyl)tetrahydro-2H-pyran-3,5-diol

To a solution of Intermediate 3 (26.0 g, 49.3 mmol) in MeOH (150 mL) is added K₂CO₃ (0.68 g, 4.93 mmol, 0.1 eq). The reaction mixture is stirred at rt for 1 h, acidified with Amber Chrom (Dowex, 50WX2 Hydrogen Form) to pH = 5, then filtered and concentrated *in vacuo.* The crude compound is triturated from TDBME and filtered to give the desired product as a beige solid (15.22 g, 77%, as a 9:1 mixture of alpha/beta isomers). LCMS (A): t_{R} = 0.74 min; [M+H]⁺ = 401.75.

¹H NMR (500 MHz, DMSO-d6) δ: 8.41 (d, J = 2.8 Hz, 1 H), 7.98 (m, 1 H), 7.57 (m, 1 H), 5.82-5.92 (m, 1H), 5.34 (m, 1H), 5.1 (m, 1 H), 5.17 (d, J = 16.0 Hz, 1 H), 5.06 (d, J = 10.3 Hz, 1 H), 4.97 (d, J = 11.8 Hz, 1 H), 4.57 (dd, J¹ = 5.8 Hz, J² = 11.0 Hz, 1 H), 3.98-4.04 (m, 1 H), 3.95 (s, 1 H), 3.78 (t, J = 6.3, 1 H), 3.38-3.52 (m, 2 H), 2.66-2.78 (m, 1 H), 2.3-2.4 (m, 1 H).

### Intermediate 5: (4aR,6R,7R,8R,8aR)-6-allyl-8-(4-(4-chloro-2,3-difluorophenyl)-1H-1,2,3-triazol-1-yl)-2,2-dimethylhexahydropyrano[3,2-d][1,3]dioxin-7-ol

A solution of Intermediate 4 (15.22 g, 36.6 mmol) in THF/ Acetone (100 mL / 20 mL) is treated with 2,2-dimethoxypropane (16.1 mL, 128 mmol, 3.5 eq) and PTSA monohydrate (0.355 g, 1.83 mmol, 0.05 eq) and the solution is stirred at 50°C for 4 h. The mixture is diluted with EA, the layers are separated and the org. layer is washed with aq. sat. NaHCO₃, water and brine, dried over MgSO₄, filtered and concentrated under reduced pressure to give the crude title intermediate as a beige foam (as a 9:1 mixture of alpha/beta isomers). LCMS (A): t_{R} = 0.93 min; [M+H]⁺ = 442.15. ¹H NMR (500 MHz, DMSO-d6) δ: 8.3 (d, J = 3.0 Hz, 1 H), 7.96 (m, 1 H), 7.57 (m, 1 H), 5.86 (m, 1 H), 5.41 (d, J = 5.5 Hz, 1 H), 5.06-5.18 (m, 3 H), 4.49 (m, 1 H), 4.33 (d, J = 2.5 Hz, 1 H), 4.09-4.12 (m, 1 H), 3.98-4.06 (m, 1 H), 3.68 (s, 1 H), 3.63 (d, J = 12.8 Hz, 1 H), 2.65-2.74 (m, 1 H), 2.33-2.43 (m, 1 H), 1.32 (s, 3 H), 1.20 (m, 3 H).

### Intermediate 6: (4aR,5aR,8aR,9R,9aR)-9-(4-(4-chloro-2,3-difluorophenyl)-1H-1,2,3-triazol-1-yl)-2,2-dimethyloctahydrofuro[2',3':5,6]pyrano[3,2-d][1,3]dioxin-7-ol

To a solution of Intermediate 5 (17.72g, 40.1 mmol, 1 eq) in 1,4-dioxane (216 mL) and water (20 mL), is added 2,6-lutidine (14.2 mL, 120 mmol, 3 eq) and NaIO₄ (25.7g, 120 mmol, 3 eq), followed by K₂[OsO₂(OH)₄] (0.074 g, 0.20 mmol, 0.005 eq). The suspension is vigorously stirred at rt over 15 h, diluted with EA (100 mL), filtered and the precipitate washed with EA (50 mL). The combined filtrate is washed with water (50 mL), aq. 1N HCl, brine, dried over Na₂SO₄, filtered and concentrated *in vacuo* to recover the crude title compound (as a 9:1 mixture of alpha/beta isomers). LCMS (A): tR = 0.91 min; [M+H]+ = 444.06

### Intermediate 7 : (4aR,6R,7R,8R,8aR)-8-(4-(4-Chloro-2,3-difluorophenyl)-1H-1,2,3-triazol-1-yl)-2,2-dimethyl-6-(prop-2-yn-1-yl)hexahydropyrano[3,2-d][1,3]dioxin-7-ol

To a cooled (-3°C) solution of Intermediate 6 (17.8 g, 40.1 mmol) in MeOH (242 mL) and MeCN (81 mL), are added dimethyl(1-diazo-2-oxopropyl)phosphonate (10.61 g, 54.1 mmol, 1.35 eq) and K₂CO₃ (11.1 g, 80.2 mmol, 2 eq). The mixture is stirred at -5°C (cryostat) for 15 h, then dimethyl(1-diazo-2-oxopropyl)phosphonate (1.57 g, 8 mmol, 0.2 eq) is added again. The solution is stirred for additional 4 h, while slowly letting the solution warm to rt, then it is partitioned between DCM and aq. sat. NH₄Cl. The layers are separated and the aq. layer is extracted once more with DCM. The combined org. layer is dried over Na₂SO₄, filtered and concentrated under reduced pressure to recover the crude. Purification by Combiflash (220g cartridge, elution gradient 20 -> 100% EA in Heptane) yields the title compound (11.78 g, 67%, as a 9:1 mixture of alpha/beta isomers). LCMS (A): tR = 0.94min; [M+H]⁺ = 440.39.

¹H NMR (400 MHz, DMSO) δ: 8.30 (d, J = 3.5 Hz, 1 H), 7.9-7.97 (m, 1 H), 7.55-7.59 (m, 1 H), 5.54 (d, J = 5.4 Hz, 1 H), 5.12 (dd, *J¹* = 11.3 Hz, *J*² = 3.2 Hz, 1 H), 4.47-4.52 (m, 1 H), 4.30 (d, *J* = 3.0 Hz, 1 H), 4.22-4.17 (m, 1 H), 4.05-4.08 (m, 1 H), 3.75-3.65 (m, 2 H), 2.89-2.97 (m, 1 H), 2.80 (s, 1 H), 2.54 (m, 1H)1.31 (s, 3 H), 1.22 (s, 3 H).

### Intermediate 8 : 4-(4-Chloro-2,3-difluorophenyl)-1-((4aR,6R,7R,8R,8aR)-7-methoxy-2,2-dimethyl-6-(prop-2-yn-1-yl)hexahydropyrano[3,2-d][1,3]dioxin-8-yl)-1H-1,2,3-triazole

To a cooled (0°C) solution of intermediate 7 (11.78 g, 26.8 mmol) in DMF (130 mL) is added Mel (2.19 mL, 34.8 mmol, 1.3 eq) followed by NaH (55%, 0.1287 g, 29.5 mmol, 1.1 eq). The solution is stirred at 0°C for 1h, partitioned between aq. sat. NH₄Cl and EA and the phases are separated. The org. layer is washed with water and brine, dried over MgSO₄, filtered and concentrated under reduced pressure to yield the crude. Purification by Combiflash (product linked on Isolute, column 80 g, elution gradient 20 -> 60% EA in Heptane) yields the title product (as a 9:1 mixture of alpha/beta isomers) as a white powder (10.57 g, 87%). LCMS (A): tR = 1.03 min; [M+H]⁺ = 453.78.

¹H NMR (400 MHz, DMSO) δ: 8.43 (d, J = 3.3 Hz, 1 H), 7.91-7.95 (m, 1 H), 7.56 (m, 1 H), 5.25 (dd, *J¹* = 11.4 Hz, *J²* = 3.3 Hz, 1 H), 4.55 (m, 1 H), 4.35-4.27 (m, 2 H), 4.07 (dd, *J¹* = 13.0 Hz, *J²* = 2.0 Hz, 1 H), 3.70 (t, *J* = 5.6 Hz, 2 H), 3.18 (s, 3 H), 2.89-2.98 (m, 1 H), 2.85 (t, J = 2.5 Hz, 1 H), 2.55-2.67 (m, 1H), 2.41-2.48 (m, 1 H), 1.32 (s, 3 H), 1.22 (s, 3 H).

### Intermediate 9a: 4-(2,3-Difluoro-4-methyl-phenyl)-1-((4aR,6R,7R,8R,8aR)-7-methoxy-2,2-dimethyl-6-(prop-2-yn-1-yl)hexahydropyrano[3,2-d][1,3]dioxin-8-yl)-1H-1,2,3-triazole

This intermediate is prepared from Intermediate 2 and 1-ethynyl-2,3-difluoro-4-methylbenzene in analogy to Intermediate 8 as the essentially pure alpha isomers. LCMS (A): t_{R} = 0.97 min; [M+H]⁺ = 434.02. ¹H NMR (400 MHz, DMSO) *δ*: 8.34 (s, 1H), 7.79 (t, *J* = 7.5 Hz, 1H), 7.23 (t, *J* = 7.5 Hz, 1 H), 5.23 (d, *J* = 12.5 Hz, 1 H), 4.55 (m, 1 H), 4.29-4.33 (m, 2 H), 4.07 (d, *J* = 13.0 Hz, 1 H), 3.70 (m, 2 H), 3.17 (s, 3H), 2.91-3.01 (m, 1H), 2.85 (t, J =2.6 Hz, 1H), 2.44 (m, 2H), 1.33 (s, 3H), 1.23 (s, 3H).

### Intermediate 10a: 4-(2,3,4-Trifluorophenyl)-1-((4aR,6R,7R,8R,8aR)-7-methoxy-2,2-dimethyl-6-(prop-2-yn-1-yl)hexahydropyrano[3,2-d][1,3]dioxin-8-yl)-1H-1,2,3-triazole

This intermediate is prepared from Intermediate 2 and 1-ethynyl-2,3,4-trifluorobenzene in analogy to Intermediate 8 as the essentially pure alpha isomers. LCMS (A): t_{R} = 1.01 min; [M+H]⁺ = 453.85. ¹H NMR (500 MHz, DMSO) *δ*: 8.39 (d, J = 3.4 Hz, 1 H), 7.76-8.02 (m, 1 H), 7.43-7.49 (m, 1 H), 5.24 (dd, *J*₁ = 11.4 Hz, *J*₂ = 3.4 Hz, 1 H), 4.55 (m, 1 H), 4.27-4.36 (m, 2 H), 4.07 (dd, *J*₁ = 13.1 Hz, *J*₂ = 2.4 Hz, 1 H), 3.69-3.73 (m, 2 H), 3.18 (s, 3 H), 2.94 (ddd, *J*₁ = 2.6 Hz, *J*₂ = 10.7 Hz, *J*₃ = 17.5 Hz, 1 H), 2.84 (t, *J* = 2.6 Hz, 1H), 2.78-2.87 (m, 2 H), 1.33 (s, 3H), 1.22 (s, 3H).

### Intermediate 11a: 4-(4-Bromo-2,3-difluorophenyl)-1-((4aR,6R,7R,8R,8aR)-7-methoxy-2,2-dimethyl-6-(prop-2-yn-1-yl)hexahydropyrano[3,2-d][1,3]dioxin-8-yl)-1H-1,2,3-triazole

This intermediate is prepared from Intermediate 2 and 1-bromo-4-ethynyl-2,3-difluorobenzene in analogy to Intermediate 8 as the essentially pure alpha isomer. LCMS (A): t_{R} = 1.03 min; [M+H]⁺ = 497.89. ¹H NMR (500 MHz, DMSO) *δ*: 8.43 (d, *J* = 3.3 Hz, 1 H), 7.88 (m, 1 H), 7.66 (m, 1 H), 5.25 (dd, *J*₁ = 11.4 Hz, *J*₂ = 3.4 Hz, 1 H), 4.55 (m, 1 H), 4.29-4.34 (m, 2 H), 4.07 (dd, *J*₁ = 13.1 Hz, *J*₂ = 2.2 Hz, 1 H), 3.70 (m, 2 H), 3.18 (s, 3 H), 2.94 (ddd, *J*₁ = 17.6 Hz, *J*₂ = 10.8 Hz, *J*₃ = 2.5 Hz, 1 H), 2.84 (t, *J* = 2.6 Hz, 1 H), 2.47 (m, 2 H), 1.33 (s, 3 H), 1.22 (s, 3 H).

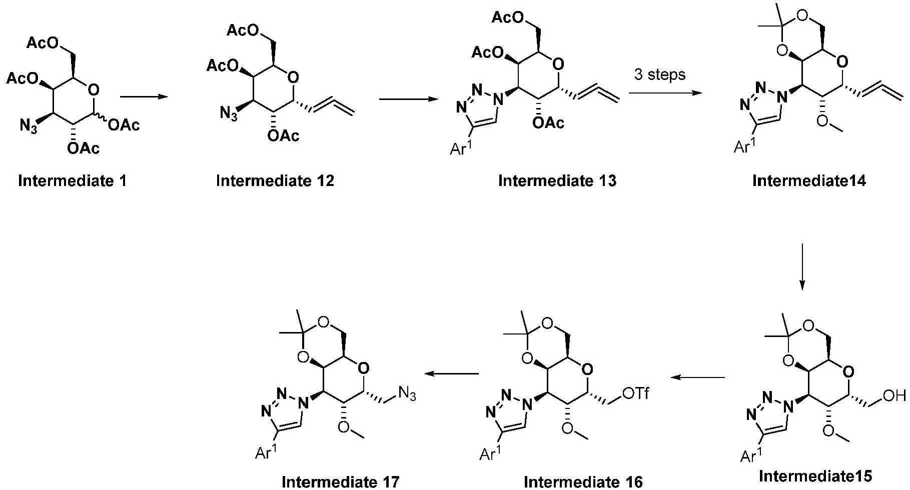

### Intermediate 12: (2R,3R,4R,5R,6R)-2-(Acetoxymethyl)-6-(propa-1,2-dien-1-yl)-4-(2l4-triaza-1,2-dien-1-yl)tetrahydro-2H-pyran-3,5-diyl diacetate

To a cooled (3°C) solution of Intermediate 1 (19.0 g, 50.9 mmol) in MeCN (120 mL) are added trimethyl(propargyl)silane (23.76 mL, 127 mmol, 2.5 eq) followed by BF₃OEt₂ (18.8 mL, 153 mmol, 3 eq) and trimethylsilyl trifluoromethanesulfonate (18.6 mL, 102 mmol, 2.0 eq) dropwise. The mixture is stirred at 0°C for 1.5 h and at rt for 1 h, then partitioned between TBME and aq. sat. NaHCO₃. The phases are separated and the org. phase is washed with brine, dried over MgSO₄, filtered and the solvent is removed *in vacuo.* The crude product is purified by FC (10% TBME in DCM) to give the desired allene intermediate as a yellowish oil, that is directly converted further (TLC: EA/Hept=2:1).

### Intermediate 13: (2R,3R,4R,5R,6R)-2-(Acetoxymethyl)-4-(4-(4-chloro-2,3-difluorophenyl)-1H-1,2,3-triazol-1-yl)-6-(propa-1,2-dien-1-yl)tetrahydro-2H-pyran-3,5-diyl diacetate

The title compound is prepared in analogy to Intermediate 3, starting from Intermediate 12 (as a 98/2 mixture of alpha/beta isomers). LCMS (A): t_{R} = 1.05 min; [M+H]⁺ = 526.10

¹H NMR (500 MHz, DMSO-d6) δ: 8.68 (d, J = 3.4 Hz, 1 H), 7.93 (m, 1 H), 7.56 (m, 1 H), 5.86 (dd, *J₁* = 5.8 Hz, *J₂* = 11.9, 1 H), 5.72 (q, J = 6.7 Hz, 1 H), 5.67 (dd, *J₁* = 3.1 Hz, *J₂* = 11.9,1 H), 5.44 (dd, *J₁* = 1.2 Hz, *J₂* = 3.1,1 H), 5.06 (dd, J¹ = 0.6 Hz, J² = 2.6 Hz, 1 H), 5.06 (dd, J¹ = 0.8 Hz, J² = 2.9 Hz, J³ = 6.7 Hz , 2 H), 5.01-4.97 (m, 1 H), 4.46 (t, J = 6.3 Hz, 1 H), 4.01-3.97 (m, 2 H), 2.01-1.99 (m, 6 H), 1.87 (s, 3 H).

### Intermediate 14: 4-(4-Chloro-2,3-difluorophenyl)-1-((4aR,6R,7R,8R,8aR)-7-methoxy-2,2-dimethyl-6-(propa-1,2-dien-1-yl)hexahydropyrano[3,2-d][1,3]dioxin-8-yl)-1H-1,2,3-triazole

The title intermediate is prepared starting from Intermediate 13 following the procedures of Intermediates 4, 5 and 8 as a 98/2 mixture of alpha/beta isomers. LCMS (A): t_{R} = 1.06 min; [M+H]⁺ = 454.12

¹H NMR (500 MHz, DMSO-d6) δ: 8.44 (d, J = 3.4 Hz 1 H), 7.91-7.96 (m, 1 H), 7.59-7.54 (m, 1 H), 5.73 (q, *J* = 7.0 Hz, 1 H), 5.19 (dd, *J*₁ = 3.9 Hz, *J*₂ = 11.4 Hz), 4.95-5.05 (m, 3 H), 4.34-4.38 (m, 2 H), 4.03-4.06 (m, 1 H), 3.82 (m, 1H), 3.73 (dd, *J*₁ = 1.5 Hz, *J*₂ = 12.8 Hz, 1 H), 3.69 (dd, *J*₁ = 1.7 Hz, *J*₂ = 13 Hz, 1 H), 3.19 (s, 3 H), 1.32 (s, 3 H), 1.22 (s, 3 H).

### Intermediate 15a: ((4aR,6R,7R,8R,8aR)-8-(4-(4-Chloro-2,3-difluorophenyl)-1H-1,2,3-triazol-1-yl)-7-methoxy-2,2-dimethylhexahydropyrano[3,2-d][1,3]dioxin-6-yl)methanol

Intermediate 14 (11.4 g, 25.1 mmol) is dissolved in DCM/MeOH (4:1, 500 mL) and cooled to -70°C. Ozone is bubbled through the solution until the KI solution in the scrubber turned brown (~2 h). Excess O₃ is purged by bubbling N₂ through the solution for 10 min. NaBH₄ (0.95 g, 25.1 mmol, 1 eq) is added at -78°C, the dry ice bath is removed and the mixture is allowed to warm up to rt within 1h. The mixture is then carefully quenched with water (25 mL) and the layers are separated. The org. layer is extracted once more with DCM, the combined organic layer is washed with water, dried over MgSO₄, filtered and concentrated under reduced pressure. The crude solid is purified by FC using CombiFlash (SiO₂ column; elution gradient 0 -> 50% EA in Hept) to give the title intermediate as a white solid as the essentially pure alpha isomer. LCMS (A): t_{R} = 0.87 min; [M+H]⁺ = 446.12.

¹H NMR (500 MHz, DMSO-d6) δ: 8.44 (d, J = 3.4 Hz 1 H), 7.91-7.96 (m, 1 H), 7.59-7.54 (m, 1 H), 5.34 (dd, *J*₁ = 3.5 Hz, *J*₂ = 11.0 Hz, 1 H), 4.81 (t, J = 5.5 Hz, 1 H), 4.36 (dd, *J*₁ = 1.1 Hz, *J*₂ = 3.5 Hz, 1H), 4.32-4.37 (m, 2 H), 3.97-4.05 (m, 2 H), 3.91 (d, *J* = 0.9 Hz, 1 H), 3.73 (dd, *J*₁ = 1.5 Hz, *J*₂ = 12.8 Hz, 1 H), 3.64 (ddd, *J*₁ = 2.3 Hz, *J*₂ = 5.6 Hz, *J*₃ = 12.2 Hz, 1 H), 3.19 (s, 3 H), 1.32 (s, 3 H), 1.22 (s, 3 H).

### Intermediate 16a: ((4aR,6R,7R,8R,8aR)-8-(4-(4-Chloro-2,3-difluorophenyl)-1H-1,2,3-triazol-1-yl)-7-methoxy-2,2-dimethylhexahydropyrano[3,2-d][1,3]dioxin-6-yl)methyl trifluoromethanesulfonate

Intermediate 15a (5.0 g, 0.011 mmol) is dissolved in DCM (50 mL), pyridine (1.81 mL, 0.024 mmol, 2.0 eq) is added and the reaction mixture is cooled to 0°C. Tf₂O (1M solution in DCM, 14.0 mL, 0.014 mmol, 1.2 eq) is added dropwise at 0°C and stirring is continued at 0°C for 1h. The mixture is diluted with DCM and washed with aq. 10% citric acid and water. The layers are separated, the org. layer is dried over MgSO₄ and concentrated under reduced pressure. The crude foam is not further purified and used as such. LCMS (A): t_{R} = 1.13 min; [M+H]⁺ = 577.76.

### Intermediate 17a: 1-((4aR,6R,7R,8R,8aR)-6-(Azidomethyl)-7-methoxy-2,2-dimethylhexahydropyrano[3,2-d][1,3]dioxin-8-yl)-4-(4-chloro-2,3-difluorophenyl)-1H-1,2,3-triazole

To a solution of Intermediate 16a (7.6 g, 13.2 mmol) in dry DMF (120 mL) is added sodium azide (0.942 g, 14.5 mmol, 1.2 eq) and the reaction mixture is heated at 70°C for 1 h. The mixture is then allowed to cool to rt, diluted with EA and water and the layers are separated. The aq. layer is extracted once with EA (2x). The combined org. layer is washed with water (2x), brine, dried over MgSO₄, filtered and concentrated under reduced pressure. The crude brown oil is purified by FC using CombiFlash (SiO₂ column; elution gradient: 0 -> 10% MeOH in DCM) to give the desired intermediate as a white foam. A second batch of desired product is obtained through a second purification of impure fractions using CombiFlash (SiO₂ column; elution gradient: 0 -> 20% EA in Hept) to give the desired intermediate as a white foam (4.89 g, 79%). LCMS (A): t_{R} = 1.05 min; [M+H]⁺ = 471.13.

¹H NMR (500 Mz, DMSO) δ: 8.45 (d, J = 3.2 Hz, 1 H), 7.94 (m, 1 H), 7.57 (m, 1 H), 5.24 (dd, J¹ = 11.6 Hz, J² = 3.4 Hz, 1 H), 4.65 (m, 1 H), 4.39 (dd, J¹ = 6.1 Hz, J² = 11.4 Hz, 1 H), 4.35 (dd, J¹ = 1.2 Hz, J² = 3.4 Hz, 1 H), 4.2 (dd, J¹ = 10.5 Hz, J² = 13.7 Hz, 1 H), 4.1 (dd, J¹ = 13.0 Hz, J² = 2.1 Hz, 1 H), 3.83 (s, 1 H), 3.7 (dd, J¹ = 12.8 Hz, J² = 1.8 Hz, 1 H), 3.26 (dd, J¹ = 13.7 Hz, J² = 3.4 Hz, 1 H), 1.34 (s, 3 H), 1.25 (s, 6 H).

### Intermediate 18a: 1-((4aR,6R,7R,8R,8aR)-6-(Azidomethyl)-7-methoxy-2,2-dimethylhexahydropyrano[3,2-d][1,3]dioxin-8-yl)-4-(2,3-difluoro-4-methylphenyl)-1H-1,2,3-triazole

The title compound is prepared in analogy to Intermediate 17a, starting from Intermediate 12 and 1-ethynyl-2,3-difluoro-4-methylbenzene. LCMS (A): t_{R} = 1.03 min; [M+H]⁺ = 451.22. ¹H NMR (500 Mz, DMSO) δ: 8.37 (d, J = 3.4 Hz, 1 H), 7.77-7.80 (m, 1 H), 7.57 (m, 1 H), 5.22 (dd, J¹ = 3.5 Hz, J² = 11.6 Hz, 1 H), 4.63-4.68 (m, 1 H), 4.7 (dd, J¹ = 6.1 Hz, J² = 11.7 Hz, 1 H), 4.35 (m, 1 H), 4.2 (dd, J¹ = 10.7 Hz, J² = 13.7 Hz, 1 H), 4.0.9 (dd, J¹ = 2 Hz, J² = 13 Hz, 1 H), 3.83 (s, 1 H), 3.69 (dd, J¹ = 1.4 Hz, J² = 13.0 Hz, 1 H), 3.26 (dd, J¹ = 3.4 Hz, J² = 13.7 Hz, 1 H), 1.34 (s, 3 H), 1.25 (s, 6 H).

### Intermediate 19a: 1-((4aR,6R,7R,8R,8aR)-6-(Azidomethyl)-7-methoxy-2,2-dimethylhexahydropyrano[3,2-d][1,3]dioxin-8-yl)-4-(2,3,4-trifluorophenyl)-1H-1,2,3-triazole

The title compound is prepared in analogy to Intermediate 17a, starting from Intermediate 12 and 1-ethynyl-2,3,4-trifluorobenzene. LCMS (A): t_{R} = 1.0 min; [M+H]⁺ = 455.18.

### Intermediate 20a: 1-((4aR,6R,7R,8R,8aR)-6-(Azidomethyl)-7-methoxy-2,2-dimethylhexahydropyrano[3,2-d][1,3]dioxin-8-yl)-4-(4-bromo-2,3-difluorophenyl)-1H-1,2,3-triazole

The title compound is prepared in analogy to Intermediate 17a, starting from Intermediate 12 and 1-bromo-4-ethynyl-2,3-difluorobenzene. LCMS (A): t_{R} = 1.04 min; [M+H]⁺ = 515.12.

### B- Preparation of examples

### Example 3.1.7.

### (2R,3R,4S,5R,6R)-2-(Hydroxymethyl)-5-methoxy-6-((1-(1-(methoxymethyl)cyclopropyl)-1H-1,2,3-triazol-4-yl)methyl)-4-(4-(2,3,4-trifluorophenyl)-1H-1,2,3-triazol-1-yl)tetrahydro-2H-pyran-3-ol

### 1. 4-(((4aR,6R,7R,8R,8aR)-7-Methoxy-2,2-dimethyl-8-(4-(2,3,4-trifluorophenyl)-1H-1,2,3-triazol-1-yl)hexahydropyrano[3,2-d][1,3]dioxin-6-yl)methyl)-1-(1-(methoxymethyl)cyclopropyl)-1H-1,2,3-triazole

To a solution of 1-(methoxymethyl)cyclopropan-1-amine hydrochloride (0.189 g, 1.37 mmol, 4.0 eq) and TEA (0.335 mL, 2.40 mmol, 7.0 eq) in MeCN (2.5 mL), is added dropwise a solution of ADMP (0.535 g, 1.78 mmol, 5.2 eq) in MeCN (2.5 mL) at rt. Upon completion of the addition, the mixture is stirred at 30°C for 40 min, then cooled to rt, and Intermediate 10 (0.15 g, 0.343 mmol, 1.0 eq) is added, followed by a solution of (+)-sodium L-ascorbate (0.007 g, 0.0343 mmol, 0.1 eq), Cul (0.007 g, 0.0343 mmol, 0.1 eq) and trans-N,N'-dimethylcyclohexane-1,2-diamine (0.00836 mL, 0.0514 mmol, 0.15 eq) in DMSO/H₂O (5/1, 2.0 mL). The reaction mixture is stirred at 50°C for 20 h, cooled to rt, partitioned between EA and water and the layers are separated. The aqueous layer is extracted with EA, the combined organic layer is dried over MgSO₄, filtered and solvent removed *in vacuo* to give a yellow oil. The crude material is purified by preparative HPLC/MS (I) to recover the title compound as a yellow oil (0.120 g, 62%). LCMS (A): t_{R} = 0.94 min; [M+H]⁺ = 565.22.

¹H NMR (400 MHz, DMSO) *δ*: 8.43 (d, 2.5 Hz, 1 H), 7.98 (s, 1 H), 7.85-7.97 (m, 1 H), 7.37-7.56 (m, 1 H), 5.32 (dd, J¹ = 2.8 Hz, J² = 12.8 Hz, 1 H), 4.54-4.65 (m, 1 H), 4.35-4.4 (m, 2 H), 4.03 (d, J = 12.8, 1 H), 3..85 (s, 1 H), 3.69 (m, 2 H), 3.62 (d, J = 12.8 Hz, 1 H), 3.27-3.32 (m, 1 H), 3.25 (s, 3 H), 3.20 (s, 3 H), 2.88 (dd, J¹ = 1.8 Hz, J² = 15 Hz, 1 H), 1.32-1.38 (m, 5 H), 1.27 (s, 3 H), 1.18 (m, 2H).

### 2. (2R,3R,4S,5R,6R)-2-(Hydroxymethyl)-5-methoxy-6-((1-(1-(methoxymethyl)cyclopropyl)-1H-1,2,3-triazol-4-yl)methyl)-4-(4-(2,3,4-trifluorophenyl)-1H-1,2,3-triazol-1-yl)tetrahydro-2H-pyran-3-ol (Example 3.1.7.)

To a solution of Example 3.1.7. *Step* 1. (0.120 g, 0.213 mmol, 1.0 eq) in THF (4.0 mL) are added glacial AcOH (8.0 mL) and H₂O (8.0 mL). The reaction mixture is stirred at 55°C for 20 h, cooled to rt, the solvent is removed *in vacuo* to give a slightly yellow oil that is directly purified by preparative HPLC/MS (I) to give the title compound as a white solid (0.045 g, 40%). LCMS (A): t_{R} = 0.77 min; [M+H]⁺ = 525.16.

¹H NMR (400 MHz, MeOD) *δ*: 8.47 (d, *J* = 3.0 Hz, 1 H), 8.12 (s, 1 H), 7.88-7.97 (m, 1 H), 7.25-7.35 (m, 1 H), 5.17 (dd, J¹ = 1.5 Hz, J² = 11.5 Hz, 1 H), 4.6-4.7 (m, 1 H), 4.5 (dd, J¹ = 6.0 Hz, J² = 11.5 Hz, 1 H), 4.15 (s, 1 H), 4.06 (t, *J* = 5.8 Hz, 1 H), 3.63-3.80 (m, 4 H), 3.42 (m, 1H), 3.35 (s, 3H), 3.34 (s, 3H), 3.10 (dd, J¹ = 3.0 Hz, J² = 15.5 Hz, 1 H), 1.42 (m, 2H), 1.26 (m, 2H).

Following examples are prepared starting from either Intermediate 8, 9a, 10a, or 11a and the corresponding amines, according to the procedures described for Example 3.1.7. LC-MS data are listed in Table 1 below. LC-MS conditions are LC-MS (A).

**Table 1**

| **Ex.** | **Compound** | **t_{R} [min]** | **MS Data m/z [M+H]⁺** |
|---|---|---|---|
| **1.1.1.** | (2R,3R,4S,5R,6R)-4-(4-(2,3-difluoro-4-methylphenyl)-1H-1,2,3-triazol-1-yl)-2-(hydroxymethyl)-6-((1-((3R,4R)-4-hydroxytetrahydro-2H-pyran-3-yl)-1H-1,2,3-triazol-4-yl)methyl)-5-methoxytetrahydro-2H-pyran-3-ol | 0.73 | 537.08 |
| **1.1.2.** | (2R,3R,4S,5R,6R)-4-(4-(2,3-difluoro-4-methylphenyl)-1H-1,2,3-triazol-1-yl)-6-((1-((1R,2R)-2-hydroxycyclopentyl)-1H-1,2,3-triazol-4-yl)methyl)-2-(hydroxymethyl)-5-methoxytetrahydro-2H-pyran-3-ol | 0.76 | 521.12 |
| **1.1.3.** | (2R,3R,4S,5R,6R)-4-(4-(2,3-difluoro-4-methylphenyl)-1H-1,2,3-triazol-1-yl)-6-((1-((1S,2S)-2-hydroxycyclopentyl)-1H-1,2,3-triazol-4-yl)methyl)-2-(hydroxymethyl)-5-methoxytetrahydro-2H-pyran-3-ol | 0.76 | 521.12 |
| **1.1.4.** | (2R,3R,4S,5R,6R)-4-(4-(2,3-difluoro-4-methylphenyl)-1H-1,2,3-triazol-1-yl)-2-(hydroxymethyl)-6-((1-(1-(hydroxymethyl)cyclopropyl)-1H-1,2,3-triazol-4-yl)methyl)-5-methoxytetrahydro-2H-pyran-3-ol | 0.73 | 507.10 |
| **1.1.5.**** | (2R,3R,4S,5R,6R)-4-(4-(2,3-difluoro-4-methylphenyl)-1H-1,2,3-triazol-1-yl)-2-(hydroxymethyl)-6-((1-(1-(hydroxymethyl)cyclopentyl)-1H-1,2,3-triazol-4-yl)methyl)-5-methoxytetrahydro-2H-pyran-3-ol | 0.80 | 535.12 |
| **1.1.6.** | (2R,3R,4S,5R,6R)-4-(4-(2,3-difluoro-4-methylphenyl)-1H-1,2,3-triazol-1-yl)-2-(hydroxymethyl)-5-methoxy-6-((1-(3-methyloxetan-3-yl)-1H-1,2,3-triazol-4-yl)methyl)tetrahydro-2H-pyran-3-ol | 0.77 | 507.113 |
| **1.1.7.** | (2R,3R,4S,5R,6R)-4-(4-(2,3-difluoro-4-methylphenyl)-1H-1,2,3-triazol-1-yl)-2-(hydroxymethyl)-5-methoxy-6-((1-(1-(methoxymethyl)cyclopropyl)-1H-1,2,3-triazol-4-yl)methyl)tetrahydro-2H-pyran-3-ol | 0.80 | 521.26 |
| **1.1.8.** | (2R,3R,4S,5R,6R)-4-(4-(2,3-difluoro-4-methylphenyl)-1H-1,2,3-triazol-1-yl)-6-((1-(4-hydroxybicyclo[2.2.2]octan-1-yl)-1H-1,2,3-triazol-4-yl)methyl)-2-(hydroxymethyl)-5-methoxytetrahydro-2H-pyran-3-ol | 0.76 | 561.09 |
| **1.1.9.** | (2R,3R,4S,5R,6R)-4-(4-(2,3-difluoro-4-methylphenyl)-1H-1,2,3-triazol-1-yl)-6-((1-(1-hydroxy-2-methylpropan-2-yl)-1H-1,2,3-triazol-4-yl)methyl)-2-(hydroxymethyl)-5-methoxytetrahydro-2H-pyran-3-ol | 0.75 | 509.23 |
| **1.1.10.** | methyl 2-(4-(((2R,3R,4S,5R,6R)-4-(4-(2,3-difluoro-4-methylphenyl)-1H-1,2,3-triazol-1-yl)-5-hydroxy-6-(hydroxymethyl)-3-methoxytetrahydro-2H-pyran-2-yl)methyl)-1H-1,2,3-triazol-1-yl)-2-methylpropanoate | 0.83 | 537.16 |
| **1.1.11.** | (2R,3R,4S,5R,6R)-4-(4-(2,3-difluoro-4-methylphenyl)-1H-1,2,3-triazol-1-yl)-2-(hydroxymethyl)-6-((1-(1-(hydroxymethyl)cyclobutyl)-1H-1,2,3-triazol-4-yl)methyl)-5-methoxytetrahydro-2H-pyran-3-ol | 0.76 | 521.17 |
| **1.1.12.** | (2R,3R,4S,5R,6R)-6-((1-(bicyclo[1.1.1]pentan-1-yl)-1H-1,2,3-triazol-4-yl)methyl)-4-(4-(2,3-difluoro-4-methylphenyl)-1H-1,2,3-triazol-1-yl)-2-(hydroxymethyl)-5-methoxytetrahydro-2H-pyran-3-ol | 0.85 | 503.12 |
| **1.1.13.*** | (2R,3R,4S,5R,6R)-4-(4-(2,3-difluoro-4-methylphenyl)-1H-1,2,3-triazol-1-yl)-2-(hydroxymethyl)-5-methoxy-6-((1-(1-methylcyclobutyl)-1H-1,2,3-triazol-4-yl)methyl)tetrahydro-2H-pyran-3-ol | 0.85 | 505.46 |
| **1.1.14.** | (2R,3R,4S,5R,6R)-4-(4-(2,3-difluoro-4-methylphenyl)-1H-1,2,3-triazol-1-yl)-2-(hydroxymethyl)-5-methoxy-6-((1-(3-methylbicyclo[1.1.1]pentan-1-yl)-1H-1,2,3-triazol-4-yl)methyl)tetrahydro-2H-pyran-3-ol | 0.89 | 517.24 |
| **1.1.15.** | (2R,3R,4S,5R,6R)-6-((1-(3,3-difluoro-1-methylcyclobutyl)-1H-1,2,3-triazol-4-yl)methyl)-4-(4-(2,3-difluoro-4-methylphenyl)-1H-1,2,3-triazol-1-yl)-2-(hydroxymethyl)-5-methoxytetrahydro-2H-pyran-3-ol | 0.87 | 541.27 |
| **1.1.16.** | (2R,3R,4S,5R,6R)-6-((1-(2-oxaspiro[3.3]heptan-6-yl)-1H-1,2,3-triazol-4-yl)methyl)-4-(4-(2,3-difluoro-4-methylphenyl)-1H-1,2,3-triazol-1-yl)-2-(hydroxymethyl)-5-methoxytetrahydro-2H-pyran-3-ol | 0.77 | 533.26 |
| **1.1.17.** | (2R,3R,4S,5R,6R)-4-(4-(2,3-difluoro-4-methylphenyl)-1H-1,2,3-triazol-1-yl)-2-(hydroxymethyl)-6-((1-((1RS,2SR)-2-isopropylcyclopropyl)-1H-1,2,3-triazol-4-yl)methyl)-5-methoxytetrahydro-2H-pyran-3-ol | 0.91 | 519.25 |
| **1.1.18.** | (2R,3R,4S,5R,6R)-6-((1-(3,3-difluoro-1-(hydroxymethyl)cyclobutyl)-1H-1,2,3-triazol-4-yl)methyl)-4-(4-(2,3-difluoro-4-methylphenyl)-1H-1,2,3-triazol-1-yl)-2-(hydroxymethyl)-5-methoxytetrahydro-2H-pyran-3-ol | 0.80 | 557.24 |
| **1.1.19.** | (2R,3R,4S,5R,6R)-4-(4-(2,3-difluoro-4-methylphenyl)-1H-1,2,3-triazol-1-yl)-6-((1-((1S,3S)-3-hydroxy-1-methylcyclobutyl)-1H-1,2,3-triazol-4-yl)methyl)-2-(hydroxymethyl)-5-methoxytetrahydro-2H-pyran-3-ol | 0.74 | 521.24 |
| **1.1.20.** | (2R,3R,4S,5R,6R)-6-((1-((1R,5S)-bicyclo[3.1.0]hexan-6-yl)-1H-1,2,3-triazol-4-yl)methyl)-4-(4-(2,3-difluoro-4-methylphenyl)-1H-1,2,3-triazol-1-yl)-2-(hydroxymethyl)-5-methoxytetrahydro-2H-pyran-3-ol | 0.89 | 517.16 |
| **1.1.21.** | (2R,3R,4S,5R,6R)-4-(4-(2,3-difluoro-4-methylphenyl)-1H-1,2,3-triazol-1-yl)-2-(hydroxymethyl)-5-methoxy-6-((1-(3-(methoxymethyl)oxetan-3-yl)-1H-1,2,3-triazol-4-yl)methyl)tetrahydro-2H-pyran-3-ol | 0.77 | 537.23 |
| **1.1.22.** | (2R,3R,4S,5R,6R)-4-(4-(2,3-difluoro-4-methylphenyl)-1H-1,2,3-triazol-1-yl)-6-((1-(3-(2-hydroxyethyl)oxetan-3-yl)-1H-1,2,3-triazol-4-yl)methyl)-2-(hydroxymethyl)-5-methoxytetrahydro-2H-pyran-3-ol | 0.71 | 537.27 |
| **1.1.23.** | (2R,3R,4S,5R,6R)-4-(4-(2,3-difluoro-4-methylphenyl)-1H-1,2,3-triazol-1-yl)-2-(hydroxymethyl)-5-methoxy-6-((1-(1-methylcyclopropyl)-1H-1,2,3-triazol-4-yl)methyl)tetrahydro-2H-pyran-3-ol | 0.82 | 491.25 |
| **1.1.24.** | (2R,3R,4S,5R,6R)-4-(4-(2,3-difluoro-4-methylphenyl)-1H-1,2,3-triazol-1-yl)-2-(hydroxymethyl)-5-methoxy-6-((1-(3-(trifluoromethyl)bicyclo[1.1.1]pentan-1-yl)-1H-1,2,3-triazol-4-yl)methyl)tetrahydro-2H-pyran-3-ol | 0.93 | 570.98 |
| **1.1.25.***** | (2R,3R,4S,5R,6R)-4-(4-(2,3-difluoro-4-methylphenyl)-1H-1,2,3-triazol-1-yl)-6-((1-(3-fluorobicyclo[1.1.1]pentan-1-yl)-1H-1,2,3-triazol-4-yl)methyl)-2-(hydroxymethyl)-5-methoxytetrahydro-2H-pyran-3-ol | 0.86 | 521.122 |
| **1.1.26.** | (2R,3R,4S,5R,6R)-4-(4-(2,3-difluoro-4-methylphenyl)-1H-1,2,3-triazol-1-yl)-2-(hydroxymethyl)-5-methoxy-6-((1-(spiro[2.3]hexan-5-yl)-1H-1,2,3-triazol-4-yl)methyl)tetrahydro-2H-pyran-3-ol | 0.89 | 517.12 |
| **1.1.27.** | (2R,3R,4S,5R,6R)-4-(4-(2,3-difluoro-4-methylphenyl)-1H-1,2,3-triazol-1-yl)-6-((1-((1R,3R)-3-hydroxy-1-methylcyclobutyl)-1H-1,2,3-triazol-4-yl)methyl)-2-(hydroxymethyl)-5-methoxytetrahydro-2H-pyran-3-ol | 0.74 | 521.22 |
| **2.1.7.** | (2R,3R,4S,5R,6R)-4-(4-(4-chloro-2,3-difluorophenyl)-1H-1,2,3-triazol-1-yl)-2-(hydroxymethyl)-5-methoxy-6-((1-(1-(methoxymethyl)cyclopropyl)-1H-1,2,3-triazol-4-yl)methyl)tetrahydro-2H-pyran-3-ol | 0.82 | 541.17 |
| **2.1.28.** | (2R,3R,4S,5R,6R)-4-(4-(4-chloro-2,3-difluorophenyl)-1H-1,2,3-triazol-1-yl)-6-((1-(1,1-difluoro-2-methylpropan-2-yl)-1H-1,2,3-triazol-4-yl)methyl)-2-(hydroxymethyl)-5-methoxytetrahydro-2H-pyran-3-ol | 0.89 | 548.98 |
| **2.1.4.** | (2R,3R,4S,5R,6R)-4-(4-(4-chloro-2,3-difluorophenyl)-1H-1,2,3-triazol-1-yl)-2-(hydroxymethyl)-6-((1-(1-(hydroxymethyl)cyclopropyl)-1H-1,2,3-triazol-4-yl)methyl)-5-methoxytetrahydro-2H-pyran-3-ol | 0.75 | 527.02. |
| **2.1.5.** | (2R,3R,4S,5R,6R)-4-(4-(4-chloro-2,3-difluorophenyl)-1H-1,2,3-triazol-1-yl)-2-(hydroxymethyl)-6-((1-(1-(hydroxymethyl)cyclopentyl)-1H-1,2,3-triazol-4-yl)methyl)-5-methoxytetrahydro-2H-pyran-3-ol | 0.82 | 555.16 |
| **2.1.11.** | (2R,3R,4S,5R,6R)-4-(4-(4-chloro-2,3-difluorophenyl)-1H-1,2,3-triazol-1-yl)-2-(hydroxymethyl)-6-((1-(1-(hydroxymethyl)cyclobutyl)-1H-1,2,3-triazol-4-yl)methyl)-5-methoxytetrahydro-2H-pyran-3-ol | 0.79 | 541.05 |
| **2.1.8.** | (2R,3R,4S,5R,6R)-4-(4-(4-chloro-2,3-difluorophenyl)-1H-1,2,3-triazol-1-yl)-6-((1-(4-hydroxybicyclo[2.2.2]octan-1-yl)-1H-1,2,3-triazol-4-yl)methyl)-2-(hydroxymethyl)-5-methoxytetrahydro-2H-pyran-3-ol | 0.79 | 581.15 |
| **2.1.9.** | (2R,3R,4S,5R,6R)-4-(4-(4-chloro-2,3-difluorophenyl)-1H-1,2,3-triazol-1-yl)-6-((1-(1-hydroxy-2-methylpropan-2-yl)-1H-1,2,3-triazol-4-yl)methyl)-2-(hydroxymethyl)-5-methoxytetrahydro-2H-pyran-3-ol | 0.77 | 529.12 |
| **2.1.10.** | methyl 2-(4-(((2R,3R,4S,5R,6R)-4-(4-(4-chloro-2,3-difluorophenyl)-1H-1,2,3-triazol-1-yl)-5-hydroxy-6-(hydroxymethyl)-3-methoxytetrahydro-2H-pyran-2-yl)methyl)-1H-1,2,3-triazol-1-yl)-2-methylpropanoate | 0.85 | 557.11 |
| **2.1.12.*** | (2R,3R,4S,5R,6R)-6-((1-(bicyclo[1.1.1]pentan-1-yl)-1H-1,2,3-triazol-4-yl)methyl)-4-(4-(4-chloro-2,3-difluorophenyl)-1H-1,2,3-triazol-1-yl)-2-(hydroxymethyl)-5-methoxytetrahydro-2H-pyran-3-ol | 0.87 | 523.10 |
| **2.1.13.*** | (2R,3R,4S,5R,6R)-4-(4-(4-chloro-2,3-difluorophenyl)-1H-1,2,3-triazol-1-yl)-2-(hydroxymethyl)-5-methoxy-6-((1-(1-methylcyclobutyl)-1H-1,2,3-triazol-4-yl)methyl)tetrahydro-2H-pyran-3-ol | 0.87 | 525.14 |
| **2.1.6.** | (2R,3R,4S,5R,6R)-4-(4-(4-chloro-2,3-difluorophenyl)-1H-1,2,3-triazol-1-yl)-2-(hydroxymethyl)-5-methoxy-6-((1-(3-methyloxetan-3-yl)-1H-1,2,3-triazol-4-yl)methyl)tetrahydro-2H-pyran-3-ol | 0.79 | 527.18 |
| **2.1.14.** | (2R,3R,4S,5R,6R)-4-(4-(4-chloro-2,3-difluorophenyl)-1H-1,2,3-triazol-1-yl)-2-(hydroxymethyl)-5-methoxy-6-((1-(3-methylbicyclo[1.1.1]pentan-1-yl)-1H-1,2,3-triazol-4-yl)methyl)tetrahydro-2H-pyran-3-ol | 0.92 | 537.19 |
| **2.1.29.** | (2R,3R,4S,5R,6R)-4-(4-(4-chloro-2,3-difluorophenyl)-1H-1,2,3-triazol-1-yl)-2-(hydroxymethyl)-5-methoxy-6-((1-(3-methoxybicyclo[1.1.1]pentan-1-yl)-1H-1,2,3-triazol-4-yl)methyl)tetrahydro-2H-pyran-3-ol | 0.85 | 553.21 |
| **2.1.15.** | (2R,3R,4S,5R,6R)-4-(4-(4-chloro-2,3-difluorophenyl)-1H-1,2,3-triazol-1-yl)-6-((1-(3,3-difluoro-1-methylcyclobutyl)-1H-1,2,3-triazol-4-yl)methyl)-2-(hydroxymethyl)-5-methoxytetrahydro-2H-pyran-3-ol | 0.89 | 562.05 |
| **2.1.16.** | (2R,3R,4S,5R,6R)-6-((1-(2-oxaspiro[3.3]heptan-6-yl)-1H-1,2,3-triazol-4-yl)methyl)-4-(4-(4-chloro-2,3-difluorophenyl)-1H-1,2,3-triazol-1-yl)-2-(hydroxymethyl)-5-methoxytetrahydro-2H-pyran-3-ol | 0.79 | 553.21 |
| **2.1.17.** | (2R,3R,4S,5R,6R)-4-(4-(4-chloro-2,3-difluorophenyl)-1H-1,2,3-triazol-1-yl)-2-(hydroxymethyl)-6-((1-((1RS,2SR)-2-isopropylcyclopropyl)-1H-1,2,3-triazol-4-yl)methyl)-5-methoxytetrahydro-2H-pyran-3-ol | 0.93 | 539.25 |
| **2.1.18.** | (2R,3R,4S,5R,6R)-4-(4-(4-chloro-2,3-difluorophenyl)-1H-1,2,3-triazol-1-yl)-6-((1-(3,3-difluoro-1-(hydroxymethyl)cyclobutyl)-1H-1,2,3-triazol-4-yl)methyl)-2-(hydroxymethyl)-5-methoxytetrahydro-2H-pyran-3-ol | 0.82 | 577.19 |
| **2.1.19.** | (2R,3R,4S,5R,6R)-4-(4-(4-chloro-2,3-difluorophenyl)-1H-1,2,3-triazol-1-yl)-6-((1-((1S,3S)-3-hydroxy-1-methylcyclobutyl)-1H-1,2,3-triazol-4-yl)methyl)-2-(hydroxymethyl)-5-methoxytetrahydro-2H-pyran-3-ol | 0.76 | 541.16 |
| **2.1.20.** | (2R,3R,4S,5R,6R)-6-((1-((1R,5S)-bicyclo[3.1.0]hexan-6-yl)-1H-1,2,3-triazol-4-yl)methyl)-4-(4-(4-chloro-2,3-difluorophenyl)-1H-1,2,3-triazol-1-yl)-2-(hydroxymethyl)-5-methoxytetrahydro-2H-pyran-3-ol | 0.91 | 537.22 |
| **2.1.21.** | (2R,3R,4S,5R,6R)-4-(4-(4-chloro-2,3-difluorophenyl)-1H-1,2,3-triazol-1-yl)-2-(hydroxymethyl)-5-methoxy-6-((1-(3-(methoxymethyl)oxetan-3-yl)-1H-1,2,3-triazol-4-yl)methyl)tetrahydro-2H-pyran-3-ol | 0.80 | 557.21 |
| **2.1.22.** | (2R,3R,4S,5R,6R)-4-(4-(4-chloro-2,3-difluorophenyl)-1H-1,2,3-triazol-1-yl)-6-((1-(3-(2-hydroxyethyl)oxetan-3-yl)-1H-1,2,3-triazol-4-yl)methyl)-2-(hydroxymethyl)-5-methoxytetrahydro-2H-pyran-3-ol | 0.73 | 557.19 |
| **2.1.23.** | (2R,3R,4S,5R,6R)-4-(4-(4-chloro-2,3-difluorophenyl)-1H-1,2,3-triazol-1-yl)-2-(hydroxymethyl)-5-methoxy-6-((1-(1-methylcyclopropyl)-1H-1,2,3-triazol-4-yl)methyl)tetrahydro-2H-pyran-3-ol | 0.84 | 511.11 |
| **2.1.24.** | (2R,3R,4S,5R,6R)-4-(4-(4-chloro-2,3-difluorophenyl)-1H-1,2,3-triazol-1-yl)-2-(hydroxymethyl)-5-methoxy-6-((1-(3-(trifluoromethyl)bicyclo[1.1.1]pentan-1-yl)-1H-1,2,3-triazol-4-yl)methyl)tetrahydro-2H-pyran-3-ol | 0.82 | 551.20 |
| **2.1.25.****** | (2R,3R,4S,5R,6R)-4-(4-(4-chloro-2,3-difluorophenyl)-1H-1,2,3-triazol-1-yl)-6-((1-(3-fluorobicyclo[1.1.1]pentan-1-yl)-1H-1,2,3-triazol-4-yl)methyl)-2-(hydroxymethyl)-5-methoxytetrahydro-2H-pyran-3-ol | 0.88 | 541.17 |
| **2.1.26.** | (2R,3R,4S,5R,6R)-4-(4-(4-chloro-2,3-difluorophenyl)-1H-1,2,3-triazol-1-yl)-2-(hydroxymethyl)-5-methoxy-6-((1-(spiro[2.3]hexan-5-yl)-1H-1,2,3-triazol-4-yl)methyl)tetrahydro-2H-pyran-3-ol | 0.91 | 537.20 |
| **2.1.27.** | (2R,3R,4S,5R,6R)-4-(4-(4-chloro-2,3-difluorophenyl)-1H-1,2,3-triazol-1-yl)-6-((1-((1R,3R)-3-hydroxy-1-methylcyclobutyl)-1H-1,2,3-triazol-4-yl)methyl)-2-(hydroxymethyl)-5-methoxytetrahydro-2H-pyran-3-ol | 0.76 | 541.17 |
| **3.1.5.** | (2R,3R,4S,5R,6R)-2-(hydroxymethyl)-6-((1-(1-(hydroxymethyl)cyclopentyl)-1H-1,2,3-triazol-4-yl)methyl)-5-methoxy-4-(4-(2,3,4-trifluorophenyl)-1H-1,2,3-triazol-1-yl)tetrahydro-2H-pyran-3-ol | 0.78 | 539.18 |
| **3.1.4.** | (2R,3R,4S,5R,6R)-2-(hydroxymethyl)-6-((1-(1-(hydroxymethyl)cyclopropyl)-1H-1,2,3-triazol-4-yl)methyl)-5-methoxy-4-(4-(2,3,4-trifluorophenyl)-1H-1,2,3-triazol-1-yl)tetrahydro-2H-pyran-3-ol | 0.71 | 511.18 |
| **3.1.7.** | (2R,3R,4S,5R,6R)-2-(hydroxymethyl)-5-methoxy-6-((1-(1-(methoxymethyl)cyclopropyl)-1H-1,2,3-triazol-4-yl)methyl)-4-(4-(2,3,4-trifluorophenyl)-1H-1,2,3-triazol-1-yl)tetrahydro-2H-pyran-3-ol | 0.77 | 525.16 |
| **3.1.12.** | (2R,3R,4S,5R,6R)-6-((1-(bicyclo[1.1.1]pentan-1-yl)-1H-1,2,3-triazol-4-yl)methyl)-2-(hydroxymethyl)-5-methoxy-4-(4-(2,3,4-trifluorophenyl)-1H-1,2,3-triazol-1-yl)tetrahydro-2H-pyran-3-ol | 0.83 | 507.15 |
| **3.1.11.** | (2R,3R,4S,5R,6R)-2-(hydroxymethyl)-6-((1-(1-(hydroxymethyl)cyclobutyl)-1H-1,2,3-triazol-4-yl)methyl)-5-methoxy-4-(4-(2,3,4-trifluorophenyl)-1H-1,2,3-triazol-1-yl)tetrahydro-2H-pyran-3-ol | 0.74 | 525.2 |
| **3.1.25.** | (2R,3R,4S,5R,6R)-6-((1-(3-fluorobicyclo[1.1.1]pentan-1-yl)-1H-1,2,3-triazol-4-yl)methyl)-2-(hydroxymethyl)-5-methoxy-4-(4-(2,3,4-trifluorophenyl)-1H-1,2,3-triazol-1-yl)tetrahydro-2H-pyran-3-ol | 0.84 | 525.21 |
| **3.1.24.** | (2R,3R,4S,5R,6R)-2-(hydroxymethyl)-5-methoxy-6-((1-(3-(trifluoromethyl)bicyclo[1.1.1]pentan-1-yl)-1H-1,2,3-triazol-4-yl)methyl)-4-(4-(2,3,4-trifluorophenyl)-1H-1,2,3-triazol-1-yl)tetrahydro-2H-pyran-3-ol | 0.91 | 575.22 |
| **3.1.15.** | (2R,3R,4S,5R,6R)-6-((1-(3,3-difluoro-1-methylcyclobutyl)-1H-1,2,3-triazol-4-yl)methyl)-2-(hydroxymethyl)-5-methoxy-4-(4-(2,3,4-trifluorophenyl)-1H-1,2,3-triazol-1-yl)tetrahydro-2H-pyran-3-ol | 0.85 | 546.05 |
| **3.1.18.** | (2R,3R,4S,5R,6R)-6-((1-(3,3-difluoro-1-(hydroxymethyl)cyclobutyl)-1H-1,2,3-triazol-4-yl)methyl)-2-(hydroxymethyl)-5-methoxy-4-(4-(2,3,4-trifluorophenyl)-1H-1,2,3-triazol-1-yl)tetrahydro-2H-pyran-3-ol | 0.78 | 562.08 |
| **3.1.16.** | (2R,3R,4S,5R,6R)-6-((1-(2-oxaspiro[3.3]heptan-6-yl)-1H-1,2,3-triazol-4-yl)methyl)-2-(hydroxymethyl)-5-methoxy-4-(4-(2,3,4-trifluorophenyl)-1H-1,2,3-triazol-1-yl)tetrahydro-2H-pyran-3-ol | 0.75 | 537.24 |
| **3.1.27.** | (2R,3R,4S,5R,6R)-6-((1-((1R,3R)-3-hydroxy-1-methylcyclobutyl)-1H-1,2,3-triazol-4-yl)methyl)-2-(hydroxymethyl)-5-methoxy-4-(4-(2,3,4-trifluorophenyl)-1H-1,2,3-triazol-1-yl)tetrahydro-2H-pyran-3-ol | 0.72 | 525.21 |
| **3.1.19.** | (2R,3R,4S,5R,6R)-6-((1-((1S,3S)-3-hydroxy-1-methylcyclobutyl)-1H-1,2,3-triazol-4-yl)methyl)-2-(hydroxymethyl)-5-methoxy-4-(4-(2,3,4-trifluorophenyl)-1H-1,2,3-triazol-1-yl)tetrahydro-2H-pyran-3-ol | 0.72 | 525.2 |
| **3.1.21.** | (2R,3R,4S,5R,6R)-2-(hydroxymethyl)-5-methoxy-6-((1-(3-(methoxymethyl)oxetan-3-yl)-1H-1,2,3-triazol-4-yl)methyl)-4-(4-(2,3,4-trifluorophenyl)-1H-1,2,3-triazol-1-yl)tetrahydro-2H-pyran-3-ol | 0.76 | 541.24 |
| **3.1.22.** | (2R,3R,4S,5R,6R)-6-((1-(3-(2-hydroxyethyl)oxetan-3-yl)-1H-1,2,3-triazol-4-yl)methyl)-2-(hydroxymethyl)-5-methoxy-4-(4-(2,3,4-trifluorophenyl)-1H-1,2,3-triazol-1-yl)tetrahydro-2H-pyran-3-ol | 0.69 | 541.22 |
| **3.1.23.** | (2R,3R,4S,5R,6R)-2-(hydroxymethyl)-5-methoxy-6-((1-(1-methylcyclopropyl)-1H-1,2,3-triazol-4-yl)methyl)-4-(4-(2,3,4-trifluorophenyl)-1H-1,2,3-triazol-1-yl)tetrahydro-2H-pyran-3-ol | 495.18 | 495.18 |
| **4.1.6.** | (2R,3R,4S,5R,6R)-4-(4-(4-bromo-2,3-difluorophenyl)-1H-1,2,3-triazol-1-yl)-2-(hydroxymethyl)-5-methoxy-6-((1-(3-methyloxetan-3-yl)-1H-1,2,3-triazol-4-yl)methyl)tetrahydro-2H-pyran-3-ol | 0.80 | 571.05 |
| **4.1.13.** | (2R,3R,4S,5R,6R)-4-(4-(4-bromo-2,3-difluorophenyl)-1H-1,2,3-triazol-1-yl)-2-(hydroxymethyl)-5-methoxy-6-((1-(1-methylcyclobutyl)-1H-1,2,3-triazol-4-yl)methyl)tetrahydro-2H-pyran-3-ol | 0.88 | 568.20 |
| **4.1.12.** | (2R,3R,4S,5R,6R)-6-((1-(bicyclo[1.1.1]pentan-1-yl)-1H-1,2,3-triazol-4-yl)methyl)-4-(4-(4-bromo-2,3-difluorophenyl)-1H-1,2,3-triazol-1-yl)-2-(hydroxymethyl)-5-methoxytetrahydro-2H-pyran-3-ol | 0.87 | 566.88 |
| **4.1.21.** | (2R,3R,4S,5R,6R)-4-(4-(4-bromo-2,3-difluorophenyl)-1H-1,2,3-triazol-1-yl)-2-(hydroxymethyl)-5-methoxy-6-((1-(1-(methoxymethyl)cyclobutyl)-1H-1,2,3-triazol-4-yl)methyl)tetrahydro-2H-pyran-3-ol | 0.87 | 566.88 |
| **4.1.4.** | (2R,3R,4S,5R,6R)-4-(4-(4-bromo-2,3-difluorophenyl)-1H-1,2,3-triazol-1-yl)-2-(hydroxymethyl)-6-((1-(1-(hydroxymethyl)cyclopropyl)-1H-1,2,3-triazol-4-yl)methyl)-5-methoxytetrahydro-2H-pyran-3-ol | 0.75 | 570.88 |
| **4.1.3.** | (2R,3R,4S,5R,6R)-4-(4-(4-bromo-2,3-difluorophenyl)-1H-1,2,3-triazol-1-yl)-6-((1-((1S,2S)-2-hydroxycyclopentyl)-1H-1,2,3-triazol-4-yl)methyl)-2-(hydroxymethyl)-5-methoxytetrahydro-2H-pyran-3-ol | 0.78 | 584.89 |
| **2.1.40.** | (2R,3R,4S,5R,6R)-4-(4-(4-chloro-2,3-difluorophenyl)-1H-1,2,3-triazol-1-yl)-6-((1-(1-(fluoromethyl)cyclobutyl)-1H-1,2,3-triazol-4-yl)methyl)-2-(hydroxymethyl)-5-methoxytetrahydro-2H-pyran-3-ol | 0.86 | 543.21 |
| **2.1.41.** | (2R,3R,4S,5R,6R)-4-(4-(4-chloro-2,3-difluorophenyl)-1H-1,2,3-triazol-1-yl)-2-(hydroxymethyl)-5-methoxy-6-((1-(3-(trifluoromethyl)cyclobutyl)-1H-1,2,3-triazol-4-yl)methyl)tetrahydro-2H-pyran-3-ol | 0.92 | 579.05 |
| **2.1.42.** | (2R,3R,4S,5R,6R)-4-(4-(4-chloro-2,3-difluorophenyl)-1H-1,2,3-triazol-1-yl)-2-(hydroxymethyl)-5-methoxy-6-((1-(3-methyltetrahydrofuran-3-yl)-1H-1,2,3-triazol-4-yl)methyl)tetrahydro-2H-pyran-3-ol | 0.8 | 541.22 |
| **2.1.43.** | (2R,3R,4S,5R,6R)-4-(4-(4-chloro-2,3-difluorophenyl)-1H-1,2,3-triazol-1-yl)-6-((1-(1-(difluoromethyl)cyclobutyl)-1H-1,2,3-triazol-4-yl)methyl)-2-(hydroxymethyl)-5-methoxytetrahydro-2H-pyran-3-ol | 0.88 | 561.25 |
| **2.1.44.** | (2R,3R,4S,5R,6R)-6-((1-(2-oxabicyclo[2.1.1]hexan-4-yl)-1H-1,2,3-triazol-4-yl)methyl)-4-(4-(4-chloro-2,3-difluorophenyl)-1H-1,2,3-triazol-1-yl)-2-(hydroxymethyl)-5-methoxytetrahydro-2H-pyran-3-ol | 0.79 | 539.12 |
| **2.1.46.** | (2R,3R,4S,5R,6R)-4-(4-(4-chloro-2,3-difluorophenyl)-1H-1,2,3-triazol-1-yl)-6-((1-(1-(fluoromethyl)cyclopropyl)-1H-1,2,3-triazol-4-yl)methyl)-2-(hydroxymethyl)-5-methoxytetrahydro-2H-pyran-3-ol | 0.82 | 528.91 |
| **3.1.26.** | (2R,3R,4S,5R,6R)-2-(hydroxymethyl)-5-methoxy-6-((1-(spiro[2.3]hexan-5-yl)-1H-1,2,3-triazol-4-yl)methyl)-4-(4-(2,3,4-trifluorophenyl)-1H-1,2,3-triazol-1-yl)tetrahydro-2H-pyran-3-ol | 0.87 | 521.24 |
| **3.1.14.** | (2R,3R,4S,5R,6R)-2-(hydroxymethyl)-5-methoxy-6-((1-(3-methylbicyclo[1.1.1]pentan-1-yl)-1H-1,2,3-triazol-4-yl)methyl)-4-(4-(2,3,4-trifluorophenyl)-1H-1,2,3-triazol-1-yl)tetrahydro-2H-pyran-3-ol | 0.88 | 521.23 |
| **3.1.13.** | (2R,3R,4S,5R,6R)-2-(hydroxymethyl)-5-methoxy-6-((1-(1-methylcyclobutyl)-1H-1,2,3-triazol-4-yl)methyl)-4-(4-(2,3,4-trifluorophenyl)-1H-1,2,3-triazol-1-yl)tetrahydro-2H-pyran-3-ol | 0.83 | 509.22 |
| **3.1.44.** | (2R,3R,4S,5R,6R)-6-((1-(2-oxabicyclo[2.1.1]hexan-4-yl)-1H-1,2,3-triazol-4-yl)methyl)-2-(hydroxymethyl)-5-methoxy-4-(4-(2,3,4-trifluorophenyl)-1H-1,2,3-triazol-1-yl)tetrahydro-2H-pyran-3-ol | 0.75 | 523.21 |
| **3.1.82.** | (2R,3R,4S,5R,6R)-2-(hydroxymethyl)-5-methoxy-6-((1-(spiro[3.3]heptan-1-yl)-1H-1,2,3-triazol-4-yl)methyl)-4-(4-(2,3,4-trifluorophenyl)-1H-1,2,3-triazol-1-yl)tetrahydro-2H-pyran-3-ol | 0.89 | 535.27 |
| **3.1.62.** | (2R,3R,4S,5R,6R)-2-(hydroxymethyl)-5-methoxy-6-((1-(1-methyl-2-oxabicyclo[3.1.1]heptan-5-yl)-1H-1,2,3-triazol-4-yl)methyl)-4-(4-(2,3,4-trifluorophenyl)-1H-1,2,3-triazol-1-yl)tetrahydro-2H-pyran-3-ol | 0.79 | 551.24 |
| **3.1.51.** | (2R,3R,4S,5R,6R)-6-((1-(1-(difluoromethyl)cyclopropyl)-1H-1,2,3-triazol-4-yl)methyl)-2-(hydroxymethyl)-5-methoxy-4-(4-(2,3,4-trifluorophenyl)-1H-1,2,3-triazol-1-yl)tetrahydro-2H-pyran-3-ol | 0.81 | 531.00 |
| **4.1.15.** | (2R,3R,4S,5R,6R)-4-(4-(4-bromo-2,3-difluorophenyl)-1H-1,2,3-triazol-1-yl)-6-((1-(3,3-difluoro-1-methylcyclobutyl)-1H-1,2,3-triazol-4-yl)methyl)-2-(hydroxymethyl)-5-methoxytetrahydro-2H-pyran-3-ol | 0.89 | 605.17 |
| **4.1.19.** | (2R,3R,4S,5R,6R)-4-(4-(4-bromo-2,3-difluorophenyl)-1H-1,2,3-triazol-1-yl)-6-((1-((1S,3S)-3-hydroxy-1-methylcyclobutyl)-1H-1,2,3-triazol-4-yl)methyl)-2-(hydroxymethyl)-5-methoxytetrahydro-2H-pyran-3-ol | 0.76 | 587.18 |
| **4.1.22.** | (2R,3R,4S,5R,6R)-4-(4-(4-bromo-2,3-difluorophenyl)-1H-1,2,3-triazol-1-yl)-6-((1-(3-(2-hydroxyethyl)oxetan-3-yl)-1 H-1,2,3-triazol-4-yl)methyl)-2-(hydroxymethyl)-5-methoxytetrahydro-2H-pyran-3-ol | 0.74 | 601.18 |
| **4.1.23** | (2R,3R,4S,5R,6R)-4-(4-(4-bromo-2,3-difluorophenyl)-1H-1,2,3-triazol-1-yl)-2-(hydroxymethyl)-5-methoxy-6-((1-(1-methylcyclopropyl)-1H-1,2,3-triazol-4-yl)methyl)tetrahydro-2H-pyran-3-ol | 0.84 | 557.15 |
| **4.1.44.** | (2R,3R,4S,5R,6R)-6-((1-(2-oxabicyclo[2.1.1]hexan-4-yl)-1H-1,2,3-triazol-4-yl)methyl)-4-(4-(4-bromo-2,3-difluorophenyl)-1H-1,2,3-triazol-1-yl)-2-(hydroxymethyl)-5-methoxytetrahydro-2H-pyran-3-ol | 0.80 | 583.06 |
| **4.1.24.** | (2R,3R,4S,5R,6R)-4-(4-(4-bromo-2,3-difluorophenyl)-1H-1,2,3-triazol-1-yl)-2-(hydroxymethyl)-5-methoxy-6-((1-(3-(trifluoromethyl)bicyclo[1.1.1]pentan-1-yl)-1H-1,2,3-triazol-4-yl)methyl)tetrahydro-2H-pyran-3-ol | 0.89 | 605.21 |
| **4.1.29.** | (2R,3R,4S,5R,6R)-4-(4-(4-bromo-2,3-difluorophenyl)-1H-1,2,3-triazol-1-yl)-2-(hydroxymethyl)-5-methoxy-6-((1-(3-methoxybicyclo[1.1.1]pentan-1-yl)-1H-1,2,3-triazol-4-yl)methyl)tetrahydro-2H-pyran-3-ol | 0.86 | 597.08 |

| | | | |
|---|---|---|---|
| *An additional purification on chiral preparative HPLC/MS(I) has been performed. **An additional purification on chiral preparative HPLC/MS(II) has been performed. ***An additional purification on chiral preparative HPLC/MS(III) has been performed. ****An additional purification on chiral preparative HPLC/MS(IV) has been performed. | | | |

### Example 2.1.65.A

### (2R,3R,4S,5R,6R)-4-(4-(4-Chloro-2,3-difluorophenyl)-1H-1,2,3-triazol-1-yl)-6-((1-((3R,4R)-4-fluorotetrahydro-2H-pyran-3-yl)-1H-1,2,3-triazol-4-yl)methyl)-2-(hydroxymethyl)-5-methoxytetrahydro-2H-pyran-3-ol

### 1. 4-(4-Chloro-2,3-difluorophenyl)-1-((4aR,6R,7R,8R,8aR)-6-((1-((3R,4R)-4-fluorotetrahydro-2H-pyran-3-yl)-1H-1,2,3-triazol-4-yl)methyl)-7-methoxy-2,2-dimethylhexahydropyrano[3,2-d][1,3]dioxin-8-yl)-1H-1,2,3-triazole

To a solution of (3R,4R)-4-fluorooxan-3-amine hydrochloride (0.034 g, 0.22 mmol, 2.0 eq ) and DBU (0.049 mL, 0.33 mmol, 3.0 eq) in MeCN (2.5 mL) is added ADMP (0.066 g, 0.22 mmol, 2.0 eq) and the reaction mixture is stirred at rt for 17 h. This solution is then added to a suspension of Intermediate 8 (50 mg, 0.11 mmol, 1.0 eq), Copper (35 mg, 0.551 mmol, 5.0 eq), AcOH (0.12 mL, 2.2 mmol, 20 eq) and aq. sat CuSO₄ (0.203 mL, 1.1 mmol, 10 eq) in THF (2.0 ml) and stirred at rt for 1.0 h. Copper (0.035 g, 0.551 mmol, 5.0 eq) is added again and the reaction mixture is stirred at rt over 17 h. The reaction mixture is filtered, the filtrate is extracted with EA and the phases are separated. The organic phase is washed with aq sat NaCl, dried over MgSO₄, filtered and the solvent is removed in vacuo to recover the crude as an oil. Purification over preparative HPLC(I) yields the desired product as a white solid (0.012 g, 19%). LCMS (A): t_{R} =0.99 min; [M+H]⁺ = 599.05.

### 2. (2R,3R,4S,5R,6R)-4-(4-(4-Chloro-2,3-difluorophenyl)-1H-1,2,3-triazol-1-yl)-6-((1-((3R,4R)-4-fluorotetrahydro-2H-pyran-3-yl)-1H-1,2,3-triazol-4-yl)methyl)-2-(hydroxymethyl)-5-methoxytetrahydro-2H-pyran-3-ol (Example 2.1.65.A)

To a solution of Example 2.1.65.A *Step 1*. (0.012 g, 0.021 mmol, 1.0 eq) in dioxane (1.0 mL) is added water (0.5 mL), followed by TFA (0.023 mL, 0.417 mmol, 20 eq) and it is stirred at rt for 17 h. The reaction mixture is quenched with 25% NH₄OH (pH = 10) and directly purified over preparatory HPLC(I) to recover the desired product as a white solid (0.008 g, 66%). LCMS (A): t_{R} = 0.82 min; [M+H]⁺ = 559.

¹H NMR (400 MHz, MeOD) *δ*: 8.51 (d, *J* = 3.0 Hz, 1 H), 8.19 (s, 1H), 7.95 (t, *J* = 7.8 Hz, 1 H), 7.44 (t, *J* = 8.5 Hz, 1 H), 5.1-5.30 (m, 2 H), 4.62-4.79 (m, 2 H), 4.51 (dd, *J*₁ = 6.0 Hz, *J*₂ = 11.5 Hz), 4.18-4.27 (m, 1 H), 4.16 (s), 4.04-4.13 (m, 2 H), 3.78-3.88 (m, 1 H), 3.6-3.75 (m, 3 H), 3.41 (dd, *J*₁ = 12.0 Hz, *J*₂ = 16.0 Hz), 3.14 (dd, *J*₁ = 3.3 Hz, *J*₂ = 16.5 Hz), 2.25-2.35 (m, 1 H), 1.95-2.05 (m, 1 H).

Following examples are prepared starting from Intermediate 8, 9a, 10a or 11a and the corresponding amines in analogy to the procedures described for Example 2.1.65.A.. *Step 2.* is performed either with TFA, as described or with AcOH, as described for Example 3.1.7. *Step 2..* Selected examples, synthesized with chiral amines, have yielded mixtures of diastereomers, that are separated by chiral preparatory HPLC.

LC-MS data are listed in Table 2 below. LC-MS conditions are LC-MS (A). Chiral analytical HPLC (I) (conditions and retention time) of the diastereomers of selected Examples are also listed.

**Table 2**

| **Example** | **Name** | **t_{R} [min]** | **[M+H]⁺** | **Chiral HPLC conditions** | **t_{R} chiral [min]** |
|---|---|---|---|---|---|
| **2.1.65.A** | (2R,3R,4S,5R,6R)-4-(4-(4-chloro-2,3-difluorophenyl)-1H-1,2,3-triazol-1-yl)-6-((1-((3R,4R)-4-fluorotetrahydro-2H-pyran-3-yl)-1H-1,2,3-triazol-4-yl)methyl)-2-(hydroxymethyl)-5-methoxytetrahydro-2H-pyran-3-ol | 0.82 | 559 | Chiralpak IB B: 30% MeCN/MeOH (1:1) | 2.23 |
| | | | | 5 min run | |
| **2.1.52.** | (2R,3R,4S,5R,6R)-4-(4-(4-chloro-2,3-difluorophenyl)-1H-1,2,3-triazol-1-yl)-6-((1-(3-(fluoromethyl)oxetan-3-yl)-1H-1,2,3-triazol-4-yl)methyl)-2-(hydroxymethyl)-5-methoxytetrahydro-2H-pyran-3-ol [1-(1,2,3-tri-deoxy-2-methoxy-3-[4-(4-chloro-2,3-difluorophenyl)-1H-1,2,3-triazol-1-yl]-α-D-galacto-pyranose)-1-(1-(3-fluoromethyl-3-oxetanyl)-1H-triazol-4-yl)-methane] | 0.79 | 544.82 | | |
| **2.1.53.** | (2R,3R,4S,5R,6R)-4-(4-(4-chloro-2,3-difluorophenyl)-1H-1,2,3-triazol-1-yl)-6-((1-(3-(difluoromethyl)oxetan-3-yl)-1H-1,2,3-triazol-4-yl)methyl)-2-(hydroxymethyl)-5-methoxytetrahydro-2H-pyran-3-ol [1-(1,2,3-tri-deoxy-2-methoxy-3-[4-(4-chloro-2,3-difluorophenyl)-1H-1,2,3-triazol-1-yl]-α-D-galacto-pyranose)-1-(1-(3-difluoromethyl-3-oxetanyl)-1H-triazol-4-yl)-methane] | 0.82 | 562.95 | | |
| **2.1.58.** | (2R,3R,4S,5R,6R)-4-(4-(4-chloro-2,3-difluorophenyl)-1H-1,2,3-triazol-1-yl)-2-(hydroxymethyl)-5-methoxy-6-((1-(3-(trifluoromethyl)oxetan-3-yl)-1H-1,2,3-triazol-4-yl)methyl)tetrahydro-2H-pyran-3-ol [1-(1,2,3-tri-deoxy-2-methoxy-3-[4-(4-chloro-2,3-difluorophenyl)-1H-1,2,3-triazol-1-yl]-α-D-galacto-pyranose)-1-(1-(3-trifluoromethyl-3-oxetanyl)-1H-triazol-4-yl)-methane] | 0.87 | 581.13 | | |
| **2.1.59.** | (2R,3R,4S,5R,6R)-4-(4-(4-chloro-2,3-difluorophenyl)-1H-1,2,3-triazol-1-yl)-2-(hydroxymethyl)-5-methoxy-6-((1-(1-methyl-2-oxabicyclo[2.1.1]hexan-4-yl)-1 H-1,2,3-triazol-4-yl)methyl)tetrahydro-2H-pyran-3-ol | 0.82 | 552.83 | | |
| **2.1.62.** | (2R,3R,4S,5R,6R)-4-(4-(4-chloro-2,3-difluorophenyl)-1H-1,2,3-triazol-1-yl)-2-(hydroxymethyl)-5-methoxy-6-((1-(1-methyl-2-oxabicyclo[3.1.1]heptan-5-yl)-1H-1,2,3-triazol-4-yl)methyl)tetrahydro-2H-pyran-3-ol | 0.83 | 567 | | |
| **2.1.63.A** | (2R,3R,4S,5R,6R)-4-(4-(4-chloro-2,3-difluorophenyl)-1H-1,2,3-triazol-1-yl)-6-((1-(2,2-difluorocyclobutyl)-1H-1,2,3-triazol-4-yl)methyl)-2-(hydroxymethyl)-5-methoxytetrahydro-2H-pyran-3-ol | .87 | 547.07 | Chiralpak IG B: 40% MeCN/ MeOH (1:1) | 1.9 |
| **2.1.63.B** | | | | | 2.77 |
| | | | | 5 min run | |
| **2.1.64.A** | (2R,3R,4S,5R,6R)-4-(4-(4-chloro-2,3-difluorophenyl)-1H-1,2,3-triazol-1-yl)-6-((1-((R)-2,2-difluorocyclopentyl)-1H-1,2,3-triazol-4-yl)methyl)-2-(hydroxymethyl)-5-methoxytetrahydro-2H-pyran-3-ol | 0.88 | 560.88 | Chiralpak IG B: 40% MeCN/MeOH (1:1) 5 min run | 2.12 |
| **2.1.64.B** | (2R,3R,4S,5R,6R)-4-(4-(4-chloro-2,3-difluorophenyl)-1H-1,2,3-triazol-1-yl)-6-((1-((S)-2,2-difluorocyclopentyl)-1H-1,2,3-triazol-4-yl)methyl)-2-(hydroxymethyl)-5-methoxytetrahydro-2H-pyran-3-ol | 0.88 | 560.89 | Chiralpak IG B: 40% MeCN/MeOH (1:1) 5 min run | 2.28 |
| **2.1.66.C** | (2R,3R,4S,5R,6R)-4-(4-(4-chloro-2,3-difluorophenyl)-1H-1,2,3-triazol-1-yl)-6-((1-((3S,4R)-3-fluorotetrahydro-2H-pyran-4-yl)-1H-1,2,3-triazol-4-yl)methyl)-2-(hydroxymethyl)-5-methoxytetrahydro-2H-pyran-3-ol [1-(1,2,3-tri-deoxy-2-methoxy-3-[4-(4-chloro-2,3-difluorophenyl)-1H-1,2,3-triazol-1-yl]-α-D-galacto-pyranose)-1-(1-((3S,4R)-3-fluorotetrahydropyran-4-yl)-1H-triazol-4-yl)-methane] | 0.81 | 560.88 | Chiralpak ID B: 30% MeCN/MeOH (1:1) 5 min run | 4.17 |
| **2.1.66.D** | (2R,3R,4S,5R,6R)-4-(4-(4-chloro-2,3-difluorophenyl)-1H-1,2,3-triazol-1-yl)-6-((1-((3R,4S)-3-fluorotetrahydro-2H-pyran-4-yl)-1H-1,2,3-triazol-4-yl)methyl)-2-(hydroxymethyl)-5-methoxytetrahydro-2H-pyran-3-ol [1-(1,2,3-tri-deoxy-2-methoxy-3-[4-(4-chloro-2,3-difluorophenyl)-1H-1,2,3-triazol-1-yl]-α-D-galacto-pyranose)-1-(1-((3R,4S)-3-fluoro-tetrahydropyran-4-yl)-1H-triazol-4-yl)-methane] | 0.82 | 560.88 | Chiralpak ID B: 30% MeCN/MeOH (1:1) 5 min run | 3.47 |
| **2.1.68.A** | (2R,3R,4S,5R,6R)-4-(4-(4-chloro-2,3-difluorophenyl)-1H-1,2,3-triazol-1-yl)-6-((1-((2R*,3R*)-2,3-dimethyltetrahydrofuran-3-yl)-1 H-1,2,3-triazol-4-yl)methyl)-2-(hydroxymethyl)-5-methoxytetrahydro-2H-pyran-3-ol | 0.84 | 554.8 | Chiralpak IA B: 30% MeCN/MeOH (1:1) gradient 0.5 to 60% in 2 min 5 min run | 3.1 |
| **2.1.68.B** | | | | | 3.24 |
| **2.1.71.** | (2R,3R,4S,5R,6R)-4-(4-(4-chloro-2,3-difluorophenyl)-1H-1,2,3-triazol-1-yl)-6-((1-(4,4-difluoro-1-(trifluoromethyl)cyclohexyl)-1H-1,2,3-triazol-4-yl)methyl)-2-(hydroxymethyl)-5-methoxytetrahydro-2H-pyran-3-ol | 1.0 | 643.11 | | |
| **2.1.72.** | (2R,3R,4S,5R,6R)-4-(4-(4-chloro-2,3-difluorophenyl)-1H-1,2,3-triazol-1-yl)-2-(hydroxymethyl)-5-methoxy-6-((1-(4-(trifluoromethyl)tetrahydro-2H-pyran-4-yl)-1H-1,2,3-triazol-4-yl)methyl)tetrahydro-2H-pyran-3-ol | 1.0 | 611.11 | | |
| **2.1.74.A** | (2R,3R,4S,5R,6R)-4-(4-(4-chloro-2,3-difluorophenyl)-1H-1,2,3-triazol-1-yl)-6-((1-((3R*,4R*)-4-fluorotetrahydrofuran-3-yl)-1H-1,2,3-triazol-4-yl)methyl)-2-(hydroxymethyl)-5-methoxytetrahydro-2H-pyran-3-ol [1-(1,2,3-tri-deoxy-2-methoxy-3-[4-(4-chloro-2,3-difluorophenyl)-1H-1,2,3-triazol-1-yl]-α-D-galacto-pyranose)-1-(1-((3R,4R)-3-fluoro-tetrahydrofuran-4-yl)-1H-triazol-4-yl)-methane] and /or [1-(1,2,3-tri-deoxy-2-methoxy-3-[4-(4-chloro-2,3-difluorophenyl)-1H-1,2,3-triazol-1-yl]-α-D-galacto-pyranose)-1-(1-((3S,4S)-3-fluoro-tetrahydrofuran-4-yl)-1H-triazol-4-yl)-methane] | 0.79 | 545.05 | Chiralpak ID B: 35% MeCN/MeOH (1:1) | 1.97 |
| **2.1.74.B** | | | | | 2.45 |
| | | | | 5 min run | |
| **2.1.74.C** | (2R,3R,4S,5R,6R)-4-(4-(4-chloro-2,3-difluorophenyl)-1H-1,2,3-triazol-1-yl)-6-((1-((3R*,4S*)-4-fluorotetrahydrofuran-3-yl)-1H-1,2,3-triazol-4-yl)methyl)-2-(hydroxymethyl)-5-methoxytetrahydro-2H-pyran-3-ol | 0.79 | 545.24 | Chiralpak AY-H B:35% MeCN/MeOH (1:1) | 2.15 |
| **2.1.74.D** | | | | | 3.83 |
| | | | | 5 min run | |
| **2.1.75.** | (2R,3R,4S,5R,6R)-4-(4-(4-chloro-2,3-difluorophenyl)-1H-1,2,3-triazol-1-yl)-6-((1-(4,4-difluorospiro[2.2]pentan-1-yl)-1H-1,2,3-triazol-4-yl)methyl)-2-(hydroxymethyl)-5-methoxytetrahydro-2H-pyran-3-ol | 0.9 | 559.22 | | |
| **2.1.76.** | (2R,3R,4S,5R,6R)-4-(4-(4-chloro-2,3-difluorophenyl)-1H-1,2,3-triazol-1-yl)-6-((1-(2,2-difluoro-1-(fluoromethyl)cyclopropyl)-1H-1,2,3-triazol-4-yl)methyl)-2-(hydroxymethyl)-5-methoxytetrahydro-2H-pyran-3-ol | 0.88 | 565.22 | | |
| **2.1.77.A** | (2R,3R,4S,5R,6R)-4-(4-(4-chloro-2,3-difluorophenyl)-1H-1,2,3-triazol-1-yl)-2-(hydroxymethyl)-5-methoxy-6-((1-(3-(trifluoromethyl)tetrahydrofuran-3-yl)-1H-1,2,3-triazol-4-yl)methyl)tetrahydro-2H-pyran-3-ol | 0.9 | 596.13 | Chiralpak IE A: 30% Hept | 6.47 |
| **2.1.77.B** | | | | B: 70% EtOH | 8.64 |
| | | | | 5 min run | |
| **2.1.78.A** | (2R,3R,4S,5R,6R)-4-(4-(4-chloro-2,3-difluorophenyl)-1H-1,2,3-triazol-1-yl)-6-((1-(3-(difluoromethyl)tetrahydrofuran-3-yl)-1H-1,2,3-triazol-4-yl)methyl)-2-(hydroxymethyl)-5-methoxytetrahydro-2H-pyran-3-ol | 0.85 | 577.26 | Chiralpak ID B: 30% MeCN/MeOH (1:1) | 2.24 |
| **2.1.78.B** | | | | | 3.1 |
| | | | | 5 min run | |
| **2.1.79.A** | (2R,3R,4S,5R,6R)-4-(4-(4-chloro-2,3-difluorophenyl)-1H-1,2,3-triazol-1-yl)-6-((1-((1R,2S)-2-fluorocyclopentyl)-1H-1,2,3-triazol-4-yl)methyl)-2-(hydroxymethyl)-5-methoxytetrahydro-2H-pyran-3-ol | 0.87 | 543.27 | Chiralpak IG B: 45% EtOH | 3.08 |
| | | | | 5 min run | |
| **2.1.79.B** | (2R,3R,4S,5R,6R)-4-(4-(4-chloro-2,3-difluorophenyl)-1H-1,2,3-triazol-1-yl)-6-((1-((1S,2R)-2-fluorocyclopentyl)-1H-1,2,3-triazol-4-yl)methyl)-2-(hydroxymethyl)-5-methoxytetrahydro-2H-pyran-3-ol | 0.87 | 543.27 | Chiralpak IG B: 45% EtOH | 4.09 |
| | | | | 5 min run | |
| **2.1.79.CD** | (2R,3R,4S,5R,6R)-4-(4-(4-chloro-2,3-difluorophenyl)-1H-1,2,3-triazol-1-yl)-6-((1-((1R*,2R*)-2-fluorocyclopentyl)-1H-1,2,3-triazol-4-yl)methyl)-2-(hydroxymethyl)-5-methoxytetrahydro-2H-pyran-3-ol | 0.87 | 543.01 | | |
| **2.1.80.A** | (2R,3R,4S,5R,6R)-4-(4-(4-chloro-2,3-difluorophenyl)-1H-1,2,3-triazol-1-yl)-6-((1-(2,2-difluorocyclohexyl)-1H-1,2,3-triazol-4-yl)methyl)-2-(hydroxymethyl)-5-methoxytetrahydro-2H-pyran-3-ol | 0.92 | 575.21 | Chiralpak ID B: 40% MeCN/MeOH (1:1) | 2.49 |
| **2.1.80.B** | | | | | 3.44 |
| | | | | 5 min run | |
| **2.1.81.B** | (2R,3R,4S,5R,6R)-4-(4-(4-chloro-2,3-difluorophenyl)-1H-1,2,3-triazol-1-yl)-6-((1-((1S,2R)-2-fluorocyclohexyl)-1H-1,2,3-triazol-4-yl)methyl)-2-(hydroxymethyl)-5-methoxytetrahydro-2H-pyran-3-ol | 0.9 | 557.27 | Chiralpak IC B:40% MeCN/MeOH (1:1) | 3.47 |
| | | | | 5 min run | |
| **2.1.81.C** | (2R,3R,4S,5R,6R)-4-(4-(4-chloro-2,3-difluorophenyl)-1H-1,2,3-triazol-1-yl)-6-((1-((1R,2R)-2-fluorocyclohexyl)-1H-1,2,3-triazol-4-yl)methyl)-2-(hydroxymethyl)-5-methoxytetrahydro-2H-pyran-3-ol | 0.9 | 557.2 | Chiralpak IC B:40% MeCN/MeOH (1:1) | 3.1 |
| | | | | 5 min run | |
| **2.1.81.D** | (2R,3R,4S,5R,6R)-4-(4-(4-chloro-2,3-difluorophenyl)-1H-1,2,3-triazol-1-yl)-6-((1-((1S,2S)-2-fluorocyclohexyl)-1H-1,2,3-triazol-4-yl)methyl)-2-(hydroxymethyl)-5-methoxytetrahydro-2H-pyran-3-ol | 0.9 | 557.27 | Chiralpak IC B:40% MeCN/MeOH (1:1) | 4.05 |
| | | | | 5 min run | |
| **2.1.88.A** | (2R,3R,4S,5R,6R)-4-(4-(4-chloro-2,3-difluorophenyl)-1H-1,2,3-triazol-1-yl)-6-((1-((1R*,2S*)-2-fluorocyclobutyl)-1H-1,2,3-triazol-4-yl)methyl)-2-(hydroxymethyl)-5-methoxytetrahydro-2H-pyran-3-ol | 0.86 | 530.27 | Chiralpak IC B: 40% MeOH | 4.16 |
| **2.1.88.B** | | | | 5 min run | 3.65 |
| **2.1.88.C** | (2R,3R,4S,5R,6R)-4-(4-(4-chloro-2,3-difluorophenyl)-1H-1,2,3-triazol-1-yl)-6-((1-((1R*,2R*)-2-fluorocyclobutyl)-1H-1,2,3-triazol-4-yl)methyl)-2-(hydroxymethyl)-5-methoxytetrahydro-2H-pyran-3-ol | 0.86 | 530.14 | Chiralpak IC B: 40% MeOH | 2.95 |
| **2.1.88.D** | | | | 5 min run | 2.93 |
| **2.1.89.A** | (2R,3R,4S,5R,6R)-4-(4-(4-chloro-2,3-difluorophenyl)-1H-1,2,3-triazol-1-yl)-6-((1-((RS)-3,3-difluorotetrahydro-2H-pyran-4-yl)-1H-1,2,3-triazol-4-yl)methyl)-2-(hydroxymethyl)-5-methoxytetrahydro-2H-pyran-3-ol [1-(1,2,3-tri-deoxy-2-methoxy-3-[4-(4-chloro-2,3-difluorophenyl)-1H-1,2,3-triazol-1-yl]-α-D-galacto-pyranose)-1-(1-((4R)-3,3-difluoro-tetrahydropyran-4-yl)-1H-triazol-4-yl)-methane] and / or [1-(1,2,3-tri-deoxy-2-methoxy-3-[4-(4-chloro-2,3-difluorophenyl)-1H-1,2,3-triazol-1-yl]-α-D-galacto-pyranose)-1-(1-((4S)-3,3-difluoro-tetrahydropyran-4-yl)-1H-triazol-4-yl)-methane] | 0.85 | 579.23 | Chiralpak IG B: 40% EtOH | 2.65 |
| **2.1.89.B** | | | | 5 min run | 3.39 |
| **2.1.90.A** | (2R,3R,4S,5R,6R)-4-(4-(4-chloro-2,3-difluorophenyl)-1H-1,2,3-triazol-1-yl)-6-((1-((RS)-4,4-difluorotetrahydrofuran-3-yl)-1H-1,2,3-triazol-4-yl)methyl)-2-(hydroxymethyl)-5-methoxytetrahydro-2H-pyran-3-ol [1-(1,2,3-tri-deoxy-2-methoxy-3-[4-(4-chloro-2,3-difluorophenyl)-1H-1,2,3-triazol-1-yl]-α-D-galacto-pyranose)-1-(1-((3R)-4,4-difluorotetrahydrofuran-3-yl)-1H-triazol-4-yl)-methane] and /or [1-(1,2,3-tri-deoxy-2-methoxy-3-[4-(4-chloro-2,3-difluorophenyl)-1H-1,2,3-triazol-1-yl]-α-D-galacto-pyranose)-1-(1-((3S)-4,4-difluoro-tetrahydrofuran-3-yl)-1H-triazol-4-yl)-methane] | 0.85 | 563.22 | Chiralpak IG B: 40% MeCN/MeOH (1:1) 5 min run | 2.25 |
| **2.1.90.B** | | | | | 2.95 |
| **3.1.6.** | (2R,3R,4S,5R,6R)-2-(hydroxymethyl)-5-methoxy-6-((1-(3-methyloxetan-3-yl)-1H-1,2,3-triazol-4-yl)methyl)-4-(4-(2,3,4-trifluorophenyl)-1H-1,2,3-triazol-1-yl)tetrahydro-2H-pyran-3-ol | 0.74 | 511.03 | | |
| **3.1.46.** | (2R,3R,4S,5R,6R)-6-((1-(1-(fluoromethyl)cyclopropyl)-1H-1,2,3-triazol-4-yl)methyl)-2-(hydroxymethyl)-5-methoxy-4-(4-(2,3,4-trifluorophenyl)-1H-1,2,3-triazol-1-yl)tetrahydro-2H-pyran-3-ol | 0.78 | 512.92 | | |
| **3.1.43.** | (2R,3R,4S,5R,6R)-6-((1-(1-(difluoromethyl)cyclobutyl)-1H-1,2,3-triazol-4-yl)methyl)-2-(hydroxymethyl)-5-methoxy-4-(4-(2,3,4-trifluorophenyl)-1H-1,2,3-triazol-1-yl)tetrahydro-2H-pyran-3-ol | 0.85 | 544.79 | | |
| **3.1.28.** | (2R,3R,4S,5R,6R)-6-((1-(1,1-difluoro-2-methylpropan-2-yl)-1H-1,2,3-triazol-4-yl)methyl)-2-(hydroxymethyl)-5-methoxy-4-(4-(2,3,4-trifluorophenyl)-1H-1,2,3-triazol-1-yl)tetrahydro-2H-pyran-3-ol | 0.84 | 532.87 | | |
| **3.1.40.** | (2R,3R,4S,5R,6R)-6-((1-(1-(fluoromethyl)cyclobutyl)-1H-1,2,3-triazol-4-yl)methyl)-2-(hydroxymethyl)-5-methoxy-4-(4-(2,3,4-trifluorophenyl)-1H-1,2,3-triazol-1-yl)tetrahydro-2H-pyran-3-ol | 0.81 | 527.02 | | |
| **3.1.55.** | (2R,3R,4S,5R,6R)-2-(hydroxymethyl)-5-methoxy-6-((1-(1-(trifluoromethyl)cyclobutyl)-1H-1,2,3-triazol-4-yl)methyl)-4-(4-(2,3,4-trifluorophenyl)-1H-1,2,3-triazol-1-yl)tetrahydro-2H-pyran-3-ol | 0.88 | 563.00 | | |
| **3.1.58.** | (2R,3R,4S,5R,6R)-2-(hydroxymethyl)-5-methoxy-6-((1-(3-(trifluoromethyl)oxetan-3-yl)-1H-1,2,3-triazol-4-yl)methyl)-4-(4-(2,3,4-trifluorophenyl)-1H-1,2,3-triazol-1-yl)tetrahydro-2H-pyran-3-ol | 0.83 | 565.00 | | |
| **3.1.66.C** | (2R,3R,4S,5R,6R)-6-((1-((3S*,4R*)-3-fluorotetrahydro-2H-pyran-4-yl)-1H-1,2,3-triazol-4-yl)methyl)-2-(hydroxymethyl)-5-methoxy-4-(4-(2,3,4-trifluorophenyl)-1H-1,2,3-triazol-1-yl)tetrahydro-2H-pyran-3-ol [1-(1,2,3-tri-deoxy-2-methoxy-3-[4-(2,3,4-trifluorophenyl)-1H-1,2,3-triazol-1-yl]-α-D-galacto-pyranose)-1-(1-((3S,4R)-3-fluoro-tetrahydropyran-4-yl)-1H-triazol-4-yl)-methane] and / or [1-(1,2,3-tri-deoxy-2-methoxy-3-[4-(2,3,4-trifluorophenyl)-1H-1,2,3-triazol-1-yl]-α-D-galacto-pyranose)-1-(1-((3R,4S)-3-fluoro-tetrahydropyran-4-yl)-1H-triazol-4-yl)-methane] | 0.79 | 543.17 | Chiralpak AZ-H B:45% MeCN/MeOH (1:1) | 1.87 |
| **3.1.66.D** | | | | | 2.92 |
| | | | | 5 min run | |
| **4.1.46.** | (2R,3R,4S,5R,6R)-4-(4-(4-bromo-2,3-difluorophenyl)-1H-1,2,3-triazol-1-yl)-6-((1-(1-(fluoromethyl)cyclopropyl)-1H-1,2,3-triazol-4-yl)methyl)-2-(hydroxymethyl)-5-methoxytetrahydro-2H-pyran-3-ol | 0.83 | 572.99 | | |
| **4.1.59.** | (2R,3R,4S,5R,6R)-4-(4-(4-bromo-2,3-difluorophenyl)-1H-1,2,3-triazol-1-yl)-2-(hydroxymethyl)-5-methoxy-6-((1-(1-methyl-2-oxabicyclo[2.1.1]hexan-4-yl)-1 H-1,2,3-triazol-4-yl)methyl)tetrahydro-2H-pyran-3-ol | 0.83 | 596.77 | | |
| **4.1.52.** | (2R,3R,4S,5R,6R)-4-(4-(4-bromo-2,3-difluorophenyl)-1H-1,2,3-triazol-1-yl)-6-((1-(3-(fluoromethyl)oxetan-3-yl)-1H-1,2,3-triazol-4-yl)methyl)-2-(hydroxymethyl)-5-methoxytetrahydro-2H-pyran-3-ol | 0.81 | 589.07 | | |
| **4.1.62.** | (2R,3R,4S,5R,6R)-4-(4-(4-bromo-2,3-difluorophenyl)-1H-1,2,3-triazol-1-yl)-2-(hydroxymethyl)-5-methoxy-6-((1-(1-methyl-2-oxabicyclo[3.1.1]heptan-5-yl)-1H-1,2,3-triazol-4-yl)methyl)tetrahydro-2H-pyran-3-ol | 0.84 | 610.96 | | |
| **4.1.25.** | (2R,3R,4S,5R,6R)-4-(4-(4-bromo-2,3-difluorophenyl)-1H-1,2,3-triazol-1-yl)-6-((1-(3-fluorobicyclo[1.1.1]pentan-1-yl)-1H-1,2,3-triazol-4-yl)methyl)-2-(hydroxymethyl)-5-methoxytetrahydro-2H-pyran-3-ol | 0.88 | 584.93 | | |
| **4.1.55.** | (2R,3R,4S,5R,6R)-4-(4-(4-bromo-2,3-difluorophenyl)-1H-1,2,3-triazol-1-yl)-2-(hydroxymethyl)-5-methoxy-6-((1-(1-(trifluoromethyl)cyclobutyl)-1H-1,2,3-triazol-4-yl)methyl)tetrahydro-2H-pyran-3-ol | 0.95 | 622.95 | | |
| **4.1.40.** | (2R,3R,4S,5R,6R)-4-(4-(4-bromo-2,3-difluorophenyl)-1H-1,2,3-triazol-1-yl)-6-((1-(1-(fluoromethyl)cyclobutyl)-1H-1,2,3-triazol-4-yl)methyl)-2-(hydroxymethyl)-5-methoxytetrahydro-2H-pyran-3-ol | 0.86 | 586.97 | | |
| **4.1.64.A** | (2R,3R,4S,5R,6R)-4-(4-(4-bromo-2,3-difluorophenyl)-1H-1,2,3-triazol-1-yl)-6-((1-((R)-2,2-difluorocyclopentyl)-1H-1,2,3-triazol-4-yl)methyl)-2-(hydroxymethyl)-5-methoxytetrahydro-2H-pyran-3-ol | 0.88 | 604.98 | Chiralpak IG B: 40% MeCN/MeOH (1:1) | 2.35 |
| | | | | 5 min run | |
| **4.1.64.B** | (2R,3R,4S,5R,6R)-4-(4-(4-bromo-2,3-difluorophenyl)-1H-1,2,3-triazol-1-yl)-6-((1-((S)-2,2-difluorocyclopentyl)-1H-1,2,3-triazol-4-yl)methyl)-2-(hydroxymethyl)-5-methoxytetrahydro-2H-pyran-3-ol | 0.88 | 605 | Chiralpak IG B: 40% MeCN/MeOH (1:1) | 3.12 |
| | | | | 5 min run | |
| **4.1.14.** | (2R,3R,4S,5R,6R)-4-(4-(4-bromo-2,3-difluorophenyl)-1H-1,2,3-triazol-1-yl)-2-(hydroxymethyl)-5-methoxy-6-((1-(3-methylbicyclo[1.1.1]pentan-1-yl)-1H-1,2,3-triazol-4-yl)methyl)tetrahydro-2H-pyran-3-ol | 0.92 | 580.99 | | |
| **4.1.79.CD** | (2R,3R,4S,5R,6R)-4-(4-(4-bromo-2,3-difluorophenyl)-1H-1,2,3-triazol-1-yl)-6-((1-((1S*,2S*)-2-fluorocyclopentyl)-1H-1,2,3-triazol-4-yl)methyl)-2-(hydroxymethyl)-5-methoxytetrahydro-2H-pyran-3-ol | 0.88 | 586.97 | | |
| **4.1.63.AB** | (2R,3R,4S,5R,6R)-4-(4-(4-bromo-2,3-difluorophenyl)-1H-1,2,3-triazol-1-yl)-6-((1-(2,2-difluorocyclobutyl)-1H-1,2,3-triazol-4-yl)methyl)-2-(hydroxymethyl)-5-methoxytetrahydro-2H-pyran-3-ol | 0.87 | 590.94 | | |
| **4.1.88.AB** | (2R,3R,4S,5R,6R)-4-(4-(4-bromo-2,3-difluorophenyl)-1H-1,2,3-triazol-1-yl)-6-((1-((1R*,2S*)-2-fluorocyclobutyl)-1H-1,2,3-triazol-4-yl)methyl)-2-(hydroxymethyl)-5-methoxytetrahydro-2H-pyran-3-ol | 0.84 | 572.96 | | |
| **1.1.53** | (2R,3R,4S,5R,6R)-4-(4-(2,3-difluoro-4-methylphenyl)-1H-1,2,3-triazol-1-yl)-6-((1-(3-(difluoromethyl)oxetan-3-yl)-1H-1,2,3-triazol-4-yl)methyl)-2-(hydroxymethyl)-5-methoxytetrahydro-2H-pyran-3-ol | 0.8 | 543.23 | | |

### Example 2.1.45.

### (2R,3R,4S,5R,6R)-4-(4-(4-Chloro-2,3-difluorophenyl)-1H-1,2,3-triazol-1-yl)-6-((1-(1-fluoro-2-methylpropan-2-yl)-1H-1,2,3-triazol-4-yl)methyl)-2-(hydroxymethyl)-5-methoxytetrahydro-2H-pyran-3-ol

### 1. 4-(4-Chloro-2,3-difluorophenyl)-1-((4aR,6R,7R,8R,8aR)-6-((1-(1-fluoro-2-methylpropan-2-yl)-1H-1,2,3-triazol-4-yl)methyl)-7-methoxy-2,2-dimethylhexahydropyrano[3,2-d][1,3]dioxin-8-yl)-1H-1,2,3-triazole

To a solution of 1-fluoro-2-methylpropan-2-amine hydrochloride (0.1 g, 0.745 mmol) in MeOH (1.0 mL) are added K₂CO₃ (0.210g, 1.49 mmol, 2.0 eq), Copper(II) sulfate pentahydrate (0.02 g, 0.074 mmol, 0.1 eq), 1H-imidazole-1-sulfonyl azide hydrochloride (0.2 g, 0.893 mmol, 1.2 eq) and the reaction mixture is stirred for 17 h at rt. A solution of Intermediate 8 (0.2 g, 0.44 mmol, 1.0 eq) and Copper(I) thiophene-2-carboxylate (0.025 g, 0.132 mmol, 0.3 eq) in THF (6.0 mL) is added to the mixture and stirred at 50 °C for 4 days. The reaction mixture is quenched with aq. sat. NH₄Cl (10.0 mL) and water and diluted with EA (10.0 mL). The aq. phase is extracted once more with EA (10.0 mL), the phases are separated and the combined organic phase is dried over a phase separator and the solvent is removed *in vacuo* to recover the crude as a brown oil. Purification over preparative HPLC(I) yields the desired product as a white foam (0.038 g, 15%). LCMS (A): t_{R} =1.01 min; [M+H]⁺ = 571.09.

### 2. (2R,3R,4S,5R,6R)-4-(4-(4-Chloro-2,3-difluorophenyl)-1H-1,2,3-triazol-1-yl)-6-((1-(1-fluoro-2-methylpropan-2-yl)-1H-1,2,3-triazol-4-yl)methyl)-2-(hydroxymethyl)-5-methoxytetrahydro-2H-pyran-3-ol (Example 2.1.45.)

To a solution of Example 2.1.45. *Step* 1. (0.035 g, 0.061 mmol, 1.0 eq) in dioxane (1.0 mL) is added water (0.5 mL) and the reaction mixture is cooled to 0°C (ice bath). TFA (0.38 mL, 4.9 mmol, 80 eq) is added dropwise and the solution is stirred at rt for 20 h. The reaction mixture is quenched with 25% NH₄OH (pH = 10) and directly purified over preparatory HPLC(I) to recover the desired product as a white solid (0.026 g, 88%). LCMS (A): t_{R} = 0.84 min; [M+H]⁺ = 531.02.

¹H NMR (500 MHz, DMSO) *δ*: 8.57 (d, *J* = 3.2 Hz, 1 H), 8.22 (s, 1 H), 7.95-7.98 (m, 1 H), 7.55-7.9 (m, 1 H), 5.32 (d, *J* = 6.8 Hz, 1 H), 5.21 (dd, *J*₁ = 2.9 Hz, *J*₂ = 11.0 Hz), 4.85 (t, *J* = 5.5 Hz, 1 H), 4.68 (d, *J* = 47.1 Hz , 2 H), 4.44-4.49 (m, 2 H), 3.93-4.0 (m, 2 H), 3.47-3.53 (m, 2 H), 3.38-3.333 (m, 1 H), 3.22 (s, 3 H), 2.87-2.91 (m, 1H), 1.62 (s, 6 H)

Following examples are prepared starting from Intermediate 8 and the corresponding amines according to the procedures described for Example 2.1.45. LC-MS data are listed in Table 3 below. LC-MS conditions are LC-MS (A).

**Table 3**

| **Ex.** | **Compound** | **t_{R} [min]** | **MS Data m/z [M+H]⁺** | **Chiral HPLC conditions** | **t_{R} chiral [min]** |
|---|---|---|---|---|---|
| **2.1.45.** | (2R,3R,4S,5R,6R)-4-(4-(4-chloro-2,3-difluorophenyl)-1H-1,2,3-triazol-1-yl)-6-((1-(1-fluoro-2-methylpropan-2-yl)-1H-1,2,3-triazol-4-yl)methyl)-2-(hydroxymethyl)-5-methoxytetrahydro-2H-pyran-3-ol | 0.84 | 531.02 | | |
| **2.1.47.A** | (2R,3R,4S,5R,6R)-4-(4-(4-chloro-2,3-difluorophenyl)-1H-1,2,3-triazol-1-yl)-6-((1-((1S,3S)-3-fluoro-1-methylcyclobutyl)-1H-1,2,3-triazol-4-yl)methyl)-2-(hydroxymethyl)-5-methoxytetrahydro-2H-pyran-3-ol | 0.86 | 543.04 | Chiralpak IG B: 40% EtOH 5 min run | 3.4 |
| **2.1.48.B** | (2R,3R,4S,5R,6R)-4-(4-(4-chloro-2,3-difluorophenyl)-1H-1,2,3-triazol-1-yl)-6-((1-((1R,3R)-3-fluoro-1-methylcyclobutyl)-1H-1,2,3-triazol-4-yl)methyl)-2-(hydroxymethyl)-5-methoxytetrahydro-2H-pyran-3-ol | 0.87 | 543.03 | Chiralpak IG B: 40% EtOH 5 min run | 2.47 |

### Example 2.1.54.

### (2R,3R,4S,5R,6R)-4-(4-(4-Chloro-2,3-difluorophenyl)-1H-1,2,3-triazol-1-yl)-2-(hydroxymethyl)-5-methoxy-6-((1-(1-(trifluoromethyl)cyclopropyl)-1H-1,2,3-triazol-4-yl)methyl)tetrahydro-2H-pyran-3-ol

### 1. 4-(4-Chloro-2,3-difluorophenyl)-1-((4aR,6R,7R,8R,8aR)-7-methoxy-2,2-dimethyl-6-((1-(1-(trifluoromethyl)cyclopropyl)-1H-1,2,3-triazol-4-yl)methyl)hexahydropyrano[3,2-d][1,3]dioxin-8-yl)-1H-1,2,3-triazole

To a suspension of 1H-imidazole-1-sulfonyl azide hydrochloride (0.16 g, 0.71 mmol, 1.2 eq) in MeOH (2.0 mL) are added K₂CO₃ (0.210g, 1.2 mmol, 2.0 eq), and copper(II) sulfate pentahydrate (0.015 g, 0.059 mmol, 0.1 eq), followed by 1-(trifluoromethyl)cyclopropan-1-amine hydrochloride (0.1 g, 0.59 mmol) and stirred at rt for 17 h. The suspension is added to a solution of Intermediate 8 (0.12 g, 0.44 mmol, 1.0 eq) in THF (3.0 ml) with Copper(I) (0.085 g, 1.32 mmol, 5.0 eq), AcOH ( 0.61 mL, 10.6 mmol, 40 eq) and aq. sat. CuSO₄ (0.54 mL) and stirred at rt for 1.5 h. The reaction mixture is quenched with aq. sat. NH₄Cl (10.0 mL) and water, diluted with EA (10.0 mL) and the aq. phase is extracted once more with EA (10.0 mL). The combined organic phase is extracted with aq. sat. NaHCO₃ (10 mL), aq. sat. NaCl (10 mL), dried over MgSO₄, filtered and the solvent is removed *in vacuo* to recover the crude as an oil. Purification over preparative HPLC(I) yields the title product as a white solid (0.134 g, 82%). LCMS (A): t_{R} =1.05 min; [M+H]⁺ = 604.83.

### 2. (2R,3R,4S,5R,6R)-4-(4-(4-Chloro-2,3-difluorophenyl)-1H-1,2,3-triazol-1-yl)-2-(hydroxymethyl)-5-methoxy-6-((1-(1-(trifluoromethyl)cyclopropyl)-1H-1,2,3-triazol-4-yl)methyl)tetrahydro-2H-pyran-3-ol (Example 2.1.54.)

To a solution of Example 2.1.54. *Step* 1. (0.128 g, 0.212 mmol, 1.0 eq) in dioxane (3.0 mL) is added water (1.5 mL) and the reaction mixture is cooled to 0°C (ice bath). TFA (0.33 mL, 4.24 mmol, 20 eq) is added dropwise and the solution is stirred at rt for 20 h. The reaction mixture is quenched with 25% NH₄OH (pH = 10) and directly purified over preparatory HPLC(I) to recover the title product as a white solid (0.098 g, 82%). LCMS (A): t_{R} = 0. 0.9 min; [M+H]⁺ = 564.91.

¹H NMR (500 MHz, DMSO) *δ*: 8.57 (d, *J* = 3.2 Hz, 1 H), 8.32 (s, 1 H), 7.96 (m, 1 H), 7.57 (m, 1 H), 5.33 (d, *J* = 6.8 Hz, 1 H), 5.21 (dd, *J*₁ = 11.3 Hz, *J*₂ = 3.0 Hz, 1 H), 4.77 (t, *J* = 5.6 Hz, 1 H), 4.45-4.53 (m, 2 H), 3.94 (m, 2 H), 3.48 (t, *J* = 5.7 Hz, 2 H), 3.39 (dd, *J*₁ = 15.7 Hz, *J*₂ = 11.5 Hz, 1 H), 3.22 (s, 3 H), 2.91 (dd, *J*₁ = 3.2 Hz, *J*₂ = 15.7 Hz, 1H), 1.63-1.83 (m, 4 H) .

Following examples are prepared starting from Intermediate 8, 9a, 10a or 11a and the corresponding amines, in analogy to the procedures described for Example 2.1.54.. *Step 2.* is performed either with TFA, as described or with AcOH, as described for Example 3.1.7. *Step 2..* Selected examples, synthesized with chiral amines, have yielded mixtures of diastereomers, that are separated by chiral preparatory HPLC.

LC-MS data are listed in Table 4 below. LC-MS conditions are LC-MS (A). Chiral analytical HPLC (I) (conditions and retention time) of the diastereomers of selected Examples are also listed.

**Table 4**

| **Ex.** | **Compound** | **t_{R} [min]** | **MS Data m/z [M+H]⁺** | **Chiral HPLC conditions** | **t_{R} chiral [min]** |
|---|---|---|---|---|---|
| **2.1.54.** | (2R,3R,4S,5R,6R)-4-(4-(4-chloro-2,3-difluorophenyl)-1H-1,2,3-triazol-1-yl)-2-(hydroxymethyl)-5-methoxy-6-((1-(1-(trifluoromethyl)cyclopropyl)-1H-1,2,3-triazol-4-yl)methyl)tetrahydro-2H-pyran-3-ol | 0.9 | 564.91 | | |
| **2.1.55.** | (2R,3R,4S,5R,6R)-4-(4-(4-chloro-2,3-difluorophenyl)-1H-1,2,3-triazol-1-yl)-2-(hydroxymethyl)-5-methoxy-6-((1-(1-(trifluoromethyl)cyclobutyl)-1H-1,2,3-triazol-4-yl)methyl)tetrahydro-2H-pyran-3-ol | 0.93 | 578.91 | | |
| **2.1.56.** | (2R,3R,4S,5R,6R)-4-(4-(4-chloro-2,3-difluorophenyl)-1H-1,2,3-triazol-1-yl)-2-(hydroxymethyl)-5-methoxy-6-((1-(1,1,1-trifluoro-2-methylpropan-2-yl)-1H-1,2,3-triazol-4-yl)methyl)tetrahydro-2H-pyran-3-ol | 0.91 | 566.92 | | |
| **2.1.57.** | (2R,3R,4S,5R,6R)-6-((1-(3,3-bis(trifluoromethyl)cyclobutyl)-1H-1,2,3-triazol-4-yl)methyl)-4-(4-(4-chloro-2,3-difluorophenyl)-1H-1,2,3-triazol-1-yl)-2-(hydroxymethyl)-5-methoxytetrahydro-2H-pyran-3-ol | 0.98 | 646.94 | | |
| **2.1.60.A** | (2R,3R,4S,5R,6R)-4-(4-(4-chloro-2,3-difluorophenyl)-1H-1,2,3-triazol-1-yl)-6-((1-((1R,3R)-3-fluorocyclobutyl)-1H-1,2,3-triazol-4-yl)methyl)-2-(hydroxymethyl)-5-methoxytetrahydro-2H-pyran-3-ol | 0.93 | 596.95 | Chiralpak AD-H B: 30% EtOH 5 min run | 1.38 |
| **2.1.60.B** | (2R,3R,4S,5R,6R)-4-(4-(4-chloro-2,3-difluorophenyl)-1H-1,2,3-triazol-1-yl)-6-((1-((1S,3S)-3-fluorocyclobutyl)-1H-1,2,3-triazol-4-yl)methyl)-2-(hydroxymethyl)-5-methoxytetrahydro-2H-pyran-3-ol | 0.92 | 596.96 | Chiralpak AD-H B: 30% EtOH 5 min run | 2.11 |
| **2.1.67.** | (2R,3R,4S,5R,6R)-4-(4-(4-chloro-2,3-difluorophenyl)-1H-1,2,3-triazol-1-yl)-2-(hydroxymethyl)-5-methoxy-6-((1-(1-(trifluoromethyl)cyclopentyl)-1H-1,2,3-triazol-4-yl)methyl)tetrahydro-2H-pyran-3-ol | 0.95 | 592.9 | | |
| **2.1.69.** | (2R,3R,4S,5R,6R)-4-(4-(4-chloro-2,3-difluorophenyl)-1H-1,2,3-triazol-1-yl)-2- | 0.97 | 604.85 | | |
| | (hydroxymethyl)-5-methoxy-6-((1-((1R,3R,5S)-3-(trifluoromethyl)bicyclo[3.1.0]hexan-3-yl)-1H-1,2,3-triazol-4-yl)methyl)tetrahydro-2H-pyran-3-ol | | | | |
| **2.1.70.** | (2R,3R,4S,5R,6R)-4-(4-(4-chloro-2,3-difluorophenyl)-1H-1,2,3-triazol-1-yl)-2-(hydroxymethyl)-5-methoxy-6-((1-(2-(trifluoromethyl)bicyclo[2.2.1]heptan-2-yl)-1H-1,2,3-triazol-4-yl)methyl)tetrahydro-2H-pyran-3-ol | 1.0 | 618.86 | | |
| **2.1.66.A** | (2R,3R,4S,5R,6R)-4-(4-(4-chloro-2,3-difluorophenyl)-1H-1,2,3-triazol-1-yl)-6-((1-((3R,4R)-3-fluorotetrahydro-2H-pyran-4-yl)-1H-1,2,3-triazol-4-yl)methyl)-2-(hydroxymethyl)-5-methoxytetrahydro-2H-pyran-3-ol [1-(1,2,3-tri-deoxy-2-methoxy-3-[4-(4-chloro-2,3-difluorophenyl)-1H-1,2,3-triazol-1-yl]-α-D-galacto-pyranose)-1-(1-((3R,4R)-3-fluoro-tetrahydropyran-4-yl)-1H-triazol-4-yl)-methane] | 0.8 | 558.85 | Chiralpak IC B: 40% MeCN/MeOH (1:1) 5 min run | 2.89 |
| **2.1.66.B** | (2R,3R,4S,5R,6R)-4-(4-(4-chloro-2,3-difluorophenyl)-1H-1,2,3-triazol-1-yl)-6-((1-((3S,4S)-3-fluorotetrahydro-2H-pyran-4-yl)-1H-1,2,3-triazol-4-yl)methyl)-2-(hydroxymethyl)-5-methoxytetrahydro-2H-pyran-3-ol [1-(1,2,3-tri-deoxy-2-methoxy-3-[4-(4-chloro-2,3-difluorophenyl)-1H-1,2,3-triazol-1-yl]-α-D-galacto-pyranose)-1-(1-((3S,4S)-3-fluoro-tetrahydropyran-4-yl)-1H-triazol-4-yl)-methane] | 0.8 | 558.85 | Chiralpak IC B: 40% MeCN/MeOH (1:1) 5 min run | 3.4 |
| **2.1.73.A** | (2R,3R,4S,5R,6R)-4-(4-(4-chloro-2,3-difluorophenyl)-1H-1,2,3-triazol-1-yl)-6-((1-(4,4-difluorotetrahydro-2H-pyran-3-yl)-1H-1,2,3-triazol-4-yl)methyl)-2-(hydroxymethyl)-5-methoxytetrahydro-2H-pyran-3-ol | 0.85 | 578.14 | Chiralcel OJ-H B: 25% MeCN/MeOH (1:1) 3 min run | 1.43 |
| **2.1.73.B** | | | | | 1.96 |
| **3.1.59.** | (2R,3R,4S,5R,6R)-2-(hydroxymethyl)-5-methoxy-6-((1-(1-methyl-2-oxabicyclo[2.1.1]hexan-4-yl)-1H-1,2,3-triazol-4-yl)methyl)-4-(4-(2,3,4-trifluorophenyl)-1H-1,2,3-triazol-1-yl)tetrahydro-2H-pyran-3-ol | 0.78 | 537.05 | | |
| **3.1.29.** | (2R,3R,4S,5R,6R)-2-(hydroxymethyl)-5-methoxy-6-((1-(3-methoxybicyclo[1.1.1]pentan-1-yl)-1H-1,2,3-triazol-4-yl)methyl)-4-(4-(2,3,4-trifluorophenyl)-1H-1,2,3-triazol-1-yl)tetrahydro-2H-pyran-3-ol | 0.81 | 537.03 | | |
| **3.1.52.** | (2R,3R,4S,5R,6R)-6-((1-(3-(fluoromethyl)oxetan-3-yl)-1H-1,2,3-triazol-4-yl)methyl)-2-(hydroxymethyl)-5-methoxy-4-(4-(2,3,4-trifluorophenyl)-1H-1,2,3-triazol-1-yl)tetrahydro-2H-pyran-3-ol | 0.75 | 528.90 | | |
| **3.1.53.** | (2R,3R,4S,5R,6R)-6-((1-(3-(difluoromethyl)oxetan-3-yl)-1H-1,2,3-triazol-4-yl)methyl)-2-(hydroxymethyl)-5-methoxy-4-(4-(2,3,4-trifluorophenyl)-1H-1,2,3-triazol-1-yl)tetrahydro-2H-pyran-3-ol | 0.78 | 546.97 | | |
| **4.1.28.** | (2R,3R,4S,5R,6R)-4-(4-(4-bromo-2,3-difluorophenyl)-1H-1,2,3-triazol-1-yl)-6-((1-(1,1-difluoro-2-methylpropan-2-yl)-1H-1,2,3-triazol-4-yl)methyl)-2-(hydroxymethyl)-5-methoxytetrahydro-2H-pyran-3-ol | 0.88 | 593.17 | | |
| **4.1.43.** | (2R,3R,4S,5R,6R)-4-(4-(4-bromo-2,3-difluorophenyl)-1H-1,2,3-triazol-1-yl)-6-((1-(1-(difluoromethyl)cyclobutyl)-1H-1,2,3-triazol-4-yl)methyl)-2-(hydroxymethyl)-5-methoxytetrahydro-2H-pyran-3-ol | 0.89 | 604.76 | | |
| **4.1.53.** | (2R,3R,4S,5R,6R)-4-(4-(4-bromo-2,3-difluorophenyl)-1H-1,2,3-triazol-1-yl)-6-((1-(3-(difluoromethyl)oxetan-3-yl)-1H-1,2,3-triazol-4-yl)methyl)-2-(hydroxymethyl)-5-methoxytetrahydro-2H-pyran-3-ol [1-(1,2,3-tri-deoxy-2-methoxy-3-[4-(4-bromo-2,3-difluorophenyl)-1H-1,2,3-triazol-1-yl]-α-D-galacto-pyranose)-1-(1-(3-difluoromethyl-3-oxetanyl)-1H-triazol-4-yl)-methane] | 0.84 | 607.05 | | |
| **4.1.58.** | (2R,3R,4S,5R,6R)-4-(4-(4-bromo-2,3-difluorophenyl)-1H-1,2,3-triazol-1-yl)-2-(hydroxymethyl)-5-methoxy-6-((1-(3-(trifluoromethyl)oxetan-3-yl)-1H-1,2,3-triazol-4-yl)methyl)tetrahydro-2H-pyran-3-ol [1-(1,2,3-tri-deoxy-2-methoxy-3-[4-(4-bromo-2,3- | 0.88 | 624.67 | | |
| | difluorophenyl)-1H-1,2,3-triazol-1-yl]-α-D-galacto-pyranose)-1-(1-(3-trifluoromethyl-3-oxetanyl)-1H-triazol-4-yl)-methane] | | | | |
| **1.1.58.** | (2R,3R,4S,5R,6R)-4-(4-(2,3-difluoro-4-methylphenyl)-1H-1,2,3-triazol-1-yl)-2-(hydroxymethyl)-5-methoxy-6-((1-(3-(trifluoromethyl)oxetan-3-yl)-1H-1,2,3-triazol-4-yl)methyl)tetrahydro-2H-pyran-3-ol | 0.84 | 561.09 | | |

### Example 2.1.51.

### (2R,3R,4S,5R,6R)-4-(4-(4-chloro-2,3-difluorophenyl)-1H-1,2,3-triazol-1-yl)-6-((1-(1-(difluoromethyl)cyclopropyl)-1H-1,2,3-triazol-4-yl)methyl)-2-(hydroxymethyl)-5-methoxytetrahydro-2H-pyran-3-ol

### 1. 4-(4-Chloro-2,3-difluorophenyl)-1-((4aR,6R,7R,8R,8aR)-6-((1-(1-(difluoromethyl)cyclopropyl)-1H-1,2,3-triazol-4-yl)methyl)-7-methoxy-2,2-dimethylhexahydropyrano[3,2-d][1,3]dioxin-8-yl)-1H-1,2,3-triazole

To a solution of Intermediate 8 (0.1 g, 0.22 mmol, 1.0 eq) in DMF (2.0 mL) are added 1-azido-1-(difluoromethyl)cyclopropane (14,5% in TBME, 0.22 g, 0.242 mmol, 1.1 eq), copper (I) iodide (0.0042 g, 0.022 mmol, 0.1 eq) and DIPEA (0.115 mL, 0.661 mmol, 3.0 eq) and the reaction mixture is stirred at 50°C for 3 h. The mixture is partitioned between EA and water/NH₄Cl and the layers are separated. The aq. layer is extracted (1x) with EA and the combined org layer is washed with aq. sat. NH₄Cl, water and brine, dried over MgSO₄, filtered and the solvent reduced under pressure to recover the crude. Purification by preparatory HPLC/MS (I) yields the desired product as a beige solid (0.1 g, 77%). LCMS (A): t_{R} = 1.01 min; [M+H]⁺ = 587.07.

### 2. (2R,3R,4S,5R,6R)-4-(4-(4-chloro-2,3-difluorophenyl)-1H-1,2,3-triazol-1-yl)-6-((1-(1-(difluoromethyl)cyclopropyl)-1H-1,2,3-triazol-4-yl)methyl)-2-(hydroxymethyl)-5-methoxytetrahydro-2H-pyran-3-ol (Example 2.1.51.)

To a solution of Example 2.1.51. *Step* 1. (99 mg, 0.169 mmol, 1 eq) in water (4.0 mL) is added acetic acid (4.0 mL) and the solution is stirred at 55°C for 48 h. The solvent is removed *in vacuo* and the crude material is purified by preparative HPLC/MS (I) to give the title compound as a white solid (0.045 g, 49%). LC-MS (A): t_{R} = 0.84 min; [M+H]⁺: 547.01.

¹H NMR (400 MHz, MeOD) *δ*: 8.50 (d, *J* = 3.5 Hz, 1 H), 8.2 (s, 1 H), 7.85 (m, 1 H), 7.45 (m, 1 H), 6.02 (t, *J* = 55 Hz, 1 H), 5.18 (dd, *J*₁ = 3.0 Hz, *J*₂ = 11.5 Hz, 1 Hz), 4.64-4.7 (m, 1 H), 4.50 (dd, *J*₁ = 6.0 Hz, *J*₂ = 11.5 Hz, 1 H), 4.15 (d, *J* = 2.3 Hz, 1 H), 4.06 (t, *J* = 6.3 Hz, 1 H), 3.65-3.75 (m, 2 H), 3.41 (dd, *J*₁ = 11.3 Hz, *J*₂ = 15.8 Hz, 1 H), 3.1-3.17 (m, 1 H ), 1.54-1.6 (m, 4 H)

Following examples are prepared starting from Intermediate 8, 10a or 11a and the corresponding azides, according to the procedures described for Example 2.1.51.. *Step* 2 is performed either with AcOH, as described or with TFA, as described for Example 2.1.54. *Step 2.* LC-MS data are listed in Table 5 below. LC-MS conditions are LC-MS (A).

**Table 5**

| **Ex.** | **Compound** | **t_{R} [min]** | **MS Data m/z [M+H]⁺** |
|---|---|---|---|
| **2.1.51.** | (2R,3R,4S,5R,6R)-4-(4-(4-chloro-2,3-difluorophenyl)-1H-1,2,3-triazol-1-yl)-6-((1-(1-(difluoromethyl)cyclopropyl)-1H-1,2,3-triazol-4-yl)methyl)-2-(hydroxymethyl)-5-methoxytetrahydro-2H-pyran-3-ol | 0.84 | 546.88 |
| **3.1.54.** | (2R,3R,4S,5R,6R)-2-(hydroxymethyl)-5-methoxy-6-((1-(1-(trifluoromethyl)cyclopropyl)-1H-1,2,3-triazol-4-yl)methyl)-4-(4-(2,3,4-trifluorophenyl)-1H-1,2,3-triazol-1-yl)tetrahydro-2H-pyran-3-ol | 0.86 | 548.99 |
| **4.1.51.** | (2R,3R,4S,5R,6R)-4-(4-(4-bromo-2,3-difluorophenyl)-1H-1,2,3-triazol-1-yl)-6-((1-(1-(difluoromethyl)cyclopropyl)-1H-1,2,3-triazol-4-yl)methyl)-2-(hydroxymethyl)-5-methoxytetrahydro-2H-pyran-3-ol | 0.85 | 591 |
| **4.1.54.** | (2R,3R,4S,5R,6R)-4-(4-(4-bromo-2,3-difluorophenyl)-1H-1,2,3-triazol-1-yl)-2-(hydroxymethyl)-5-methoxy-6-((1-(1-(trifluoromethyl)cyclopropyl)-1H-1,2,3-triazol-4-yl)methyl)tetrahydro-2H-pyran-3-ol | 0.90 | 608.94 |

### Example 2.1.49.

### (2R,3R,4S,5R,6R)-4-(4-(4-Chloro-2,3-difluorophenyl)-1H-1,2,3-triazol-1-yl)-2-(hydroxymethyl)-5-methoxy-6-((1-(tert-pentyl)-1H-1,2,3-triazol-4-yl)methyl)tetrahydro-2H-pyran-3-ol

### 1. 4-(4-Chloro-2,3-difluorophenyl)-1-((4aR,6R,7R,8R,8aR)-7-methoxy-2,2-dimethyl-6-((1-(tert-pentyl)-1H-1,2,3-triazol-4-yl)methyl)hexahydropyrano[3,2-d][1,3]dioxin-8-yl)-1H-1,2,3-triazole

The triazole synthesis is conducted on a commercial continuous-flow reactor (Vapourtec) using a PFA (2.0 mL internal volume) and a copper coil (10.0 mL internal volume) and a back-pressure regulator (7.0 bar). tert-Amilamine (0.0315 mL, 0.264 mmol, 1.2 eq, 0.12 M in DMSO) and diethylamine (0.165 mL, 1.59 mmol, 7.2 eq) are dissolved in DMSO (1.96 mL). 2-Azido-1,3-dimethylimidazolinium hexafluorophosphate (95.2 mg, 0.317 mmol, 1.44 eq, 0.15 M in DMSO) is dissolved in DMSO (2.15 mL). The two solutions are pumped at a flow of 0.063 mL/ min at a T= 50°C through the PFA coil. The rector outlet is fed directly into the copper coil, kept at a temperature of 145°C, together with a solution of Intermediate 8 (0.1 g, 0.22 mmol, 1 eq, 0.05 M in DMSO/water), (+)-sodium L-ascorbate (4.41 mg, 0.022 mmol, 0.1 eq) and trans-N,N'-dimethylcyclohexane-1,2-diamine (0.00537 mL, 0.033 mmol, 0.15 eq) in DMSO/Water (5/1) (4.3 mL) at a flow rate of 0.125 mL/min through. The reactor outlet is collected, diluted with EA (20 mL) and sat. aq. NH₄Cl soln. (20 mL). The layers are separated and the organic layer is washed with sat. aq. NH₄Cl (20 mL) and brine (20 mL). The layers are separated and the remaining aq. layer is extracted once more with EA (20 mL). The combined organic layer is dried over MgSO₄, filtered, concentrated in vacuo to recover the crude, that is purified over preparatory HPLC(l) to yield a white foam as the title compound (0.058 g). LC-MS (A): t_{R} = 1.05 min; [M+H]⁺: 567.05.

### 2. (2R,3R,4S,5R,6R)-4-(4-(4-Chloro-2,3-difluorophenyl)-1H-1,2,3-triazol-1-yl)-2-(hydroxymethyl)-5-methoxy-6-((1-(tert-pentyl)-1H-1,2, 3-triazol-4-yl)methyl)tetrahydro-2H-pyran-3-ol (Example 2.1.49.)

The title compound is prepared from Example 2.1.49. *Step 1.* in TFA in analogy to Example 2.1.51. *Step* 2. as a colorless glass (0.014, 25%). LCMS (A): t_{R} = 0.88 min; [M+H]⁺ = 527.01.

Following examples are prepared starting from Intermediate 8 and the corresponding amines according to the procedures described for Example 2.1.49. LC-MS data are listed in Table 6 below. LC-MS conditions are LC-MS (A).

**Table 6**

| **Ex.** | **Compound** | **t_{R} [min]** | **MS Data m/z [M+H]⁺** |
|---|---|---|---|
| **2.1.49.** | (2R,3R,4S,5R,6R)-4-(4-(4-chloro-2,3-difluorophenyl)-1H-1,2,3-triazol-1-yl)-2-(hydroxymethyl)-5-methoxy-6-((1-(tert-pentyl)-1H-1,2,3-triazol-4-yl)methyl)tetrahydro-2H-pyran-3-ol | 0.88 | 527.01 |
| **2.1.50.** | (2R,3R,4S,5R,6R)-4-(4-(4-chloro-2,3-difluorophenyl)-1H-1,2,3-triazol-1-yl)-2-(hydroxymethyl)-5-methoxy-6-((1-(3-methylpentan-3-yl)-1H-1,2,3-triazol-4-yl)methyl)tetrahydro-2H-pyran-3-ol | 0.91 | 541.04 |

### Example 5.1.53.

### 4-(1-((2R,3R,4S,5R,6R)-2-((1-(3-(Difluoromethyl)oxetan-3-yl)-1H-1,2,3-triazol-4-yl)methyl)-5-hydroxy-6-(hydroxymethyl)-3-methoxytetrahydro-2H-pyran-4-yl)-1H-1,2,3-triazol-4-yl)-2,3-difluorobenzonitrile

### 1. 4-(4-Bromo-2,3-difluorophenyl)-1-((4aR,6R, 7R,8R,8aR)-6-((1-(3-(difluoromethyl)oxetan-3-yl)-1H-1,2,3-triazol-4-yl)methyl)-7-methoxy-2,2-dimethylhexahydropyrano[3,2-d][1,3]dioxin-8-yl)-1H-1,2,3-triazole

The title compound is prepared from Intermediate 11a and 3-(difluoromethyl)oxetan-3-amine in analogy to Example 2.1.51. *Step 1.* as a light green glass (0.21, 65%). LCMS (A): t_{R} = 0.99 min; [M+H]⁺ = 647.09.

### 2. 4-(1-((4aR,6R,7R,8R,8aR)-6-((1-(3-(Difluoromethyl)oxetan-3-yl)-1H-1,2,3-triazol-4-yl)methyl)-7-methoxy-2,2-dimethylhexahydropyrano[3,2-d][1,3]dioxin-8-yl)-1H-1,2,3-triazol-4-yl)-2,3-difluorobenzonitrile

To a mixture of Example 5.1.53. *Step 1*.(0.15 g, 0.232 mmol, 1.0 eq) and Copper(I) cyanide (0.042 g, 0.463 mmol, 2.0 eq) in DMF (3.0 mL) is added copper(I) iodide (0.0004 g, 0.0232 mmol, 0.1 eq) and the resulting yellow solution is stirred at 120°C for 72 h. The reaction mixture is cooled to rt and quenched with EA and water. The phases are separated, the organic phase is washed with brine, dried over MgSO4, filtered and concentrated under reduced pressure. The crude material is purified by ISCO to recover (0.112 g, 81%) of desired product as colorless solid. LCMS (A): t_{R} = 0.94 min; [M+H]⁺ = 593.99.

### 3. 4-(1-((2R,3R,4S,5R,6R)-2-((1-(3-(Difluoromethyl)oxetan-3-yl)-1H-1,2,3-triazol-4-yl)methyl)-5-hydroxy-6-(hydroxymethyl)-3-methoxytetrahydro-2H-pyran-4-yl)-1H-1,2,3-triazol-4-yl)-2,3-difluorobenzonitrile (Example 5.1.53)

The title compound is prepared from Example 5.1.53. *Step 2.* in TFA in analogy to Example 2.1.51. *Step 2.* as a colorless glass (0.087, 65%). LCMS (A): t_{R} = 0.77 min; [M+H]⁺ = 554.16.

¹H NMR (500 MHz, MeOD) δ: 8.63 (d, *J* = 3.5 Hz, 1 H), 8.26 (s, 1 H), 8.16 (m, 1 H), 7.70 (m, 1 H), 6.57 (t, *J* = 54.5 Hz), 5.19-5.24 (m, 3 H), 5.15 (dd, *J*₁ = 2.3 Hz, *J*₂ = 7.7 Hz, 2H), 4.70 (m, 1 H,), 4.53 (dd, *J*₁=59 Hz, *J*₂ = 11.5 Hz 2 H, 1H), 4.15 (d, *J* = 2.4 Hz, 1 H), 4.07-4.10 (m, 1 H), 3.67-3.76 (m, 2 H), 3.46 (dd, *J*₁ = 15.8 Hz, *J*₂ = 11.9 Hz, 1 H), 3.15-3.19 (m, 1 H).

Following examples are prepared starting from Intermediate 11a and the corresponding amines according to the procedures described for Example 5.1.53. *Step 3.* is performed either with TFA a described, or with AcOH, as reported for Example 3.1.7. *Step 2..* LC-MS data are listed in Table 7 below. LC-MS conditions are LC-MS (A).

**Table 7**

| **Ex.** | **Compound** | **t_{R} [min]** | **MS Data m/z [M+H]⁺** |
|---|---|---|---|
| **5.1.53.** | 4-(1-((2R,3R,4S,5R,6R)-2-((1-(3-(difluoromethyl)oxetan-3-yl)-1H-1,2,3-triazol-4-yl)methyl)-5-hydroxy-6-(hydroxymethyl)-3-methoxytetrahydro-2H-pyran-4-yl)-1H-1,2,3-triazol-4-yl)-2,3-difluorobenzonitrile | 0.77 | 554.16 |
| **5.1.58.** | 2,3-difluoro-4-(1-((2R,3R,4S,5R,6R)-3-hydroxy-2-(hydroxymethyl)-5-methoxy-6-((1-(3-(trifluoromethyl)oxetan-3-yl)-1H-1,2,3-triazol-4-yl)methyl)tetrahydro-2H-pyran-4-yl)-1H-1,2,3-triazol-4-yl)benzonitrile | 0.82 | 572.17 |
| **5.1.6.** | 2,3-difluoro-4-(1-((2R,3R,4S,5R,6R)-3-hydroxy-2-(hydroxymethyl)-5-methoxy-6-((1-(3-methyloxetan-3-yl)-1H-1,2,3-triazol-4-yl)methyl)tetrahydro-2H-pyran-4-yl)-1H-1,2,3-triazol-4-yl)benzonitrile | 0.75 | 518.22 |
| **5.1.43.** | 4-(1-((2R,3R,4S,5R,6R)-2-((1-(1-(difluoromethyl)cyclobutyl)-1 H-1 ,2,3-triazol-4-yl)methyl)-5-hydroxy-6-(hydroxymethyl)-3-methoxytetrahydro-2H-pyran-4-yl)-1H-1,2,3-triazol-4-yl)-2,3-difluorobenzonitrile | 0.85 | 552.21 |
| **5.1.66CD.** | 2,3-difluoro-4-(1-((2R,3R,4S,5R,6R)-2-((1-((3R*,4S*)-3-fluorotetrahydro-2H-pyran-4-yl)-1H-1,2,3-triazol-4-yl)methyl)-5-hydroxy-6-(hydroxymethyl)-3-methoxytetrahydro-2H-pyran-4-yl)-1H-1,2,3-triazol-4-yl)benzonitrile [1-(1,2,3-tri-deoxy-2-methoxy-3-[4-(4-cyano-2,3-diifluorophenyl)-1H-1,2,3-triazol-1-yl]-α-D-galacto-pyranose)-1-(1-((3R*,4S*)-3-fluoro-tetrahydropyran-4-yl)-1H-triazol-4-yl)-methane] | 0.77 | 550.28 |

### Example 1.2.34.

### (2R,3R,4S,5R,6R)-4-(4-(2,3-Difluoro-4-methylphenyl)-1H-1,2,3-triazol-1-yl)-2-(hydroxymethyl)-6-((4-(2-hydroxypropan-2-yl)-1H-1,2,3-triazol-1-yl)methyl)-5-methoxytetrahydro-2H-pyran-3-ol

### 1. 2-(1-(((4aR,6R,7R,8R,8aR)-8-(4-(2,3-Difluoro-4-methylphenyl)-1H-1,2,3-triazol-1-yl)-7-methoxy-2,2-dimethylhexahydropyrano[3,2-d][1,3]dioxin-6-y0methy0-1H-1,2,3-triazol-4-y0propan-2-ol

2-Methylbut-3-yn-2-ol (0.017 g, 0.2 mmol, 1 eq) and Intermediate 18a (90.1 mg, 0.2 mmol) are dissolved in DMF (2 mL) and Copper(I) iodide (3.89 mg, 0.02 mmol, 0.1 eq) and DIPEA (0.10 mL, 0.6 mmol, 3.0 eq) are added. The mixture is stirred at rt for 15 h, then filtered and directly purified by prep HPLC/MS (I) to recover the desired compound (0.094 g, 88%). LCMS (A): t_{R} = 0.86 min; [M+H]⁺ = 535.23.

### 2. (2R,3R,4S,5R,6R)-4-(4-(2,3-Difluoro-4-methylphenyl)-1H-1,2,3-triazol-1-yl)-2-(hydroxymethyl)-6-((4-(2-hydroxypropan-2-yl)-1H-1,2,3-triazol-1-yl)methyl)-5-methoxytetrahydro-2H-pyran-3-ol (Example 1.2.34.)

To a solution of Example 1.2.34. *1. Step* (0.094, 0.18 mmol) in THF (6.0 mL) is added a mixture of AcOH/ water (1/1, 10 mL) and the solution is stirred at 65°C for 24h. The reaction mixture is partitioned between EA and aq. sat NaHCO₃. The layers are separated and the aq. phase is extracted with EA (2 x 15 mL), the combined organic phase is dried over Na₂SO₄, filtered and concentrated *in vacuo.* The crude is purified by prep HPLC/MS (I) to recover the desired product (0.072 g, 83%). LCMS (A): t_{R} = 0.73 min; [M+H]⁺ = 495.20.

¹H NMR (400 MHz, MeOD) δ: 8.47 (d, J = 3.3 Hz, 1 H), 8.06 (s, 1 H), 7.78 (m, 1 H), 7.15 (t, J = 7.3 Hz, 1H), 5.21 (dd, J¹ = 2.8 Hz, J² = 11.5 Hz, 1 H), 5.09 (dd, J¹ = 11.5 Hz, J² = 15.1 Hz, 1 H), 4.85-4.75 (m, 2 H), 4.59 (dd, J¹ = 6.5 Hz, J² = 11.5 Hz, 1 H), 4.25-4.21 (m, 2 H), 3.71 (m, 2 H), 3.37 (s, 3 H), 2.36 (s, 3 H), 1.63 (s, 6 H).

Following examples are prepared starting from either Intermediate 17a, or 18a, and the corresponding alkynes according to the procedures described for Example 1.2.34. LC-MS data are listed in Table 8 below. LC-MS conditions are LC-MS (A).

**Table 8**

| **Ex.** | **Compound** | **t_{R} [min]** | **MS Data m/z [M+H]⁺** |
|---|---|---|---|
| **1.2.31.** | (2R,3R,4S,5R,6R)-4-(4-(2,3-difluoro-4-methylphenyl)-1H-1,2,3-triazol-1-yl)-6-((4-(1-hydroxycyclobutyl)-1H-1,2,3-triazol-1-yl)methyl)-2-(hydroxymethyl)-5-methoxytetrahydro-2H-pyran-3-ol | 0.76 | 507.20 |
| **1.2.32.** | (2R,3R,4S,5R,6R)-6-((4-cyclopentyl-1H-1,2,3-triazol-1-yl)methyl)-4-(4-(2,3-difluoro-4-methylphenyl)-1H-1,2,3-triazol-1-yl)-2-(hydroxymethyl)-5-methoxytetrahydro-2H-pyran-3-ol | 0.89 | 505.23 |
| 1.2.33. | (2R,3R,4S,5R,6R)-4-(4-(2,3-difluoro-4-methylphenyl)-1H-1,2,3-triazol-1-yl)-6-((4-(1-hydroxycyclopentyl)-1H-1,2,3-triazol-1-yl)methyl)-2-(hydroxymethyl)-5-methoxytetrahydro-2H-pyran-3-ol | 0.78 | 521.21 |
| **1.2.34.** | (2R,3R,4S,5R,6R)-4-(4-(2,3-difluoro-4-methylphenyl)-1H-1,2,3-triazol-1-yl)-2-(hydroxymethyl)-6-((4-(2-hydroxypropan-2-yl)-1H-1,2,3-triazol-1-yl)methyl)-5-methoxytetrahydro-2H-pyran-3-ol | 0.73 | 495.20 |
| **1.2.6.** | (2R,3R,4S,5R,6R)-4-(4-(2,3-difluoro-4-methylphenyl)-1H-1,2,3-triazol-1-yl)-2-(hydroxymethyl)-5-methoxy-6-((4-(3-methyloxetan-3-yl)-1H-1,2,3-triazol-1-yl)methyl)tetrahydro-2H-pyran-3-ol | 0.77 | 507.20 |
| **1.2.35.** | (2R,3R,4S,5R,6R)-4-(4-(2,3-difluoro-4-methylphenyl)-1H-1,2,3-triazol-1-yl)-2-(hydroxymethyl)-6-((4-(4-hydroxytetrahydro-2H-pyran-4-yl)-1H-1,2,3-triazol-1-yl)methyl)-5-methoxytetrahydro-2H-pyran-3-ol | 0.71 | 537.23 |
| **1.2.36.** | (2R,3R,4S,5R,6R)-4-(4-(2,3-difluoro-4-methylphenyl)-1H-1,2,3-triazol-1-yl)-6-((4-(1-hydroxycyclopropyl)-1H-1,2,3-triazol-1-yl)methyl)-2-(hydroxymethyl)-5-methoxytetrahydro-2H-pyran-3-ol | 0.74 | 493.17 |
| **1.2.4.** | (2R,3R,4S,5R,6R)-4-(4-(2,3-difluoro-4-methylphenyl)-1H-1,2,3-triazol-1-yl)-2-(hydroxymethyl)-6-((4-(1-(hydroxymethyl)cyclopropyl)-1H-1,2,3-triazol-1-yl)methyl)-5-methoxytetrahydro-2H-pyran-3-ol | 0.74 | 507.23 |
| **1.2.37.** | tert-butyl (4-(1-(((2R,3R,4S,5R,6R)-4-(4-(2,3-difluoro-4-methylphenyl)-1H-1,2,3-triazol-1-yl)-5-hydroxy-6-(hydroxymethyl)-3-methoxytetrahydro-2H-pyran-2-yl)methyl)-1H-1,2,3-triazol-4-yl)bicyclo[2.2.2]octan-1-yl)carbamate | 0.97 | 660.38 |
| **1.2.38.** | (2R,3R,4S,5R,6R)-4-(4-(2,3-difluoro-4-methylphenyl)-1H-1,2,3-triazol-1-yl)-6-((4-(3-ethyloxetan-3-yl)-1H-1,2,3-triazol-1-yl)methyl)-2-(hydroxymethyl)-5-methoxytetrahydro-2H-pyran-3-ol | 0.80 | 521.22 |
| **1.2.39.** | (2R,3R,4S,5R 6R)-6-((1-(4-aminobicyclo[2.2.2]octan-1-yl)-1H-1,2,3-triazol-4-yl)methyl)-4-(4-(2,3-difluoro-4-methylphenyl)-1H-1,2,3-triazol-1-yl)-2-(hydroxymethyl)-5-methoxytetrahydro-2H-pyran-3-ol | 0.66 | 560.32 |
| **2.2.31.** | (2R,3R,4S,5R,6R)-4-(4-(4-chloro-2,3-difluorophenyl)-1H-1,2,3-triazol-1-yl)-6-((4-(1-hydroxycyclobutyl)-1H-1,2,3-triazol-1-yl)methyl)-2-(hydroxymethyl)-5-methoxytetrahydro-2H-pyran-3-ol | 0.78 | 527.16 |
| **2.2.32.** | (2R,3R,4S,5R,6R)-4-(4-(4-chloro-2,3-difluorophenyl)-1H-1,2,3-triazol-1-yl)-6-((4-cyclopentyl-1H-1,2,3-triazol-1-yl)methyl)-2-(hydroxymethyl)-5-methoxytetrahydro-2H-pyran-3-ol | 0.91 | 525.17 |
| **2.2.33.** | (2R,3R,4S,5R,6R)-4-(4-(4-chloro-2,3-difluorophenyl)-1H-1,2,3-triazol-1-yl)-6-((4-(1-hydroxycyclopentyl)-1H-1,2,3-triazol-1-yl)methyl)-2-(hydroxymethyl)-5-methoxytetrahydro-2H-pyran-3-ol | 0.80 | 541.18 |
| **2.2.34.** | (2R,3R,4S,5R,6R)-4-(4-(4-chloro-2,3-difluorophenyl)-1H-1,2,3-triazol-1-yl)-2-(hydroxymethyl)-6-((4-(2-hydroxypropan-2-yl)-1H-1,2,3-triazol-1-yl)methyl)-5-methoxytetrahydro-2H-pyran-3-ol | 0.75 | 515.14 |
| **2.2.6.** | (2R,3R,4S,5R,6R)-4-(4-(4-chloro-2,3-difluorophenyl)-1H-1,2,3-triazol-1-yl)-2-(hydroxymethyl)-5-methoxy-6-((4-(3-methyloxetan-3-yl)-1H-1,2,3-triazol-1-yl)methyl)tetrahydro-2H-pyran-3-ol | 0.80 | 527.17 |
| **2.2.35.** | (2R,3R,4S,5R,6R)-4-(4-(4-chloro-2,3-difluorophenyl)-1H-1,2,3-triazol-1-yl)-2-(hydroxymethyl)-6-((4-(4-hydroxytetrahydro-2H-pyran-4-yl)-1H-1,2,3-triazol-1-yl)methyl)-5-methoxytetrahydro-2H-pyran-3-ol | 0.74 | 557.18 |
| **2.2.36.** | (2R,3R,4S,5R,6R)-4-(4-(4-chloro-2,3-difluorophenyl)-1H-1,2,3-triazol-1-yl)-6-((4-(1-hydroxycyclopropyl)-1H-1,2,3-triazol-1-yl)methyl)-2-(hydroxymethyl)-5-methoxytetrahydro-2H-pyran-3-ol | 0.76 | 513.13 |
| **2.2.4.** | (2R,3R,4S,5R,6R)-4-(4-(4-chloro-2,3-difluorophenyl)-1H-1,2,3-triazol-1-yl)-2-(hydroxymethyl)-6-((4-(1-(hydroxymethyl)cyclopropyl)-1H-1,2,3-triazol-1-yl)methyl)-5-methoxytetrahydro-2H-pyran-3-ol | 0.76 | 527.24 |
| **2.2.37.** | tert-butyl (4-(1-(((2R,3R,4S,5R,6R)-4-(4-(4-chloro-2,3-difluorophenyl)-1H-1,2,3-triazol-1-yl)-5-hydroxy-6-(hydroxymethyl)-3-methoxytetrahydro-2H-pyran-2-yl)methyl)-1H-1,2,3-triazol-4-yl)bicyclo[2.2.2]octan-1-yl)carbamate | 0.98 | 680.27 |
| **2.2.38.** | (2R,3R,4S,5R,6R)-4-(4-(4-chloro-2,3-difluorophenyl)-1H-1,2,3-triazol-1-yl)-6-((4-(3-ethyloxetan-3-yl)-1H-1,2,3-triazol-1-yl)methyl)-2-(hydroxymethyl)-5-methoxytetrahydro-2H-pyran-3-ol | 0.82 | 551.20 |
| **2.2.39.** | (2R,3R,4S,5R,6R)-6-((1-(4-aminobicyclo[2.2.2]octan-1-yl)-1H-1,2,3-triazol-4-yl)methyl)-4-(4-(4-chloro-2,3-difluorophenyl)-1H-1,2,3-triazol-1-yl)-2-(hydroxymethyl)-5-methoxytetrahydro-2H-pyran-3-ol | 0.68 | 580.27 |

Following examples are / can be prepared starting from either Intermediate 18a, 19a, or 20a, and the corresponding alkynes according to the procedures described for Example 1.2.34. LC-MS data are listed in Table 9 below. LC-MS conditions are LC-MS (A).

**Table 9**

| **Ex.** | **Compound** | **t_{R} [min]** | **MS Data m/z [M+H]⁺** |
|---|---|---|---|
| **3.2.31.** | (2R,3R,4S,5R,6R)-6-((4-(1-hydroxycyclobutyl)-1H-1,2,3-triazol-1-yl)methyl)-2-(hydroxymethyl)-5-methoxy-4-(4-(2,3,4-trifluorophenyl)-1H-1,2,3-triazol-1-yl)tetrahydro-2H-pyran-3-ol | 0.73 | 511.19 |
| **3.2.32.** | (2R,3R,4S,5R,6R)-6-((4-cyclopentyl-1H-1,2,3-triazol-1-yl)methyl)-2-(hydroxymethyl)-5-methoxy-4-(4-(2,3,4-trifluorophenyl)-1H-1,2,3-triazol-1-yl)tetrahydro-2H-pyran-3-ol | 0.87 | 509.21 |
| **3.2.33.** | (2R,3R,4S,5R,6R)-6-((4-(1-hydroxycyclopentyl)-1H-1,2,3-triazol-1-yl)methyl)-2-(hydroxymethyl)-5-methoxy-4-(4-(2,3,4-trifluorophenyl)-1H-1,2,3-triazol-1-yl)tetrahydro-2H-pyran-3-ol | 0.76 | 525.24 |
| **3.2.34.** | (2R,3R,4S,5R,6R)-2-(hydroxymethyl)-6-((4-(2-hydroxypropan-2-yl)-1H-1,2,3-triazol-1-yl)methyl)-5-methoxy-4-(4-(2,3,4-trifluorophenyl)-1H-1,2,3-triazol-1-yl)tetrahydro-2H-pyran-3-ol | 0.71 | 499.21 |
| **3.2.6.** | (2R,3R,4S,5R,6R)-2-(hydroxymethyl)-5-methoxy-6-((4-(3-methyloxetan-3-yl)-1H-1,2,3-triazol-1-yl)methyl)-4-(4-(2,3,4-trifluorophenyl)-1H-1,2,3-triazol-1-yl)tetrahydro-2H-pyran-3-ol | 0.75 | 511.21 |
| **3.2.35.** | (2R,3R,4S,5R,6R)-2-(hydroxymethyl)-6-((4-(4-hydroxytetrahydro-2H-pyran-4-yl)-1H-1,2,3-triazol-1-yl)methyl)-5-methoxy-4-(4-(2,3,4-trifluorophenyl)-1H-1,2,3-triazol-1-yl)tetrahydro-2H-pyran-3-ol | 0.69 | 541.23 |
| **3.2.36.** | (2R,3R,4S,5R,6R)-6-((4-(1-hydroxycyclopropyl)-1H-1,2,3-triazol-1-yl)methyl)-2-(hydroxymethyl)-5-methoxy-4-(4-(2,3,4-trifluorophenyl)-1H-1,2,3-triazol-1-yl)tetrahydro-2H-pyran-3-ol | 0.71 | 497.02 |
| **3.2.4.** | (2R,3R,4S,5R,6R)-2-(hydroxymethyl)-6-((4-(1-(hydroxymethyl)cyclopropyl)-1H-1,2,3-triazol-1-yl)methyl)-5-methoxy-4-(4-(2,3,4-trifluorophenyl)-1H-1,2,3-triazol-1-yl)tetrahydro-2H-pyran-3-ol | 0.72 | 511.2 |
| **3.2.38.** | (2R,3R,4S,5R,6R)-6-((4-(3-ethyloxetan-3-yl)-1H-1,2,3-triazol-1-yl)methyl)-2-(hydroxymethyl)-5-methoxy-4-(4-(2,3,4-trifluorophenyl)-1H-1,2,3-triazol-1-yl)tetrahydro-2H-pyran-3-ol | 0.78 | 525.24 |
| **3.2.37.** | tert-butyl (4-(1-(((2R,3R,4S,5R,6R)-5-hydroxy-6-(hydroxymethyl)-3-methoxy-4-(4-(2,3,4-trifluorophenyl)-1H-1,2,3-triazol-1-yl)tetrahydro-2H-pyran-2-yl)methyl)-1H-1,2,3-triazol-4-yl)bicyclo[2.2.2]octan-1-yl)carbamate | 0.95 | 664.36 |
| **3.2.39.** | (2R,3R,4S,5R,6R)-6-((1-(4-aminobicyclo[2.2.2]octan-1-yl)-1H-1,2,3-triazol-4-yl)methyl)-2-(hydroxymethyl)-5-methoxy-4-(4-(2,3,4-trifluorophenyl)-1H-1,2,3-triazol-1-yl)tetrahydro-2H-pyran-3-ol | 0.63 | 564.33 |
| **2.2.12.** | (2R,3R,4S,5R,6R)-6-((4-(bicyclo[1.1.1]pentan-1-yl)-1H-1,2,3-triazol-1-yl)methyl)-4-(4-(4-chloro-2,3-difluorophenyl)-1H-1,2,3-triazol-1-yl)-2-(hydroxymethyl)-5-methoxytetrahydro-2H-pyran-3-ol | 0.90 | 522.97 |
| **2.2.23.** | (2R,3R,4S,5R,6R)-4-(4-(4-chloro-2,3-difluorophenyl)-1H-1,2,3-triazol-1-yl)-2-(hydroxymethyl)-5-methoxy-6-((4-(1-methylcyclopropyl)-1H-1,2,3-triazol-1-yl)methyl)tetrahydro-2H-pyran-3-ol | 0.88 | 511.2 |
| **2.2.13.** | (2R,3R,4S,5R,6R)-4-(4-(4-chloro-2,3-difluorophenyl)-1H-1,2,3-triazol-1-yl)-2-(hydroxymethyl)-5-methoxy-6-((4-(1-methylcyclobutyl)-1H-1,2,3-triazol-1-yl)methyl)tetrahydro-2H-pyran-3-ol | 0.91 | 525.21 |
| **2.2.83.** | (2R,3R,4S,5R,6R)-4-(4-(4-chloro-2,3-difluorophenyl)-1H-1,2,3-triazol-1-yl)-6-((4-(3,3-difluorocyclobutyl)-1H-1,2,3-triazol-1-yl)methyl)-2-(hydroxymethyl)-5-methoxytetrahydro-2H-pyran-3-ol | 0.88 | 547.16 |
| **2.2.84.** | (2R,3R,4S,5R,6R)-4-(4-(4-chloro-2,3-difluorophenyl)-1H-1,2,3-triazol-1-yl)-6-((4-(6,6-difluorospiro[3.3]heptan-2-yl)-1H-1,2,3-triazol-1-yl)methyl)-2-(hydroxymethyl)-5-methoxytetrahydro-2H-pyran-3-ol | 0.95 | 587.23 |
| **2.2.51.** | (2R,3R,4S,5R,6R)-4-(4-(4-chloro-2,3-difluorophenyl)-1H-1,2,3-triazol-1-yl)-6-((4-(1-(difluoromethyl)cyclopropyl)-1H-1,2,3-triazol-1-yl)methyl)-2-(hydroxymethyl)-5-methoxytetrahydro-2H-pyran-3-ol | 0.87 | 547.17 |
| **2.2.85.** | (2R,3R,4S,5R,6R)-4-(4-(4-chloro-2,3-difluorophenyl)-1H-1,2,3-triazol-1-yl)-6-((4-(3,3-difluoro-1-hydroxycyclobutyl)-1H-1,2,3-triazol-1-yl)methyl)-2-(hydroxymethyl)-5-methoxytetrahydro-2H-pyran-3-ol | 0.82 | 563.13 |
| **2.2.46.** | (2R,3R,4S,5R,6R)-4-(4-(4-chloro-2,3-difluorophenyl)-1H-1,2,3-triazol-1-yl)-6-((4-(1-(fluoromethyl)cyclopropyl)-1H-1,2,3-triazol-1-yl)methyl)-2-(hydroxymethyl)-5-methoxytetrahydro-2H-pyran-3-ol | 0.86 | 528.79 |
| **2.2.54.** | (2R,3R,4S,5R,6R)-4-(4-(4-chloro-2,3-difluorophenyl)-1H-1,2,3-triazol-1-yl)-2-(hydroxymethyl)-5-methoxy-6-((4-(1-(trifluoromethyl)cyclopropyl)-1H-1,2,3-triazol-1-yl)methyl)tetrahydro-2H-pyran-3-ol | 0.92 | 564.83 |
| **2.2.86.** | tert-butyl ((1S,3S)-3-(1-(((2R,3R,4S,5R,6R)-4-(4-(4-chloro-2,3-difluorophenyl)-1H-1,2,3-triazol-1-yl)-5-hydroxy-6-(hydroxymethyl)-3-methoxytetrahydro-2H-pyran-2-yl)methyl)-1H-1,2,3-triazol-4-yl)cyclobutyl)carbamate | 0.92 | 625.81 |
| **2.2.87.** | (2R,3R,4S,5R,6R)-6-((4-((1S,3S)-3-aminocyclobutyl)-1H-1,2,3-triazol-1-yl)methyl)-4-(4-(4-chloro-2,3-difluorophenyl)-1H-1,2,3-triazol-1-yl)-2-(hydroxymethyl)-5-methoxytetrahydro-2H-pyran-3-ol | 0.65 | 526.02 |
| **3.2.23.** | (2R,3R,4S,5R,6R)-2-(hydroxymethyl)-5-methoxy-6-((4-(1-methylcyclopropyl)-1H-1,2,3-triazol-1-yl)methyl)-4-(4-(2,3,4-trifluorophenyl)-1H-1,2,3-triazol-1-yl)tetrahydro-2H-pyran-3-ol | 0.83 | 494.9 |
| **3.2.13.** | (2R,3R,4S,5R,6R)-2-(hydroxymethyl)-5-methoxy-6-((4-(1-methylcyclobutyl)-1H-1,2,3-triazol-1-yl)methyl)-4-(4-(2,3,4-trifluorophenyl)-1H-1,2,3-triazol-1-yl)tetrahydro-2H-pyran-3-ol | 0.86 | 508.9 |
| **3.2.12.** | (2R,3R,4S,5R,6R)-6-((4-(bicyclo[1.1.1]pentan-1-yl)-1H-1,2,3-triazol-1-yl)methyl)-2-(hydroxymethyl)-5-methoxy-4-(4-(2,3,4-trifluorophenyl)-1H-1,2,3-triazol-1-yl)tetrahydro-2H-pyran-3-ol | 0.85 | 506.88 |
| **4.2.31.** | (2R,3R,4S,5R,6R)-4-(4-(4-bromo-2,3-difluorophenyl)-1H-1,2,3-triazol-1-yl)-6-((4-(1-hydroxycyclobutyl)-1H-1,2,3-triazol-1-yl)methyl)-2-(hydroxymethyl)-5-methoxytetrahydro-2H-pyran-3-ol | 0.77 | 573.17 |
| **4.2.32.** | (2R,3R,4S,5R,6R)-4-(4-(4-bromo-2,3-difluorophenyl)-1H-1,2,3-triazol-1-yl)-6-((4-cyclopentyl-1H-1,2,3-triazol-1-yl)methyl)-2-(hydroxymethyl)-5-methoxytetrahydro-2H-pyran-3-ol | 0.9 | 569.23 |
| **4.2.33.** | (2R,3R,4S,5R,6R)-4-(4-(4-bromo-2,3-difluorophenyl)-1H-1,2,3-triazol-1-yl)-6-((4-(1-hydroxycyclopentyl)-1H-1,2,3-triazol-1-yl)methyl)-2-(hydroxymethyl)-5-methoxytetrahydro-2H-pyran-3-ol | 0.8 | 585.2 |
| **4.2.34.** | (2R,3R,4S,5R,6R)-4-(4-(4-bromo-2,3-difluorophenyl)-1H-1,2,3-triazol-1-yl)-2-(hydroxymethyl)-6-((4-(2-hydroxypropan-2-yl)-1H-1,2,3-triazol-1-yl)methyl)-5-methoxytetrahydro-2H-pyran-3-ol | 0.75 | 559.15 |
| **4.2.6.** | (2R,3R,4S,5R,6R)-4-(4-(4-bromo-2,3-difluorophenyl)-1H-1,2,3-triazol-1-yl)-2-(hydroxymethyl)-5-methoxy-6-((4-(3-methyloxetan-3-yl)-1H-1,2,3-triazol-1-yl)methyl)tetrahydro-2H-pyran-3-ol | 0.79 | 571.19 |
| **4.2.35.** | (2R,3R,4S,5R,6R)-4-(4-(4-bromo-2,3-difluorophenyl)-1H-1,2,3-triazol-1-yl)-2-(hydroxymethyl)-6-((4-(4-hydroxytetrahydro-2H-pyran-4-yl)-1H-1,2,3-triazol-1-yl)methyl)-5-methoxytetrahydro-2H-pyran-3-ol | 0.73 | 601.21 |
| **4.2.36.** | (2R,3R,4S,5R,6R)-4-(4-(4-bromo-2,3-difluorophenyl)-1H-1,2,3-triazol-1-yl)-6-((4-(1-hydroxycyclopropyl)-1H-1,2,3-triazol-1-yl)methyl)-2-(hydroxymethyl)-5-methoxytetrahydro-2H-pyran-3-ol | 0.76 | 557.17 |
| **4.2.4.** | (2R,3R,4S,5R,6R)-4-(4-(4-bromo-2,3-difluorophenyl)-1H-1,2,3-triazol-1-yl)-2-(hydroxymethyl)-6-((4-(1-(hydroxymethyl)cyclopropyl)-1H-1,2,3-triazol-1-yl)methyl)-5-methoxytetrahydro-2H-pyran-3-ol | 0.76 | 573.19 |
| **4.2.38.** | (2R,3R,4S,5R,6R)-4-(4-(4-bromo-2,3-difluorophenyl)-1H-1,2,3-triazol-1-yl)-6-((4-(3-ethyloxetan-3-yl)-1H-1,2,3-triazol-1-yl)methyl)-2-(hydroxymethyl)-5-methoxytetrahydro-2H-pyran-3-ol | 0.82 | 585.19 |
| **4.2.37.** | tert-butyl (4-(1-(((2R,3R,4S,5R,6R)-4-(4-(4-bromo-2,3-difluorophenyl)-1H-1,2,3-triazol-1-yl)-5-hydroxy-6-(hydroxymethyl)-3-methoxytetrahydro-2H-pyran-2-yl)methyl)-1H-1,2,3-triazol-4-yl)bicyclo[2.2.2]octan-1-yl)carbamate | 0.98 | 724.25 |
| **4.2.39.** | (2R,3R,4S,5R,6R)-6-((4-(4-aminobicyclo[2.2.2]octan-1-yl)-1H-1,2,3-triazol-1-yl)methyl)-4-(4-(4-bromo-2,3-difluorophenyl)-1H-1,2,3-triazol-1-yl)-2-(hydroxymethyl)-5-methoxytetrahydro-2H-pyran-3-ol | 0.69 | 623.97 |
| **4.2.23.** | (2R,3R,4S,5R,6R)-4-(4-(4-bromo-2,3-difluorophenyl)-1H-1,2,3-triazol-1-yl)-2-(hydroxymethyl)-5-methoxy-6-((4-(1-methylcyclopropyl)-1H-1,2,3-triazol-1-yl)methyl)tetrahydro-2H-pyran-3-ol | 0.88 | 554.8 |
| **4.2.13.** | (2R,3R,4S,5R,6R)-4-(4-(4-bromo-2,3-difluorophenyl)-1H-1,2,3-triazol-1-yl)-2-(hydroxymethyl)-5-methoxy-6-((4-(1-methylcyclobutyl)-1H-1,2,3-triazol-1-yl)methyl)tetrahydro-2H-pyran-3-ol | 0.91 | 568.8 |
| **4.2.12.** | (2R,3R,4S,5R,6R)-6-((4-(bicyclo[1.1.1]pentan-1-yl)-1H-1,2,3-triazol-1-yl)methyl)-4-(4-(4-bromo-2,3-difluorophenyl)-1H-1,2,3-triazol-1-yl)-2-(hydroxymethyl)-5-methoxytetrahydro-2H-pyran-3-ol | 0.9 | 566.97 |

### II. Biological Assays

### Evaluation of compound inhibitory activity (IC₅₀)

The inhibitory activity of compounds is determined in competitive binding assays. This spectrophotometric assay measures the binding of biotinylated human Gal-3 (hGal-3) or human Gal-1 (hGal-1), respectively, to a microplate-adsorbed glycoprotein, asialofetuin (ASF) (Proc Natl Acad Sci U S A. 2013 Mar 26;110(13):5052-7.). Alternatively, and preferably, a human Gal-1 version in which all six cysteines are substituted by serines may be used.

Briefly, compounds are serially diluted in DMSO (working dilutions). ASF-coated 384well plates are supplemented with 22.8 µL/well of biotinylated hGal-3 or hGal-1 in assay buffer (i.e. 300-1000 ng/mL biotinylated hGal-3 or hGal-1) to which 1.2 µL of compound working dilutions are added and mixed.

Plates are incubated for 3 hours at 4°C, then washed with cold assay buffer (3x50uL), incubated for 1 hour with 25 µL/well of a streptavidin-peroxidase solution (diluted in assay buffer to 80 ng/mL) at 4°C, followed by further washing steps with assay buffer (3x50uL). Finally, 25 µL/well of ABTS substrate is added. OD (410nm) is recorded after 30 to 45min and IC₅₀ values are calculated.

The calculated IC₅₀ values may fluctuate depending on the daily assay performance. Fluctuations of this kind are known to those skilled in the art. IC₅₀ values from several measurements are given as geomean values.

### Activity on hGal-3 (IC₅₀ in µM)

**Table 10**

| **Ex** | **Gal-3 IC₅₀** | **Ex** | **Gal-3 IC₅₀** | **Ex** | **Gal-3 IC₅₀** | **Ex** | **Gal-3 IC₅₀** |
|---|---|---|---|---|---|---|---|
| **1.1.1.** | 0.05 | **1.1.2.** | 0.04 | **1.1.3.** | 0.04 | **1.1.4.** | 0.04 |
| **1.1.5.** | 0.05 | **1.1.6.** | 0.04 | **1.1.7.** | 0.06 | **1.1.8.** | 0.05 |
| **1.1.9.** | 0.07 | **1.1.10.** | 0.06 | **1.1.11.** | 0.04 | **1.1.12.** | 0.05 |
| **1.1.13.** | 0.03 | **1.1.14.** | 0.04 | **1.1.15.** | 0.03 | **1.1.16.** | 0.05 |
| **1.1.17.** | 0.11 | **1.1.18.** | 0.05 | **1.1.19.** | 0.02 | **1.1.20.** | 0.05 |
| **1.1.21.** | 0.04 | **1.1.22.** | 0.04 | **1.1.23.** | 0.02 | **1.1.24.** | 0.08 |
| **1.1.25.** | 0.04 | **1.1.26.** | 0.06 | **1.1.27.** | 0.03 | **2.1.7.** | 0.06 |
| **2.1.28.** | 0.04 | **2.1.4.** | 0.04 | **2.1.5.** | 0.06 | **2.1.11.** | 0.05 |
| **2.1.8.** | 0.03 | **2.1.9.** | 0.04 | **2.1.10.** | 0.06 | **2.1.12.** | 0.05 |
| **2.1.13.** | 0.02 | **2.1.6.** | 0.04 | **2.1.14.** | 0.05 | **2.1.29.** | 0.06 |
| **2.1.15.** | 0.05 | **2.1.16.** | 0.09 | **2.1.17.** | 0.16 | **2.1.18.** | 0.04 |
| **2.1.19.** | 0.04 | **2.1.20.** | 0.06 | **2.1.21.** | 0.05 | **2.1.22.** | 0.05 |
| **2.1.23.** | 0.02 | **2.1.24.** | 0.08 | **2.1.25.** | 0.05 | **2.1.26.** | 0.1 |
| **2.1.27.** | 0.03 | **3.1.5.** | 0.06 | **3.1.4.** | 0.06 | **3.1.7.** | 0.12 |
| **3.1.12.** | 0.06 | **3.1.11.** | 0.04 | **3.1.25.** | 0.04 | **3.1.24.** | 0.09 |
| **3.1.15.** | 0.05 | **3.1.18.** | 0.09 | **3.1.16.** | 0.12 | **3.1.27.** | 0.03 |
| **3.1.19.** | 0.06 | **3.1.21.** | 0.06 | **3.1.22.** | 0.06 | **3.1.23.** | 0.05 |
| **4.1.6.** | 0.04 | **4.1.13.** | 0.02 | **4.1.12.** | 0.04 | **4.1.21.** | 0.07 |
| **4.1.4.** | 0.04 | **4.1.3.** | 0.04 | | | | |
| **2.1.40.** | 0.05 | **2.1.41.** | 0.13 | **2.1.42.** | 0.04 | **2.1.43.** | 0.02 |
| **2.1.44.** | 0.03 | **2.1.46.** | 0.03 | **3.1.26.** | 0.07 | **3.1.14.** | 0.06 |
| **3.1.13.** | 0.03 | **3.1.44** | 0.04 | **3.1.82.** | 0.05 | **3.1.62.** | 0.06 |
| **3.1.51.** | 0.04 | **4.1.15.** | 0.05 | **4.1.19.** | 0.05 | **4.1.22.** | 0.07 |
| **4.1.23.** | 0.03 | **4.1.44.** | 0.04 | **4.1.24.** | 0.13 | **4.1.29.** | 0.05 |
| **2.1.65.A** | 0.06 | **2.1.52.** | 0.02 | **2.1.53.** | 0.02 | **2.1.58.** | 0.02 |
| **2.1.59.** | 0.05 | **2.1.62.** | 0.03 | **2.1.63.A** | 0.06 | **2.1.63.B** | 0.04 |
| **2.1.64.A** | 0.03 | **2.1.64.B** | 0.03 | **2.1.66.C** | 0.02 | **2.1.66.D** | 0.01 |
| **2.1.68.A** | 0.03 | **2.1.68.B** | 0.02 | **2.1.71.** | 0.05 | **2.1.72.** | 0.05 |
| **2.1.74.A** | 0.01 | **2.1.74.B** | 0.04 | **2.1.74.C** | 0.06 | **2.1.74.D** | 0.07 |
| **2.1.75.** | 0.05 | **2.1.76.** | 0.02 | **2.1.77.A** | 0.03 | **2.1.77.B** | 0.03 |
| **2.1.78.A** | 0.04 | **2.1.78.B** | 0.02 | **2.1.79.A** | 0.02 | **2.1.79.B** | 0.03 |
| **2.1.79.CD** | 0.06 | **2.1.80.A** | 0.02 | **2.1.80.B** | 0.03 | **2.1.81.B** | 0.04 |
| **2.1.81.C** | 0.06 | **2.1.81.D** | 0.06 | **2.1.88.A** | 0.03 | **2.1.88.B** | 0.03 |
| **2.1.88.C** | 0.05 | **2.1.88.D** | 0.04 | **2.1.89.A** | 0.01 | **2.1.89.B** | 0.02 |
| **2.1.90.A** | 0.03 | **2.1.90.B** | 0.02 | | | | |
| **3.1.6.** | 0.05 | **3.1.46.** | 0.07 | **3.1.43.** | 0.03 | **3.1.28.** | 0.03 |
| **3.1.40.** | 0.07 | **3.1.55.** | 0.04 | **3.1.58.** | 0.04 | **3.1.66.C** | 0.04 |
| **3.1.66.D** | 0.02 | **4.1.46.** | 0.03 | **4.1.59.** | 0.03 | **4.1.52.** | 0.03 |
| **4.1.62.** | 0.02 | **4.1.25.** | 0.05 | **4.1.55.** | 0.03 | **4.1.40.** | 0.06 |
| **4.1.64.A** | 0.03 | **4.1.64.B** | 0.05 | **4.1.14.** | 0.06 | **4.1.79.CD** | 0.07 |
| **4.1.63.AB** | 0.07 | **4.1.88.AB** | 0.03 | **1.1.53.** | 0.02 | **2.1.45.** | 0.04 |
| **2.1.47.A** | 0.03 | **2.1.47.B** | 0.03 | **2.1.54.** | 0.03 | **2.1.55.** | 0.02 |
| **2.1.56.** | 0.05 | **2.1.57.** | 0.5 | **2.1.60.A** | 0.02 | **2.1.60.B** | 0.04 |
| **2.1.67.** | 0.04 | **2.1.69.** | 0.04 | **2.1.70.** | 0.05 | **2.1.66.A** | 0.01 |
| **2.1.66.B** | 0.04 | **2.1.73.A** | 0.03 | **2.1.73.B** | 0.06 | **3.1.59.** | 0.04 |
| **3.1.29.** | 0.03 | **3.1.52.** | 0.03 | **3.1.53.** | 0.02 | **4.1.28.** | 0.05 |
| **4.1.43.** | 0.03 | **4.1.53.** | 0.02 | **4.1.58.** | 0.02 | **1.1.58.** | 0.03 |
| **2.1.51.** | 0.02 | **3.1.54.** | 0.05 | **4.1.51.** | 0.03 | **4.1.54.** | 0.03 |
| | | **2.1.49.** | 0.03 | **2.1.50.** | 0.07 | **5.1.53.** | 0.06 |
| **5.1.58.** | 0.07 | **5.1.6.** | 0.07 | **5.1.43.** | 0.03 | **5.1.66.CD** | 0.03 |
| **1.2.31.** | 0.06 | **1.2.32.** | 0.06 | **1.2.33.** | 0.11 | **1.2.34.** | 0.08 |
| **1.2.6.** | 0.04 | **1.2.35.** | 0.07 | **1.2.36.** | 0.08 | **1.2.4.** | 0.04 |
| **1.2.37.** | 0.02 | **1.2.38.** | 0.03 | **1.2.39.** | 0.06 | **2.2.31.** | 0.09 |
| **2.2.32.** | 0.05 | **2.2.33.** | 0.08 | **2.2.34.** | 0.07 | **2.2.6.** | 0.03 |
| **2.2.35.** | 0.06 | **2.2.36.** | 0.09 | **2.2.4.** | 0.05 | **2.2.37.** | 0.07 |
| **2.2.38.** | 0.03 | **2.2.39.** | 0.07 | **3.2.31.** | 0.1 | **3.2.32.** | 0.06 |
| **3.2.33.** | 0.09 | **3.2.34.** | 0.44 | **3.2.6.** | 0.07 | **3.2.35.** | 0.06 |
| **3.2.36.** | 0.13 | **3.2.4.** | 0.1 | **3.2.38.** | 0.05 | **3.2.37.** | 0.04 |
| **3.2.39.** | 0.05 | | | | | | |
| **2.2.12.** | 0.05 | **2.2.23.** | 0.06 | **2.2.13.** | 0.03 | **2.2.83.** | 0.16 |
| **2.2.84.** | 0.24 | **2.2.51.** | 0.06 | **2.2.85.** | 0.17 | **2.2.46.** | 0.06 |
| **2.2.54.** | 0.07 | **2.2.86.** | 0.06 | **2.2.87.** | 0.12 | **3.2.23.** | 0.08 |
| **3.2.13.** | 0.08 | **3.2.12.** | 0.04 | **3.2.31.** | 0.09 | **4.2.32.** | 0.06 |
| **4.2.33.** | 0.1 | **4.2.34.** | 0.1 | **4.2.6.** | 0.07 | **4.2.35.** | 0.07 |
| **4.2.36.** | 0.13 | **4.2.4.** | 0.07 | **4.2.38.** | 0.05 | **4.2.23.** | 0.05 |
| **4.2.37.** | 0.05 | **4.2.13.** | 0.05 | **4.2.39.** | 0.06 | **4.2.12.** | 0.09 |

### Activities on hGal-1 IC50 (µM)

**Table 11**

| **Ex** | **Gal-1 IC₅₀** | **Ex** | **Gal-1 IC₅₀** | **Ex** | **Gal-1 IC₅₀** | **Ex** | **Gal-1 IC₅₀** |
|---|---|---|---|---|---|---|---|
| **1.1.1.** | 2.61 | **1.1.2.** | 6.46 | **1.1.3.** | 3.15 | **1.1.4.** | 2.57 |
| **1.1.5.** | 2.57 | **1.1.6.** | 2.21 | **1.1.7.** | 5.1 | **1.1.8.** | 4.01 |
| **1.1.9.** | 44.01 | **1.1.10.** | 4.37 | **1.1.11.** | 2.09 | **1.1.12.** | 4.15 |
| **1.1.13.** | 1.45 | **1.1.14.** | 2.61 | **1.1.15.** | 1.76 | **1.1.16.** | 3.69 |
| **1.1.17.** | 5 | **1.1.18.** | 1.47 | **1.1.19.** | 0.67 | **1.1.20.** | 3.44 |
| **1.1.21.** | 0.84 | **1.1.22.** | 0.69 | **1.1.23.** | 1.06 | **1.1.24.** | 5.04 |
| **1.1.25.** | 3.95 | **1.1.26.** | 17.1 | **1.1.27.** | 1.41 | **2.1.7.** | 2.95 |
| **2.1.28.** | 0.78 | **2.1.4.** | 3.05 | **2.1.5.** | 2.25 | **2.1.11.** | 2.51 |
| **2.1.8.** | 4.43 | **2.1.9.** | 1.67 | **2.1.10.** | 2.26 | **2.1.12.** | 3.25 |
| **2.1.13.** | 0.69 | **2.1.6.** | 0.76 | **2.1.14.** | 3.45 | **2.1.29.** | 2.1 |
| **2.1.15.** | 1.71 | **2.1.16.** | 5.93 | **2.1.17.** | 11.5 | **2.1.18.** | 2.81 |
| **2.1.19.** | 0.63 | **2.1.20.** | 2.06 | **2.1.21.** | 0.9 | **2.1.22.** | 0.68 |
| **2.1.23.** | 1.09 | **2.1.24.** | 6.7 | **2.1.25.** | 1.96 | **2.1.26.** | 7.86 |
| **2.1.27.** | 0.7 | **3.1.5.** | 0.6 | **3.1.4.** | 0.81 | **3.1.7.** | 1.2 |
| **3.1.12.** | 0.95 | **3.1.11.** | 0.89 | **3.1.25.** | 1.55 | **3.1.24.** | 2.08 |
| **3.1.15.** | 0.46 | **3.1.18.** | 1.39 | **3.1.16.** | 1.11 | **3.1.27.** | 0.14 |
| **3.1.19.** | 0.26 | **3.1.21.** | 0.77 | **3.1.22.** | 0.42 | **3.1.23.** | 0.39 |
| **4.1.6.** | 1.46 | **4.1.13.** | 2.2 | **4.1.12.** | 3.92 | **4.1.21.** | 1.53 |
| **4.1.4.** | 2.93 | **4.1.3.** | 4.7 | | | | |
| **2.1.40.** | 1.63 | **2.1.41.** | 9.17 | **2.1.42.** | 1.83 | **2.1.43.** | 0.61 |
| **2.1.44.** | 2.1 | **2.1.46.** | 1.07 | **3.1.26.** | 0.76 | **3.1.14.** | 0.7 |
| **3.1.13.** | 0.29 | **3.1.44.** | 0.8 | **3.1.82.** | 0.37 | **3.1.62.** | 0.81 |
| **3.1.51.** | 0.24 | **4.1.15.** | 2.41 | **4.1.19.** | 1.82 | **4.1.22.** | 1.14 |
| **4.1.23.** | 1.8 | **4.1.44.** | 3.3 | **4.1.24.** | 28.4 | **4.1.29.** | 3.63 |
| **2.1.65.A** | 1.8 | **2.1.52.** | 0.65 | **2.1.53.** | 0.42 | **2.1.58.** | 0.47 |
| **2.1.59.** | 2.37 | **2.1.62.** | 1.2 | **2.1.63.A** | 2.17 | **2.1.63.B** | 2 |
| **2.1.64.A** | 0.94 | **2.1.64.B** | 2.34 | **2.1.66.C** | 0.95 | **2.1.66.D** | 0.78 |
| **2.1.68.A** | 1.1 | **2.1.68.B** | 0.67 | **2.1.71.** | 0.67 | **2.1.72.** | 0.48 |
| **2.1.74.A** | 0.45 | **2.1.74.B** | 1.62 | **2.1.74.C** | 3.94 | **2.1.74.D** | 3.54 |
| **2.1.75.** | 2.78 | **2.1.76.** | 0.81 | **2.1.77.A** | 0.76 | **2.1.77.B** | 0.59 |
| **2.1.78.A** | 0.36 | **2.1.78.B** | 0.24 | **2.1.79.A** | 1.51 | **2.1.79.B** | 3.16 |
| **2.1.79.CD** | 3.48 | **2.1.80.A** | 1.02 | **2.1.80.B** | 1.74 | **2.1.81.B** | 3.05 |
| **2.1.81.C** | 2.92 | **2.1.81.D** | 2.18 | **2.1.88.A** | 1.77 | **2.1.88.B** | 0.92 |
| **2.1.88.C** | 1.68 | **2.1.88.D** | 2.27 | **2.1.89.A** | 0.1 | **2.1.89.B** | 0.87 |
| **2.1.90.A** | 1.13 | **2.1.90.B** | 0.91 | | | | |
| **3.1.6.** | 0.19 | **3.1.46.** | 0.4 | **3.1.43.** | 0.21 | **3.1.28.** | 0.29 |
| **3.1.40.** | 0.26 | **3.1.55.** | 0.18 | **3.1.58.** | 0.2 | **3.1.66.C** | 0.27 |
| **3.1.66.D** | 0.31 | **4.1.46.** | 1.94 | **4.1.59.** | 2.03 | **4.1.52.** | 0.85 |
| **4.1.62.** | 2.03 | **4.1.25.** | 3.22 | **4.1.55.** | 1.48 | **4.1.40.** | 1.03 |
| **4.1.64.A** | 1.74 | **4.1.64.B** | 1.81 | **4.1.14.** | 1.36 | **4.1.79.CD** | 4.96 |
| **4.163.AB** | 3.19 | **4.1.88.AB** | 2.45 | **1.1.53.** | 0.52 | **2.1.45.** | 1.49 |
| **2.1.47.A** | 1.37 | **2.1.47.B** | 1.2 | **2.1.54.** | 0.7 | **2.1.55.** | 0.62 |
| **2.1.56.** | 0.82 | **2.1.57.** | 28.1 | **2.1.60.A** | 0.69 | **2.1.60.B** | 1.19 |
| **2.1.67.** | 1.03 | **2.1.69.** | 0.47 | **2.1.70.** | 0.83 | **2.1.66.A** | 0.57 |
| **2.1.66.B** | 2.9 | **2.1.73.A** | 1.4 | **2.1.73.B** | 1.2 | **3.1.59.** | 0.52 |
| **3.1.29.** | 0.76 | **3.1.52.** | 0.24 | **3.1.53.** | 0.13 | **4.1.28.** | 1.02 |
| **4.1.43.** | 0.96 | **4.1.53.** | 0.65 | **4.1.58.** | 1.07 | **1.1.58.** | 0.8 |
| **2.1.51.** | 0.83 | **3.1.54.** | 0.28 | **4.1.51.** | 1.29 | **4.1.54.** | 1.17 |
| | | **2.1.49.** | 1.33 | **2.1.50.** | 1.6 | **5.1.53.** | 1.35 |
| **5.1.58.** | 1.91 | **5.1.6.** | 3.99 | **5.1.43.** | 1.32 | **5.1.66.CD** | 1.6 |
| **1.2.31.** | 3.76 | **1.2.32.** | 5.13 | **1.2.33.** | 5.7 | **1.2.34.** | 5.33 |
| **1.2.6.** | 2.97 | **1.2.35.** | 4.5 | **1.2.36.** | 7.71 | **1.2.4.** | 2.46 |
| **1.2.37.** | 9.4 | **1.2.38.** | 2.65 | **1.2.39.** | 11 | **2.2.31.** | 2.63 |
| **2.2.32.** | 3.81 | **2.2.33.** | 4.78 | **2.2.34.** | 5.12 | **2.2.6.** | 1.17 |
| **2.2.35.** | 3.91 | **2.2.36.** | 3.16 | **2.2.4.** | 1.48 | **2.2.37.** | 13.1 |
| **2.2.38.** | 1.4 | **2.2.39.** | 6.07 | **3.2.31.** | 0.73 | **3.2.32.** | 1.15 |
| **3.2.33.** | 1.25 | **3.2.34.** | 0.72 | **3.2.6.** | 0.58 | **3.2.35.** | 0.86 |
| **3.2.36.** | 2.14 | **3.2.4.** | 0.65 | **3.2.38.** | 0.47 | **3.2.37.** | 1.72 |
| **3.2.39.** | 0.81 | | | | | | |
| **2.2.12.** | 3.4 | **2.2.23.** | 1.71 | **2.2.13.** | 1.51 | **2.2.83.** | 8.8 |
| **2.2.84.** | 11 | **2.2.51.** | 2.55 | **2.2.85.** | 5.4 | **2.2.46.** | 2.3 |
| **2.2.54.** | 2.46 | **2.2.86.** | 2.57 | **2.2.87.** | 7.15 | **3.2.23.** | 0.3 |
| **3.2.13.** | 0.67 | **3.2.12.** | 0.97 | **4.2.31.** | 2.55 | **4.2.32.** | 2.68 |
| **4.2.33.** | 4.11 | **4.2.34.** | 5.49 | **4.2.6.** | 2.53 | **4.2.35.** | 4.6 |
| **4.2.36.** | 5.91 | **4.2.4.** | 4.64 | **4.2.38.** | 2.26 | **4.2.23.** | 2.48 |
| **4.2.37.** | 11.3 | **4.2.13.** | 1.93 | **4.2.39.** | 2.19 | **4.2.12.** | 3.31 |

Compounds of the present invention may be further characterized with regard to their potency /selectivity in competitive binding assays, using for example Gal-1, Gal-2, Gal-4N, Gal-4C, Gal-8N, Gal-8C, Gal-9N, Gal-9C, Gal-10 as probes; with regard to their potency using impedance-based cellular assays measuring inhibition of Gal-3-induced cellular shape changes; with regard to their potency in inhibiting hepatic stellate cell activation or inhibiting T cell apoptosis; with regard to their potency in thermal shift assays measuring the ability of compounds preventing thermal denaturation of purified Gal-3 or Gal-3 in cells including those in blood and organs; with regard to their potency in inhibiting intracellular Gal-3 recruitment to sites of organellar injury (Stegmayr et al., 2019; doi.org/10.1038/s41598-019-38497-8); or with regard to their thermodynamic and kinetics interaction profile with Gal-3 in using conventional assays well known in the art, for example using surface plasma resonance assays (Biacore) or isothermal calorimetry assays (ITC) measuring binding enthalpy, binding entropy, binding affinity, on-rates and off-rates of the Gal-3-compound interaction.

Compounds of the present invention may be further characterized with regard to their general pharmacokinetic and pharmacological properties using conventional assays well known in the art; for example for their properties with regard to drug safety and/or toxicological properties using conventional assays well known in the art, for example relating to cytochrome P450 enzyme inhibition and time dependent inhibition, pregnane X receptor (PXR) activation, glutathione binding, or their ability to bind to different proteins using for example plasma protein binding assay, or their ability to enter the blood cells using for example a blood to plasma distribution coefficient assay; for example for their in vitro metabolic stability using (human) liver microsomes assay or fresh (human) hepatocytes assay; or their permeation ability using for example a Caco-2 (human colon carcinoma cell line) or MDCK (Madin Darby canine kidney cell line) cell assay; or relating to their ability to cross the blood-brain barrier, using for example a human P-glycoprotein 1 (MDR 1) substrate assay, or relating to their bioavailability in different species (such as rat or dog);

Compounds of the present invention may be further characterized with regard to their cardiovascular safety behavior, using for example a human induced pluripotent stem cell (iPSC)-derived cardiomyocytes assay or their effect on the Kv11.1 channel, a potassium ion channel encoded by the human ether-à-go-go-related gene (hERG channel), using for example the patch-clamp measurement of effect on the hERG K+ currents in Chinese Hamster Ovary (CHO) cell assay.

Compounds of the present invention may be further characterized with regard to their effect on cell viability using the commercial CellTiter-Glo luminescent assay to quantify cellular ATP as marker of metabolically active cells. Compounds may be further assessed for their cytotoxic effect using fluorescent marker dyes to label and quantify live cells from dead cells.

## Claims

1. A compound of formula (I) 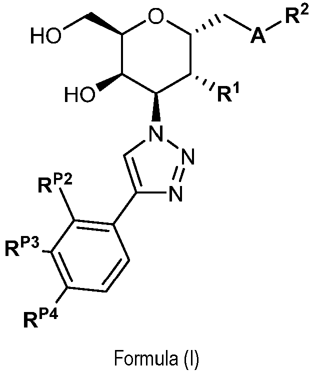 wherein
**R^{P2}** represents halogen;
**R^{P3}** represents halogen;
**R^{P4}** represents halogen, methyl or cyano;
**R¹** represents
• hydroxy;
• C₁₋₄-alkoxy;
• -O-CO-C₁₋₃-alkyl;
• O-CO-NH-**R**^{**N1**1} wherein **R^{N11}** represents hydrogen or C₁₋₃-alkyl;
• -O-CH₂-C₁-fluoroalkyl;
• -O-CH₂-**HET¹** wherein **HET¹** represents a 5-membered heteroaryl wherein said 5-membered heteroaryl independently is unsubstituted or mono-substituted with methyl; or
• -O-CH₂-CO-**R^{1X}** wherein **R^{1X}**represents
➢ -hydroxy;
➢ C₁₋₃-alkoxy;
➢ morpholin-4-yl; or
❖ -**NR^{N21}R^{N22}** wherein **R^{N21}** and **R^{N22}** both independently represent hydrogen or methyl; or **R^{N21}** and **R^{N22}** together with the nitrogen atom to which they are attached form a 4- to 6-membered mono-cyclic heterocycloalkyl selected from azetidine-1-yl, pyrrolidine-1-yl, and piperidine-1-yl, wherein said 4- to 6-membered heterocycloalkyl is mono-substituted with hydroxy;
A represents [1,2,3]triazol-1,4-diyl; and
• **R²** represents branched C₃₋₆-alkyl wherein said branched C₃₋₆-alkyl is
➢ mono-substituted with hydroxy,
➢ mono-substituted with -CO-O-C₁₋₄-alkyl, or
➢ mono-substituted with C₁-fluoroalkyl;
• or **R²** represents a saturated 3- to 8-membered mono- or bicyclic group, wherein said mono- or bicyclic group is
❖ mono-cyclic C₃₋₆-cycloalkyl,
❖ mono-cyclic 4- to 6-membered heterocycloalkyl containing one ring oxygen atom,
❖ bridged bicyclic C₅₋₈-cycloalkyl,
❖ fused bicyclic C₆₋₈-cycloalkyl,
❖ spiro-bicyclic C₆₋₈-cycloalkyl, or
❖ spiro-bicyclic 7- or 8-membered heterocycloalkyl containing one ring oxygen atom;
wherein said mono-cyclic or bicyclic group independently is
➢ unsubstituted;
➢ mono-substituted with hydroxy;
➢ mono-substituted with C₁₋₃-alkyl;
➢ mono-substituted with C₁₋₃-alkoxy;
➢ mono-substituted with -C₁₋₃-alkylene-OH;
➢ mono-substituted with -C₁₋₃-alkylene-O-C₁₋₃-alkyl;
➢ mono-substituted with C₁-fluoroalkyl;
➢ mono-substituted with -NR^{N1}R^{N2}, wherein R^{N1} represents hydrogen, and R^{N2} represents hydrogen or -CO-O-C₁₋₄-alkyl;
➢ mono- or di-substituted with fluoro;
> di-substituted wherein one substituent is hydroxy and the other is C₁₋₃-alkyl; or
➢ tri-substituted wherein two of said substituents are fluoro; and the remaining substituent is C₁₋₃-alkyl or -C₁₋₃-alkylene-OH;
or a pharmaceutically acceptable salt thereof.

2. A compound according to claim 1, wherein
➢ **R^{P2}** represents fluoro or chloro;
➢ **R^{P3}** represents fluoro or chloro; and
> **R^{P4}** represents halogen, methyl or cyano.
or a pharmaceutically acceptable salt thereof.

3. A compound according to claim 1, wherein
➢ **R^{P2}** represents fluoro;
➢ **R^{P3}** represents fluoro; and
➢ **R^{P4}** represents fluoro, chloro, bromo, or methyl;
or a pharmaceutically acceptable salt thereof.

4. A compound according to any one of claims 1 to 3, wherein **R¹** represents methoxy;
or a pharmaceutically acceptable salt thereof.

5. A compound according to any one of claims 1 to 4, wherein **A** represents [1,2,3]triazol-1,4-diyl wherein R²is attached to position 1 of said [1,2,3]triazol-1,4-diyl;
or a pharmaceutically acceptable salt thereof.

6. A compound according to any one of claims 1 to 4; wherein **A** represents [1,2,3]triazol-1,4-diyl wherein R²is attached to position 4 of said [1,2,3]triazol-1,4-diyl;
or a pharmaceutically acceptable salt thereof.

7. A compound according to any one of claims 1 to 4, wherein
a) the group **A-R²** represents a group 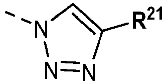 selected from:
(i) 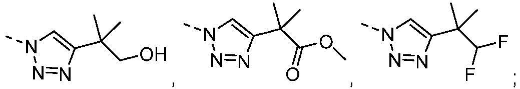
(ii) 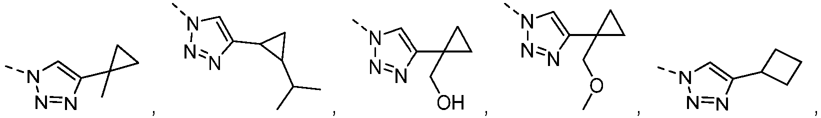 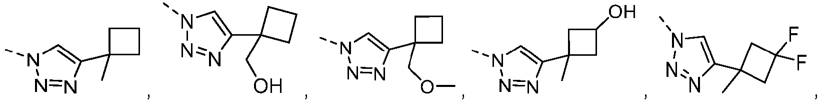 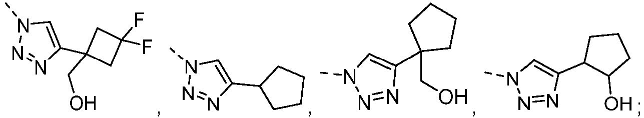
(iii) 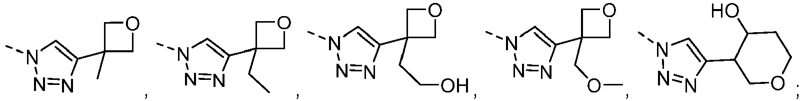
(iv) 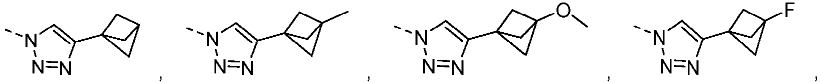 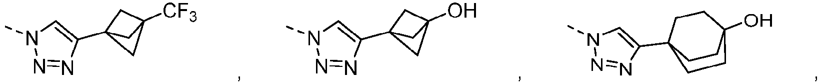 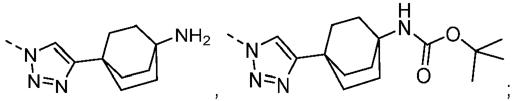 or
(v) 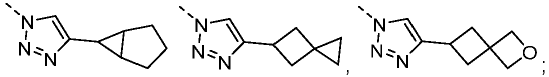 or
b) the group **A-R²** represents a group 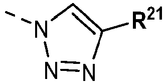 selected from:
(i) 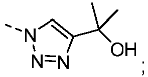
(ii) 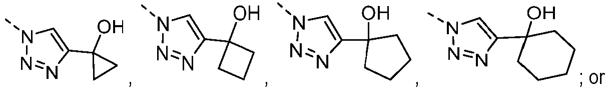
(iii) 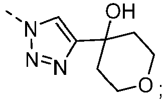 or
c) the group **A-R²** represents a group 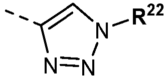 selected from:
(i) 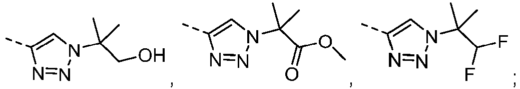
(ii) 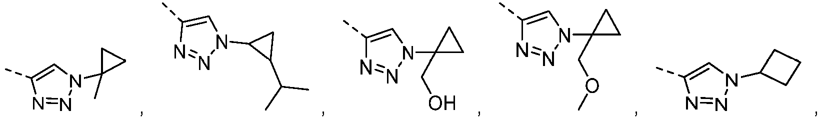 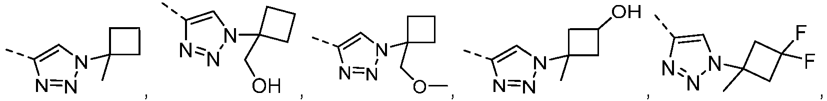 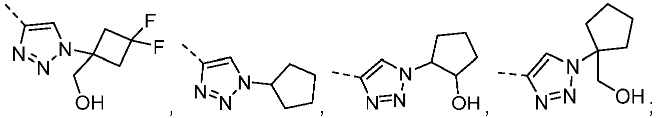
(iii) 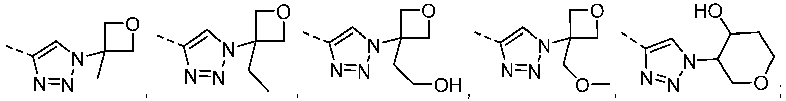
(iv) 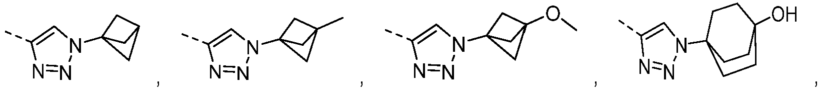 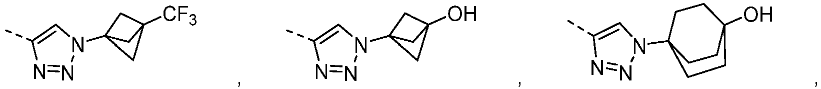 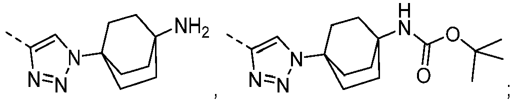 or
(v) 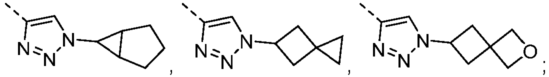
or a pharmaceutically acceptable salt thereof.

8. A compound according to claim 1, wherein said compound is:
(2R,3R,4S,5R,6R)-4-(4-(2,3-difluoro-4-methylphenyl)-1H-1,2,3-triazol-1-yl)-2-(hydroxymethyl)-6-((1-((3R,4R)-4-hydroxytetrahydro-2H-pyran-3-yl)-1H-1,2,3-triazol-4-yl)methyl)-5-methoxytetrahydro-2H-pyran-3-ol;
(2R,3R,4S,5R,6R)-4-(4-(2,3-difluoro-4-methylphenyl)-1H-1,2,3-triazol-1-yl)-6-((1-((1R,2R)-2-hydroxycyclopentyl)-1H-1,2,3-triazol-4-yl)methyl)-2-(hydroxymethyl)-5-methoxytetrahydro-2H-pyran-3-ol;
(2R,3R,4S,5R,6R)-4-(4-(2,3-difluoro-4-methylphenyl)-1H-1,2,3-triazol-1-yl)-6-((1-((1S,2S)-2-hydroxycyclopentyl)-1H-1,2,3-triazol-4-yl)methyl)-2-(hydroxymethyl)-5-methoxytetrahydro-2H-pyran-3-ol;
(2R,3R,4S,5R,6R)-4-(4-(2,3-difluoro-4-methylphenyl)-1H-1,2,3-triazol-1-yl)-2-(hydroxymethyl)-6-((1-(1-(hydroxymethyl)cyclopropyl)-1H-1,2,3-triazol-4-yl)methyl)-5-methoxytetrahydro-2H-pyran-3-ol;
(2R,3R,4S,5R,6R)-4-(4-(2,3-difluoro-4-methylphenyl)-1H-1,2,3-triazol-1-yl)-2-(hydroxymethyl)-6-((1-(1-(hydroxymethyl)cyclopentyl)-1H-1,2,3-triazol-4-yl)methyl)-5-methoxytetrahydro-2H-pyran-3-ol;
(2R,3R,4S,5R,6R)-4-(4-(2,3-difluoro-4-methylphenyl)-1H-1,2,3-triazol-1-yl)-2-(hydroxymethyl)-5-methoxy-6-((1-(3-methyloxetan-3-yl)-1H-1,2,3-triazol-4-yl)methyl)tetrahydro-2H-pyran-3-ol;
(2R,3R,4S,5R,6R)-4-(4-(2,3-difluoro-4-methylphenyl)-1H-1,2,3-triazol-1-yl)-2-(hydroxymethyl)-5-methoxy-6-((1-(1-(methoxymethyl)cyclopropyl)-1H-1,2,3-triazol-4-yl)methyl)tetrahydro-2H-pyran-3-ol;
(2R,3R,4S,5R,6R)-4-(4-(2,3-difluoro-4-methylphenyl)-1H-1,2,3-triazol-1-yl)-6-((1-(4-hydroxybicyclo[2.2.2]octan-1-yl)-1H-1,2,3-triazol-4-yl)methyl)-2-(hydroxymethyl)-5-methoxytetrahydro-2H-pyran-3-ol;
(2R,3R,4S,5R,6R)-4-(4-(2,3-difluoro-4-methylphenyl)-1H-1,2,3-triazol-1-yl)-6-((1-(1-hydroxy-2-methylpropan-2-yl)-1H-1,2,3-triazol-4-yl)methyl)-2-(hydroxymethyl)-5-methoxytetrahydro-2H-pyran-3-ol;
methyl 2-(4-(((2R,3R,4S,5R,6R)-4-(4-(2,3-difluoro-4-methylphenyl)-1H-1,2,3-triazol-1-yl)-5-hydroxy-6-(hydroxymethyl)-3-methoxytetrahydro-2H-pyran-2-yl)methyl)-1H-1,2,3-triazol-1-yl)-2-methylpropanoate;
(2R,3R,4S,5R,6R)-4-(4-(2,3-difluoro-4-methylphenyl)-1H-1,2,3-triazol-1-yl)-2-(hydroxymethyl)-6-((1-(1-(hydroxymethyl)cyclobutyl)-1H-1,2,3-triazol-4-yl)methyl)-5-methoxytetrahydro-2H-pyran-3-ol;
(2R,3R,4S,5R,6R)-6-((1-(bicyclo[1.1.1]pentan-1-yl)-1H-1,2,3-triazol-4-yl)methyl)-4-(4-(2,3-difluoro-4-methylphenyl)-1H-1,2,3-triazol-1-yl)-2-(hydroxymethyl)-5-methoxytetrahydro-2H-pyran-3-ol;
(2R,3R,4S,5R,6R)-4-(4-(2,3-difluoro-4-methylphenyl)-1H-1,2,3-triazol-1-yl)-2-(hydroxymethyl)-5-methoxy-6-((1-(1-methylcyclobutyl)-1H-1,2,3-triazol-4-yl)methyl)tetrahydro-2H-pyran-3-ol;
(2R,3R,4S,5R,6R)-4-(4-(2,3-difluoro-4-methylphenyl)-1H-1,2,3-triazol-1-yl)-2-(hydroxymethyl)-5-methoxy-6-((1-(3-methylbicyclo[1.1.1]pentan-1-yl)-1H-1,2,3-triazol-4-yl)methyl)tetrahydro-2H-pyran-3-ol;
(2R,3R,4S,5R,6R)-6-((1-(3,3-difluoro-1-methylcyclobutyl)-1H-1,2,3-triazol-4-yl)methyl)-4-(4-(2,3-difluoro-4-methylphenyl)-1H-1,2,3-triazol-1-yl)-2-(hydroxymethyl)-5-methoxytetrahydro-2H-pyran-3-ol;
(2R,3R,4S,5R,6R)-6-((1-(2-oxaspiro[3.3]heptan-6-yl)-1H-1,2,3-triazol-4-yl)methyl)-4-(4-(2,3-difluoro-4-methylphenyl)-1H-1,2,3-triazol-1-yl)-2-(hydroxymethyl)-5-methoxytetrahydro-2H-pyran-3-ol;
(2R,3R,4S,5R,6R)-4-(4-(2,3-difluoro-4-methylphenyl)-1H-1,2,3-triazol-1-yl)-2-(hydroxymethyl)-6-((1-((1RS,2SR)-2-isopropylcyclopropyl)-1H-1,2,3-triazol-4-yl)methyl)-5-methoxytetrahydro-2H-pyran-3-ol;
(2R,3R,4S,5R,6R)-6-((1-(3,3-difluoro-1-(hydroxymethyl)cyclobutyl)-1H-1,2,3-triazol-4-yl)methyl)-4-(4-(2,3-difluoro-4-methylphenyl)-1H-1,2,3-triazol-1-yl)-2-(hydroxymethyl)-5-methoxytetrahydro-2H-pyran-3-ol;
(2R,3R,4S,5R,6R)-4-(4-(2,3-difluoro-4-methylphenyl)-1H-1,2,3-triazol-1-yl)-6-((1-((1S,3S)-3-hydroxy-1-methylcyclobutyl)-1H-1,2,3-triazol-4-yl)methyl)-2-(hydroxymethyl)-5-methoxytetrahydro-2H-pyran-3-ol;
(2R,3R,4S,5R,6R)-6-((1-((1 R,5S)-bicyclo[3.1.0]hexan-6-yl)-1H-1,2,3-triazol-4-yl)methyl)-4-(4-(2,3-difluoro-4-methylphenyl)-1H-1,2,3-triazol-1-yl)-2-(hydroxymethyl)-5-methoxytetrahydro-2H-pyran-3-ol;
(2R,3R,4S,5R,6R)-4-(4-(2,3-difluoro-4-methylphenyl)-1H-1,2,3-triazol-1-yl)-2-(hydroxymethyl)-5-methoxy-6-((1-(3-(methoxymethyl)oxetan-3-yl)-1H-1,2,3-triazol-4-yl)methyl)tetrahydro-2H-pyran-3-ol;
(2R,3R,4S,5R,6R)-4-(4-(2,3-difluoro-4-methylphenyl)-1H-1,2,3-triazol-1-yl)-6-((1-(3-(2-hydroxyethyl)oxetan-3-yl)-1H-1,2,3-triazol-4-yl)methyl)-2-(hydroxymethyl)-5-methoxytetrahydro-2H-pyran-3-ol;
(2R,3R,4S,5R,6R)-4-(4-(2,3-difluoro-4-methylphenyl)-1H-1,2,3-triazol-1-yl)-2-(hydroxymethyl)-5-methoxy-6-((1-(1-methylcyclopropyl)-1H-1,2,3-triazol-4-yl)methyl)tetrahydro-2H-pyran-3-ol;
(2R,3R,4S,5R,6R)-4-(4-(2,3-difluoro-4-methylphenyl)-1H-1,2,3-triazol-1-yl)-2-(hydroxymethyl)-5-methoxy-6-((1-(3-(trifluoromethyl)bicyclo[1.1.1]pentan-1-yl)-1H-1,2,3-triazol-4-yl)methyl)tetrahydro-2H-pyran-3-ol;
(2R,3R,4S,5R,6R)-4-(4-(2,3-difluoro-4-methylphenyl)-1H-1,2,3-triazol-1-yl)-6-((1-(3-fluorobicyclo[1.1.1]pentan-1-yl)-1H-1,2,3-triazol-4-yl)methyl)-2-(hydroxymethyl)-5-methoxytetrahydro-2H-pyran-3-ol;
(2R,3R,4S,5R,6R)-4-(4-(2,3-difluoro-4-methylphenyl)-1H-1,2,3-triazol-1-yl)-2-(hydroxymethyl)-5-methoxy-6-((1-(spiro[2.3]hexan-5-yl)-1H-1,2,3-triazol-4-yl)methyl)tetrahydro-2H-pyran-3-ol;
(2R,3R,4S,5R,6R)-4-(4-(2,3-difluoro-4-methylphenyl)-1H-1,2,3-triazol-1-yl)-6-((1-((1R,3R)-3-hydroxy-1-methylcyclobutyl)-1H-1,2,3-triazol-4-yl)methyl)-2-(hydroxymethyl)-5-methoxytetrahydro-2H-pyran-3-ol;
(2R,3R,4S,5R,6R)-4-(4-(4-chloro-2,3-difluorophenyl)-1H-1,2,3-triazol-1-yl)-2-(hydroxymethyl)-5-methoxy-6-((1-(1-(methoxymethyl)cyclopropyl)-1H-1,2,3-triazol-4-yl)methyl)tetrahydro-2H-pyran-3-ol;
(2R,3R,4S,5R,6R)-4-(4-(4-chloro-2,3-difluorophenyl)-1H-1,2,3-triazol-1-yl)-6-((1-(1,1-difluoro-2-methylpropan-2-yl)-1H-1,2,3-triazol-4-yl)methyl)-2-(hydroxymethyl)-5-methoxytetrahydro-2H-pyran-3-ol;
(2R,3R,4S,5R,6R)-4-(4-(4-chloro-2,3-difluorophenyl)-1H-1,2,3-triazol-1-yl)-2-(hydroxymethyl)-6-((1-(1-(hydroxymethyl)cyclopropyl)-1H-1,2,3-triazol-4-yl)methyl)-5-methoxytetrahydro-2H-pyran-3-ol;
(2R,3R,4S,5R,6R)-4-(4-(4-chloro-2,3-difluorophenyl)-1H-1,2,3-triazol-1-yl)-2-(hydroxymethyl)-6-((1-(1-(hydroxymethyl)cyclopentyl)-1H-1,2,3-triazol-4-yl)methyl)-5-methoxytetrahydro-2H-pyran-3-ol;
(2R,3R,4S,5R,6R)-4-(4-(4-chloro-2,3-difluorophenyl)-1H-1,2,3-triazol-1-yl)-2-(hydroxymethyl)-6-((1-(1-(hydroxymethyl)cyclobutyl)-1H-1,2,3-triazol-4-yl)methyl)-5-methoxytetrahydro-2H-pyran-3-ol;
(2R,3R,4S,5R,6R)-4-(4-(4-chloro-2,3-difluorophenyl)-1H-1,2,3-triazol-1-yl)-6-((1-(4-hydroxybicyclo[2.2.2]octan-1-yl)-1H-1,2,3-triazol-4-yl)methyl)-2-(hydroxymethyl)-5-methoxytetrahydro-2H-pyran-3-ol;
(2R,3R,4S,5R,6R)-4-(4-(4-chloro-2,3-difluorophenyl)-1H-1,2,3-triazol-1-yl)-6-((1-(1-hydroxy-2-methylpropan-2-yl)-1H-1,2,3-triazol-4-yl)methyl)-2-(hydroxymethyl)-5-methoxytetrahydro-2H-pyran-3-ol;
methyl 2-(4-(((2R,3R,4S,5R,6R)-4-(4-(4-chloro-2,3-difluorophenyl)-1H-1,2,3-triazol-1-yl)-5-hydroxy-6-(hydroxymethyl)-3-methoxytetrahydro-2H-pyran-2-yl)methyl)-1H-1,2,3-triazol-1-yl)-2-methylpropanoate;
(2R,3R,4S,5R,6R)-6-((1-(bicyclo[1.1.1]pentan-1-yl)-1H-1,2,3-triazol-4-yl)methyl)-4-(4-(4-chloro-2,3-difluorophenyl)-1H-1,2,3-triazol-1-yl)-2-(hydroxymethyl)-5-methoxytetrahydro-2H-pyran-3-ol;
(2R,3R,4S,5R,6R)-4-(4-(4-chloro-2,3-difluorophenyl)-1H-1,2,3-triazol-1-yl)-2-(hydroxymethyl)-5-methoxy-6-((1-(1-methylcyclobutyl)-1H-1,2,3-triazol-4-yl)methyl)tetrahydro-2H-pyran-3-ol;
(2R,3R,4S,5R,6R)-4-(4-(4-chloro-2,3-difluorophenyl)-1H-1,2,3-triazol-1-yl)-2-(hydroxymethyl)-5-methoxy-6-((1-(3-methyloxetan-3-yl)-1H-1,2,3-triazol-4-yl)methyl)tetrahydro-2H-pyran-3-ol;
(2R,3R,4S,5R,6R)-4-(4-(4-chloro-2,3-difluorophenyl)-1H-1,2,3-triazol-1-yl)-2-(hydroxymethyl)-5-methoxy-6-((1-(3-methylbicyclo[1.1.1]pentan-1-yl)-1H-1,2,3-triazol-4-yl)methyl)tetrahydro-2H-pyran-3-ol;
(2R,3R,4S,5R,6R)-4-(4-(4-chloro-2,3-difluorophenyl)-1H-1,2,3-triazol-1-yl)-2-(hydroxymethyl)-5-methoxy-6-((1-(3-methoxybicyclo[1.1.1]pentan-1-yl)-1H-1,2,3-triazol-4-yl)methyl)tetrahydro-2H-pyran-3-ol;
(2R,3R,4S,5R,6R)-4-(4-(4-chloro-2,3-difluorophenyl)-1H-1,2,3-triazol-1-yl)-6-((1-(3,3-difluoro-1-methylcyclobutyl)-1H-1,2,3-triazol-4-yl)methyl)-2-(hydroxymethyl)-5-methoxytetrahydro-2H-pyran-3-ol;
(2R,3R,4S,5R,6R)-6-((1-(2-oxaspiro[3.3]heptan-6-yl)-1H-1,2,3-triazol-4-yl)methyl)-4-(4-(4-chloro-2,3-difluorophenyl)-1H-1,2,3-triazol-1-yl)-2-(hydroxymethyl)-5-methoxytetrahydro-2H-pyran-3-ol;
(2R,3R,4S,5R,6R)-4-(4-(4-chloro-2,3-difluorophenyl)-1H-1,2,3-triazol-1-yl)-2-(hydroxymethyl)-6-((1-(2-isopropylcyclopropyl)-1H-1,2,3-triazol-4-yl)methyl)-5-methoxytetrahydro-2H-pyran-3-ol;
(2R,3R,4S,5R,6R)-4-(4-(4-chloro-2,3-difluorophenyl)-1H-1,2,3-triazol-1-yl)-6-((1-(3,3-difluoro-1-(hydroxymethyl)cyclobutyl)-1H-1,2,3-triazol-4-yl)methyl)-2-(hydroxymethyl)-5-methoxytetrahydro-2H-pyran-3-ol;
(2R,3R,4S,5R,6R)-4-(4-(4-chloro-2,3-difluorophenyl)-1H-1,2,3-triazol-1-yl)-6-((1-((1S,3S)-3-hydroxy-1-methylcyclobutyl)-1H-1,2,3-triazol-4-yl)methyl)-2-(hydroxymethyl)-5-methoxytetrahydro-2H-pyran-3-ol;
(2R,3R,4S,5R,6R)-6-((1-((1R,5S)-bicyclo[3.1.0]hexan-6-yl)-1H-1,2,3-triazol-4-yl)methyl)-4-(4-(4-chloro-2,3-difluorophenyl)-1H-1,2,3-triazol-1-yl)-2-(hydroxymethyl)-5-methoxytetrahydro-2H-pyran-3-ol;
(2R,3R,4S,5R,6R)-4-(4-(4-chloro-2,3-difluorophenyl)-1H-1,2,3-triazol-1-yl)-2-(hydroxymethyl)-5-methoxy-6-((1-(3-(methoxymethyl)oxetan-3-yl)-1H-1,2,3-triazol-4-yl)methyl)tetrahydro-2H-pyran-3-ol;
(2R,3R,4S,5R,6R)-4-(4-(4-chloro-2,3-difluorophenyl)-1H-1,2,3-triazol-1-yl)-6-((1-(3-(2-hydroxyethyl)oxetan-3-yl)-1H-1,2,3-triazol-4-yl)methyl)-2-(hydroxymethyl)-5-methoxytetrahydro-2H-pyran-3-ol;
(2R,3R,4S,5R,6R)-4-(4-(4-chloro-2,3-difluorophenyl)-1H-1,2,3-triazol-1-yl)-2-(hydroxymethyl)-5-methoxy-6-((1-(1-methylcyclopropyl)-1H-1,2,3-triazol-4-yl)methyl)tetrahydro-2H-pyran-3-ol;
(2R,3R,4S,5R,6R)-4-(4-(4-chloro-2,3-difluorophenyl)-1H-1,2,3-triazol-1-yl)-2-(hydroxymethyl)-5-methoxy-6-((1-(3-(trifluoromethyl)bicyclo[1.1.1]pentan-1-yl)-1H-1,2,3-triazol-4-yl)methyl)tetrahydro-2H-pyran-3-ol;
(2R,3R,4S,5R,6R)-4-(4-(4-chloro-2,3-difluorophenyl)-1H-1,2,3-triazol-1-yl)-6-((1-(3-fluorobicyclo[1.1.1]pentan-1-yl)-1H-1,2,3-triazol-4-yl)methyl)-2-(hydroxymethyl)-5-methoxytetrahydro-2H-pyran-3-ol;
(2R,3R,4S,5R,6R)-4-(4-(4-chloro-2,3-difluorophenyl)-1H-1,2,3-triazol-1-yl)-2-(hydroxymethyl)-5-methoxy-6-((1-(spiro[2.3]hexan-5-yl)-1H-1,2,3-triazol-4-yl)methyl)tetrahydro-2H-pyran-3-ol;
(2R,3R,4S,5R,6R)-4-(4-(4-chloro-2,3-difluorophenyl)-1H-1,2,3-triazol-1-yl)-6-((1-((1R,3R)-3-hydroxy-1-methylcyclobutyl)-1H-1,2,3-triazol-4-yl)methyl)-2-(hydroxymethyl)-5-methoxytetrahydro-2H-pyran-3-ol;
(2R,3R,4S,5R,6R)-2-(hydroxymethyl)-6-((1-(1-(hydroxymethyl)cyclopentyl)-1H-1,2,3-triazol-4-yl)methyl)-5-methoxy-4-(4-(2,3,4-trifluorophenyl)-1H-1,2,3-triazol-1-yl)tetrahydro-2H-pyran-3-ol;
(2R,3R,4S,5R,6R)-2-(hydroxymethyl)-6-((1-(1-(hydroxymethyl)cyclopropyl)-1H-1,2,3-triazol-4-yl)methyl)-5-methoxy-4-(4-(2,3,4-trifluorophenyl)-1H-1,2,3-triazol-1-yl)tetrahydro-2H-pyran-3-ol;
(2R,3R,4S,5R,6R)-2-(hydroxymethyl)-5-methoxy-6-((1-(1-(methoxymethyl)cyclopropyl)-1H-1,2,3-triazol-4-yl)methyl)-4-(4-(2,3,4-trifluorophenyl)-1H-1,2,3-triazol-1-yl)tetrahydro-2H-pyran-3-ol;
(2R,3R,4S,5R,6R)-6-((1-(bicyclo[1.1.1]pentan-1-yl)-1H-1,2,3-triazol-4-yl)methyl)-2-(hydroxymethyl)-5-methoxy-4-(4-(2,3,4-trifluorophenyl)-1H-1,2,3-triazol-1-yl)tetrahydro-2H-pyran-3-ol;
(2R,3R,4S,5R,6R)-2-(hydroxymethyl)-6-((1-(1-(hydroxymethyl)cyclobutyl)-1H-1,2,3-triazol-4-yl)methyl)-5-methoxy-4-(4-(2,3,4-trifluorophenyl)-1H-1,2,3-triazol-1-yl)tetrahydro-2H-pyran-3-ol;
(2R,3R,4S,5R,6R)-6-((1-(3-fluorobicyclo[1.1.1]pentan-1-yl)-1H-1,2,3-triazol-4-yl)methyl)-2-(hydroxymethyl)-5-methoxy-4-(4-(2,3,4-trifluorophenyl)-1H-1,2,3-triazol-1-yl)tetrahydro-2H-pyran-3-ol;
(2R,3R,4S,5R,6R)-2-(hydroxymethyl)-5-methoxy-6-((1-(3-(trifluoromethyl)bicyclo[1.1.1]pentan-1-yl)-1H-1,2,3-triazol-4-yl)methyl)-4-(4-(2,3,4-trifluorophenyl)-1H-1,2,3-triazol-1-yl)tetrahydro-2H-pyran-3-ol;
(2R,3R,4S,5R,6R)-6-((1-(3,3-difluoro-1-methylcyclobutyl)-1H-1,2,3-triazol-4-yl)methyl)-2-(hydroxymethyl)-5-methoxy-4-(4-(2,3,4-trifluorophenyl)-1H-1,2,3-triazol-1-yl)tetrahydro-2H-pyran-3-ol;
(2R,3R,4S,5R,6R)-6-((1-(3,3-difluoro-1-(hydroxymethyl)cyclobutyl)-1H-1,2,3-triazol-4-yl)methyl)-2-(hydroxymethyl)-5-methoxy-4-(4-(2,3,4-trifluorophenyl)-1H-1,2,3-triazol-1-yl)tetrahydro-2H-pyran-3-ol;
(2R,3R,4S,5R,6R)-6-((1-(2-oxaspiro[3.3]heptan-6-yl)-1H-1,2,3-triazol-4-yl)methyl)-2-(hydroxymethyl)-5-methoxy-4-(4-(2,3,4-trifluorophenyl)-1H-1,2,3-triazol-1-yl)tetrahydro-2H-pyran-3-ol;
(2R,3R,4S,5R,6R)-6-((1-((1R,3R)-3-hydroxy-1-methylcyclobutyl)-1H-1,2,3-triazol-4-yl)methyl)-2-(hydroxymethyl)-5-methoxy-4-(4-(2,3,4-trifluorophenyl)-1H-1,2,3-triazol-1-yl)tetrahydro-2H-pyran-3-ol;
(2R,3R,4S,5R,6R)-6-((1-((1S,3S)-3-hydroxy-1-methylcyclobutyl)-1H-1,2,3-triazol-4-yl)methyl)-2-(hydroxymethyl)-5-methoxy-4-(4-(2,3,4-trifluorophenyl)-1H-1,2,3-triazol-1-yl)tetrahydro-2H-pyran-3-ol;
(2R,3R,4S,5R,6R)-2-(hydroxymethyl)-5-methoxy-6-((1-(3-(methoxymethyl)oxetan-3-yl)-1H-1,2,3-triazol-4-yl)methyl)-4-(4-(2,3,4-trifluorophenyl)-1H-1,2,3-triazol-1-yl)tetrahydro-2H-pyran-3-ol;
(2R,3R,4S,5R,6R)-6-((1-(3-(2-hydroxyethyl)oxetan-3-yl)-1H-1,2,3-triazol-4-yl)methyl)-2-(hydroxymethyl)-5-methoxy-4-(4-(2,3,4-trifluorophenyl)-1H-1,2,3-triazol-1-yl)tetrahydro-2H-pyran-3-ol;
(2R,3R,4S,5R,6R)-2-(hydroxymethyl)-5-methoxy-6-((1-(1-methylcyclopropyl)-1H-1,2,3-triazol-4-yl)methyl)-4-(4-(2,3,4-trifluorophenyl)-1H-1,2,3-triazol-1-yl)tetrahydro-2H-pyran-3-ol;
(2R,3R,4S,5R,6R)-4-(4-(4-bromo-2,3-difluorophenyl)-1H-1,2,3-triazol-1-yl)-2-(hydroxymethyl)-5-methoxy-6-((1-(3-methyloxetan-3-yl)-1H-1,2,3-triazol-4-yl)methyl)tetrahydro-2H-pyran-3-ol;
(2R,3R,4S,5R,6R)-4-(4-(4-bromo-2,3-difluorophenyl)-1H-1,2,3-triazol-1-yl)-2-(hydroxymethyl)-5-methoxy-6-((1-(1-methylcyclobutyl)-1H-1,2,3-triazol-4-yl)methyl)tetrahydro-2H-pyran-3-ol;
(2R,3R,4S,5R,6R)-6-((1-(bicyclo[1.1.1]pentan-1-yl)-1H-1,2,3-triazol-4-yl)methyl)-4-(4-(4-bromo-2,3-difluorophenyl)-1H-1,2,3-triazol-1-yl)-2-(hydroxymethyl)-5-methoxytetrahydro-2H-pyran-3-ol;
(2R,3R,4S,5R,6R)-4-(4-(4-bromo-2,3-difluorophenyl)-1H-1,2,3-triazol-1-yl)-2-(hydroxymethyl)-5-methoxy-6-((1-(1-(methoxymethyl)cyclobutyl)-1H-1,2,3-triazol-4-yl)methyl)tetrahydro-2H-pyran-3-ol;
(2R,3R,4S,5R,6R)-4-(4-(4-bromo-2,3-difluorophenyl)-1H-1,2,3-triazol-1-yl)-2-(hydroxymethyl)-6-((1-(1-(hydroxymethyl)cyclopropyl)-1H-1,2,3-triazol-4-yl)methyl)-5-methoxytetrahydro-2H-pyran-3-ol;
(2R,3R,4S,5R,6R)-4-(4-(4-bromo-2,3-difluorophenyl)-1H-1,2,3-triazol-1-yl)-6-((1-((1S,2S)-2-hydroxycyclopentyl)-1H-1,2,3-triazol-4-yl)methyl)-2-(hydroxymethyl)-5-methoxytetrahydro-2H-pyran-3-ol;
(2R,3R,4S,5R,6R)-4-(4-(2,3-difluoro-4-methylphenyl)-1H-1,2,3-triazol-1-yl)-6-((4-(1-hydroxycyclobutyl)-1H-1,2,3-triazol-1-yl)methyl)-2-(hydroxymethyl)-5-methoxytetrahydro-2H-pyran-3-ol;
(2R,3R,4S,5R,6R)-6-((4-cyclopentyl-1H-1,2,3-triazol-1-yl)methyl)-4-(4-(2,3-difluoro-4-methylphenyl)-1H-1,2,3-triazol-1-yl)-2-(hydroxymethyl)-5-methoxytetrahydro-2H-pyran-3-ol;
(2R,3R,4S,5R,6R)-4-(4-(2,3-difluoro-4-methylphenyl)-1H-1,2,3-triazol-1-yl)-6-((4-(1-hydroxycyclopentyl)-1H-1,2,3-triazol-1-yl)methyl)-2-(hydroxymethyl)-5-methoxytetrahydro-2H-pyran-3-ol;
(2R,3R,4S,5R,6R)-4-(4-(2,3-difluoro-4-methylphenyl)-1H-1,2,3-triazol-1-yl)-2-(hydroxymethyl)-6-((4-(2-hydroxypropan-2-yl)-1H-1,2,3-triazol-1-yl)methyl)-5-methoxytetrahydro-2H-pyran-3-ol;
(2R,3R,4S,5R,6R)-4-(4-(2,3-difluoro-4-methylphenyl)-1H-1,2,3-triazol-1-yl)-2-(hydroxymethyl)-5-methoxy-6-((4-(3-methyloxetan-3-yl)-1H-1,2,3-triazol-1-yl)methyl)tetrahydro-2H-pyran-3-ol;
(2R,3R,4S,5R,6R)-4-(4-(2,3-difluoro-4-methylphenyl)-1H-1,2,3-triazol-1-yl)-2-(hydroxymethyl)-6-((4-(4-hydroxytetrahydro-2H-pyran-4-yl)-1H-1,2,3-triazol-1-yl)methyl)-5-methoxytetrahydro-2H-pyran-3-ol;
(2R,3R,4S,5R,6R)-4-(4-(2,3-difluoro-4-methylphenyl)-1H-1,2,3-triazol-1-yl)-6-((4-(1-hydroxycyclopropyl)-1H-1,2,3-triazol-1-yl)methyl)-2-(hydroxymethyl)-5-methoxytetrahydro-2H-pyran-3-ol;
(2R,3R,4S,5R,6R)-4-(4-(2,3-difluoro-4-methylphenyl)-1H-1,2,3-triazol-1-yl)-2-(hydroxymethyl)-6-((4-(1-(hydroxymethyl)cyclopropyl)-1H-1,2,3-triazol-1-yl)methyl)-5-methoxytetrahydro-2H-pyran-3-ol;
tert-butyl (4-(1-(((2R,3R,4S,5R,6R)-4-(4-(2,3-difluoro-4-methylphenyl)-1H-1,2,3-triazol-1-yl)-5-hydroxy-6-(hydroxymethyl)-3-methoxytetrahydro-2H-pyran-2-yl)methyl)-1H-1,2,3-triazol-4-yl)bicyclo[2.2.2]octan-1-yl)carbamate;
(2R,3R,4S,5R,6R)-4-(4-(2,3-difluoro-4-methylphenyl)-1H-1,2,3-triazol-1-yl)-6-((4-(3-ethyloxetan-3-yl)-1H-1,2,3-triazol-1-yl)methyl)-2-(hydroxymethyl)-5-methoxytetrahydro-2H-pyran-3-ol;
(2R,3R,4S,5R,6R)-6-((1-(4-aminobicyclo[2.2.2]octan-1-yl)-1H-1,2,3-triazol-4-yl)methyl)-4-(4-(2,3-difluoro-4-methylphenyl)-1H-1,2,3-triazol-1-yl)-2-(hydroxymethyl)-5-methoxytetrahydro-2H-pyran-3-ol;
(2R,3R,4S,5R,6R)-4-(4-(4-chloro-2,3-difluorophenyl)-1H-1,2,3-triazol-1-yl)-6-((4-(1-hydroxycyclobutyl)-1H-1,2,3-triazol-1-yl)methyl)-2-(hydroxymethyl)-5-methoxytetrahydro-2H-pyran-3-ol;
(2R,3R,4S,5R,6R)-4-(4-(4-chloro-2,3-difluorophenyl)-1H-1,2,3-triazol-1-yl)-6-((4-cyclopentyl-1H-1,2,3-triazol-1-yl)methyl)-2-(hydroxymethyl)-5-methoxytetrahydro-2H-pyran-3-ol;
(2R,3R,4S,5R,6R)-4-(4-(4-chloro-2,3-difluorophenyl)-1H-1,2,3-triazol-1-yl)-6-((4-(1-hydroxycyclopentyl)-1H-1,2,3-triazol-1-yl)methyl)-2-(hydroxymethyl)-5-methoxytetrahydro-2H-pyran-3-ol;
(2R,3R,4S,5R,6R)-4-(4-(4-chloro-2,3-difluorophenyl)-1H-1,2,3-triazol-1-yl)-2-(hydroxymethyl)-6-((4-(2-hydroxypropan-2-yl)-1H-1,2,3-triazol-1-yl)methyl)-5-methoxytetrahydro-2H-pyran-3-ol;
(2R,3R,4S,5R,6R)-4-(4-(4-chloro-2,3-difluorophenyl)-1H-1,2,3-triazol-1-yl)-2-(hydroxymethyl)-5-methoxy-6-((4-(3-methyloxetan-3-yl)-1H-1,2,3-triazol-1-yl)methyl)tetrahydro-2H-pyran-3-ol;
(2R,3R,4S,5R,6R)-4-(4-(4-chloro-2,3-difluorophenyl)-1H-1,2,3-triazol-1-yl)-2-(hydroxymethyl)-6-((4-(4-hydroxytetrahydro-2H-pyran-4-yl)-1H-1,2,3-triazol-1-yl)methyl)-5-methoxytetrahydro-2H-pyran-3-ol;
(2R,3R,4S,5R,6R)-4-(4-(4-chloro-2,3-difluorophenyl)-1H-1,2,3-triazol-1-yl)-6-((4-(1-hydroxycyclopropyl)-1H-1,2,3-triazol-1-yl)methyl)-2-(hydroxymethyl)-5-methoxytetrahydro-2H-pyran-3-ol;
(2R,3R,4S,5R,6R)-4-(4-(4-chloro-2,3-difluorophenyl)-1H-1,2,3-triazol-1-yl)-2-(hydroxymethyl)-6-((4-(1-(hydroxymethyl)cyclopropyl)-1H-1,2,3-triazol-1-yl)methyl)-5-methoxytetrahydro-2H-pyran-3-ol;
tert-butyl (4-(1-(((2R,3R,4S,5R,6R)-4-(4-(4-chloro-2,3-difluorophenyl)-1H-1,2,3-triazol-1-yl)-5-hydroxy-6-(hydroxymethyl)-3-methoxytetrahydro-2H-pyran-2-yl)methyl)-1H-1,2,3-triazol-4-yl)bicyclo[2.2.2]octan-1-yl)carbamate;
(2R,3R,4S,5R,6R)-4-(4-(4-chloro-2,3-difluorophenyl)-1H-1,2,3-triazol-1-yl)-6-((4-(3-ethyloxetan-3-yl)-1H-1,2,3-triazol-1-yl)methyl)-2-(hydroxymethyl)-5-methoxytetrahydro-2H-pyran-3-ol;
(2R,3R,4S,5R,6R)-6-((1-(4-aminobicyclo[2.2.2]octan-1-yl)-1H-1,2,3-triazol-4-yl)methyl)-4-(4-(4-chloro-2,3-difluorophenyl)-1H-1,2,3-triazol-1-yl)-2-(hydroxymethyl)-5-methoxytetrahydro-2H-pyran-3-ol;
(2R,3R,4S,5R,6R)-6-((4-(1-hydroxycyclobutyl)-1H-1,2,3-triazol-1-yl)methyl)-2-(hydroxymethyl)-5-methoxy-4-(4-(2,3,4-trifluorophenyl)-1H-1,2,3-triazol-1-yl)tetrahydro-2H-pyran-3-ol;
(2R,3R,4S,5R,6R)-6-((4-cyclopentyl-1H-1,2,3-triazol-1-yl)methyl)-2-(hydroxymethyl)-5-methoxy-4-(4-(2,3,4-trifluorophenyl)-1H-1,2,3-triazol-1-yl)tetrahydro-2H-pyran-3-ol;
(2R,3R,4S,5R,6R)-6-((4-(1-hydroxycyclopentyl)-1H-1,2,3-triazol-1-yl)methyl)-2-(hydroxymethyl)-5-methoxy-4-(4-(2,3,4-trifluorophenyl)-1H-1,2,3-triazol-1-yl)tetrahydro-2H-pyran-3-ol;
(2R,3R,4S,5R,6R)-2-(hydroxymethyl)-6-((4-(2-hydroxypropan-2-yl)-1H-1,2,3-triazol-1-yl)methyl)-5-methoxy-4-(4-(2,3,4-trifluorophenyl)-1H-1,2,3-triazol-1-yl)tetrahydro-2H-pyran-3-ol;
(2R,3R,4S,5R,6R)-2-(hydroxymethyl)-5-methoxy-6-((4-(3-methyloxetan-3-yl)-1H-1,2,3-triazol-1-yl)methyl)-4-(4-(2,3,4-trifluorophenyl)-1H-1,2,3-triazol-1-yl)tetrahydro-2H-pyran-3-ol;
(2R,3R,4S,5R,6R)-2-(hydroxymethyl)-6-((4-(4-hydroxytetrahydro-2H-pyran-4-yl)-1H-1,2,3-triazol-1-yl)methyl)-5-methoxy-4-(4-(2,3,4-trifluorophenyl)-1H-1,2,3-triazol-1-yl)tetrahydro-2H-pyran-3-ol;
(2R,3R,4S,5R,6R)-6-((4-(1-hydroxycyclopropyl)-1H-1,2,3-triazol-1-yl)methyl)-2-(hydroxymethyl)-5-methoxy-4-(4-(2,3,4-trifluorophenyl)-1H-1,2,3-triazol-1-yl)tetrahydro-2H-pyran-3-ol;
(2R,3R,4S,5R,6R)-2-(hydroxymethyl)-6-((4-(1-(hydroxymethyl)cyclopropyl)-1H-1,2,3-triazol-1-yl)methyl)-5-methoxy-4-(4-(2,3,4-trifluorophenyl)-1H-1,2,3-triazol-1-yl)tetrahydro-2H-pyran-3-ol;
(2R,3R,4S,5R,6R)-6-((4-(3-ethyloxetan-3-yl)-1H-1,2,3-triazol-1-yl)methyl)-2-(hydroxymethyl)-5-methoxy-4-(4-(2,3,4-trifluorophenyl)-1H-1,2,3-triazol-1-yl)tetrahydro-2H-pyran-3-ol;
tert-butyl (4-(1-(((2R,3R,4S,5R,6R)-5-hydroxy-6-(hydroxymethyl)-3-methoxy-4-(4-(2,3,4-trifluorophenyl)-1H-1,2,3-triazol-1-yl)tetrahydro-2H-pyran-2-yl)methyl)-1 H-1 ,2,3-triazol-4-yl)bicyclo[2.2.2]octan-1-yl)carbamate;
(2R,3R,4S,5R,6R)-6-((1-(4-aminobicyclo[2.2.2]octan-1-yl)-1H-1,2,3-triazol-4-yl)methyl)-2-(hydroxymethyl)-5-methoxy-4-(4-(2,3,4-trifluorophenyl)-1H-1,2,3-triazol-1-yl)tetrahydro-2H-pyran-3-ol;
(2R,3R,4S,5R,6R)-6-((4-(bicyclo[1.1.1]pentan-1-yl)-1H-1,2,3-triazol-1-yl)methyl)-4-(4-(4-chloro-2,3-difluorophenyl)-1H-1,2,3-triazol-1-yl)-2-(hydroxymethyl)-5-methoxytetrahydro-2H-pyran-3-ol;
(2R,3R,4S,5R,6R)-4-(4-(4-chloro-2,3-difluorophenyl)-1H-1,2,3-triazol- 1-yl)-2-(hydroxymethyl)-5-methoxy-6-((4-(1-methylcyclopropyl)-1H-1,2,3-triazol-1-yl)methyl)tetrahydro-2H-pyran-3-ol; or
(2R,3R,4S,5R,6R)-4-(4-(4-chloro-2,3-difluorophenyl)-1H-1,2,3-triazol-1-yl)-2-(hydroxymethyl)-5-methoxy-6-((4-(1-methylcyclobutyl)-1H-1,2,3-triazol-1-yl)methyl)tetrahydro-2H-pyran-3-ol;
or a pharmaceutically acceptable salt thereof.

9. A compound according to claim 1, wherein said compound is:
(2R,3R,4S,5R,6R)-4-(4-(4-chloro-2,3-difluorophenyl)-1H-1,2,3-triazol-1-yl)-6-((1-(3-(fluoromethyl)oxetan-3-yl)-1H-1,2,3-triazol-4-yl)methyl)-2-(hydroxymethyl)-5-methoxytetrahydro-2H-pyran-3-ol;
(2R,3R,4S,5R,6R)-4-(4-(4-chloro-2,3-difluorophenyl)-1H-1,2,3-triazol-1-yl)-6-((1-(3-(difluoromethyl)oxetan-3-yl)-1H-1,2,3-triazol-4-yl)methyl)-2-(hydroxymethyl)-5-methoxytetrahydro-2H-pyran-3-ol;
(2R,3R,4S,5R,6R)-4-(4-(4-chloro-2,3-difluorophenyl)-1H-1,2,3-triazol-1-yl)-2-(hydroxymethyl)-5-methoxy-6-((1-(3-(trifluoromethyl)oxetan-3-yl)-1H-1,2,3-triazol-4-yl)methyl)tetrahydro-2H-pyran-3-ol;
(2R,3R,4S,5R,6R)-4-(4-(4-chloro-2,3-difluorophenyl)-1H-1,2,3-triazol-1-yl)-6-((1-((3R,4S)-3-fluorotetrahydro-2H-pyran-4-yl)-1 H-1,2,3-triazol-4-yl)methyl)-2-(hydroxymethyl)-5-methoxytetrahydro-2H-pyran-3-ol;
(2R,3R,4S,5R,6R)-4-(4-(4-chloro-2,3-difluorophenyl)-1H-1,2,3-triazol-1-yl)-6-((1-((3R,4R)-4-fluorotetrahydrofuran-3-yl)-1H-1,2,3-triazol-4-yl)methyl)-2-(hydroxymethyl)-5-methoxytetrahydro-2H-pyran-3-ol;
(2R,3R,4S,5R,6R)-4-(4-(4-chloro-2,3-difluorophenyl)-1H-1,2,3-triazol-1-yl)-6-((1-((3S,4S)-4-fluorotetrahydrofuran-3-yl)-1H-1,2,3-triazol-4-yl)methyl)-2-(hydroxymethyl)-5-methoxytetrahydro-2H-pyran-3-ol;
(2R,3R,4S,5R,6R)-4-(4-(4-chloro-2,3-difluorophenyl)-1H-1,2,3-triazol-1-yl)-6-((1-((R)-3,3-difluorotetrahydro-2H-pyran-4-yl)-1 H-1,2,3-triazol-4-yl)methyl)-2-(hydroxymethyl)-5-methoxytetrahydro-2H-pyran-3-ol;
(2R,3R,4S,5R,6R)-4-(4-(4-chloro-2,3-difluorophenyl)-1H-1,2,3-triazol-1-yl)-6-((1-((S)-3,3-difluorotetrahydro-2H-pyran-4-yl)-1H-1,2,3-triazol-4-yl)methyl)-2-(hydroxymethyl)-5-methoxytetrahydro-2H-pyran-3-ol;
(2R,3R,4S,5R,6R)-4-(4-(4-chloro-2,3-difluorophenyl)-1H-1,2,3-triazol-1-yl)-6-((1-((R)-4,4-difluorotetrahydrofuran-3-yl)-1H-1,2,3-triazol-4-yl)methyl)-2-(hydroxymethyl)-5-methoxytetrahydro-2H-pyran-3-ol;
(2R,3R,4S,5R,6R)-4-(4-(4-chloro-2,3-difluorophenyl)-1H-1,2,3-triazol-1-yl)-6-((1-((S)-4,4-difluorotetrahydrofuran-3-yl)-1H-1,2,3-triazol-4-yl)methyl)-2-(hydroxymethyl)-5-methoxytetrahydro-2H-pyran-3-ol;
(2R,3R,4S,5R,6R)-6-((1-((3S,4R)-3-fluorotetrahydro-2H-pyran-4-yl)-1H-1,2,3-triazol-4-yl)methyl)-2-(hydroxymethyl)-5-methoxy-4-(4-(2,3,4-trifluorophenyl)-1H-1,2,3-triazol-1-yl)tetrahydro-2H-pyran-3-ol;
(2R,3R,4S,5R,6R)-6-((1-((3R,4S)-3-fluorotetrahydro-2H-pyran-4-yl)-1H-1,2,3-triazol-4-yl)methyl)-2-(hydroxymethyl)-5-methoxy-4-(4-(2,3,4-trifluorophenyl)-1H-1,2,3-triazol-1-yl)tetrahydro-2H-pyran-3-ol;
(2R,3R,4S,5R,6R)-4-(4-(4-chloro-2,3-difluorophenyl)-1H-1,2,3-triazol-1-yl)-6-((1-((3R,4R)-3-fluorotetrahydro-2H-pyran-4-yl)-1H-1,2,3-triazol-4-yl)methyl)-2-(hydroxymethyl)-5-methoxytetrahydro-2H-pyran-3-ol;
(2R,3R,4S,5R,6R)-4-(4-(4-chloro-2,3-difluorophenyl)-1H-1,2,3-triazol-1-yl)-6-((1-((3S,4S)-3-fluorotetrahydro-2H-pyran-4-yl)-1H-1,2,3-triazol-4-yl)methyl)-2-(hydroxymethyl)-5-methoxytetrahydro-2H-pyran-3-ol;
(2R,3R,4S,5R,6R)-4-(4-(4-bromo-2,3-difluorophenyl)-1H-1,2,3-triazol-1-yl)-6-((1-(3-(difluoromethyl)oxetan-3-yl)-1H-1,2,3-triazol-4-yl)methyl)-2-(hydroxymethyl)-5-methoxytetrahydro-2H-pyran-3-ol;
(2R,3R,4S,5R,6R)-4-(4-(4-bromo-2,3-difluorophenyl)-1H-1,2,3-triazol-1-yl)-2-(hydroxymethyl)-5-methoxy-6-((1-(3-(trifluoromethyl)oxetan-3-yl)-1H-1,2,3-triazol-4-yl)methyl)tetrahydro-2H-pyran-3-ol;
2,3-difluoro-4-(1-((2R,3R,4S,5R,6R)-2-((1-((3R,4S)-3-fluorotetrahydro-2H-pyran-4-yl)-1H-1,2,3-triazol-4-yl)methyl)-5-hydroxy-6-(hydroxymethyl)-3-methoxytetrahydro-2H-pyran-4-yl)-1H-1,2,3-triazol-4-yl)benzonitrile; or
2,3-difluoro-4-(1-((2R,3R,4S,5R,6R)-2-((1-((3S,4R)-3-fluorotetrahydro-2H-pyran-4-yl)-1H-1,2,3-triazol-4-yl)methyl)-5-hydroxy-6-(hydroxymethyl)-3-methoxytetrahydro-2H-pyran-4-yl)-1H-1,2,3-triazol-4-yl)benzonitrile;
or a pharmaceutically acceptable salt thereof.

10. A compound according to claim 1, wherein said compound is:
(2R,3R,4S,5R,6R)-4-(4-(4-chloro-2,3-difluorophenyl)-1H-1,2,3-triazol-1-yl)-6-((1-((3R,4R)-4-fluorotetrahydrofuran-3-yl)-1H-1,2,3-triazol-4-yl)methyl)-2-(hydroxymethyl)-5-methoxytetrahydro-2H-pyran-3-ol;
(2R,3R,4S,5R,6R)-4-(4-(4-chloro-2,3-difluorophenyl)-1H-1,2,3-triazol-1-yl)-6-((1-((3S,4R)-3-fluorotetrahydro-2H-pyran-4-yl)-1H-1,2,3-triazol-4-yl)methyl)-2-(hydroxymethyl)-5-methoxytetrahydro-2H-pyran-3-ol;
(2R,3R,4S,5R,6R)-4-(4-(4-chloro-2,3-difluorophenyl)-1H-1,2,3-triazol-1-yl)-6-((1-((3R,4R)-3-fluorotetrahydro-2H-pyran-4-yl)-1H-1,2,3-triazol-4-yl)methyl)-2-(hydroxymethyl)-5-methoxytetrahydro-2H-pyran-3-ol;
(2R,3R,4S,5R,6R)-4-(4-(4-chloro-2,3-difluorophenyl)-1H-1,2,3-triazol-1-yl)-6-((1-(3-(difluoromethyl)oxetan-3-yl)-1H-1,2,3-triazol-4-yl)methyl)-2-(hydroxymethyl)-5-methoxytetrahydro-2H-pyran-3-ol: or
(2R,3R,4S,5R,6R)-4-(4-(4-chloro-2,3-difluorophenyl)-1H-1,2,3-triazol-1-yl)-6-((1-((S)-3,3-difluorotetrahydro-2H-pyran-4-yl)-1H-1,2,3-triazol-4-yl)methyl)-2-(hydroxymethyl)-5-methoxytetrahydro-2H-pyran-3-ol;
or a pharmaceutically acceptable salt thereof.

11. A pharmaceutical composition comprising a compound according to any one of claims 1 to 10, or a pharmaceutically acceptable salt thereof, and a pharmaceutically acceptable carrier.

12. A compound according to any one of claims 1 to 10, or a pharmaceutically acceptable salt thereof, for use as a medicament.

13. A compound according to any one of claims 1 to 10, or a pharmaceutically acceptable salt thereof, for use in the prevention or treatment of fibrosis of organs; liver diseases and disorders; acute kidney injury and chronic kidney disease; cardiovascular diseases and disorders; interstitial lung diseases and disorders; cell proliferative diseases and cancers; inflammatory and autoimmune diseases and disorders; gastrointestinal tract diseases and disorders; pancreatic diseases and disorders; abnormal angiogenesis-associated diseases and disorders; brain-associated diseases and disorders; neuropathic pain and peripheral neuropathy; ocular diseases and disorders; or transplant rejection.

14. Use of a compound according to any one of claims 1 to 10, or a pharmaceutically acceptable salt thereof, in the preparation of a medicament for the prevention or treatment of fibrosis of organs; liver diseases and disorders; acute kidney injury and chronic kidney disease; cardiovascular diseases and disorders; interstitial lung diseases and disorders; cell proliferative diseases and cancers; inflammatory and autoimmune diseases and disorders; gastrointestinal tract diseases and disorders; pancreatic diseases and disorders; abnormal angiogenesis-associated diseases and disorders; brain-associated diseases and disorders; neuropathic pain and peripheral neuropathy; ocular diseases and disorders; or transplant rejection.

## Patentansprüche

1. Verbindung von Formel (I) 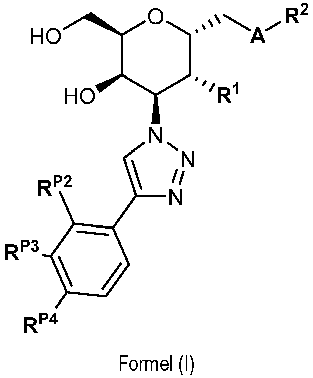 wobei
**R^{P2}** Halogen repräsentiert;
**R^{P3}** Halogen repräsentiert;
**R^{P4}** Halogen, Methyl oder Cyano repräsentiert;
**R¹** Folgendes repräsentiert:
• Hydroxy;
• C₁₋₄-Alkoxy;
• -O-CO-C₁₋₃-Alkyl;
• O-CO-NH-R^{N11}, wobei R^{N11} Wasserstoff oder C₁₋₃-Alkyl repräsentiert;
• -O-CH₂-C₄-Fluoralkyl;
• -O-CH₂-**HET¹**, wobei **HET¹** ein 5-gliedriges Heteroaryl repräsentiert, wobei das genannte 5-gliedrige Heteroaryl unabhängig unsubstituiert oder mit Methyl monosubstituiert ist; oder
• -O-CH₂-CO-**R^{1X}**, wobei **R^{1X}** Folgendes repräsentiert:
> -Hydroxy;
> C₁₋₃-Alkoxy;
> Morpholin-4-yl; oder
❖ -N**R^{N21}R^{N22}** wobei **R^{N21}** und **R^{N22}** beide unabhängig Wasserstoff oder Methyl repräsentieren; oder **R^{N21}** und **R^{N22}** zusammen mit dem Stickstoffatom, an das sie gebunden sind, ein 4- bis 6-gliedriges monocyclisches Heterocycloalkyl bilden, das ausgewählt ist aus Azetidin-1-yl, Pyrrolidin-1-yl und Piperidin-1-yl, wobei das genannte 4- bis 6-gliedrige Heterocycloalkyl mit Hydroxy monosubstituiert ist;
A [1,2,3]Triazol-1,4-diyl repräsentiert; und
• **R²** verzweigtes C₃₋₆-Alkyl repräsentiert, wobei das genannte verzweigte C₃₋₆-Alkyl Folgendes ist:
> mit Hydroxy monosubstituiert,
> mit -CO-O-C₁₋₄-Alkyl monosubstituiert, oder
> mit C₁-Fluoralkyl monosubstituiert;
• oder **R²** eine gesättigte 3- bis 8-gliedrige mono- oder bicyclische Gruppe repräsentiert, wobei die genannte mono- oder bicyclische Gruppe Folgendes ist:
❖ monocyclisches C₃₋₆-Cycloalkyl,
❖ monocyclisches 4- bis 6-gliedriges Heterocycloalkyl, das ein Ringsauerstoffatom enthält,
❖ verbrücktes bicyclisches C₅₋₈-Cycloalkyl,
❖ kondensiertes bicyclisches C₆₋₈-Cycloalkyl,
❖ spiro-bicyclisches C₆₋₈-Cycloalkyl oder
❖ spiro-bicyclisches 7- oder 8-gliedriges Heterocycloalkyl, das ein Ringsauerstoffatom enthält;
wobei die genannte monocyclische oder bicyclische Gruppe unabhängig Folgendes ist:
> unsubstituiert;
➢ mit Hydroxy monosubstituiert;
➢ mit C₁₋₃-Alkyl monosubstituiert;
➢ mit C₁₋₃-Alkoxy monosubstituiert;
➢ mit -C₁₋₃-Alkylen-OH monosubstituiert;
➢ mit -C₁₋₃-Alkylen-O-C₁₋₃-Alkyl monosubstituiert;
➢ mit C₁-Fluoralkyl monosubstituiert;
➢ mit -NR^{N1}R^{N2} monosubstituiert, wobei R^{N1} Wasserstoff repräsentiert und R^{N2} Wasserstoff oder -CO-O-C₁₋₄-Alkyl repräsentiert;
➢ mit Fluor mono- oder disubstituiert;
➢ disubstituiert, wobei ein Substituent Hydroxy und der andere C₁₋₃-Alkyl ist; oder
➢ trisubstituiert, wobei zwei der genannten Substituenten Fluor sind; und der verbleibende Substituent C₁₋₃-Alkyl oder -C₁₋₃-Alkylen-OH ist;
oder ein pharmazeutisch akzeptables Salz davon.

2. Verbindung nach Anspruch 1, wobei
> R^{P2} Fluor oder Chlor repräsentiert;
> **R^{P3}** Fluor oder Chlor repräsentiert; und
> **R^{P4}** Halogen, Methyl oder Cyano repräsentiert.
oder ein pharmazeutisch akzeptables Salz davon.

3. Verbindung nach Anspruch 1, wobei
> **R^{P2}** Fluor repräsentiert;
> **R^{P3}** Fluor repräsentiert; und
> **R^{P4}** Fluor, Chlor, Brom oder Methyl repräsentiert;
oder ein pharmazeutisch akzeptables Salz davon.

4. Verbindung nach einem der Ansprüche 1 bis 3, wobei **R¹** Methoxy repräsentiert;
oder ein pharmazeutisch akzeptables Salz davon.

5. Verbindung nach einem der Ansprüche 1 bis 4, wobei **A** [1,2,3]Triazol-1,4-diyl repräsentiert, wobei **R²** an Position 1 des genannten [1,2,3]Triazol-1,4-diyls gebunden ist;
oder ein pharmazeutisch akzeptables Salz davon.

6. Verbindung nach einem der Ansprüche 1 bis 4; wobei **A** [1,2,3]Triazol-1,4-diyl repräsentiert, wobei **R²** an Position 4 des genannten [1,2,3]Triazol-1,4-diyls gebunden ist;
oder ein pharmazeutisch akzeptables Salz davon.

7. Verbindung nach einem der Ansprüche 1 bis 4, wobei
a) die Gruppe **A-R²** eine Gruppe 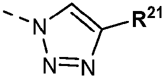 repräsentiert, ausgewählt aus:
(i) 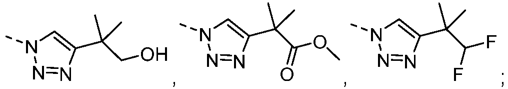
(ii) 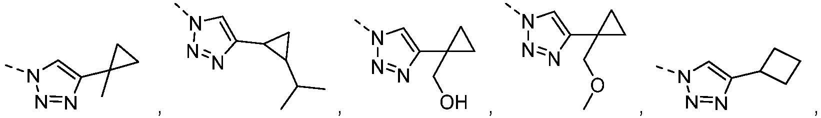 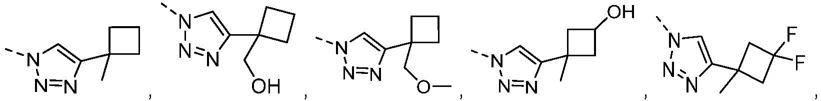 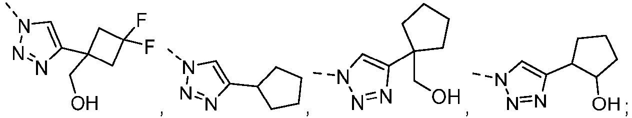
(iii) 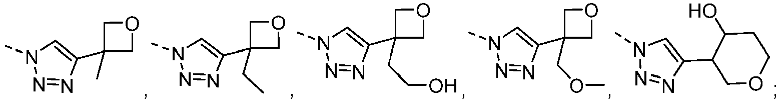
(iv) 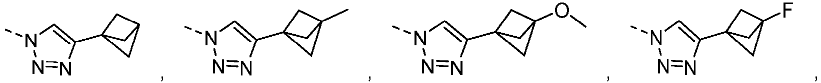 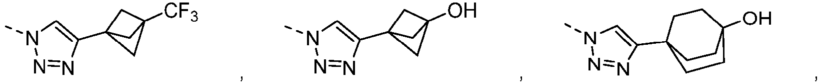 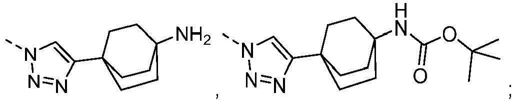 oder
(v) 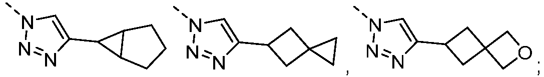 oder
b) die Gruppe **A-R²** eine Gruppe 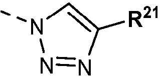 repräsentiert, ausgewählt aus:
(i) 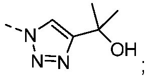
(ii) 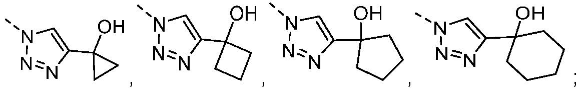 oder
(iii) 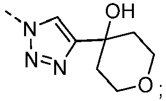 oder
c) die Gruppe **A-R²** eine Gruppe 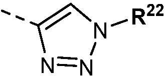 repräsentiert, ausgewählt aus:
(i) 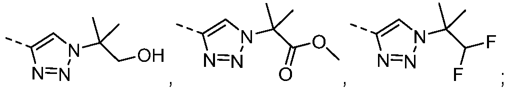
(ii) 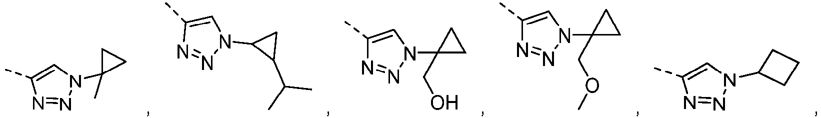 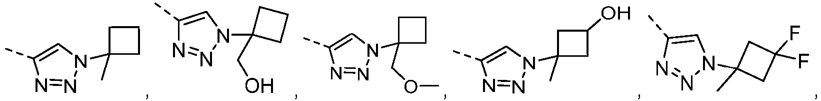 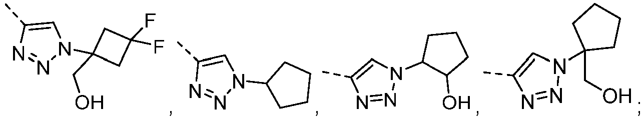
(iii) 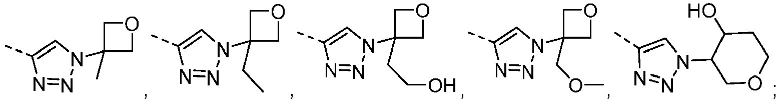
(iv) 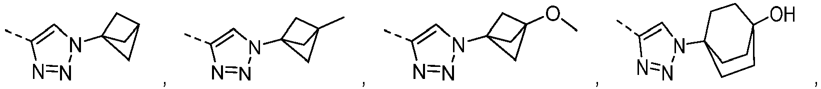 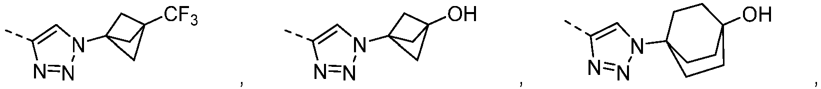 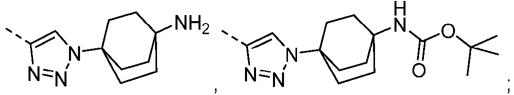 oder
(v) 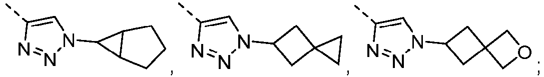
oder ein pharmazeutisch akzeptables Salz davon.

8. Verbindung nach Anspruch 1, wobei die genannte Verbindung Folgendes ist:
(2R,3R,4S,5R,6R)-4-(4-(2,3-Difluor-4-methylphenyl)-1H-1,2,3-triazol-1-yl)-2-(hydroxymethyl)-6-((1-((3R,4R)-4-hydroxytetrahydro-2H-pyran-3-yl)-1H-1,2,3-triazol-4-yl)methyl)-5-methoxytetrahydro-2H-pyran-3-ol;
(2R,3R,4S,5R,6R)-4-(4-(2,3-Difluor-4-methylphenyl)-1H-1,2,3-triazol-1-yl)-6-((1-((1R,2R)-2-hydroxycyclopentyl)-1H-1,2,3-triazol-4-yl)methyl)-2-(hydroxymethyl)-5-methoxytetrahydro-2H-pyran-3-ol;
(2R,3R,4S,5R,6R)-4-(4-(2,3-Difluor-4-methylphenyl)-1H-1,2,3-triazol-1-yl)-6-((1-((1S,2S)-2-hydroxycyclopentyl)-1H-1,2,3-triazol-4-yl)methyl)-2-(hydroxymethyl)-5-methoxytetrahydro-2H-pyran-3-ol;
(2R,3R,4S,5R,6R)-4-(4-(2,3-Difluor-4-methylphenyl)-1H-1,2,3-triazol-1-yl)-2-(hydroxymethyl)-6-((1-(1-(hydroxymethyl)cyclopropyl)-1H-1,2,3-triazol-4-yl)methyl)-5-methoxytetrahydro-2H-pyran-3-ol;
(2R,3R,4S,5R,6R)-4-(4-(2,3-Difluor-4-methylphenyl)-1H-1,2,3-triazol-1-yl)-2-(hydroxymethyl)-6-((1-(1-(hydroxymethyl)cyclopentyl)-1H-1,2,3-triazol-4-yl)methyl)-5-methoxytetrahydro-2H-pyran-3-ol;
(2R,3R,4S,5R,6R)-4-(4-(2,3-Difluor-4-methylphenyl)-1H-1,2,3-triazol-1-yl)-2-(hydroxymethyl)-5-methoxy-6-((1-(3-methyloxetan-3-yl)-1H-1,2,3-triazol-4-yl)methyl)tetrahydro-2H-pyran-3-ol;
(2R,3R,4S,5R,6R)-4-(4-(2,3-Difluor-4-methylphenyl)-1H-1,2,3-triazol-1-yl)-2-(hydroxymethyl)-5-methoxy-6-((1-(1-(methoxymethyl)cyclopropyl)-1H-1,2,3-triazol-4-yl)methyl)tetrahydro-2H-pyran-3-ol;
(2R,3R,4S,5R,6R)-4-(4-(2,3-Difluor-4-methylphenyl)-1H-1,2,3-triazol-1-yl)-6-((1-(4-hydroxybicyclo[2.2.2]octan-1-yl)-1H-1,2,3-triazol-4-yl)methyl)-2-(hydroxymethyl)-5-methoxytetrahydro-2H-pyran-3-ol;
(2R,3R,4S,5R,6R)-4-(4-(2,3-Difluor-4-methylphenyl)-1H-1,2,3-triazol-1-yl)-6-((1-(1-hydroxy-2-methylpropan-2-yl)-1H-1,2,3-triazol-4-yl)methyl)-2-(hydroxymethyl)-5-methoxytetrahydro-2H-pyran-3-ol;
Methyl 2-(4-(((2R,3R,4S,5R,6R)-4-(4-(2,3-difluor-4-methylphenyl)-1H-1,2,3-triazol-1-yl)-5-hydroxy-6-(hydroxymethyl)-3-methoxytetrahydro-2H-pyran-2-yl)methyl)-1H-1,2,3-triazol-1-yl)-2-methylpropanoat;
(2R,3R,4S,5R,6R)-4-(4-(2,3-Difluor-4-methylphenyl)-1H-1,2,3-triazol-1-yl)-2-(hydroxymethyl)-6-((1-(1-(hydroxymethyl)cyclobutyl)-1H-1,2,3-triazol-4-yl)methyl)-5-methoxytetrahydro-2H-pyran-3-ol;
(2R,3R,4S,5R,6R)-6-((1-(Bicyclo[1.1.1]pentan-1-yl)-1H-1,2,3-triazol-4-yl)methyl)-4-(4-(2,3-difluor-4-methylphenyl)-1H-1,2,3-triazol-1-yl)-2-(hydroxymethyl)-5-methoxytetrahydro-2H-pyran-3-ol;
(2R,3R,4S,5R,6R)-4-(4-(2,3-Difluor-4-methylphenyl)-1H-1,2,3-triazol-1-yl)-2-(hydroxymethyl)-5-methoxy-6-((1-(1-methylcyclobutyl)-1H-1,2,3-triazol-4-yl)methyl)tetrahydro-2H-pyran-3-ol;
(2R,3R,4S,5R,6R)-4-(4-(2,3-Difluor-4-methylphenyl)-1H-1,2,3-triazol-1-yl)-2-(hydroxymethyl)-5-methoxy-6-((1-(3-methylbicyclo[1.1.1]pentan-1-yl)-1H-1,2,3-triazol-4-yl)methyl)tetrahydro-2H-pyran-3-ol;
(2R,3R,4S,5R,6R)-6-((1-(3,3-Difluor-1-methylcyclobutyl)-1H-1,2,3-triazol-4-yl)methyl)-4-(4-(2,3-difluor-4-methylphenyl)-1H-1,2,3-triazol-1-yl)-2-(hydroxymethyl)-5-methoxytetrahydro-2H-pyran-3-ol;
(2R,3R,4S,5R, 6R)-6-((1-(2-Oxaspiro[3.3]heptan-6-yl)-1H-1,2,3-triazol-4-yl)methyl)-4-(4-(2,3-difluor-4-methylphenyl)-1H-1,2,3-triazol-1-yl)-2-(hydroxymethyl)-5-methoxytetrahydro-2H-pyran-3-ol;
(2R,3R,4S,5R,6R)-4-(4-(2,3-Difluor-4-methylphenyl)-1H-1,2,3-triazol-1-yl)-2-(hydroxymethyl)-6-((1-((1RS,2SR)-2-isopropylcyclopropyl)-1H-1,2,3-triazol-4-yl)methyl)-5-methoxytetrahydro-2H-pyran-3-ol;
(2R,3R,4S,5R,6R)-6-((1-(3,3-Difluor-1-(hydroxymethyl)cyclobutyl)-1H-1,2,3-triazol-4-yl)methyl)-4-(4-(2,3-difluor-4-methylphenyl)-1H-1,2,3-triazol-1-yl)-2-(hydroxymethyl)-5-methoxytetrahydro-2H-pyran-3-ol;
(2R,3R,4S,5R,6R)-4-(4-(2,3-Difluor-4-methylphenyl)-1H-1,2,3-triazol-1-yl)-6-((1-((1S,3S)-3-hydroxy-1-methylcyclobutyl)-1H-1,2,3-triazol-4-yl)methyl)-2-(hydroxymethyl)-5-methoxytetrahydro-2H-pyran-3-ol;
(2R,3R,4S,5R,6R)-6-((1-((1R,5S)-Bicyclo[3.1.0]hexan-6-yl)-1H-1,2,3-triazol-4-yl)methyl)-4-(4-(2,3-difluor-4-methylphenyl)-1H-1,2,3-triazol-1-yl)-2-(hydroxymethyl)-5-methoxytetrahydro-2H-pyran-3-ol;
(2R,3R,4S,5R,6R)-4-(4-(2,3-Difluor-4-methylphenyl)-1H-1,2,3-triazol-1-yl)-2-(hydroxymethyl)-5-methoxy-6-((1-(3-(methoxymethyl)oxetan-3-yl)-1H-1,2,3-triazol-4-yl)methyl)tetrahydro-2H-pyran-3-ol;
(2R,3R,4S,5R,6R)-4-(4-(2,3-Difluor-4-methylphenyl)-1H-1,2,3-triazol-1-yl)-6-((1-(3-(2-hydroxyethyl)oxetan-3-yl)-1H-1,2,3-triazol-4-yl)methyl)-2-(hydroxymethyl)-5-methoxytetrahydro-2H-pyran-3-ol;
(2R,3R,4S,5R,6R)-4-(4-(2,3-Difluor-4-methylphenyl)-1H-1,2,3-triazol-1-yl)-2-(hydroxymethyl)-5-methoxy-6-((1-(1-methylcyclopropyl)-1H-1,2,3-triazol-4-yl)methyl)tetrahydro-2H-pyran-3-ol;
(2R,3R,4S,5R,6R)-4-(4-(2,3-Difluor-4-methylphenyl)-1H-1,2,3-triazol-1-yl)-2-(hydroxymethyl)-5-methoxy-6-((1-(3-(trifluormethyl)bicyclo[1.1.1]pentan-1-yl)-1H-1,2,3-triazol-4-yl)methyl)tetrahydro-2H-pyran-3-ol;
(2R,3R,4S,5R,6R)-4-(4-(2,3-Difluor-4-methylphenyl)-1H-1,2,3-triazol-1-yl)-6-((1-(3-fluorbicyclo[1.1.1]pentan-1-yl)-1H-1,2,3-triazol-4-yl)methyl)-2-(hydroxymethyl)-5-methoxytetrahydro-2H-pyran-3-ol;
(2R,3R,4S,5R,6R)-4-(4-(2,3-Difluor-4-methylphenyl)-1H-1,2,3-triazol-1-yl)-2-(hydroxymethyl)-5-methoxy-6-((1-(spiro[2.3]hexan-5-yl)-1H-1,2,3-triazol-4-yl)methyl)tetrahydro-2H-pyran-3-ol;
(2R,3R,4S,5R,6R)-4-(4-(2,3-Difluor-4-methylphenyl)-1H-1,2,3-triazol-1-yl)-6-((1-((1R,3R)-3-hydroxy-1-methylcyclobutyl)-1H-1,2,3-triazol-4-yl)methyl)-2-(hydroxymethyl)-5-methoxytetrahydro-2H-pyran-3-ol;
(2R,3R,4S,5R,6R)-4-(4-(4-Chlor-2,3-difluorphenyl)-1H-1,2,3-triazol-1-yl)-2-(hydroxymethyl)-5-methoxy-6-((1-(1-(methoxymethyl)cyclopropyl)-1H-1,2,3-triazol-4-yl)methyl)tetrahydro-2H-pyran-3-ol;
(2R,3R,4S,5R,6R)-4-(4-(4-Chlor-2,3-difluorphenyl)-1H-1,2,3-triazol-1-yl)-6-((1-(1,1-difluor-2-methylpropan-2-yl)-1H-1,2,3-triazol-4-yl)methyl)-2-(hydroxymethyl)-5-methoxytetrahydro-2H-pyran-3-ol;
(2R,3R,4S,5R,6R)-4-(4-(4-Chlor-2,3-difluorphenyl)-1H-1,2,3-triazol-1-yl)-2-(hydroxymethyl)-6-((1-(1-(hydroxymethyl)cyclopropyl)-1H-1,2,3-triazol-4-yl)methyl)-5-methoxytetrahydro-2H-pyran-3-ol;
(2R,3R,4S,5R,6R)-4-(4-(4-Chlor-2,3-difluorphenyl)-1H-1,2,3-triazol-1-yl)-2-(hydroxymethyl)-6-((1-(1-(hydroxymethyl)cyclopentyl)-1H-1,2,3-triazol-4-yl)methyl)-5-methoxytetrahydro-2H-pyran-3-ol;
(2R,3R,4S,5R,6R)-4-(4-(4-Chlor-2,3-difluorphenyl)-1H-1,2,3-triazol-1-yl)-2-(hydroxymethyl)-6-((1-(1-(hydroxymethyl)cyclobutyl)-1H-1,2,3-triazol-4-yl)methyl)-5-methoxytetrahydro-2H-pyran-3-ol;
(2R,3R,4S,5R,6R)-4-(4-(4-Chlor-2,3-difluorphenyl)-1H-1,2,3-triazol-1-yl)-6-((1-(4-hydroxybicyclo[2.2.2]octan-1-yl)-1H-1,2,3-triazol-4-yl)methyl)-2-(hydroxymethyl)-5-methoxytetrahydro-2H-pyran-3-ol;
(2R,3R,4S,5R,6R)-4-(4-(4-Chlor-2,3-difluorphenyl)-1H-1,2,3-triazol-1-yl)-6-((1-(1-hydroxy-2-methylpropan-2-yl)-1H-1,2,3-triazol-4-yl)methyl)-2-(hydroxymethyl)-5-methoxytetrahydro-2H-pyran-3-ol;
Methyl 2-(4-(((2R,3R,4S,5R,6R)-4-(4-(4-chlor-2,3-difluorphenyl)-1H-1,2,3-triazol-1-yl)-5-hydroxy-6-(hydroxymethyl)-3-methoxytetrahydro-2H-pyran-2-yl)methyl)-1H-1,2,3-triazol-1-yl)-2-methylpropanoat;
(2R,3R,4S,5R,6R)-6-((1-(Bicyclo[1.1.1]pentan-1-yl)-1H-1,2,3-triazol-4-yl)methyl)-4-(4-(4-chlor-2,3-difluorphenyl)-1H-1,2,3-triazol-1-yl)-2-(hydroxymethyl)-5-methoxytetrahydro-2H-pyran-3-ol;
(2R,3R,4S,5R,6R)-4-(4-(4-Chlor-2,3-difluorphenyl)-1H-1,2,3-triazol-1-yl)-2-(hydroxymethyl)-5-methoxy-6-((1-(1-methylcyclobutyl)-1H-1,2,3-triazol-4-yl)methyl)tetrahydro-2H-pyran-3-ol;
(2R,3R,4S,5R,6R)-4-(4-(4-Chlor-2,3-difluorphenyl)-1H-1,2,3-triazol-1-yl)-2-(hydroxymethyl)-5-methoxy-6-((1-(3-methyloxetan-3-yl)-1H-1,2,3-triazol-4-yl)methyl)tetrahydro-2H-pyran-3-ol;
(2R,3R,4S,5R,6R)-4-(4-(4-Chlor-2,3-difluorphenyl)-1H-1,2,3-triazol-1-yl)-2-(hydroxymethyl)-5-methoxy-6-((1-(3-methylbicyclo[1.1.1]pentan-1-yl)-1H-1,2,3-triazol-4-yl)methyl)tetrahydro-2H-pyran-3-ol;
(2R,3R,4S,5R,6R)-4-(4-(4-Chlor-2,3-difluorphenyl)-1H-1,2,3-triazol-1-yl)-2-(hydroxymethyl)-5-methoxy-6-((1-(3-methoxybicyclo[1.1.1]pentan-1-yl)-1H-1,2,3-triazol-4-yl)methyl)tetrahydro-2H-pyran-3-ol;
(2R,3R,4S,5R,6R)-4-(4-(4-Chlor-2,3-difluorphenyl)-1H-1,2,3-triazol-1-yl)-6-((1-(3,3-difluor-1-methylcyclobutyl)-1H-1,2,3-triazol-4-yl)methyl)-2-(hydroxymethyl)-5-methoxytetrahydro-2H-pyran-3-ol;
(2R,3R,4S,5R,6R)-6-((1-(2-Oxaspiro[3.3]heptan-6-yl)-1H-1,2,3-triazol-4-yl)methyl)-4-(4-(4-chlor-2,3-difluorphenyl)-1H-1,2,3-triazol-1-yl)-2-(hydroxymethyl)-5-methoxytetrahydro-2H-pyran-3-ol;
(2R,3R,4S,5R,6R)-4-(4-(4-Chlor-2,3-difluorphenyl)-1H-1,2,3-triazol-1-yl)-2-(hydroxymethyl)-6-((1-(2-isopropylcyclopropyl)-1H-1,2,3-triazol-4-yl)methyl)-5-methoxytetrahydro-2H-pyran-3-ol;
(2R,3R,4S,5R,6R)-4-(4-(4-Chlor-2,3-difluorphenyl)-1H-1,2,3-triazol-1-yl)-6-((1-(3,3-difluor-1-(hydroxymethyl)cyclobutyl)-1H-1,2,3-triazol-4-yl)methyl)-2-(hydroxymethyl)-5-methoxytetrahydro-2H-pyran-3-ol;
(2R,3R,4S,5R,6R)-4-(4-(4-Chlor-2,3-difluorphenyl)-1H-1,2,3-triazol-1-yl)-6-((1-((1S,3S)-3-hydroxy-1-methylcyclobutyl)-1H-1,2,3-triazol-4-yl)methyl)-2-(hydroxymethyl)-5-methoxytetrahydro-2H-pyran-3-ol;
(2R,3R,4S,5R,6R)-6-((1-((1R,5S)-Bicyclo[3.1.0]hexan-6-yl)-1H-1,2,3-triazol-4-yl)methyl)-4-(4-(4-chlor-2,3-difluorphenyl)-1 H-1,2,3-triazol-1-yl)-2-(hydroxymethyl)-5-methoxytetrahydro-2H-pyran-3-ol;
(2R,3R,4S,5R,6R)-4-(4-(4-Chlor-2,3-difluorphenyl)-1H-1,2,3-triazol-1-yl)-2-(hydroxymethyl)-5-methoxy-6-((1-(3-(methoxymethyl)oxetan-3-yl)-1H-1,2,3-triazol-4-yl)methyl)tetrahydro-2H-pyran-3-ol;
(2R,3R,4S,5R,6R)-4-(4-(4-Chlor-2,3-difluorphenyl)-1H-1,2,3-triazol-1-yl)-6-((1-(3-(2-hydroxyethyl)oxetan-3-yl)-1H-1,2,3-triazol-4-yl)methyl)-2-(hydroxymethyl)-5-methoxytetrahydro-2H-pyran-3-ol;
(2R,3R,4S,5R,6R)-4-(4-(4-Chlor-2,3-difluorphenyl)-1H-1,2,3-triazol-1-yl)-2-(hydroxymethyl)-5-methoxy-6-((1-(1-methylcyclopropyl)-1H-1,2,3-triazol-4-yl)methyl)tetrahydro-2H-pyran-3-ol;
(2R,3R,4S,5R,6R)-4-(4-(4-Chlor-2,3-difluorphenyl)-1H-1,2,3-triazol-1-yl)-2-(hydroxymethyl)-5-methoxy-6-((1-(3-(trifluormethyl)bicyclo[1.1.1]pentan-1-yl)-1H-1,2,3-triazol-4-yl)methyl)tetrahydro-2H-pyran-3-ol;
(2R,3R,4S,5R,6R)-4-(4-(4-Chlor-2,3-difluorphenyl)-1H-1,2,3-triazol-1-yl)-6-((1-(3-fluorbicyclo[1.1.1]pentan-1-yl)-1H-1,2,3-triazol-4-yl)methyl)-2-(hydroxymethyl)-5-methoxytetrahydro-2H-pyran-3-ol;
(2R,3R,4S,5R,6R)-4-(4-(4-Chlor-2,3-difluorphenyl)-1H-1,2,3-triazol-1-yl)-2-(hydroxymethyl)-5-methoxy-6-((1-(spiro[2.3]hexan-5-yl)-1H-1,2,3-triazol-4-yl)methyl)tetrahydro-2H-pyran-3-ol;
(2R,3R,4S,5R,6R)-4-(4-(4-Chlor-2,3-difluorphenyl)-1 H-1,2,3-triazol-1-yl)-6-((1-((1R,3R)-3-hydroxy-1-methylcyclobutyl)-1H-1,2,3-triazol-4-yl)methyl)-2-(hydroxymethyl)-5-methoxytetrahydro-2H-pyran-3-ol;
(2R,3R,4S,5R,6R)-2-(Hydroxymethyl)-6-((1-(1-(hydroxymethyl)cyclopentyl)-1H-1,2,3-triazol-4-yl)methyl)-5-methoxy-4-(4-(2,3,4-trifluorphenyl)-1H-1,2,3-triazol-1-yl)tetrahydro-2H-pyran-3-ol;
(2R,3R,4S,5R,6R)-2-(Hydroxymethyl)-6-((1-(1-(hydroxymethyl)cyclopropyl)-1H-1,2,3-triazol-4-yl)methyl)-5-methoxy-4-(4-(2,3,4-trifluorphenyl)-1H-1,2,3-triazol-1-yl)tetrahydro-2H-pyran-3-ol;
(2R,3R,4S,5R,6R)-2-(Hydroxymethyl)-5-methoxy-6-((1-(1-(methoxymethyl)cyclopropyl)-1H-1,2,3-triazol-4-yl)methyl)-4-(4-(2,3,4-trifluorphenyl)-1H-1,2,3-triazol-1-yl)tetrahydro-2H-pyran-3-ol;
(2R,3R,4S,5R,6R)-6-((1-(Bicyclo[1.1.1]pentan-1-yl)-1 H-1 ,2,3-triazol-4-yl)methyl)-2-(hydroxymethyl)-5-methoxy-4-(4-(2,3,4-trifluorphenyl)-1H-1,2,3-triazol-1-yl)tetrahydro-2H-pyran-3-ol;
(2R,3R,4S,5R,6R)-2-(Hydroxymethyl)-6-((1-(1-(hydroxymethyl)cyclobutyl)-1H-1,2,3-triazol-4-yl)methyl)-5-methoxy-4-(4-(2,3,4-trifluorphenyl)-1H-1,2,3-triazol-1-yl)tetrahydro-2H-pyran-3-ol;
(2R,3R,4S,5R,6R)-6-((1-(3-Fluorbicyclo[1.1.1]pentan-1-yl)-1 H-1 ,2,3-triazol-4-yl)methyl)-2-(hydroxymethyl)-5-methoxy-4-(4-(2,3,4-trifluorphenyl)-1H-1,2,3-triazol-1-yl)tetrahydro-2H-pyran-3-ol;
(2R,3R,4S,5R, 6R)-2-(Hydroxymethyl)-5-methoxy-6-((1-(3-(trifluormethyl)bicyclo[1.1.1]pentan-1-yl)-1H-1,2,3-triazol-4-yl)methyl)-4-(4-(2,3,4-trifluorphenyl)-1H-1,2,3-triazol-1-yl)tetrahydro-2H-pyran-3-ol;
(2R,3R,4S,5R,6R)-6-((1-(3,3-Difluor-1-methylcyclobutyl)-1H-1,2,3-triazol-4-yl)methyl)-2-(hydroxymethyl)-5-methoxy-4-(4-(2,3,4-trifluorphenyl)-1H-1,2,3-triazol-1-yl)tetrahydro-2H-pyran-3-ol;
(2R,3R,4S,5R,6R)-6-((1-(3,3-Difluor-1-(hydroxymethyl)cyclobutyl)-1H-1,2,3-triazol-4-yl)methyl)-2-(hydroxymethyl)-5-methoxy-4-(4-(2,3,4-trifluorphenyl)-1H-1,2,3-triazol-1-yl)tetrahydro-2H-pyran-3-ol;
(2R,3R,45S,5R,6R)-6-((1-(2-Oxaspiro[3.3]heptan-6-yl)-1H-1,2,3-triazol-4-yl)methyl)-2-(hydroxymethyl)-5-methoxy-4-(4-(2,3,4-trifluorphenyl)-1H-1,2,3-triazol-1-yl)tetrahydro-2H-pyran-3-ol;
(2R,3R,4S,5R,6R)-6-((1-((1R,3R)-3-Hydroxy-1-methylcyclobutyl)-1H-1,2,3-triazol-4-yl)methyl)-2-(hydroxymethyl)-5-methoxy-4-(4-(2,3,4-trifluorphenyl)-1H-1,2,3-triazol-1-yl)tetrahydro-2H-pyran-3-ol;
(2R,3R,4S,5R,6R)-6-((1-((1S,3S)-3-Hydroxy-1-methylcyclobutyl)-1H-1,2,3-triazol-4-yl)methyl)-2-(hydroxymethyl)-5-methoxy-4-(4-(2,3,4-trifluorphenyl)-1H-1,2,3-triazol-1-yl)tetrahydro-2H-pyran-3-ol;
(2R,3R,4S,5R,6R)-2-(Hydroxymethyl)-5-methoxy-6-((1-(3-(methoxymethyl)oxetan-3-yl)-1H-1,2,3-triazol-4-yl)methyl)-4-(4-(2,3,4-trifluorphenyl)-1H-1,2,3-triazol-1-yl)tetrahydro-2H-pyran-3-ol;
(2R,3R,4S,5R, 6R)-6-((1-(3-(2-Hydroxyethyl)oxetan-3-yl)-1H-1,2,3-triazol-4-yl)methyl)-2-(hydroxymethyl)-5-methoxy-4-(4-(2,3,4-trifluorphenyl)-1H-1,2,3-triazol-1-yl)tetrahydro-2H-pyran-3-ol;
(2R,3R,45,5R,6R)-2-(Hydroxymethyl)-5-methoxy-6-((1-(1-methylcyclopropyl)-1H-1,2,3-triazol-4-yl)methyl)-4-(4-(2,3,4-trifluorphenyl)-1H-1,2,3-triazol-1-yl)tetrahydro-2H-pyran-3-ol;
(2R,3R,4S,5R,6R)-4-(4-(4-Brom-2,3-difluorphenyl)-1H-1,2,3-triazol-1-yl)-2-(hydroxymethyl)-5-methoxy-6-((1-(3-methyloxetan-3-yl)-1H-1,2,3-triazol-4-yl)methyl)tetrahydro-2H-pyran-3-ol;
(2R,3R,4S,5R,6R)-4-(4-(4-Brom-2,3-difluorphenyl)-1H-1,2,3-triazol-1-yl)-2-(hydroxymethyl)-5-methoxy-6-((1-(1-methylcyclobutyl)-1H-1,2,3-triazol-4-yl)methyl)tetrahydro-2H-pyran-3-ol;
(2R,3R,4S,5R,6R)-6-((1-(Bicyclo[1.1.1]pentan-1-yl)-1H-1,2,3-triazol-4-yl)methyl)-4-(4-(4-brom-2,3-difluorphenyl)-1H-1,2,3-triazol-1-yl)-2-(hydroxymethyl)-5-methoxytetrahydro-2H-pyran-3-ol;
(2R,3R,4S,5R,6R)-4-(4-(4-Brom-2,3-difluorphenyl)-1H-1,2,3-triazol-1-yl)-2-(hydroxymethyl)-5-methoxy-6-((1-(1-(methoxymethyl)cyclobutyl)-1H-1,2,3-triazol-4-yl)methyl)tetrahydro-2H-pyran-3-ol;
(2R,3R,4S,5R,6R)-4-(4-(4-Brom-2,3-difluorphenyl)-1H-1,2,3-triazol-1-yl)-2-(hydroxymethyl)-6-((1-(1-(hydroxymethyl)cyclopropyl)-1H-1,2,3-triazol-4-yl)methyl)-5-methoxytetrahydro-2H-pyran-3-ol;
(2R,3R,4S,5R,6R)-4-(4-(4-Brom-2,3-difluorphenyl)-1H-1,2,3-triazol-1-yl)-6-((1-((1S,2S)-2-hydroxycyclopentyl)-1H-1,2,3-triazol-4-yl)methyl)-2-(hydroxymethyl)-5-methoxytetrahydro-2H-pyran-3-ol;
(2R,3R,4S,5R,6R)-4-(4-(2,3-Difluor-4-methylphenyl)-1H-1,2,3-triazol-1-yl)-6-((4-(1-hydroxycyclobutyl)-1H-1,2,3-triazol-1-yl)methyl)-2-(hydroxymethyl)-5-methoxytetrahydro-2H-pyran-3-ol;
(2R,3R,4S,5R,6R)-6-((4-Cyclopentyl-1H-1,2,3-triazol-1-yl)methyl)-4-(4-(2,3-difluor-4-methylphenyl)-1H-1,2,3-triazol-1-yl)-2-(hydroxymethyl)-5-methoxytetrahydro-2H-pyran-3-ol;
(2R,3R,4S,5R,6R)-4-(4-(2,3-Difluor-4-methylphenyl)-1H-1,2,3-triazol-1-yl)-6-((4-(1-hydroxycyclopentyl)-1H-1,2,3-triazol-1-yl)methyl)-2-(hydroxymethyl)-5-methoxytetrahydro-2H-pyran-3-ol;
(2R,3R,4S,5R,6R)-4-(4-(2,3-Difluor-4-methylphenyl)-1H-1,2,3-triazol-1-yl)-2-(hydroxymethyl)-6-((4-(2-hydroxypropan-2-yl)-1H-1,2,3-triazol-1-yl)methyl)-5-methoxytetrahydro-2H-pyran-3-ol;
(2R,3R,4S,5R,6R)-4-(4-(2,3-Difluor-4-methylphenyl)-1H-1,2,3-triazol-1-yl)-2-(hydroxymethyl)-5-methoxy-6-((4-(3-methyloxetan-3-yl)-1H-1,2,3-triazol-1-yl)methyl)tetrahydro-2H-pyran-3-ol;
(2R,3R,4S,5R,6R)-4-(4-(2,3-Difluor-4-methylphenyl)-1H-1,2,3-triazol-1-yl)-2-(hydroxymethyl)-6-((4-(4-hydroxytetrahydro-2H-pyran-4-yl)-1H-1,2,3-triazol-1-yl)methyl)-5-methoxytetrahydro-2H-pyran-3-ol;
(2R,3R,4S,5R,6R)-4-(4-(2,3-Difluor-4-methylphenyl)-1H-1,2,3-triazol-1-yl)-6-((4-(1-hydroxycyclopropyl)-1 H-1,2,3-triazol-1-yl)methyl)-2-(hydroxymethyl)-5-methoxytetrahydro-2H-pyran-3-ol;
(2R,3R,4S,5R,6R)-4-(4-(2,3-Difluor-4-methylphenyl)-1H-1,2,3-triazol-1-yl)-2-(hydroxymethyl)-6-((4-(1-(hydroxymethyl)cyclopropyl)-1H-1,2,3-triazol-1-yl)methyl)-5-methoxytetrahydro-2H-pyran-3-ol;
tert-Butyl (4-(1-(((2R,3R,4S,5R,6R)-4-(4-(2,3-difluor-4-methylphenyl)-1H-1,2,3-triazol-1-yl)-5-hydroxy-6-(hydroxymethyl)-3-methoxytetrahydro-2H-pyran-2-yl)methyl)-1H-1,2,3-triazol-4-yl)bicyclo[2.2.2]octan-1-yl)carbamat;
(2R,3R,4S,5R,6R)-4-(4-(2,3-Difluor-4-methylphenyl)-1H-1,2,3-triazol-1-yl)-6-((4-(3-ethyloxetan-3-yl)-1H-1,2,3-triazol-1-yl)methyl)-2-(hydroxymethyl)-5-methoxytetrahydro-2H-pyran-3-ol;
(2R,3R,4S,5R,6R)-6-((1-(4-Aminobicyclo[2.2.2]octan-1-yl)-1H-1,2,3-triazol-4-yl)methyl)-4-(4-(2,3-difluor-4-methylphenyl)-1H-1,2,3-triazol-1-yl)-2-(hydroxymethyl)-5-methoxytetrahydro-2H-pyran-3-ol;
(2R,3R,4S,5R,6R)-4-(4-(4-Chlor-2,3-difluorphenyl)-1H-1,2,3-triazol-1-yl)-6-((4-(1-hydroxycyclobutyl)-1H-1,2,3-triazol-1-yl)methyl)-2-(hydroxymethyl)-5-methoxytetrahydro-2H-pyran-3-ol;
(2R,3R,4S,5R,6R)-4-(4-(4-Chlor-2,3-difluorphenyl)-1H-1,2,3-triazol-1-yl)-6-((4-cyclopentyl-1H-1,2,3-triazol-1-yl)methyl)-2-(hydroxymethyl)-5-methoxytetrahydro-2H-pyran-3-ol;
(2R,3R,4S,5R,6R)-4-(4-(4-Chlor-2,3-difluorphenyl)-1H-1,2,3-triazol-1-yl)-6-((4-(1-hydroxycyclopentyl)-1H-1,2,3-triazol-1-yl)methyl)-2-(hydroxymethyl)-5-methoxytetrahydro-2H-pyran-3-ol;
(2R,3R,4S,5R,6R)-4-(4-(4-Chlor-2,3-difluorphenyl)-1H-1,2,3-triazol-1-yl)-2-(hydroxymethyl)-6-((4-(2-hydroxypropan-2-yl)-1H-1,2,3-triazol-1-yl)methyl)-5-methoxytetrahydro-2H-pyran-3-ol;
(2R,3R,4S,5R,6R)-4-(4-(4-Chlor-2,3-difluorphenyl)-1H-1,2,3-triazol-1-yl)-2-(hydroxymethyl)-5-methoxy-6-((4-(3-methyloxetan-3-yl)-1H-1,2,3-triazol-1-yl)methyl)tetrahydro-2H-pyran-3-ol;
(2R,3R,4S,5R,6R)-4-(4-(4-Chlor-2,3-difluorphenyl)-1H-1,2,3-triazol-1-yl)-2-(hydroxymethyl)-6-((4-(4-hydroxytetrahydro-2H-pyran-4-yl)-1H-1,2,3-triazol-1-yl)methyl)-5-methoxytetrahydro-2H-pyran-3-ol;
(2R,3R,4S,5R,6R)-4-(4-(4-Chlor-2,3-difluorphenyl)-1H-1,2,3-triazol-1-yl)-6-((4-(1-hydroxycyclopropyl)-1H-1,2,3-triazol-1-yl)methyl)-2-(hydroxymethyl)-5-methoxytetrahydro-2H-pyran-3-ol;
(2R,3R,4S,5R,6R)-4-(4-(4-Chlor-2,3-difluorphenyl)-1H-1,2,3-triazol-1-yl)-2-(hydroxymethyl)-6-((4-(1-(hydroxymethyl)cyclopropyl)-1H-1,2,3-triazol-1-yl)methyl)-5-methoxytetrahydro-2H-pyran-3-ol;
tert-Butyl (4-(1-(((2R,3R,4S,5R,6R)-4-(4-(4-chlor-2,3-difluorphenyl)-1H-1,2,3-triazol-1-yl)-5-hydroxy-6-(hydroxymethyl)-3-methoxytetrahydro-2H-pyran-2-yl)methyl)-1H-1,2,3-triazol-4-yl)bicyclo[2.2.2]octan-1-yl)carbamat;
(2R,3R,4S,5R,6R)-4-(4-(4-Chlor-2,3-difluorphenyl)-1H-1,2,3-triazol-1-yl)-6-((4-(3-ethyloxetan-3-yl)-1H-1,2,3-triazol-1-yl)methyl)-2-(hydroxymethyl)-5-methoxytetrahydro-2H-pyran-3-ol;
(2R,3R,4S,5R,6R)-6-((1-(4-Aminobicyclo[2.2.2]octan-1-yl)-1H-1,2,3-triazol-4-yl)methyl)-4-(4-(4-chlor-2,3-difluorphenyl)-1H-1,2,3-triazol-1-yl)-2-(hydroxymethyl)-5-methoxytetrahydro-2H-pyran-3-ol;
(2R,3R,4S,5R,6R)-6-((4-(1-Hydroxycyclobutyl)-1H-1,2,3-triazol-1-yl)methyl)-2-(hydroxymethyl)-5-methoxy-4-(4-(2,3,4-trifluorphenyl)-1H-1,2,3-triazol-1-yl)tetrahydro-2H-pyran-3-ol;
(2R,3R,4S,5R,6R)-6-((4-Cyclopentyl-1H-1,2,3-triazol-1-yl)methyl)-2-(hydroxymethyl)-5-methoxy-4-(4-(2,3,4-trifluorphenyl)-1H-1,2,3-triazol-1-yl)tetrahydro-2H-pyran-3-ol;
(2R,3R,4S,5R,6R)-6-((4-(1-Hydroxycyclopentyl)-1H-1,2,3-triazol-1-yl)methyl)-2-(hydroxymethyl)-5-methoxy-4-(4-(2,3,4-trifluorphenyl)-1H-1,2,3-triazol-1-yl)tetrahydro-2H-pyran-3-ol;
(2R,3R,4S,5R,6R)-2-(Hydroxymethyl)-6-((4-(2-hydroxypropan-2-yl)-1H-1,2,3-triazol-1-yl)methyl)-5-methoxy-4-(4-(2,3,4-trifluorphenyl)-1H-1,2,3-triazol-1-yl)tetrahydro-2H-pyran-3-ol;
(2R,3R,4S,5R,6R)-2-(Hydroxymethyl)-5-methoxy-6-((4-(3-methyloxetan-3-yl)-1H-1,2,3-triazol-1-yl)methyl)-4-(4-(2,3,4-trifluorphenyl)-1H-1,2,3-triazol-1-yl)tetrahydro-2H-pyran-3-ol;
(2R,3R,4S,5R,6R)-2-(Hydroxymethyl)-6-((4-(4-hydroxytetrahydro-2H-pyran-4-yl)-1H-1,2,3-triazol-1-yl)methyl)-5-methoxy-4-(4-(2,3,4-trifluorphenyl)-1H-1,2,3-triazol-1-yl)tetrahydro-2H-pyran-3-ol;
(2R,3R,4S,5R,6R)-6-((4-(1-Hydroxycyclopropyl)-1H-1,2,3-triazol-1-yl)methyl)-2-(hydroxymethyl)-5-methoxy-4-(4-(2,3,4-trifluorphenyl)-1H-1,2,3-triazol-1-yl)tetrahydro-2H-pyran-3-ol;
(2R,3R,4S,5R,6R)-2-(Hydroxymethyl)-6-((4-(1-(hydroxymethyl)cyclopropyl)-1H-1,2,3-triazol-1-yl)methyl)-5-methoxy-4-(4-(2,3,4-trifluorphenyl)-1H-1,2,3-triazol-1-yl)tetrahydro-2H-pyran-3-ol;
(2R,3R,4S,5R,6R)-6-((4-(3-Ethyloxetan-3-yl)-1H-1,2,3-triazol-1-yl)methyl)-2-(hydroxymethyl)-5-methoxy-4-(4-(2,3,4-trifluorphenyl)-1H-1,2,3-triazol-1-yl)tetrahydro-2H-pyran-3-ol;
tert-Butyl (4-(1-(((2R,3R,4S,5R,6R)-5-hydroxy-6-(hydroxymethyl)-3-methoxy-4-(4-(2,3,4-trifluorphenyl)-1H-1,2,3-triazol-1-yl)tetrahydro-2H-pyran-2-yl)methyl)-1H-1,2,3-triazol-4-yl)bicyclo[2.2.2]octan-1-yl)carbamat;
(2R,3R,4S,5R,6R)-6-((1-(4-Aminobicyclo[2.2.2]octan-1-yl)-1H-1,2,3-triazol-4-yl)methyl)-2-(hydroxymethyl)-5-methoxy-4-(4-(2,3,4-trifluorphenyl)-1H-1,2,3-triazol-1-yl)tetrahydro-2H-pyran-3-ol;
(2R,3R,4S,5R,6R)-6-((4-(Bicyclo[1.1.1]pentan-1-yl)-1H-1,2,3-triazol-1-yl)methyl)-4-(4-(4-chlor-2,3-difluorphenyl)-1H-1,2,3-triazol-1-yl)-2-(hydroxymethyl)-5-methoxytetrahydro-2H-pyran-3-ol;
(2R,3R,4S,5R,6R)-4-(4-(4-Chlor-2,3-difluorphenyl)-1H-1,2,3-triazol-1-yl)-2-(hydroxymethyl)-5-methoxy-6-((4-(1-methylcyclopropyl)-1H-1,2,3-triazol-1-yl)methyl)tetrahydro-2H-pyran-3-ol; oder
(2R,3R,4S,5R,6R)-4-(4-(4-Chlor-2,3-difluorphenyl)-1H-1,2,3-triazol-1-yl)-2-(hydroxymethyl)-5-methoxy-6-((4-(1-methylcyclobutyl)-1H-1,2,3-triazol-1-yl)methyl)tetrahydro-2H-pyran-3-ol;
oder ein pharmazeutisch akzeptables Salz davon.

9. Verbindung nach Anspruch 1, wobei die genannte Verbindung Folgendes ist:
(2R,3R,4S,5R,6R)-4-(4-(4-Chlor-2,3-difluorphenyl)-1H-1,2,3-triazol-1-yl)-6-((1-(3-(fluormethyl)oxetan-3-yl)-1H-1,2,3-triazol-4-yl)methyl)-2-(hydroxymethyl)-5-methoxytetrahydro-2H-pyran-3-ol;
(2R,3R,4S,5R,6R)-4-(4-(4-Chlor-2,3-difluorphenyl)-1H-1,2,3-triazol-1-yl)-6-((1-(3-(difluormethyl)oxetan-3-yl)-1H-1,2,3-triazol-4-yl)methyl)-2-(hydroxymethyl)-5-methoxytetrahydro-2H-pyran-3-ol;
(2R,3R,4S,5R,6R)-4-(4-(4-Chlor-2,3-difluorphenyl)-1H-1,2,3-triazol-1-yl)-2-(hydroxymethyl)-5-methoxy-6-((1-(3-(trifluormethyl)oxetan-3-yl)-1H-1,2,3-triazol-4-yl)methyl)tetrahydro-2H-pyran-3-ol;
(2R,3R,4S,5R,6R)-4-(4-(4-Chlor-2,3-difluorphenyl)-1H-1,2,3-triazol-1-yl)-6-((1-((3R,4S)-3-fluortetrahydro-2H-pyran-4-yl)-1H-1,2,3-triazol-4-yl)methyl)-2-(hydroxymethyl)-5-methoxytetrahydro-2H-pyran-3-ol;
(2R,3R,4S,5R,6R)-4-(4-(4-Chlor-2,3-difluorphenyl)-1H-1,2,3-triazol-1-yl)-6-((1-((3R,4R)-4-fluortetrahydrofuran-3-yl)-1H-1,2,3-triazol-4-yl)methyl)-2-(hydroxymethyl)-5-methoxytetrahydro-2H-pyran-3-ol;
(2R,3R,4S,5R,6R)-4-(4-(4-Chlor-2,3-difluorphenyl)-1H-1,2,3-triazol-1-yl)-6-((1-((3S,4S)-4-fluortetrahydrofuran-3-yl)-1H-1,2,3-triazol-4-yl)methyl)-2-(hydroxymethyl)-5-methoxytetrahydro-2H-pyran-3-ol;
(2R,3R,4S,5R,6R)-4-(4-(4-Chlor-2,3-difluorphenyl)-1H-1,2,3-triazol-1-yl)-6-((1-((R)-3,3-difluortetrahydro-2H-pyran-4-yl)-1H-1,2,3-triazol-4-yl)methyl)-2-(hydroxymethyl)-5-methoxytetrahydro-2H-pyran-3-ol;
(2R,3R,4S,5R,6R)-4-(4-(4-Chlor-2,3-difluorphenyl)-1H-1,2,3-triazol-1-yl)-6-((1-((S)-3,3-difluortetrahydro-2H-pyran-4-yl)-1H-1,2,3-triazol-4-yl)methyl)-2-(hydroxymethyl)-5-methoxytetrahydro-2H-pyran-3-ol;
(2R,3R,4S,5R,6R)-4-(4-(4-Chlor-2,3-difluorphenyl)-1H-1,2,3-triazol-1-yl)-6-((1-((R)-4,4-difluortetrahydrofuran-3-yl)-1H-1,2,3-triazol-4-yl)methyl)-2-(hydroxymethyl)-5-methoxytetrahydro-2H-pyran-3-ol;
(2R,3R,4S,5R,6R)-4-(4-(4-Chlor-2,3-difluorphenyl)-1H-1,2,3-triazol-1-yl)-6-((1-((S)-4,4-difluortetrahydrofuran-3-yl)-1H-1,2,3-triazol-4-yl)methyl)-2-(hydroxymethyl)-5-methoxytetrahydro-2H-pyran-3-ol;
(2R, 3R,4S,5R,6R)-6-((1-((3S,4R)-3-Fluortetrahydro-2H-pyran-4-yl)-1H-1,2,3-triazol-4-yl)methyl)-2-(hydroxymethyl)-5-methoxy-4-(4-(2,3,4-trifluorphenyl)-1H-1,2,3-triazol-1-yl)tetrahydro-2H-pyran-3-ol;
(2R,3R,4S,5R,6R)-6-((1-((3R,4S)-3-Fluortetrahydro-2H-pyran-4-yl)-1H-1,2,3-triazol-4-yl)methyl)-2-(hydroxymethyl)-5-methoxy-4-(4-(2,3,4-trifluorphenyl)-1H-1,2,3-triazol-1-yl)tetrahydro-2H-pyran-3-ol;
(2R,3R,4S,5R,6R)-4-(4-(4-Chlor-2,3-difluorphenyl)-1H-1,2,3-triazol-1-yl)-6-((1-((3R,4R)-3-fluortetrahydro-2H-pyran-4-yl)-1H-1,2,3-triazol-4-yl)methyl)-2-(hydroxymethyl)-5-methoxytetrahydro-2H-pyran-3-ol;
(2R,3R,4S,5R,6R)-4-(4-(4-Chlor-2,3-difluorphenyl)-1H-1,2,3-triazol-1-yl)-6-((1-((3S,4S)-3-fluortetrahydro-2H-pyran-4-yl)-1H-1,2,3-triazol-4-yl)methyl)-2-(hydroxymethyl)-5-methoxytetrahydro-2H-pyran-3-ol;
(2R,3R,4S,5R,6R)-4-(4-(4-Brom-2,3-difluorphenyl)-1H-1,2,3-triazol-1-yl)-6-((1-(3-(difluormethyl)oxetan-3-yl)-1H-1,2,3-triazol-4-yl)methyl)-2-(hydroxymethyl)-5-methoxytetrahydro-2H-pyran-3-ol;
(2R,3R,4S,5R,6R)-4-(4-(4-Brom-2,3-difluorphenyl)-1H-1,2,3-triazol-1-yl)-2-(hydroxymethyl)-5-methoxy-6-((1-(3-(trifluormethyl)oxetan-3-yl)-1H-1,2,3-triazol-4-yl)methyl)tetrahydro-2H-pyran-3-ol;
2,3-Difluor-4-(1-((2R,3R,4S,5R,6R)-2-((1-((3R,4S)-3-fluortetrahydro-2H-pyran-4-yl)-1H-1,2,3-triazol-4-yl)methyl)-5-hydroxy-6-(hydroxymethyl)-3-methoxytetrahydro-2H-pyran-4-yl)-1H-1,2,3-triazol-4-yl)benzonitril; oder
2,3-Difluor-4-(1-((2R,3R,4S,5R,6R)-2-((1-((3S,4R)-3-fluortetrahydro-2H-pyran-4-yl)-1H-1,2,3-triazol-4-yl)methyl)-5-hydroxy-6-(hydroxymethyl)-3-methoxytetrahydro-2H-pyran-4-yl)-1H-1,2,3-triazol-4-yl)benzonitril;
oder ein pharmazeutisch akzeptables Salz davon.

10. Verbindung nach Anspruch 1, wobei die genannte Verbindung Folgendes ist:
(2R,3R,4S,5R,6R)-4-(4-(4-Chlor-2,3-difluorphenyl)-1H-1,2,3-triazol-1-yl)-6-((1-((3R,4R)-4-fluortetrahydrofuran-3-yl)-1H-1,2,3-triazol-4-yl)methyl)-2-(hydroxymethyl)-5-methoxytetrahydro-2H-pyran-3-ol;
(2R,3R,4S,5R,6R)-4-(4-(4-Chlor-2,3-difluorphenyl)-1H-1,2,3-triazol-1-yl)-6-((1-((3S,4R)-3-fluortetrahydro-2H-pyran-4-yl)-1H-1,2,3-triazol-4-yl)methyl)-2-(hydroxymethyl)-5-methoxytetrahydro-2H-pyran-3-ol;
(2R,3R,4S,5R,6R)-4-(4-(4-Chlor-2,3-difluorphenyl)-1H-1,2,3-triazol-1-yl)-6-((1-((3R,4R)-3-fluortetrahydro-2H-pyran-4-yl)-1H-1,2,3-triazol-4-yl)methyl)-2-(hydroxymethyl)-5-methoxytetrahydro-2H-pyran-3-ol;
(2R,3R,4S,5R,6R)-4-(4-(4-Chlor-2,3-difluorphenyl)-1H-1,2,3-triazol-1-yl)-6-((1-(3-(difluormethyl)oxetan-3-yl)-1H-1,2,3-triazol-4-yl)methyl)-2-(hydroxymethyl)-5-methoxytetrahydro-2H-pyran-3-ol: oder (2R,3R,4S,5R,6R)-4-(4-(4-Chlor-2,3-difluorphenyl)-1H-1,2,3-triazol-1-yl)-6-((1-((S)-3,3-difluortetrahydro-2H-pyran-4-yl)-1H-1,2,3-triazol-4-yl)methyl)-2-(hydroxymethyl)-5-methoxytetrahydro-2H-pyran-3-ol;
oder ein pharmazeutisch akzeptables Salz davon.

11. Pharmazeutische Zusammensetzung, die eine Verbindung nach einem der Ansprüche 1 bis 10 oder ein pharmazeutisch akzeptables Salz davon und einen pharmazeutisch akzeptablen Träger umfasst.

12. Verbindung nach einem der Ansprüche 1 bis 10 oder ein pharmazeutisch akzeptables Salz davon zur Verwendung als Medikament.

13. Verbindung nach einem der Ansprüche 1 bis 10 oder ein pharmazeutisch akzeptables Salz davon zur Verwendung bei der Vorbeugung oder Behandlung von Organfibrose; Lebererkrankungen und -störungen; akuter Nierenverletzung und chronischer Nierenerkrankung; kardiovaskulären Erkrankungen und Störungen; interstitiellen Lungenerkrankungen und -störungen; zellproliferativen Erkrankungen und Krebserkrankungen; Entzündungs- und Autoimmunerkrankungen und -störungen; Erkrankungen und Störungen des Magen-Darm-Trakts; Erkrankungen und Störungen der Bauchspeicheldrüse; mit abnormaler Angiogenese assoziierten Erkrankungen und Störungen; mit dem Gehirn assoziierten Erkrankungen und Störungen; neuropathischen Schmerzen und peripherer Neuropathie; Augenerkrankungen und -störungen; oder Transplantatabstoßung.

14. Verwendung einer Verbindung nach einem der Ansprüche 1 bis 10 oder eines pharmazeutisch akzeptablen Salzes davon bei der Herstellung eines Medikaments zur Vorbeugung oder Behandlung von Organfibrose; Lebererkrankungen und -störungen; akuter Nierenverletzung und chronischer Nierenerkrankung; kardiovaskulären Erkrankungen und Störungen; interstitiellen Lungenerkrankungen und -störungen; zellproliferativen Erkrankungen und Krebserkrankungen; Entzündungs- und Autoimmunerkrankungen und -störungen; Erkrankungen und Störungen des Magen-Darm-Trakts; Erkrankungen und Störungen der Bauchspeicheldrüse; mit abnormaler Angiogenese assoziierten Erkrankungen und Störungen; mit dem Gehirn assoziierten Erkrankungen und Störungen; neuropathischen Schmerzen und peripherer Neuropathie; Augenerkrankungen und -störungen; oder Transplantatabstoßung.

## Revendications

1. Composé de formule (I) 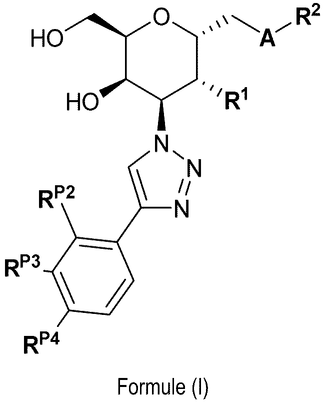
où
**R^{P2}** représente un halogène ;
**R^{P3}** représente un halogène ;
**R^{P4}** représente un halogène, méthyle ou cyano ;
**R¹** représente un
• hydroxy ;
• C₁₋₄-alkoxy ;
• -O-CO-(C₁₋₃-alkyle) ;
• O-CO-NH-**R^{N11}**, où **R^{N11}** représente un hydrogène ou un C₁₋₃-alkyle ;
• -O-CH₂-(C₁-fluoroalkyle) ;
• -O-CH₂-**HET¹**, où **HET¹** représente un hétéroaryle à 5 chaînons, ledit hétéroaryle à 5 chaînons étant indépendamment non substitué ou monosubstitué par un méthyle ; ou
• -O-CH₂-CO-**R^{1X}**, où **R^{1X}** représente un
➢ -hydroxy;
➢ C₁₋₃-alkoxy ;
➢ morpholin-4-yle ; ou
❖ -N**R^{N21}R^{N22}**, où **R^{N21}** et **R^{N22}** représentent chacun indépendamment un hydrogène ou un méthyle ; ou **R^{N21}** et **R^{N22}** forment, avec l'atome d'azote auquel ils sont rattachés, un hétérocycloalkyle monocyclique à 4 à 6 chaînons sélectionné parmi un azétidin-1-yle, pyrrolidin-1-yle et pipéridin-1-yle, ledit hétérocycloalkyle à 4 à 6 chaînons étant monosubstitué par un hydroxy ;
**A** représente un [1,2,3]triazol-1,4-diyle ; et
• **R²** représente un C₃₋₆-alkyle ramifié, ledit C₃₋₆-alkyle ramifié étant
➢ monosubstitué par un hydroxy,
➢ monosubstitué par un -CO-O-(C₁₋₄-alkyle) ou
➢ monosubstitué par un C₁-fluoroalkyle ;
• ou **R²** représente un groupement mono- ou bicyclique saturé à 3 à 8 chaînons, où ledit groupement mono- ou bicyclique est un
❖ C₃₋₆-cycloalkyle monocyclique,
❖ hétérocycloalkyle monocyclique à 4 à 6 chaînons contenant un atome d'oxygène sur le cycle, ❖ C₅₋₈-cycloalkyle bicyclique ponté,
❖ C₆₋₈-cycloalkyle bicyclique fusionné,
❖ C₆₋₈-cycloalkyle spirobicyclique ou
❖ hétérocycloalkyle spirobicyclique à 7 ou 8 chaînons contenant un atome d'oxygène sur le cycle ; où ledit groupement monocyclique ou bicyclique est indépendamment
➢ non substitué ;
➢ monosubstitué par un hydroxy ;
➢ monosubstitué par un C₁₋₃-alkyle ;
➢ monosubstitué par un C₁₋₃-alkoxy ;
➢ monosubstitué par un -(C₁₋₃-alkylène)-OH ;
➢ monosubstitué par un -(C₁₋₃-alkylène)-O-(C₁₋₃-alkyle) ;
➢ monosubstitué par un C₁-fluoroalkyle ;
➢ monosubstitué par -NR^{N1}R^{N2}, où R^{N1} représente un hydrogène et R^{N2} représente un hydrogène ou un -CO-O-(C₁₋₄-alkyle) ;
➢ mono- ou disubstitué par un/des fluoros ;
➢ disubstitué, où un substituant est un hydroxy et l'autre est un C₁₋₃-alkyle ; ou ➢ trisubstitué, où deux desdits substituants sont des fluoros ; et le substituant restant est un C₁₋₃-alkyle ou un -(C₁₋₃-alkylène)-OH ;
ou sel pharmaceutiquement acceptable de celui-ci.

2. Composé selon la revendication 1, où
➢ **R^{P2}** représente un fluoro ou un chloro ;
➢ **R^{P3}** représente un fluoro ou un chloro ; et
➢ **R^{P4}** représente un halogène, méthyle ou cyano.
ou sel pharmaceutiquement acceptable de celui-ci.

3. Composé selon la revendication 1, où
➢ **R^{P2}** représente un fluoro ;
➢ **R^{P3}** représente un fluoro ; et
➢ **R^{P4}** représente un fluoro, chloro, bromo ou méthyle ;
ou sel pharmaceutiquement acceptable de celui-ci.

4. Composé selon l'une quelconque des revendications 1 à 3, où **R¹** représente un méthoxy ;
ou sel pharmaceutiquement acceptable de celui-ci.

5. Composé selon l'une quelconque des revendications 1 à 4, **A** représentant un [1,2,3]triazol-1,4-diyle où **R²** est rattaché en position 1 dudit [1,2,3]triazol-1,4-diyle ;
ou sel pharmaceutiquement acceptable de celui-ci.

6. Composé selon l'une quelconque des revendications 1 à 4 ; **A** représentant un [1,2,3]triazol-1,4-diyle où **R²** est rattaché en position 4 dudit [1,2,3]triazol-1,4-diyle ;
ou sel pharmaceutiquement acceptable de celui-ci.

7. Composé selon l'une quelconque des revendications 1 à 4, où
a) le groupement **A-R²** représente un groupement 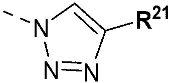 sélectionné parmi les suivants :
(i) 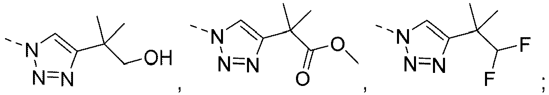
(ii) 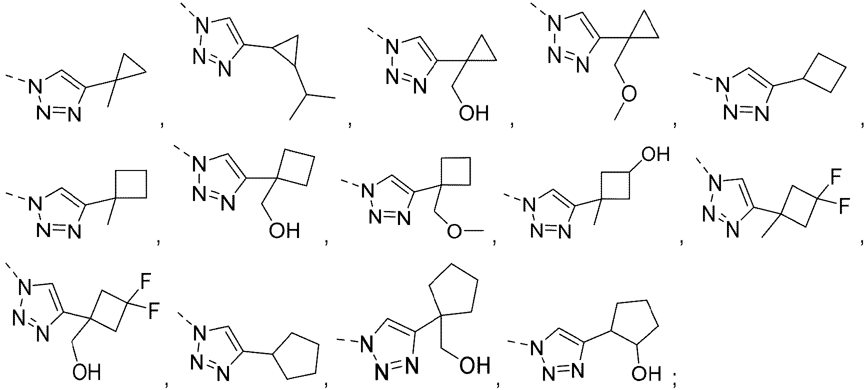
(iii) 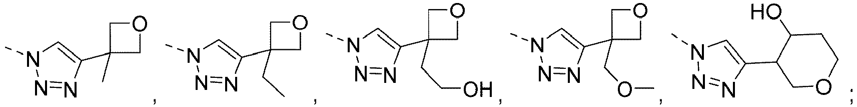
(iv) 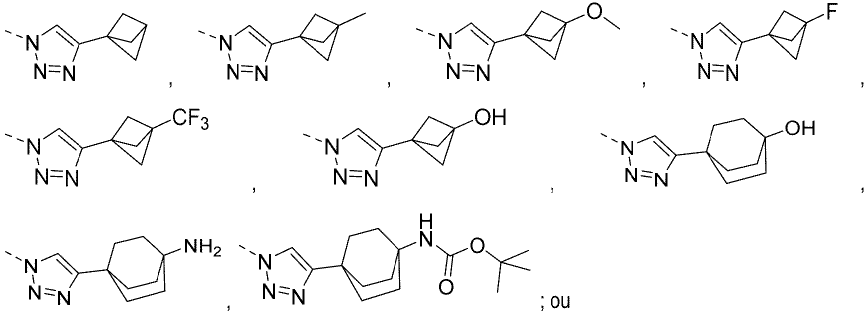
(v) 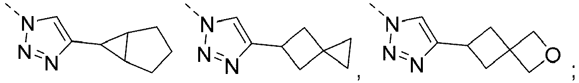 ou
b) le groupement **A-R²** représente un groupement 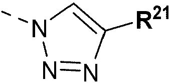 sélectionné parmi les suivants :
(i) 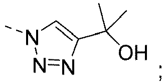
(ii) 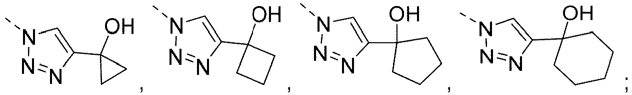 ou
(iii) 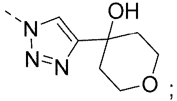 ou
c) le groupement **A-R²** représente un groupement 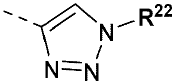 sélectionné parmi les suivants :
(i) 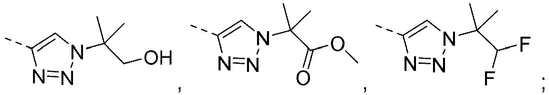
(ii) 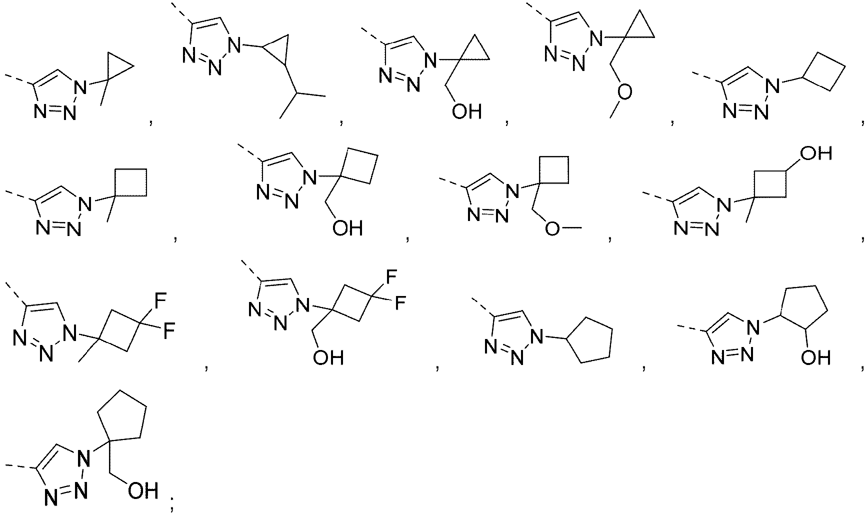
(iii) 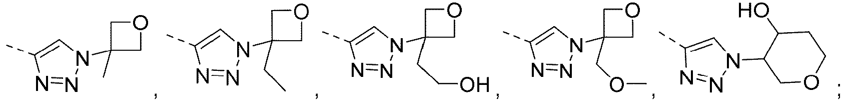
(iv) 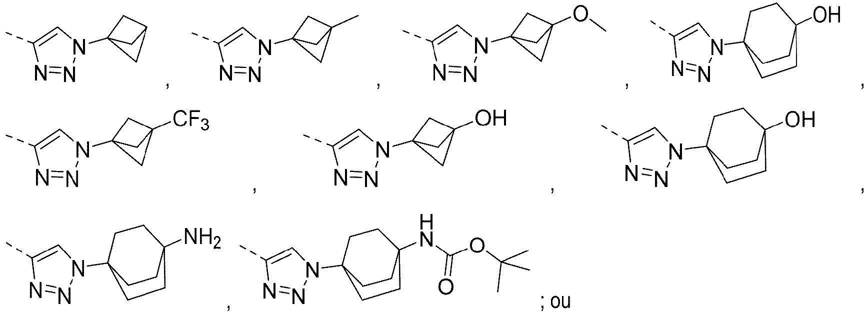
(v) 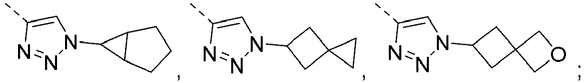 ou sel pharmaceutiquement acceptable de celui-ci.

8. Composé selon la revendication 1, où ledit composé est l'un des suivants :
(2R,3R,4S,5R,6R)-4-(4-(2,3-difluoro-4-méthylphényl)-1H-1,2,3-triazol-1-yl)-2-(hydroxyméthyl)-6-((1-((3R,4R)-4-hydroxytétrahydro-2H-pyran-3-yl)-1H-1,2,3-triazol-4-yl)méthyl)-5-méthoxytétrahydro-2H-pyran-3-ol ;
(2R,3R,4S,5R,6R)-4-(4-(2,3-difluoro-4-méthylphényl)-1H-1,2,3-triazol-1-yl)-6-((1-((1R,2R)-2-hydroxycyclopentyl)-1H-1,2,3-triazol-4-yl)méthyl)-2-(hydroxyméthyl)-5-méthoxytétrahydro-2H-pyran-3-ol ;
(2R,3R,4S,5R,6R)-4-(4-(2,3-difluoro-4-méthylphényl)-1H-1,2,3-triazol-1-yl)-6-((1-((1S,2S)-2-hydroxycyclopentyl)-1H-1,2,3-triazol-4-yl)méthyl)-2-(hydroxyméthyl)-5-méthoxytétrahydro-2H-pyran-3-ol ;
(2R,3R,4S,5R,6R)-4-(4-(2,3-difluoro-4-méthylphényl)-1H-1,2,3-triazol-1-yl)-2-(hydroxyméthyl)-6-((1-(1-(hydroxyméthyl)cyclopropyl)-1H-1,2,3-triazol-4-yl)méthyl)-5-méthoxytétrahydro-2H-pyran-3-ol ;
(2R,3R,4S,5R,6R)-4-(4-(2,3-difluoro-4-méthylphényl)-1H-1,2,3-triazol-1-yl)-2-(hydroxyméthyl)-6-((1-(1-(hydroxyméthyl)cyclopentyl)-1H-1,2,3-triazol-4-yl)méthyl)-5-méthoxytétrahydro-2H-pyran-3-ol ;
(2R,3R,4S,5R,6R)-4-(4-(2,3-difluoro-4-méthylphényl)-1H-1,2,3-triazol-1-yl)-2-(hydroxyméthyl)-5-méthoxy-6-((1-(3-méthyloxetan-3-yl)-1H-1,2,3-triazol-4-yl)méthyl)tétrahydro-2H-pyran-3-ol ;
(2R,3R,4S,5R,6R)-4-(4-(2,3-difluoro-4-méthylphényl)-1H-1,2,3-triazol-1-yl)-2-(hydroxyméthyl)-5-méthoxy-6-((1-(1-(méthoxyméthyl)cyclopropyl)-1H-1,2,3-triazol-4-yl)méthyl)tétrahydro-2H-pyran-3-ol ;
(2R,3R,4S,5R,6R)-4-(4-(2,3-difluoro-4-méthylphényl)-1H-1,2,3-triazol-1-yl)-6-((1-(4-hydroxybicyclo[2.2.2]octan-1-yl)-1H-1,2,3-triazol-4-yl)méthyl)-2-(hydroxyméthyl)-5-méthoxytétrahydro-2H-pyran-3-ol ;
(2R,3R,4S,5R,6R)-4-(4-(2,3-difluoro-4-méthylphényl)-1H-1,2,3-triazol-1-yl)-6-((1-(1-hydroxy-2-méthylpropan-2-yl)-1H-1,2,3-triazol-4-yl)méthyl)-2-(hydroxyméthyl)-5-méthoxytétrahydro-2H-pyran-3-ol ;
2-(4-(((2R,3R,4S,5R,6R)-4-(4-(2,3-difluoro-4-méthylphényl)-1H-1,2,3-triazol-1-yl)-5-hydroxy-6-(hydroxyméthyl)-3-méthoxytétrahydro-2H-pyran-2-yl)méthyl)-1H-1,2,3-triazol-1-yl)-2-méthylpropanoate de méthyle ;
(2R,3R,4S,5R,6R)-4-(4-(2,3-difluoro-4-méthylphényl)-1H-1,2,3-triazol-1-yl)-2-(hydroxyméthyl)-6-((1-(1-(hydroxyméthyl)cyclobutyl)-1H-1,2,3-triazol-4-yl)méthyl)-5-méthoxytétrahydro-2H-pyran-3-ol ;
(2R,3R,4S,5R,6R)-6-((1-(bicyclo[1.1.1]pentan-1-yl)-1H-1,2,3-triazol-4-yl)méthyl)-4-(4-(2,3-difluoro-4-méthylphényl)-1H-1,2,3-triazol-1-yl)-2-(hydroxyméthyl)-5-méthoxytétrahydro-2H-pyran-3-ol ;
(2R,3R,4S,5R,6R)-4-(4-(2,3-difluoro-4-méthylphényl)-1H-1,2,3-triazol-1-yl)-2-(hydroxyméthyl)-5-méthoxy-6-((1-(1-méthylcyclobutyl)-1H-1,2,3-triazol-4-yl)méthyl)tétrahydro-2H-pyran-3-ol ;
(2R,3R,4S,5R,6R)-4-(4-(2,3-difluoro-4-méthylphényl)-1H-1,2,3-triazol-1-yl)-2-(hydroxyméthyl)-5-méthoxy-6-((1-(3-méthylbicyclo[1.1.1]pentan-1-yl)-1H-1,2,3-triazol-4-yl)méthyl)tétrahydro-2H-pyran-3-ol ;
(2R,3R,4S,5R,6R)-6-((1-(3,3-difluoro-1-méthylcyclobutyl)-1H-1,2,3-triazol-4-yl)méthyl)-4-(4-(2,3-difluoro-4-méthylphényl)-1H-1,2,3-triazol-1-yl)-2-(hydroxyméthyl)-5-méthoxytétrahydro-2H-pyran-3-ol ;
(2R,3R,4S,5R,6R)-6-((1-(2-oxaspiro[3.3]heptan-6-yl)-1H-1,2,3-triazol-4-yl)méthyl)-4-(4-(2,3-difluoro-4-méthylphényl)-1H-1,2,3-triazol-1-yl)-2-(hydroxyméthyl)-5-méthoxytétrahydro-2H-pyran-3-ol ;
(2R,3R,4S,5R,6R)-4-(4-(2,3-difluoro-4-méthylphényl)-1H-1,2,3-triazol-1-yl)-2-(hydroxyméthyl)-6-((1-((1RS,2SR)-2-isopropylcyclopropyl)-1H-1,2,3-triazol-4-yl)méthyl)-5-méthoxytétrahydro-2H-pyran-3-ol ;
(2R,3R,4S,5R,6R)-6-((1-(3,3-difluoro-1-(hydroxyméthyl)cyclobutyl)-1H-1,2,3-triazol-4-yl)méthyl)-4-(4-(2,3-difluoro-4-méthylphényl)-1H-1,2,3-triazol-1-yl)-2-(hydroxyméthyl)-5-méthoxytétrahydro-2H-pyran-3-ol ;
(2R,3R,4S,5R,6R)-4-(4-(2,3-difluoro-4-méthylphényl)-1H-1,2,3-triazol-1-yl)-6-((1-((1S,3S)-3-hydroxy-1-méthylcyclobutyl)-1H-1,2,3-triazol-4-yl)méthyl)-2-(hydroxyméthyl)-5-méthoxytétrahydro-2H-pyran-3-ol ;
(2R,3R,4S,5R,6R)-6-((1-((1R,5S)-bicyclo[3.1.0]hexan-6-yl)-1H-1,2,3-triazol-4-yl)méthyl)-4-(4-(2,3-difluoro-4-méthylphényl)-1H-1,2,3-triazol-1-yl)-2-(hydroxyméthyl)-5-méthoxytétrahydro-2H-pyran-3-ol ;
(2R,3R,4S,5R,6R)-4-(4-(2,3-difluoro-4-méthylphényl)-1H-1,2,3-triazol-1-yl)-2-(hydroxyméthyl)-5-méthoxy-6-((1-(3-(méthoxyméthyl)oxetan-3-yl)-1H-1,2,3-triazol-4-yl)méthyl)tétrahydro-2H-pyran-3-ol ;
(2R,3R,4S,5R,6R)-4-(4-(2,3-difluoro-4-méthylphényl)-1H-1,2,3-triazol-1-yl)-6-((1-(3-(2-hydroxyéthyl)oxetan-3-yl)-1H-1,2,3-triazol-4-yl)méthyl)-2-(hydroxyméthyl)-5-méthoxytétrahydro-2H-pyran-3-ol ;
(2R,3R,4S,5R,6R)-4-(4-(2,3-difluoro-4-méthylphényl)-1H-1,2,3-triazol-1-yl)-2-(hydroxyméthyl)-5-méthoxy-6-((1-(1-méthylcyclopropyl)-1H-1,2,3-triazol-4-yl)méthyl)tétrahydro-2H-pyran-3-ol ;
(2R,3R,4S,5R,6R)-4-(4-(2,3-difluoro-4-méthylphényl)-1H-1,2,3-triazol-1-yl)-2-(hydroxyméthyl)-5-méthoxy-6-((1-(3-(trifluorométhyl)bicyclo[1.1.1]pentan-1-yl)-1H-1,2,3-triazol-4-yl)méthyl)tétrahydro-2H-pyran-3-ol ;
(2R,3R,4S,5R,6R)-4-(4-(2,3-difluoro-4-méthylphényl)-1H-1,2,3-triazol-1-yl)-6-((1-(3-fluorobicyclo[1.1.1]pentan-1-yl)-1H-1,2,3-triazol-4-yl)méthyl)-2-(hydroxyméthyl)-5-méthoxytétrahydro-2H-pyran-3-ol ;
(2R,3R,4S,5R,6R)-4-(4-(2,3-difluoro-4-méthylphényl)-1H-1,2,3-triazol-1-yl)-2-(hydroxyméthyl)-5-méthoxy-6-((1-(spiro[2.3]hexan-5-yl)-1H-1,2,3-triazol-4-yl)méthyl)tétrahydro-2H-pyran-3-ol ;
(2R,3R,4S,5R,6R)-4-(4-(2,3-difluoro-4-méthylphényl)-1H-1,2,3-triazol-1-yl)-6-((1-((1R,3R)-3-hydroxy-1-méthylcyclobutyl)-1H-1,2,3-triazol-4-yl)méthyl)-2-(hydroxyméthyl)-5-méthoxytétrahydro-2H-pyran-3-ol ;
(2R,3R,4S,5R,6R)-4-(4-(4-chloro-2,3-difluorophényl)-1H-1,2,3-triazol-1-yl)-2-(hydroxyméthyl)-5-méthoxy-6-((1-(1-(méthoxyméthyl)cyclopropyl)-1H-1,2,3-triazol-4-yl)méthyl)tétrahydro-2H-pyran-3-ol ;
(2R,3R,4S,5R,6R)-4-(4-(4-chloro-2,3-difluorophényl)-1H-1,2,3-triazol-1-yl)-6-((1-(1,1-difluoro-2-méthylpropan-2-yl)-1H-1,2,3-triazol-4-yl)méthyl)-2-(hydroxyméthyl)-5-méthoxytétrahydro-2H-pyran-3-ol ;
(2R,3R,4S,5R,6R)-4-(4-(4-chloro-2,3-difluorophényl)-1H-1,2,3-triazol-1-yl)-2-(hydroxyméthyl)-6-((1-(1-(hydroxyméthyl)cyclopropyl)-1H-1,2,3-triazol-4-yl)méthyl)-5-méthoxytétrahydro-2H-pyran-3-ol ;
(2R,3R,4S,5R,6R)-4-(4-(4-chloro-2,3-difluorophényl)-1H-1,2,3-triazol-1-yl)-2-(hydroxyméthyl)-6-((1-(1-(hydroxyméthyl)cyclopentyl)-1H-1,2,3-triazol-4-yl)méthyl)-5-méthoxytétrahydro-2H-pyran-3-ol ;
(2R,3R,4S,5R,6R)-4-(4-(4-chloro-2,3-difluorophényl)-1H-1,2,3-triazol-1-yl)-2-(hydroxyméthyl)-6-((1-(1-(hydroxyméthyl)cyclobutyl)-1H-1,2,3-triazol-4-yl)méthyl)-5-méthoxytétrahydro-2H-pyran-3-ol ;
(2R,3R,4S,5R,6R)-4-(4-(4-chloro-2,3-difluorophényl)-1H-1,2,3-triazol-1-yl)-6-((1-(4-hydroxybicyclo[2.2.2]octan-1-yl)-1H-1,2,3-triazol-4-yl)méthyl)-2-(hydroxyméthyl)-5-méthoxytétrahydro-2H-pyran-3-ol ;
(2R,3R,4S,5R,6R)-4-(4-(4-chloro-2,3-difluorophényl)-1H-1,2,3-triazol-1-yl)-6-((1-(1-hydroxy-2-méthylpropan-2-yl)-1H-1,2,3-triazol-4-yl)méthyl)-2-(hydroxyméthyl)-5-méthoxytétrahydro-2H-pyran-3-ol ;
2-(4-(((2R,3R,4S,5R,6R)-4-(4-(4-chloro-2,3-difluorophényl)-1H-1,2,3-triazol-1-yl)-5-hydroxy-6-(hydroxyméthyl)-3-méthoxytétrahydro-2H-pyran-2-yl)méthyl)-1H-1,2,3-triazol-1-yl)-2-méthylpropanoate de méthyle ;
(2R,3R,4S,5R,6R)-6-((1-(bicyclo[1.1.1]pentan-1-yl)-1H-1,2,3-triazol-4-yl)méthyl)-4-(4-(4-chloro-2,3-difluorophényl)-1H-1,2,3-triazol-1-yl)-2-(hydroxyméthyl)-5-méthoxytétrahydro-2H-pyran-3-ol ;
(2R,3R,4S,5R,6R)-4-(4-(4-chloro-2,3-difluorophényl)-1H-1,2,3-triazol-1-yl)-2-(hydroxyméthyl)-5-méthoxy-6-((1-(1-méthylcyclobutyl)-1H-1,2,3-triazol-4-yl)méthyl)tétrahydro-2H-pyran-3-ol ;
(2R,3R,4S,5R,6R)-4-(4-(4-chloro-2,3-difluorophényl)-1H-1,2,3-triazol-1-yl)-2-(hydroxyméthyl)-5-méthoxy-6-((1-(3-méthyloxetan-3-yl)-1H-1,2,3-triazol-4-yl)méthyl)tétrahydro-2H-pyran-3-ol ;
(2R,3R,4S,5R,6R)-4-(4-(4-chloro-2,3-difluorophényl)-1H-1,2,3-triazol-1-yl)-2-(hydroxyméthyl)-5-méthoxy-6-((1-(3-méthylbicyclo[1.1.1]pentan-1-yl)-1H-1,2,3-triazol-4-yl)méthyl)tétrahydro-2H-pyran-3-ol ;
(2R,3R,4S,5R,6R)-4-(4-(4-chloro-2,3-difluorophényl)-1H-1,2,3-triazol-1-yl)-2-(hydroxyméthyl)-5-méthoxy-6-((1-(3-méthoxybicyclo[1.1.1]pentan-1-yl)-1H-1,2,3-triazol-4-yl)méthyl)tétrahydro-2H-pyran-3-ol ;
(2R,3R,4S,5R,6R)-4-(4-(4-chloro-2,3-difluorophényl)-1H-1,2,3-triazol-1-yl)-6-((1-(3,3-difluoro-1-méthylcyclobutyl)-1H-1,2,3-triazol-4-yl)méthyl)-2-(hydroxyméthyl)-5-méthoxytétrahydro-2H-pyran-3-ol ;
(2R,3R,4S,5R,6R)-6-((1-(2-oxaspiro[3.3]heptan-6-yl)-1H-1,2,3-triazol-4-yl)méthyl)-4-(4-(4-chloro-2,3-difluorophényl)-1H-1,2,3-triazol-1-yl)-2-(hydroxyméthyl)-5-méthoxytétrahydro-2H-pyran-3-ol ;
(2R,3R,4S,5R,6R)-4-(4-(4-chloro-2,3-difluorophényl)-1H-1,2,3-triazol-1-yl)-2-(hydroxyméthyl)-6-((1-(2-isopropylcyclopropyl)-1H-1,2,3-triazol-4-yl)méthyl)-5-méthoxytétrahydro-2H-pyran-3-ol ;
(2R,3R,4S,5R,6R)-4-(4-(4-chloro-2,3-difluorophényl)-1H-1,2,3-triazol-1-yl)-6-((1-(3,3-difluoro-1-(hydroxyméthyl)cyclobutyl)-1H-1,2,3-triazol-4-yl)méthyl)-2-(hydroxyméthyl)-5-méthoxytétrahydro-2H-pyran-3-ol ;
(2R,3R,4S,5R,6R)-4-(4-(4-chloro-2,3-difluorophényl)-1H-1,2,3-triazol-1-yl)-6-((1-((1S,3S)-3-hydroxy-1-méthylcyclobutyl)-1H-1,2,3-triazol-4-yl)méthyl)-2-(hydroxyméthyl)-5-méthoxytétrahydro-2H-pyran-3-ol ;
(2R,3R,4S,5R,6R)-6-((1-((1R,5S)-bicyclo[3.1.0]hexan-6-yl)-1H-1,2,3-triazol-4-yl)méthyl)-4-(4-(4-chloro-2,3-difluorophényl)-1H-1,2,3-triazol-1-yl)-2-(hydroxyméthyl)-5-méthoxytétrahydro-2H-pyran-3-ol ;
(2R,3R,4S,5R,6R)-4-(4-(4-chloro-2,3-difluorophényl)-1H-1,2,3-triazol-1-yl)-2-(hydroxyméthyl)-5-méthoxy-6-((1-(3-(méthoxyméthyl)oxetan-3-yl)-1H-1,2,3-triazol-4-yl)méthyl)tétrahydro-2H-pyran-3-ol ;
(2R,3R,4S,5R,6R)-4-(4-(4-chloro-2,3-difluorophényl)-1H-1,2,3-triazol-1-yl)-6-((1-(3-(2-hydroxyéthyl)oxetan-3-yl)-1H-1,2,3-triazol-4-yl)méthyl)-2-(hydroxyméthyl)-5-méthoxytétrahydro-2H-pyran-3-ol ;
(2R,3R,4S,5R,6R)-4-(4-(4-chloro-2,3-difluorophényl)-1H-1,2,3-triazol-1-yl)-2-(hydroxyméthyl)-5-méthoxy-6-((1-(1-méthylcyclopropyl)-1H-1,2,3-triazol-4-yl)méthyl)tétrahydro-2H-pyran-3-ol ;
(2R,3R,4S,5R,6R)-4-(4-(4-chloro-2,3-difluorophényl)-1H-1,2,3-triazol-1-yl)-2-(hydroxyméthyl)-5-méthoxy-6-((1-(3-(trifluorométhyl)bicyclo[1.1.1]pentan-1-yl)-1H-1,2,3-triazol-4-yl)méthyl)tétrahydro-2H-pyran-3-ol ;
(2R,3R,4S,5R,6R)-4-(4-(4-chloro-2,3-difluorophényl)-1H-1,2,3-triazol-1-yl)-6-((1-(3-fluorobicyclo[1.1.1]pentan-1-yl)-1H-1,2,3-triazol-4-yl)méthyl)-2-(hydroxyméthyl)-5-méthoxytétrahydro-2H-pyran-3-ol ;
(2R,3R,4S,5R,6R)-4-(4-(4-chloro-2,3-difluorophényl)-1H-1,2,3-triazol-1-yl)-2-(hydroxyméthyl)-5-méthoxy-6-((1-(spiro[2.3]hexan-5-yl)-1H-1,2,3-triazol-4-yl)méthyl)tétrahydro-2H-pyran-3-ol ;
(2R,3R,4S,5R,6R)-4-(4-(4-chloro-2,3-difluorophényl)-1H-1,2,3-triazol-1-yl)-6-((1-((1R,3R)-3-hydroxy-1-méthylcyclobutyl)-1H-1,2,3-triazol-4-yl)méthyl)-2-(hydroxyméthyl)-5-méthoxytétrahydro-2H-pyran-3-ol ;
(2R,3R,4S,5R,6R)-2-(hydroxyméthyl)-6-((1-(1-(hydroxyméthyl)cyclopentyl)-1H-1,2,3-triazol-4-yl)méthyl)-5-méthoxy-4-(4-(2,3,4-trifluorophényl)-1H-1,2,3-triazol-1-yl)tétrahydro-2H-pyran-3-ol ;
(2R,3R,4S,5R,6R)-2-(hydroxyméthyl)-6-((1-(1-(hydroxyméthyl)cyclopropyl)-1H-1,2,3-triazol-4-yl)méthyl)-5-méthoxy-4-(4-(2,3,4-trifluorophényl)-1H-1,2,3-triazol-1-yl)tétrahydro-2H-pyran-3-ol ;
(2R,3R,4S,5R,6R)-2-(hydroxyméthyl)-5-méthoxy-6-((1-(1-(méthoxyméthyl)cyclopropyl)-1H-1,2,3-triazol-4-yl)méthyl)-4-(4-(2,3,4-trifluorophényl)-1H-1,2,3-triazol-1-yl)tétrahydro-2H-pyran-3-ol ;
(2R,3R,4S,5R,6R)-6-((1-(bicyclo[1.1.1]pentan-1-yl)-1H-1,2,3-triazol-4-yl)méthyl)-2-(hydroxyméthyl)-5-méthoxy-4-(4-(2,3,4-trifluorophényl)-1H-1,2,3-triazol-1-yl)tétrahydro-2H-pyran-3-ol ;
(2R,3R,4S,5R,6R)-2-(hydroxyméthyl)-6-((1-(1-(hydroxyméthyl)cyclobutyl)-1H-1,2,3-triazol-4-yl)méthyl)-5-méthoxy-4-(4-(2,3,4-trifluorophényl)-1H-1,2,3-triazol-1-yl)tétrahydro-2H-pyran-3-ol ;
(2R,3R,4S,5R,6R)-6-((1-(3-fluorobicyclo[1.1.1]pentan-1-yl)-1H-1,2,3-triazol-4-yl)méthyl)-2-(hydroxyméthyl)-5-méthoxy-4-(4-(2,3,4-trifluorophényl)-1H-1,2,3-triazol-1-yl)tétrahydro-2H-pyran-3-ol ;
(2R,3R,4S,5R,6R)-2-(hydroxyméthyl)-5-méthoxy-6-((1-(3-(trifluorométhyl)bicyclo[1.1.1]pentan-1-yl)-1H-1,2,3-triazol-4-yl)méthyl)-4-(4-(2,3,4-trifluorophényl)-1H-1,2,3-triazol-1-yl)tétrahydro-2H-pyran-3-ol ;
(2R,3R,4S,5R,6R)-6-((1-(3,3-difluoro-1-méthylcyclobutyl)-1H-1,2,3-triazol-4-yl)méthyl)-2-(hydroxyméthyl)-5-méthoxy-4-(4-(2,3,4-trifluorophényl)-1H-1,2,3-triazol-1-yl)tétrahydro-2H-pyran-3-ol ;
(2R,3R,4S,5R,6R)-6-((1-(3,3-difluoro-1-(hydroxyméthyl)cyclobutyl)-1H-1,2,3-triazol-4-yl)méthyl)-2-(hydroxyméthyl)-5-méthoxy-4-(4-(2,3,4-trifluorophényl)-1H-1,2,3-triazol-1-yl)tétrahydro-2H-pyran-3-ol ;
(2R,3R,4S,5R,6R)-6-((1-(2-oxaspiro[3.3]heptan-6-yl)-1H-1,2,3-triazol-4-yl)méthyl)-2-(hydroxyméthyl)-5-méthoxy-4-(4-(2,3,4-trifluorophényl)-1H-1,2,3-triazol-1-yl)tétrahydro-2H-pyran-3-ol ;
(2R,3R,4S,5R,6R)-6-((1-((1R,3R)-3-hydroxy-1-méthylcyclobutyl)-1H-1,2,3-triazol-4-yl)méthyl)-2-(hydroxyméthyl)-5-méthoxy-4-(4-(2,3,4-trifluorophényl)-1H-1,2,3-triazol-1-yl)tétrahydro-2H-pyran-3-ol ;
(2R,3R,4S,5R,6R)-6-((1-((1S,3S)-3-hydroxy-1-méthylcyclobutyl)-1H-1,2,3-triazol-4-yl)méthyl)-2-(hydroxyméthyl)-5-méthoxy-4-(4-(2,3,4-trifluorophényl)-1H-1,2,3-triazol-1-yl)tétrahydro-2H-pyran-3-ol ;
(2R,3R,4S,5R,6R)-2-(hydroxyméthyl)-5-méthoxy-6-((1-(3-(méthoxyméthyl)oxetan-3-yl)-1H-1,2,3-triazol-4-yl)méthyl)-4-(4-(2,3,4-trifluorophényl)-1H-1,2,3-triazol-1-yl)tétrahydro-2H-pyran-3-ol ;
(2R,3R,4S,5R,6R)-6-((1-(3-(2-hydroxyéthyl)oxetan-3-yl)-1H-1,2,3-triazol-4-yl)méthyl)-2-(hydroxyméthyl)-5-méthoxy-4-(4-(2,3,4-trifluorophényl)-1H-1,2,3-triazol-1-yl)tétrahydro-2H-pyran-3-ol ;
(2R,3R,4S,5R,6R)-2-(hydroxyméthyl)-5-méthoxy-6-((1-(1-méthylcyclopropyl)-1H-1,2,3-triazol-4-yl)méthyl)-4-(4-(2,3,4-trifluorophényl)-1H-1,2,3-triazol-1-yl)tétrahydro-2H-pyran-3-ol ;
(2R,3R,4S,5R,6R)-4-(4-(4-bromo-2,3-difluorophényl)-1H-1,2,3-triazol-1-yl)-2-(hydroxyméthyl)-5-méthoxy-6-((1-(3-méthyloxetan-3-yl)-1H-1,2,3-triazol-4-yl)méthyl)tétrahydro-2H-pyran-3-ol ;
(2R,3R,4S,5R,6R)-4-(4-(4-bromo-2,3-difluorophényl)-1H-1,2,3-triazol-1-yl)-2-(hydroxyméthyl)-5-méthoxy-6-((1-(1-méthylcyclobutyl)-1H-1,2,3-triazol-4-yl)méthyl)tétrahydro-2H-pyran-3-ol ;
(2R,3R,4S,5R,6R)-6-((1-(bicyclo[1.1.1]pentan-1-yl)-1H-1,2,3-triazol-4-yl)méthyl)-4-(4-(4-bromo-2,3-difluorophényl)-1H-1,2,3-triazol-1-yl)-2-(hydroxyméthyl)-5-méthoxytétrahydro-2H-pyran-3-ol ;
(2R,3R,4S,5R,6R)-4-(4-(4-bromo-2,3-difluorophényl)-1H-1,2,3-triazol-1-yl)-2-(hydroxyméthyl)-5-méthoxy-6-((1-(1-(méthoxyméthyl)cyclobutyl)-1H-1,2,3-triazol-4-yl)méthyl)tétrahydro-2H-pyran-3-ol ;
(2R,3R,4S,5R,6R)-4-(4-(4-bromo-2,3-difluorophényl)-1H-1,2,3-triazol-1-yl)-2-(hydroxyméthyl)-6-((1-(1-(hydroxyméthyl)cyclopropyl)-1H-1,2,3-triazol-4-yl)méthyl)-5-méthoxytétrahydro-2H-pyran-3-ol ;
(2R,3R,4S,5R,6R)-4-(4-(4-bromo-2,3-difluorophényl)-1H-1,2,3-triazol-1-yl)-6-((1-((1S,2S)-2-hydroxycyclopentyl)-1H-1,2,3-triazol-4-yl)méthyl)-2-(hydroxyméthyl)-5-méthoxytétrahydro-2H-pyran-3-ol ;
(2R,3R,4S,5R,6R)-4-(4-(2,3-difluoro-4-méthylphényl)-1H-1,2,3-triazol-1-yl)-6-((4-(1-hydroxycyclobutyl)-1H-1,2,3-triazol-1-yl)méthyl)-2-(hydroxyméthyl)-5-méthoxytétrahydro-2H-pyran-3-ol ;
(2R,3R,4S,5R,6R)-6-((4-cyclopentyl-1H-1,2,3-triazol-1-yl)méthyl)-4-(4-(2,3-difluoro-4-méthylphényl)-1H-1,2,3-triazol-1-yl)-2-(hydroxyméthyl)-5-méthoxytétrahydro-2H-pyran-3-ol ;
(2R,3R,4S,5R,6R)-4-(4-(2,3-difluoro-4-méthylphényl)-1H-1,2,3-triazol-1-yl)-6-((4-(1-hydroxycyclopentyl)-1H-1,2,3-triazol-1-yl)méthyl)-2-(hydroxyméthyl)-5-méthoxytétrahydro-2H-pyran-3-ol ;
(2R,3R,4S,5R,6R)-4-(4-(2,3-difluoro-4-méthylphényl)-1H-1,2,3-triazol-1-yl)-2-(hydroxyméthyl)-6-((4-(2-hydroxypropan-2-yl)-1H-1,2,3-triazol-1-yl)méthyl)-5-méthoxytétrahydro-2H-pyran-3-ol ;
(2R,3R,4S,5R,6R)-4-(4-(2,3-difluoro-4-méthylphényl)-1H-1,2,3-triazol-1-yl)-2-(hydroxyméthyl)-5-méthoxy-6-((4-(3-méthyloxetan-3-yl)-1H-1,2,3-triazol-1-yl)méthyl)tétrahydro-2H-pyran-3-ol ;
(2R,3R,4S,5R,6R)-4-(4-(2,3-difluoro-4-méthylphényl)-1H-1,2,3-triazol-1-yl)-2-(hydroxyméthyl)-6-((4-(4-hydroxytétrahydro-2H-pyran-4-yl)-1H-1,2,3-triazol-1-yl)méthyl)-5-méthoxytétrahydro-2H-pyran-3-ol ;
(2R,3R,4S,5R,6R)-4-(4-(2,3-difluoro-4-méthylphényl)-1H-1,2,3-triazol-1-yl)-6-((4-(1-hydroxycyclopropyl)-1H-1,2,3-triazol-1-yl)méthyl)-2-(hydroxyméthyl)-5-méthoxytétrahydro-2H-pyran-3-ol ;
(2R,3R,4S,5R,6R)-4-(4-(2,3-difluoro-4-méthylphényl)-1H-1,2,3-triazol-1-yl)-2-(hydroxyméthyl)-6-((4-(1-(hydroxyméthyl)cyclopropyl)-1H-1,2,3-triazol-1-yl)méthyl)-5-méthoxytétrahydro-2H-pyran-3-ol ;
(4-(1-(((2R,3R,4S,5R,6R)-4-(4-(2,3-difluoro-4-méthylphényl)-1H-1,2,3-triazol-1-yl)-5-hydroxy-6-(hydroxyméthyl)-3-méthoxytétrahydro-2H-pyran-2-yl)méthyl)-1H-1,2,3-triazol-4-yl)bicyclo[2.2.2]octan-1-yl)carbamate de tert-butyle ;
(2R,3R,4S,5R,6R)-4-(4-(2,3-difluoro-4-méthylphényl)-1H-1,2,3-triazol-1-yl)-6-((4-(3-éthyloxetan-3-yl)-1H-1,2,3-triazol-1-yl)méthyl)-2-(hydroxyméthyl)-5-méthoxytétrahydro-2H-pyran-3-ol ;
(2R,3R,4S,5R,6R)-6-((1-(4-aminobicyclo[2.2.2]octan-1-yl)-1H-1,2,3-triazol-4-yl)méthyl)-4-(4-(2,3-difluoro-4-méthylphényl)-1H-1,2,3-triazol-1-yl)-2-(hydroxyméthyl)-5-méthoxytétrahydro-2H-pyran-3-ol ;
(2R,3R,4S,5R,6R)-4-(4-(4-chloro-2,3-difluorophényl)-1H-1,2,3-triazol-1-yl)-6-((4-(1-hydroxycyclobutyl)-1H-1,2,3-triazol-1-yl)méthyl)-2-(hydroxyméthyl)-5-méthoxytétrahydro-2H-pyran-3-ol ;
(2R,3R,4S,5R,6R)-4-(4-(4-chloro-2,3-difluorophényl)-1H-1,2,3-triazol-1-yl)-6-((4-cyclopentyl-1H-1,2,3-triazol-1-yl)méthyl)-2-(hydroxyméthyl)-5-méthoxytétrahydro-2H-pyran-3-ol ;
(2R,3R,4S,5R,6R)-4-(4-(4-chloro-2,3-difluorophényl)-1H-1,2,3-triazol-1-yl)-6-((4-(1-hydroxycyclopentyl)-1H-1,2,3-triazol-1-yl)méthyl)-2-(hydroxyméthyl)-5-méthoxytétrahydro-2H-pyran-3-ol ;
(2R,3R,4S,5R,6R)-4-(4-(4-chloro-2,3-difluorophényl)-1H-1,2,3-triazol-1-yl)-2-(hydroxyméthyl)-6-((4-(2-hydroxypropan-2-yl)-1H-1,2,3-triazol-1-yl)méthyl)-5-méthoxytétrahydro-2H-pyran-3-ol ;
(2R,3R,4S,5R,6R)-4-(4-(4-chloro-2,3-difluorophényl)-1H-1,2,3-triazol-1-yl)-2-(hydroxyméthyl)-5-méthoxy-6-((4-(3-méthyloxetan-3-yl)-1H-1,2,3-triazol-1-yl)méthyl)tétrahydro-2H-pyran-3-ol ;
(2R,3R,4S,5R,6R)-4-(4-(4-chloro-2,3-difluorophényl)-1H-1,2,3-triazol-1-yl)-2-(hydroxyméthyl)-6-((4-(4-hydroxytétrahydro-2H-pyran-4-yl)-1H-1,2,3-triazol-1-yl)méthyl)-5-méthoxytétrahydro-2H-pyran-3-ol ;
(2R,3R,4S,5R,6R)-4-(4-(4-chloro-2,3-difluorophényl)-1H-1,2,3-triazol-1-yl)-6-((4-(1-hydroxycyclopropyl)-1H-1,2,3-triazol-1-yl)méthyl)-2-(hydroxyméthyl)-5-méthoxytétrahydro-2H-pyran-3-ol ;
(2R,3R,4S,5R,6R)-4-(4-(4-chloro-2,3-difluorophényl)-1H-1,2,3-triazol-1-yl)-2-(hydroxyméthyl)-6-((4-(1-(hydroxyméthyl)cyclopropyl)-1H-1,2,3-triazol-1-yl)méthyl)-5-méthoxytétrahydro-2H-pyran-3-ol ;
(4-(1-(((2R,3R,4S,5R,6R)-4-(4-(4-chloro-2,3-difluorophényl)-1H-1,2,3-triazol-1-yl)-5-hydroxy-6-(hydroxyméthyl)-3-méthoxytétrahydro-2H-pyran-2-yl)méthyl)-1H-1,2,3-triazol-4-yl)bicyclo[2.2.2]octan-1-yl)carbamate de tert-butyle ;
(2R,3R,4S,5R,6R)-4-(4-(4-chloro-2,3-difluorophényl)-1H-1,2,3-triazol-1-yl)-6-((4-(3-éthyloxetan-3-yl)-1H-1,2,3-triazol-1-yl)méthyl)-2-(hydroxyméthyl)-5-méthoxytétrahydro-2H-pyran-3-ol ;
(2R,3R,4S,5R,6R)-6-((1-(4-aminobicyclo[2.2.2]octan-1-yl)-1H-1,2,3-triazol-4-yl)méthyl)-4-(4-(4-chloro-2,3-difluorophényl)-1H-1,2,3-triazol-1-yl)-2-(hydroxyméthyl)-5-méthoxytétrahydro-2H-pyran-3-ol ;
(2R,3R,4S,5R,6R)-6-((4-(1-hydroxycyclobutyl)-1H-1,2,3-triazol-1-yl)méthyl)-2-(hydroxyméthyl)-5-méthoxy-4-(4-(2,3,4-trifluorophényl)-1H-1,2,3-triazol-1-yl)tétrahydro-2H-pyran-3-ol ;
(2R,3R,4S,5R,6R)-6-((4-cyclopentyl-1H-1,2,3-triazol-1-yl)méthyl)-2-(hydroxyméthyl)-5-méthoxy-4-(4-(2,3,4-trifluorophényl)-1H-1,2,3-triazol-1-yl)tétrahydro-2H-pyran-3-ol ;
(2R,3R,4S,5R,6R)-6-((4-(1-hydroxycyclopentyl)-1H-1,2,3-triazol-1-yl)méthyl)-2-(hydroxyméthyl)-5-méthoxy-4-(4-(2,3,4-trifluorophényl)-1H-1,2,3-triazol-1-yl)tétrahydro-2H-pyran-3-ol ;
(2R,3R,4S,5R,6R)-2-(hydroxyméthyl)-6-((4-(2-hydroxypropan-2-yl)-1H-1,2,3-triazol-1-yl)méthyl)-5-méthoxy-4-(4-(2,3,4-trifluorophényl)-1H-1,2,3-triazol-1-yl)tétrahydro-2H-pyran-3-ol ;
(2R,3R,4S,5R,6R)-2-(hydroxyméthyl)-5-méthoxy-6-((4-(3-méthyloxetan-3-yl)-1H-1,2,3-triazol-1-yl)méthyl)-4-(4-(2,3,4-trifluorophényl)-1H-1,2,3-triazol-1-yl)tétrahydro-2H-pyran-3-ol ;
(2R,3R,4S,5R,6R)-2-(hydroxyméthyl)-6-((4-(4-hydroxytétrahydro-2H-pyran-4-yl)-1H-1,2,3-triazol-1-yl)méthyl)-5-méthoxy-4-(4-(2,3,4-trifluorophényl)-1H-1,2,3-triazol-1-yl)tétrahydro-2H-pyran-3-ol ;
(2R,3R,4S,5R,6R)-6-((4-(1-hydroxycyclopropyl)-1H-1,2,3-triazol-1-yl)méthyl)-2-(hydroxyméthyl)-5-méthoxy-4-(4-(2,3,4-trifluorophényl)-1H-1,2,3-triazol-1-yl)tétrahydro-2H-pyran-3-ol ;
(2R,3R,4S,5R,6R)-2-(hydroxyméthyl)-6-((4-(1-(hydroxyméthyl)cyclopropyl)-1H-1,2,3-triazol-1-yl)méthyl)-5-méthoxy-4-(4-(2,3,4-trifluorophényl)-1H-1,2,3-triazol-1-yl)tétrahydro-2H-pyran-3-ol ;
(2R,3R,4S,5R,6R)-6-((4-(3-éthyloxetan-3-yl)-1H-1,2,3-triazol-1-yl)méthyl)-2-(hydroxyméthyl)-5-méthoxy-4-(4-(2,3,4-trifluorophényl)-1H-1,2,3-triazol-1-yl)tétrahydro-2H-pyran-3-ol ;
(4-(1-(((2R,3R,4S,5R,6R)-5-hydroxy-6-(hydroxyméthyl)-3-méthoxy-4-(4-(2,3,4-trifluorophényl)-1H-1,2,3-triazol-1-yl)tétrahydro-2H-pyran-2-yl)méthyl)-1H-1,2,3-triazol-4-yl)bicyclo[2.2.2]octan-1-yl)carbamate de tert-butyle ;
(2R,3R,4S,5R,6R)-6-((1-(4-aminobicyclo[2.2.2]octan-1-yl)-1H-1,2,3-triazol-4-yl)méthyl)-2-(hydroxyméthyl)-5-méthoxy-4-(4-(2,3,4-trifluorophényl)-1H-1,2,3-triazol-1-yl)tétrahydro-2H-pyran-3-ol ;
(2R,3R,4S,5R,6R)-6-((4-(bicyclo[1.1.1]pentan-1-yl)-1H-1,2,3-triazol-1-yl)méthyl)-4-(4-(4-chloro-2,3-difluorophényl)-1H-1,2,3-triazol-1-yl)-2-(hydroxyméthyl)-5-méthoxytétrahydro-2H-pyran-3-ol ;
(2R,3R,4S,5R,6R)-4-(4-(4-chloro-2,3-difluorophényl)-1H-1,2,3-triazol-1-yl)-2-(hydroxyméthyl)-5-méthoxy-6-((4-(1-méthylcyclopropyl)-1H-1,2,3-triazol-1-yl)méthyl)tétrahydro-2H-pyran-3-ol ; ou
(2R,3R,4S,5R,6R)-4-(4-(4-chloro-2,3-difluorophényl)-1H-1,2,3-triazol-1-yl)-2-(hydroxyméthyl)-5-méthoxy-6-((4-(1-méthylcyclobutyl)-1H-1,2,3-triazol-1-yl)méthyl)tétrahydro-2H-pyran-3-ol ;
ou sel pharmaceutiquement acceptable de celui-ci.

9. Composé selon la revendication 1, où ledit composé est l'un des suivants :
(2R,3R,4S,5R,6R)-4-(4-(4-chloro-2,3-difluorophényl)-1H-1,2,3-triazol-1-yl)-6-((1-(3-(fluorométhyl)oxetan-3-yl)-1H-1,2,3-triazol-4-yl)méthyl)-2-(hydroxyméthyl)-5-méthoxytétrahydro-2H-pyran-3-ol ;
(2R,3R,4S,5R,6R)-4-(4-(4-chloro-2,3-difluorophényl)-1H-1,2,3-triazol-1-yl)-6-((1-(3-(difluorométhyl)oxetan-3-yl)-1H-1,2,3-triazol-4-yl)méthyl)-2-(hydroxyméthyl)-5-méthoxytétrahydro-2H-pyran-3-ol ;
(2R,3R,4S,5R,6R)-4-(4-(4-chloro-2,3-difluorophényl)-1H-1,2,3-triazol-1-yl)-2-(hydroxyméthyl)-5-méthoxy-6-((1-(3-(trifluorométhyl)oxetan-3-yl)-1H-1,2,3-triazol-4-yl)méthyl)tétrahydro-2H-pyran-3-ol ;
(2R,3R,4S,5R,6R)-4-(4-(4-chloro-2,3-difluorophényl)-1H-1,2,3-triazol-1-yl)-6-((1-((3R,4S)-3-fluorotétrahydro-2H-pyran-4-yl)-1H-1,2,3-triazol-4-yl)méthyl)-2-(hydroxyméthyl)-5-méthoxytétrahydro-2H-pyran-3-ol ;
(2R,3R,4S,5R,6R)-4-(4-(4-chloro-2,3-difluorophényl)-1H-1,2,3-triazol-1-yl)-6-((1-((3R,4R)-4-fluorotétrahydrofuran-3-yl)-1H-1,2,3-triazol-4-yl)méthyl)-2-(hydroxyméthyl)-5-méthoxytétrahydro-2H-pyran-3-ol ;
(2R,3R,4S,5R,6R)-4-(4-(4-chloro-2,3-difluorophényl)-1H-1,2,3-triazol-1-yl)-6-((1-((3S,4S)-4-fluorotétrahydrofuran-3-yl)-1H-1,2,3-triazol-4-yl)méthyl)-2-(hydroxyméthyl)-5-méthoxytétrahydro-2H-pyran-3-ol ;
(2R,3R,4S,5R,6R)-4-(4-(4-chloro-2,3-difluorophényl)-1H-1,2,3-triazol-1-yl)-6-((1-((R)-3,3-difluorotétrahydro-2H-pyran-4-yl)-1H-1,2,3-triazol-4-yl)méthyl)-2-(hydroxyméthyl)-5-méthoxytétrahydro-2H-pyran-3-ol ;
(2R,3R,4S,5R,6R)-4-(4-(4-chloro-2,3-difluorophényl)-1H-1,2,3-triazol-1-yl)-6-((1-((S)-3,3-difluorotétrahydro-2H-pyran-4-yl)-1H-1,2,3-triazol-4-yl)méthyl)-2-(hydroxyméthyl)-5-méthoxytétrahydro-2H-pyran-3-ol ;
(2R,3R,4S,5R,6R)-4-(4-(4-chloro-2,3-difluorophényl)-1H-1,2,3-triazol-1-yl)-6-((1-((R)-4,4-difluorotétrahydrofuran-3-yl)-1H-1,2,3-triazol-4-yl)méthyl)-2-(hydroxyméthyl)-5-méthoxytétrahydro-2H-pyran-3-ol ;
(2R,3R,4S,5R,6R)-4-(4-(4-chloro-2,3-difluorophényl)-1H-1,2,3-triazol-1-yl)-6-((1-((S)-4,4-difluorotétrahydrofuran-3-yl)-1H-1,2,3-triazol-4-yl)méthyl)-2-(hydroxyméthyl)-5-méthoxytétrahydro-2H-pyran-3-ol ;
(2R,3R,4S,5R,6R)-6-((1-((3S,4R)-3-fluorotétrahydro-2H-pyran-4-yl)-1H-1,2,3-triazol-4-yl)méthyl)-2-(hydroxyméthyl)-5-méthoxy-4-(4-(2,3,4-trifluorophényl)-1H-1,2,3-triazol-1-yl)tétrahydro-2H-pyran-3-ol ;
(2R,3R,4S,5R,6R)-6-((1-((3R,4S)-3-fluorotétrahydro-2H-pyran-4-yl)-1H-1,2,3-triazol-4-yl)méthyl)-2-(hydroxyméthyl)-5-méthoxy-4-(4-(2,3,4-trifluorophényl)-1H-1,2,3-triazol-1-yl)tétrahydro-2H-pyran-3-ol ;
(2R,3R,4S,5R,6R)-4-(4-(4-chloro-2,3-difluorophényl)-1H-1,2,3-triazol-1-yl)-6-((1-((3R,4R)-3-fluorotétrahydro-2H-pyran-4-yl)-1H-1,2,3-triazol-4-yl)méthyl)-2-(hydroxyméthyl)-5-méthoxytétrahydro-2H-pyran-3-ol ;
(2R,3R,4S,5R,6R)-4-(4-(4-chloro-2,3-difluorophényl)-1H-1,2,3-triazol-1-yl)-6-((1-((3S,4S)-3-fluorotétrahydro-2H-pyran-4-yl)-1H-1,2,3-triazol-4-yl)méthyl)-2-(hydroxyméthyl)-5-méthoxytétrahydro-2H-pyran-3-ol ;
(2R,3R,4S,5R,6R)-4-(4-(4-bromo-2,3-difluorophényl)-1H-1,2,3-triazol-1-yl)-6-((1-(3-(difluorométhyl)oxetan-3-yl)-1H-1,2,3-triazol-4-yl)méthyl)-2-(hydroxyméthyl)-5-méthoxytétrahydro-2H-pyran-3-ol ;
(2R,3R,4S,5R,6R)-4-(4-(4-bromo-2,3-difluorophényl)-1H-1,2,3-triazol-1-yl)-2-(hydroxyméthyl)-5-méthoxy-6-((1-(3-(trifluorométhyl)oxetan-3-yl)-1H-1,2,3-triazol-4-yl)méthyl)tétrahydro-2H-pyran-3-ol ;
2,3-difluoro-4-(1-((2R,3R,4S,5R,6R)-2-((1-((3R,4S)-3-fluorotétrahydro-2H-pyran-4-yl)-1H-1,2,3-triazol-4-yl)méthyl)-5-hydroxy-6-(hydroxyméthyl)-3-méthoxytétrahydro-2H-pyran-4-yl)-1H-1,2,3-triazol-4-yl)benzonitrile ; ou
2,3-difluoro-4-(1-((2R,3R,4S,5R,6R)-2-((1-((3S,4R)-3-fluorotétrahydro-2H-pyran-4-yl)-1H-1,2,3-triazol-4-yl)méthyl)-5-hydroxy-6-(hydroxyméthyl)-3-méthoxytétrahydro-2H-pyran-4-yl)-1H-1,2,3-triazol-4-yl)benzonitrile ;
ou sel pharmaceutiquement acceptable de celui-ci.

10. Composé selon la revendication 1, où ledit composé est l'un des suivants :
(2R,3R,4S,5R,6R)-4-(4-(4-chloro-2,3-difluorophényl)-1H-1,2,3-triazol-1-yl)-6-((1-((3R,4R)-4-fluorotétrahydrofuran-3-yl)-1H-1,2,3-triazol-4-yl)méthyl)-2-(hydroxyméthyl)-5-méthoxytétrahydro-2H-pyran-3-ol ;
(2R,3R,4S,5R,6R)-4-(4-(4-chloro-2,3-difluorophényl)-1H-1,2,3-triazol-1-yl)-6-((1-((3S,4R)-3-fluorotétrahydro-2H-pyran-4-yl)-1H-1,2,3-triazol-4-yl)méthyl)-2-(hydroxyméthyl)-5-méthoxytétrahydro-2H-pyran-3-ol ;
(2R,3R,4S,5R,6R)-4-(4-(4-chloro-2,3-difluorophényl)-1H-1,2,3-triazol-1-yl)-6-((1-((3R,4R)-3-fluorotétrahydro-2H-pyran-4-yl)-1H-1,2,3-triazol-4-yl)méthyl)-2-(hydroxyméthyl)-5-méthoxytétrahydro-2H-pyran-3-ol ;
(2R,3R,4S,5R,6R)-4-(4-(4-chloro-2,3-difluorophényl)-1H-1,2,3-triazol-1-yl)-6-((1-(3-(difluorométhyl)oxetan-3-yl)-1H-1,2,3-triazol-4-yl)méthyl)-2-(hydroxyméthyl)-5-méthoxytétrahydro-2H-pyran-3-ol : ou
(2R,3R,4S,5R,6R)-4-(4-(4-chloro-2,3-difluorophényl)-1H-1,2,3-triazol-1-yl)-6-((1-((S)-3,3-difluorotétrahydro-2H-pyran-4-yl)-1H-1,2,3-triazol-4-yl)méthyl)-2-(hydroxyméthyl)-5-méthoxytétrahydro-2H-pyran-3-ol ;
ou sel pharmaceutiquement acceptable de celui-ci.

11. Composition pharmaceutique comprenant un composé selon l'une quelconque des revendications 1 à 10 ou un sel pharmaceutiquement acceptable de celui-ci et un véhicule pharmaceutiquement acceptable.

12. Composé selon l'une quelconque des revendications 1 à 10 ou sel pharmaceutiquement acceptable de celui-ci pour une utilisation en tant que médicament.

13. Composé selon l'une quelconque des revendications 1 à 10 ou sel pharmaceutiquement acceptable de celui-ci pour une utilisation dans la prévention ou le traitement de la fibrose d'organes ; de maladies et d'affections hépatiques ; d'une lésion rénale aiguë et de maladies rénales chroniques ; de maladies et d'affections cardiovasculaires ; de maladies et d'affections pulmonaires interstitielles ; de maladies liées à une prolifération cellulaire et de cancers ; de maladies et d'affections inflammatoires et auto-immunes ; de maladies et d'affections des voies gastrointestinales ; de maladies et d'affections pancréatiques ; de maladies et d'affections liées à une angiogenèse anormale ; de maladies et d'affections relevant du cerveau ; de la douleur neuropathique et de la neuropathie périphérique ; de maladies et d'affections oculaires ; ou d'un rejet de greffe.

14. Utilisation d'un composé selon l'une quelconque des revendications 1 à 10 ou d'un sel pharmaceutiquement acceptable de celui-ci dans la préparation d'un médicament indiqué dans la prévention ou le traitement de la fibrose d'organes ; de maladies et d'affections hépatiques ; d'une lésion rénale aiguë et de maladies rénales chroniques ; de maladies et d'affections cardiovasculaires ; de maladies et d'affections pulmonaires interstitielles ; de maladies liées à une prolifération cellulaire et de cancers ; de maladies et d'affections inflammatoires et auto-immunes ; de maladies et d'affections des voies gastrointestinales ; de maladies et d'affections pancréatiques ; de maladies et d'affections liées à une angiogenèse anormale ; de maladies et d'affections relevant du cerveau ; de la douleur neuropathique et de la neuropathie périphérique ; de maladies et d'affections oculaires ; ou d'un rejet de greffe.
